# EUROPEAN PATENT SPECIFICATION

(11) **EP 2 877 213 B1**
(45) Date of publication and mention of the grant of the patent: **02.12.2020**
(21) Application number: 13744916.1
(22) Date of filing: 21.07.2013
(51) Int. Cl.: A61K 48/00, C12N 15/62, C12N 15/63, C12N 15/90

(54) **INDUCIBLE DNA BINDING PROTEINS AND GENOME PERTURBATION TOOLS AND APPLICATIONS THEREOF**
INDUZIERBARE DNS-BINDENDE PROTEINE UND GENOMPERTURBATIONSWERKZEUGE UND DEREN ANWENDUNGEN
PROTÉINES DE LIAISON À L'ADN INDUCTIBLES ET OUTILS POUR LA PERTURBATION DU GÉNOME ET LEURS APPLICATIONS

(30) Priority: 25.07.2012 US 201261675778 P; 01.11.2012 US 201261721283 P; 12.12.2012 US 201261736465 P; 15.03.2013 US 201361794458 P; 17.06.2013 US 201361835973 P
(43) Date of publication of application: 03.06.2015
(60) Divisional application: 18200462.2
(73) Proprietor: The Broad Institute, Inc., Cambridge, MA 02142 (US); Massachusetts Institute of Technology, Cambridge, MA 02139 (US); President and Fellows of Harvard College, Cambridge, MA 02138 (US)
(72) Inventor: ZHANG, Feng, Cambridge, MA 02139 (US); BRIGHAM, Mark, Somerville, MA 02145 (US); CONG, Le, Stanford, CA 94305 (US); KONERMANN, Silvana, CH-8005 Zurich (CH); SANJANA, Neville Espi, Cambridge, MA 02142 (US)
(74) Representative: Bird & Bird LLP
(86) International application number: PCT/US2013/051418
(87) International publication number: WO 2014/018423

(56) References cited:
- WO-A2-2013/082519
- US-A1- 2011 145 940
- US-A1- 2011 287 545
- US-A1- 2011 301 073
- MATTHEW J KENNEDY ET AL: "Rapid blue-light-mediated induction of protein interactions in living cells", NATURE METHODS, vol. 7, no. 12, 1 December 2010 (2010-12-01), pages 973-975, XP055039821, ISSN: 1548-7091, DOI: 10.1038/nmeth.1524
- FINOLA E. MOORE ET AL: "Improved Somatic Mutagenesis in Zebrafish Using Transcription Activator-Like Effector Nucleases (TALENs)", PLOS ONE, vol. 7, no. 5, 24 May 2012 (2012-05-24), page e37877, XP055086697, ISSN: 1932-6203, DOI: 10.1371/journal.pone.0037877
- DIRK HOCKEMEYER ET AL: "Genetic engineering of human pluripotent cells using TALE nucleases", NATURE BIOTECHNOLOGY, vol. 29, no. 8, 1 August 2011 (2011-08-01) , pages 731-734, XP055018244, ISSN: 1087-0156, DOI: 10.1038/nbt.1927
- NING SUN ET AL: "Optimized TAL effector nucleases (TALENs) for use in treatment of sickle cell disease", MOLECULAR BIOSYSTEMS, vol. 8, no. 4, 1 January 2012 (2012-01-01) , page 1255, XP055081815, ISSN: 1742-206X, DOI: 10.1039/c2mb05461b
- TING LI ET AL: "High-efficiency TALEN-based gene editing produces disease-resistant rice", NATURE BIOTECHNOLOGY, vol. 30, no. 5, 1 May 2012 (2012-05-01), pages 390-392, XP055086834, ISSN: 1087-0156, DOI: 10.1038/nbt.2199
- XUE WANG ET AL: "Spatiotemporal control of gene expression by a light-switchable transgene system", NATURE METHODS, vol. 9, no. 3, 12 February 2012 (2012-02-12), pages 266-269, XP055087617, ISSN: 1548-7091, DOI: 10.1038/nmeth.1892

## Description

### RELATED APPLICATIONS

This application claims priority to and claims benefit of US provisional patent application Serial Nos. 61/675,778 filed July 25, 2012, 61/721,283 filed November 1, 2012, 61/736,465 filed December 12, 2012, 61/794,458 filed March 15, 2013 and 61/835,973 filed June 17, 2013 titled INDUCIBLE DNA BINDING PROTEINS AND GENOME PERTURBATION TOOLS AND APPLICATIONS THEREOF.

Reference is also made to US Provisional Application No. 61/565,171 filed November 30, 2011 and US Application Nos. 13/554,922 filed July 30, 2012 and 13/604,945 filed September 6, 2012, titled NUCLEOTIDE-SPECIFIC RECOGNITION SEQUENCES FOR DESIGNER TAL EFFECTORS.

Reference is also made to US Provisional Application Nos. 61/736,527 filed December 12, 2012; 61/748,427 filed January 2, 2013; 61/757,972 filed January 29, 2013, 61/768,959, filed February 25, 2013 and 61/791,409 filed March 15, 2013, titled SYSTEMS METHODS AND COMPOSITIONS FOR SEQUENCE MANIPULATION.

Reference is also made to US Provisional Application Nos. 61/758,468 filed January 30, 2013 and 61/769,046 filed March 15, 2013, titled ENGINEERING AND OPTIMIZATION OF SYSTEMS, METHODS AND COMPOSITIONS FOR SEQUENCE MANIPULATION.

Reference is also made to US Provisional Application Nos. 61/835,931; 61/835,936; 61/836,080; 61/836,101; 61/836,123 and 61/836,127 filed June 17, 2013.

Reference is also made to US Provisional Application No. 61/842,322, filed July 2, 2013, titled CRISPR-CAS SYSTEMS AND METHODS FOR ALTERING EXPRESSION OF GENE PRODUCTS and US Provisional Application No. 61/847,537, filed July 17, 2013, titled DELIVERY, ENGINEERING AND OPTIMIZATION OF SYSTEMS, METHODS AND COMPOSITIONS FOR SEQUENCE MANIPULATION AND APPLICATIONS.

### FIELD OF THE INVENTION AND OF THE DISCLOSURE

The present disclosure generally relates to methods and compositions used for the spatial and temporal control of gene expression, such as genome perturbation, that may use inducible transcriptional effectors.

### FEDERAL FUNDING LEGEND

This invention was made with government support under R01NS073124 and Pioneer Award 1DP1MH100706 awarded by the National Institutes of Health. The Government has certain rights in the invention.

### BACKGROUND OF THE INVENTION

Normal gene expression is a dynamic process with carefully orchestrated temporal and spatial components, the precision of which are necessary for normal development, homeostasis, and advancement of the organism. In turn, the dysregulation of required gene expression patterns, either by increased, decreased, or altered function of a gene or set of genes, has been linked to a wide array of pathologies. Technologies capable of modulating gene expression in a spatiotemporally precise fashion will enable the elucidation of the genetic cues responsible for normal biological processes and disease mechanisms. To address this technological need, Applicants developed inducible molecular tools that may regulate gene expression, in particular, light-inducible transcriptional effectors (LITEs), which provide light-mediated control of endogenous gene expression.

Inducible gene expression systems have typically been designed to allow for chemically induced activation of an inserted open reading frame or shRNA sequence, resulting in gene overexpression or repression, respectively. Disadvantages of using open reading frames for overexpression include loss of splice variation and limitation of gene size. Gene repression via RNA interference, despite its transformative power in human biology, can be hindered by complicated off-target effects. Certain inducible systems including estrogen, ecdysone, and FKBP12/FRAP based systems are known to activate off-target endogenous genes. The potentially deleterious effects of long-term antibiotic treatment can complicate the use of tetracycline transactivator (TET) based systems. *In vivo,* the temporal precision of these chemically inducible systems is dependent upon the kinetics of inducing agent uptake and elimination. Further, because inducing agents are generally delivered systemically, the spatial precision of such systems is bounded by the precision of exogenous vector delivery.

US Patent Publication No. 20030049799 relates to engineered stimulus-responsive switches to cause a detectable output in response to a preselected stimulus

US2011/145940 discloses methods for gene targeting using TAL-effector sequences.

There is an evident need for methods and compositions that allow for efficient and precise spatial and temporal control of a genomic locus of interest. These methods and compositions may provide for the regulation and modulation of genomic expression both *in vivo* and *in vitro* as well as provide for novel treatment methods for a number of disease pathologies.

Citation or identification of any document in this application is not an admission that such document is available as prior art to the present invention.

### SUMMARY OF THE INVENTION AND OF THE DISCLOSURE

The present invention is defined by the appended claims. Aspects of the present invention and/or present disclosure are described below. Aspects of the present disclosure may also pertain to the invention as defined the appended claims.

In one aspect the disclosure provides a non-naturally occurring or engineered TALE or CRISPR-Cas system which may comprise at least one switch wherein the activity of said TALE or CRISPR-Cas system is controlled by contact with at least one inducer energy source as to the switch. In an embodiment of the disclosure the control as to the at least one switch or the activity of said TALE or CRISPR-Cas system may be activated, enhanced, terminated or repressed. The contact with the at least one inducer energy source may result in a first effect and a second effect.
The first effect may be one or more of nuclear import, nuclear export, recruitment of a secondary component (such as an effector molecule), conformational change (of protein, DNA or RNA), cleavage, release of cargo (such as a caged molecule or a co-factor), association or dissociation. The second effect may be one or more of activation, enhancement, termination or repression of the control as to the at least one switch or the activity of said TALE or CRISPR-Cas system. In one embodiment the first effect and the second effect may occur in a cascade.

In another aspect of the invention or disclosure the TALE (invention) or CRISPR-Cas (disclosure) system may further comprise at least one nuclear localization signal (NLS), nuclear export signal (NES), functional domain, flexible linker, mutation, deletion, alteration or truncation. The one or more of the NLS, the NES or the functional domain may be conditionally activated or inactivated. In another embodiment, the mutation may be one or more of a mutation in a transcription factor homology region, a mutation in a DNA binding domain (such as mutating basic residues of a basic helix loop helix), a mutation in an endogenous NLS or a mutation in an endogenous NES. The disclosure comprehends that the inducer energy source may be heat, ultrasound, electromagnetic energy or chemical. In a preferred embodiment of the disclosure, the inducer energy source may be an antibiotic, a small molecule, a hormone, a hormone derivative, a steroid or a steroid derivative. In a more preferred embodiment, the inducer energy source maybe abscisic acid (ABA), doxycycline (DOX), cumate, rapamycin, 4-hydroxytamoxifen (4OHT), estrogen or ecdysone. The disclosure provides that the at least one switch may be selected from the group consisting of antibiotic based inducible systems, electromagnetic energy based inducible systems, small molecule based inducible systems, nuclear receptor based inducible systems and hormone based inducible systems. In a more preferred embodiment the at least one switch may be selected from the group consisting of tetracycline (Tet)/DOX inducible systems, light inducible systems, ABA inducible systems, cumate repressor/operator systems, 4OHT/estrogen inducible systems, ecdysone-based inducible systems and FKBP12/FRAP (FKBP12-rapamycin complex) inducible systems.

In the invention the inducer energy source is electromagnetic energy. The electromagnetic energy may be a component of visible light having a wavelength in the range of 450nm-700nm. In a preferred embodiment the component of visible light may have a wavelength in the range of 450nm-500nm and may be blue light. The blue light may have an intensity of at least 0.2mW/cm², or more preferably at least 4mW/cm². In another embodiment, the component of visible light may have a wavelength in the range of 620-700nm and is red light.

The invention comprehends systems wherein the at least one functional domain may be selected from the group consisting of: transposase domain, integrase domain, recombinase domain, resolvase domain, invertase domain, protease domain, DNA methyltransferase domain, DNA hydroxylmethylase domain, DNA demethylase domain, histone acetylase domain, histone deacetylases domain, nuclease domain, repressor domain, activator domain, nuclear-localization signal domains, transcription-regulatory protein (or transcription complex recruiting) domain, cellular uptake activity associated domain, nucleic acid binding domain, antibody presentation domain, histone modifying enzymes, recruiter of histone modifying enzymes; inhibitor of histone modifying enzymes, histone methyltransferase, histone demethylase, histone kinase, histone phosphatase, histone ribosylase, histone deribosylase, histone ubiquitinase, histone deubiquitinase, histone biotinase and histone tail protease.

The invention also provides for use of the system for perturbing a genomic or epigenomic locus of interest. Also provided are uses of the system for the preparation of a pharmaceutical compound.

In a further aspect, the disclosure provides a method of controlling a non-naturally occurring or engineered TALE or CRISPR-Cas system, comprising providing said TALE or CRISPR-Cas system comprising at least one switch wherein the activity of said TALE or CRISPR-Cas system is controlled by contact with at least one inducer energy source as to the switch.

In an embodiment of the disclosure, the invention provides methods wherein the control as to the at least one switch or the activity of said TALE or CRISPR-Cas system may be activated, enhanced, terminated or repressed. The contact with the at least one inducer energy source may result in a first effect and a second effect. The first effect may be one or more of nuclear import, nuclear export, recruitment of a secondary component (such as an effector molecule), conformational change (of protein, DNA or RNA), cleavage, release of cargo (such as a caged molecule or a co-factor), association or dissociation. The second effect may be one or more of activation, enhancement, termination or repression of the control as to the at least one switch or the activity of said TALE or CRISPR-Cas system. In one embodiment the first effect and the second effect may occur in a cascade.

In another aspect of the methods of the invention or disclosure the TALE (invention) or CRISPR-Cas (disclosure) system may further comprise at least one nuclear localization signal (NLS), nuclear export signal (NES), functional domain, flexible linker, mutation, deletion, alteration or truncation. The one or more of the NLS, the NES or the functional domain may be conditionally activated or inactivated. In another embodiment, the mutation may be one or more of a mutation in a transcription factor homology region, a mutation in a DNA binding domain (such as mutating basic residues of a basic helix loop helix), a mutation in an endogenous NLS or a mutation in an endogenous NES. The disclosure comprehends that the inducer energy source may be heat, ultrasound, electromagnetic energy or chemical. In a preferred embodiment of the disclosure , the inducer energy source may be an antibiotic, a small molecule, a hormone, a hormone derivative, a steroid or a steroid derivative. In a more preferred embodiment, the inducer energy source maybe abscisic acid (ABA), doxycycline (DOX), cumate, rapamycin, 4-hydroxytamoxifen (4OHT), estrogen or ecdysone. The disclosure provides that the at least one switch may be selected from the group consisting of antibiotic based inducible systems, electromagnetic energy based inducible systems, small molecule based inducible systems, nuclear receptor based inducible systems and hormone based inducible systems. In a more preferred embodiment the at least one switch may be selected from the group consisting of tetracycline (Tet)/DOX inducible systems, light inducible systems, ABA inducible systems, cumate repressor/operator systems, 4OHT/estrogen inducible systems, ecdysone-based inducible systems and FKBP12/FRAP (FKBP12-rapamycin complex) inducible systems.

In the methods of the invention the inducer energy source is electromagnetic energy. The electromagnetic energy may be a component of visible light having a wavelength in the range of 450nm-700nm. In a preferred embodiment the component of visible light may have a wavelength in the range of 450nm-500nm and may be blue light. The blue light may have an intensity of at least 0.2mW/cm², or more preferably at least 4mW/cm². In another embodiment, the component of visible light may have a wavelength in the range of 620-700nm and is red light.

The invention comprehends methods wherein the at least one functional domain may be selected from the group consisting of: transposase domain, integrase domain, recombinase domain, resolvase domain, invertase domain, protease domain, DNA methyltransferase domain, DNA hydroxylmethylase domain, DNA demethylase domain, histone acetylase domain, histone deacetylases domain, nuclease domain, repressor domain, activator domain, nuclear-localization signal domains, transcription-regulatory protein (or transcription complex recruiting) domain, cellular uptake activity associated domain, nucleic acid binding domain, antibody presentation domain, histone modifying enzymes, recruiter of histone modifying enzymes; inhibitor of histone modifying enzymes, histone methyltransferase, histone demethylase, histone kinase, histone phosphatase, histone ribosylase, histone deribosylase, histone ubiquitinase, histone deubiquitinase, histone biotinase and histone tail protease.

Further aspects of the disclosure provides for systems or methods as described herein wherein the TALE system comprises a DNA binding polypeptide comprising:
(i) a DNA binding domain comprising at least five or more Transcription activator-like effector (TALE) monomers and at least one or more half-monomers specifically ordered to target a locus of interest or
   at least one or more effector domains
   linked to an energy sensitive protein or fragment thereof, wherein the energy sensitive protein or fragment thereof undergoes a conformational change upon induction by an inducer energy source allowing it to bind an interacting partner, and/or
(ii) a DNA binding domain comprising at least one or more TALE monomers or half-monomers specifically ordered to target the locus of interest or
   at least one or more effector domains
   linked to the interacting partner, wherein the energy sensitive protein or fragment thereof binds to the interacting partner upon induction by the inducer energy source.

The systems and methods of the disclosure provide for the DNA binding polypeptide comprising a (a) a N-terminal capping region (b) a DNA binding domain comprising at least 5 to 40 Transcription activator-like effector (TALE) monomers and at least one or more half-monomers specifically ordered to target the locus of interest, and (c) a C-terminal capping region wherein (a), (b) and (c) may be arranged in a predetermined N-terminus to C-terminus orientation, wherein the genomic locus comprises a target DNA sequence 5'-T₀N₁N₂....N_{z} N_{z+1} - 3', where T₀ and N = A, G, T or C, wherein the target DNA sequence binds to the DNA binding domain, and the DNA binding domain may comprise (X₁₋₁₁-X₁₂X₁₃-X_{14-33 or 34 or 35})z, wherein X_{1- 11} is a chain of 11 contiguous amino acids, wherein X₁₂X₁₃ is a repeat variable diresidue (RVD), wherein X₁₄₋₃₃ or _{34 or 35} is a chain of 21, 22 or 23 contiguous amino acids, wherein z may be at least 5 to 40, wherein the polypeptide may be encoded by and translated from a codon optimized nucleic acid molecule so that the polypeptide preferentially binds to DNA of the locus of interest.

In a further embodiment, the system or method of the invention provides the N-terminal capping region or fragment thereof comprises 147 contiguous amino acids of a wild type N-terminal capping region, or the C-terminal capping region or fragment thereof comprises 68 contiguous amino acids of a wild type C-terminal capping region, or the N-terminal capping region or fragment thereof comprises 136 contiguous amino acids of a wild type N-terminal capping region and the C-terminal capping region or fragment thereof comprises 183 contiguous amino acids of a wild type C-terminal capping region. In another embodiment, the at least one RVD may be selected from the group consisting of (a) HH, KH, NH, NK, NQ, RH, RN, SS, NN, SN, KN for recognition of guanine (G); (b) NI, KI, RI, HI, SI for recognition of adenine (A); (c) NG, HG, KG, RG for recognition of thymine (T); (d) RD, SD, HD, ND, KD, YG for recognition of cytosine (C); (e) NV, HN for recognition of A or G; and (f) H*, HA, KA, N*, NA, NC, NS, RA, S*for recognition of A or T or G or C, wherein (*) means that the amino acid at X13 is absent.

In yet another embodiment the at least one RVD may be selected from the group consisting of (a) HH, KH, NH, NK, NQ, RH, RN, SS for recognition of guanine (G); (b) SI for recognition of adenine (A); (c) HG, KG, RG for recognition of thymine (T); (d) RD, SD for recognition of cytosine (C); (e) NV, HN for recognition of A or G and (f) H*, HA, KA, N*, NA, NC, NS, RA, S*for recognition of A or T or G or C, wherein (*) means that the amino acid at X13 is absent. In a preferred embodiment, the RVD for the recognition of G is RN, NH, RH or KH; or the RVD for the recognition of A is SI; or the RVD for the recognition of T is KG or RG; and the RVD for the recognition of C is SD or RD. In yet another embodiment, at least one of the following is present [LTLD] or [LTLA] or [LTQV] at X1-4, or [EQHG] or [RDHG] at positions X30-33 or X31-34 or X32-35.

In an aspect of the invention the TALE system is packaged into a AAV or a lentivirus vector.

Further aspects of the disclosure provides for systems or methods as described herein wherein the CRISPR system may comprise a vector system comprising: a) a first regulatory element operably linked to a CRISPR-Cas system guide RNA that targets a locus of interest, b) a second regulatory inducible element operably linked to a Cas protein, wherein components (a) and (b) may be located on same or different vectors of the system, wherein the guide RNA targets DNA of the locus of interest, wherein the Cas protein and the guide RNA do not naturally occur together. In a preferred aspect, the Cas protein is a Cas9 enzyme. The disclosure also provides for the vector being a AAV or a lentivirus.

The disclosure particularly relates to inducible methods of altering expression of a genomic locus of interest and to compositions that inducibly alter expression of a genomic locus of interest wherein the genomic locus may be contacted with a non-naturally occurring or engineered composition comprising a deoxyribonucleic acid (DNA) binding polypeptide.

This polypeptide may include a DNA binding domain comprising at least five or more Transcription activator-like effector (TALE) monomers and at least one or more half-monomers specifically ordered to target the genomic locus of interest or at least one or more effector domains linked to an energy sensitive protein or fragment thereof. The energy sensitive protein or fragment thereof may undergo a conformational change upon induction by an energy source allowing it to bind an interacting partner. The polypeptide may also include a DNA binding domain comprising at least one or more variant TALE monomers or half-monomers specifically ordered to target the genomic locus of interest or at least one or more effector domains linked to the interacting partner, wherein the energy sensitive protein or fragment thereof may bind to the interacting partner upon induction by the energy source. The method may also include applying the energy source and determining that the expression of the genomic locus is altered. In preferred embodiments of the disclosure the genomic locus may be in a cell.

The disclosure also relates to inducible methods of repressing expression of a genomic locus of interest and to compositions that inducibly repress expression of a genomic locus of interest wherein the genomic locus may be contacted with a non-naturally occurring or engineered composition comprising a DNA binding polypeptide.

The polypeptide may include a DNA binding domain comprising at least five or more Transcription activator-like effector (TALE) monomers and at least one or more half-monomers specifically ordered to target the genomic locus of interest or at least one or more repressor domains linked to an energy sensitive protein or fragment thereof. The energy sensitive protein or fragment thereof may undergo a conformational change upon induction by an energy source allowing it to bind an interacting partner. The polypeptide may also include a DNA binding domain comprising at least one or more variant TALE monomers or half-monomers specifically ordered to target the genomic locus of interest or at least one or more effector domains linked to the interacting partner, wherein the energy sensitive protein or fragment thereof may bind to the interacting partner upon induction by the energy source. The method may also include applying the energy source and determining that the expression of the genomic locus is repressed. In preferred embodiments of the disclosure the genomic locus may be in a cell.

The disclosure also relates to inducible methods of activating expression of a genomic locus of interest and to compositions that inducibly activate expression of a genomic locus of interest wherein the genomic locus may be contacted with a non-naturally occurring or engineered composition comprising a DNA binding polypeptide.

The polypeptide may include a DNA binding domain comprising at least five or more TALE monomers and at least one or more half-monomers specifically ordered to target the genomic locus of interest or at least one or more activator domains linked to an energy sensitive protein or fragment thereof. The energy sensitive protein or fragment thereof may undergo a conformational change upon induction by an energy source allowing it to bind an interacting partner. The polypeptide may also include a DNA binding domain comprising at least one or more variant TALE monomers or half-monomers specifically ordered to target the genomic locus of interest or at least one or more effector domains linked to the interacting partner, wherein the energy sensitive protein or fragment thereof may bind to the interacting partner upon induction by the energy source. The method may also include applying the energy source and determining that the expression of the genomic locus is activated. In preferred embodiments of the disclosure the genomic locus may be in a cell.

In another preferred embodiment of the invention, the inducible effector may be a Light Inducible Transcriptional Effector (LITE). The modularity of the LITE system allows for any number of effector domains to be employed for transcriptional modulation.

In yet another preferred embodiment of the disclosure, the inducible effector may be a chemical.

The present disclosure also contemplates an inducible multiplex genome engineering using CRISPR (clustered regularly interspaced short palindromic repeats)/Cas systems.

The present disclosure also encompasses nucleic acid encoding the polypeptides of the present invention. The nucleic acid may comprise a promoter, advantageously human Synapsin I promoter (hSyn). In a particularly advantageous embodiment, the nucleic acid may be packaged into an adeno associated viral vector (AAV).

The disclosure further also relates to methods of treatment or therapy that encompass the methods and compositions described herein.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings.

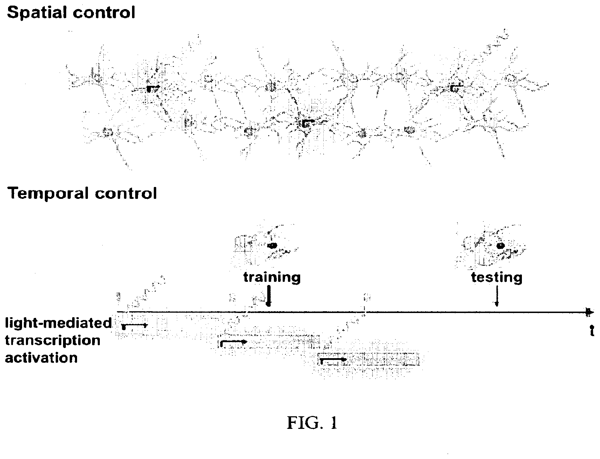

**FIG. 1** shows a schematic indicating the need for spatial and temporal precision.

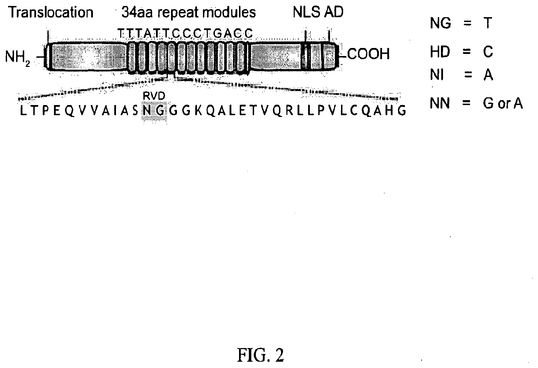

**FIG. 2** shows transcription activator like effectors (TALEs). TALEs consist of 34 aa repeats at the core of their sequence. Each repeat corresponds to a base in the target DNA that is bound by the TALE. Repeats differ only by 2 variable amino acids at positions 12 and 13. The code of this correspondence has been elucidated (Boch, J et al., Science, 2009 and Moscou, M et al. , Science, 2009) and is shown in this figure. Applicants have developed a method for the synthesis of designer TALEs incorporating this code and capable of binding a sequence of choice within the genome (Zhang, F et al., Nature Biotechnology, 2011).

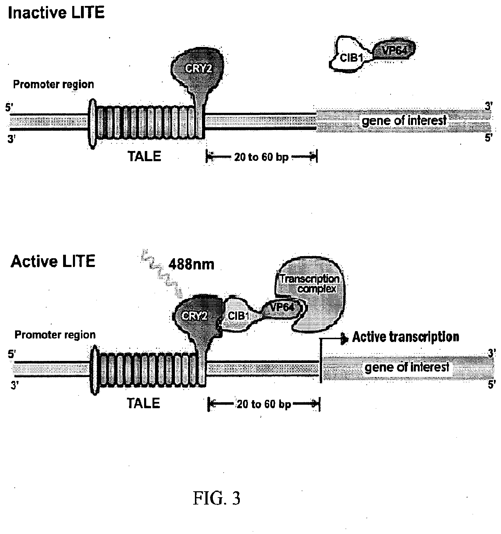

**FIG. 3** shows a design of a LITE: TALE/Cryptochrome transcriptional activation. Each LITE is a two-component system which may comprise a TALE fused to CRY2 and the cryptochrome binding partner CIB1 fused to VP64, a transcription activor. In the inactive state, the TALE localizes its fused CRY2 domain to the promoter region of the gene of interest. At this point, CIB1 is unable to bind CRY2, leaving the CIB1-VP64 unbound in the nuclear space. Upon stimulation with 488 nm (blue) light, CRY2 undergoes a conformational change, revealing its CIB1 binding site (Liu, H et al., Science, 2008). Rapid binding of CIB1 results in recruitment of the fused VP64 domain, which induces transcription of the target gene.

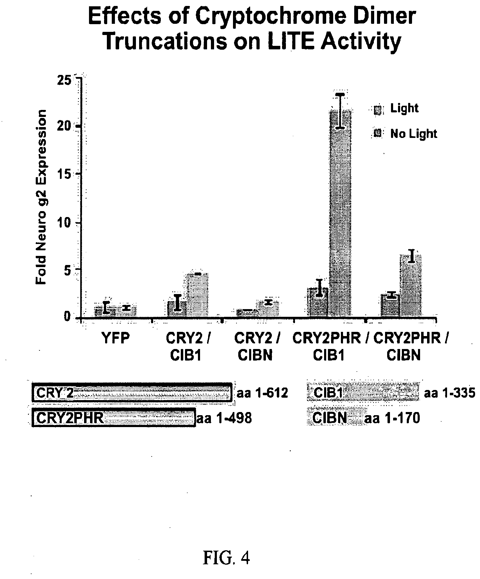

**FIG. 4** shows effects of cryptochrome dimer truncations on LITE activity. Truncations known to alter the activity of CRY2 and CIB1 (Kennedy M et al., Nature Methods 2010) were compared against the full length proteins. A LITE targeted to the promoter of Neurog2 was tested in Neuro-2a cells for each combination of domains. Following stimulation with 488 nm light, transcript levels of Neurog2 were quantified using qPCR for stimulated and unstimulated samples.

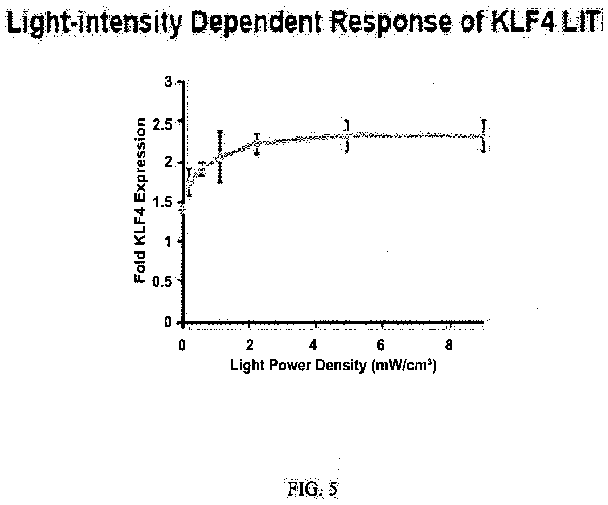

**FIG. 5** shows a light-intensity dependent response of KLF4 LITE.

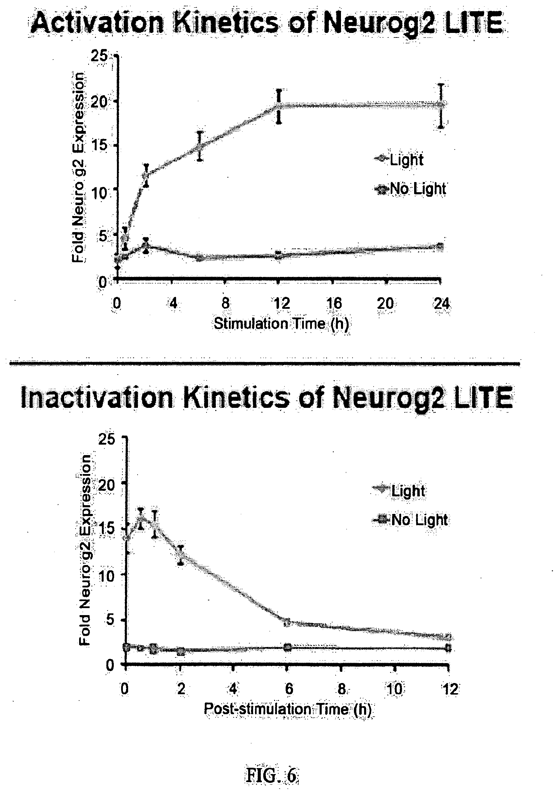

**FIG. 6** shows activation kinetics of Neurog2 LITE and inactivation kinetics of Neurog2 LITE.

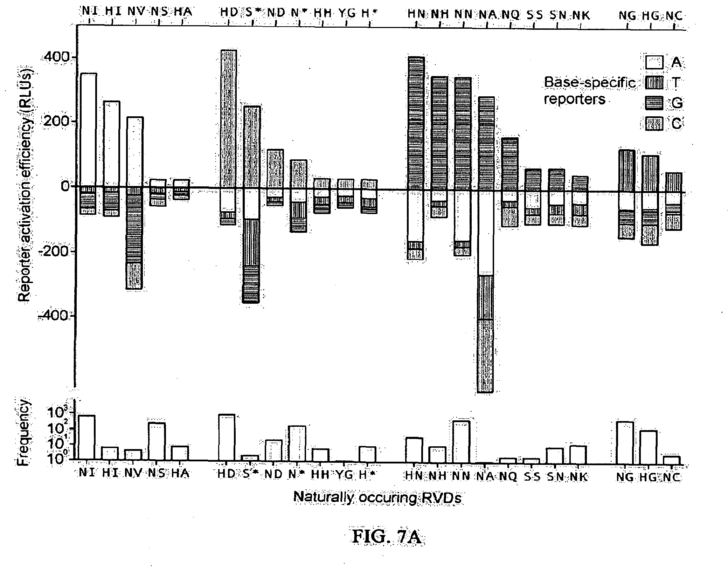

**FIG. 7A** shows the base-preference of various RVDs as determined using the Applicants' RVD screening system.

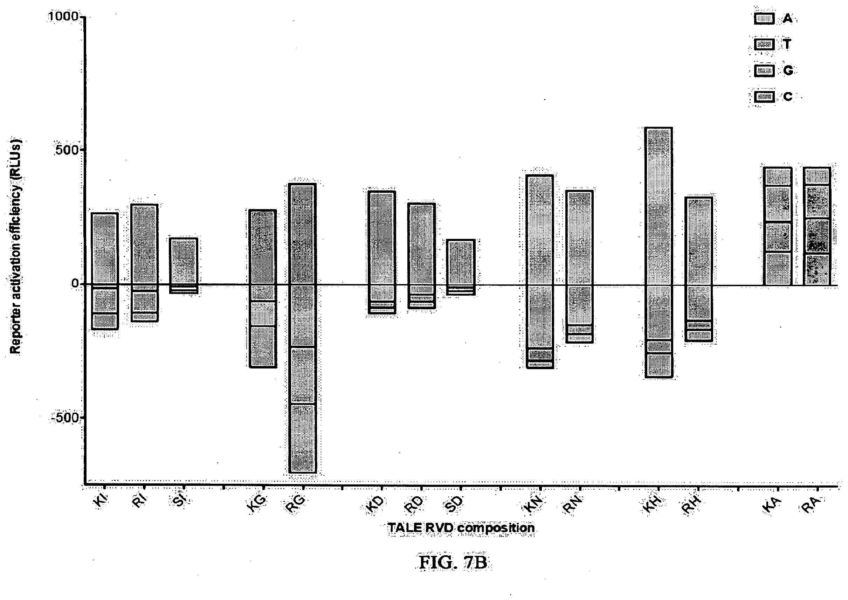

**FIG. 7B** shows the base-preference of additional RVDs as determined using the Applicants' RVD screening system.

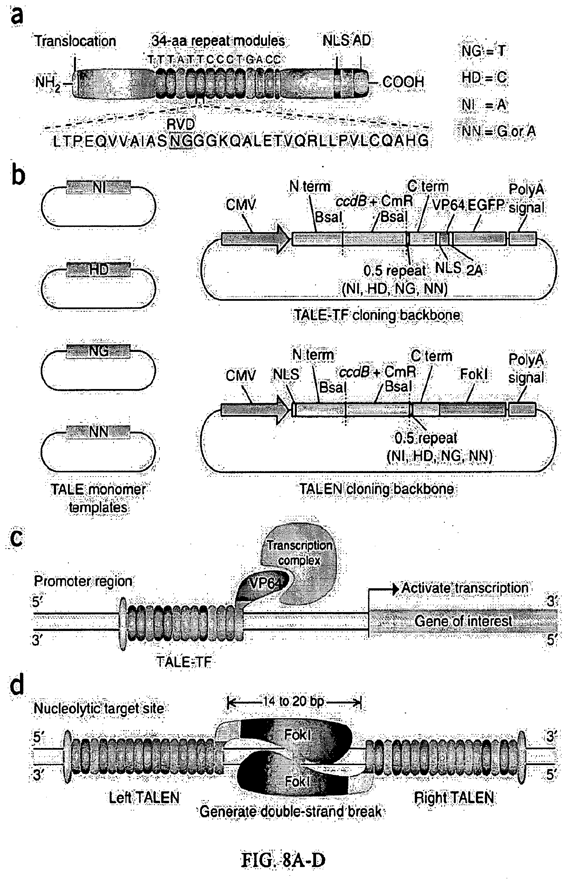

**FIGS. 8A-D** show in (**a**) Natural structure of TALEs derived from *Xanthomonas sp.* Each DNA-binding module consists of 34 amino acids, where the RVDs in the 12th and 13th amino acid positions of each repeat specify the DNA base being targeted according to the cipher NG = T, HD = C, NI = A, and NN = G or A. The DNA-binding modules are flanked by nonrepetitive N and C termini, which carry the translocation, nuclear localization (NLS) and transcription activation (AD) domains. A cryptic signal within the N terminus specifies a thymine as the first base of the target site. (**b**) The TALE toolbox allows rapid and inexpensive construction of custom TALE-TFs and TALENs. The kit consists of 12 plasmids in total: four monomer plasmids to be used as templates for PCR amplification, four TALE-TF and four TALEN cloning backbones corresponding to four different bases targeted by the 0.5 repeat. CMV, cytomegalovirus promoter; N term, nonrepetitive N terminus from the Hax3 TALE; C term, nonrepetitive C terminus from the Hax3 TALE; BsaI, type IIs restriction sites used for the insertion of custom TALE DNA-binding domains; *ccdB* + CmR, negative selection cassette containing the *ccdB* negative selection gene and chloramphenicol resistance gene; NLS, nuclear localization signal; VP64, synthetic transcriptional activator derived from VP16 protein of herpes simplex virus; 2A, 2A self-cleavage linker; EGFP, enhanced green fluorescent protein; polyA signal, polyadenylation signal; FokI, catalytic domain from the FokI endonuclease. (**c**) TALEs may be used to generate custom TALE-TFs and modulate the transcription of endogenous genes from the genome. The TALE DNA-binding domain is fused to the synthetic VP64 transcriptional activator, which recruits RNA polymerase and other factors needed to initiate transcription. (**d**) TALENs may be used to generate site-specific double-strand breaks to facilitate genome editing through nonhomologous repair or homology directed repair. Two TALENs target a pair of binding sites flanking a 16-bp spacer. The left and right TALENs recognize the top and bottom strands of the target sites, respectively. Each TALE DNA-binding domain is fused to the catalytic domain of Fokl endonuclease; when FokI dimerizes, it cuts the DNA in the region between the left and right TALEN-binding sites.

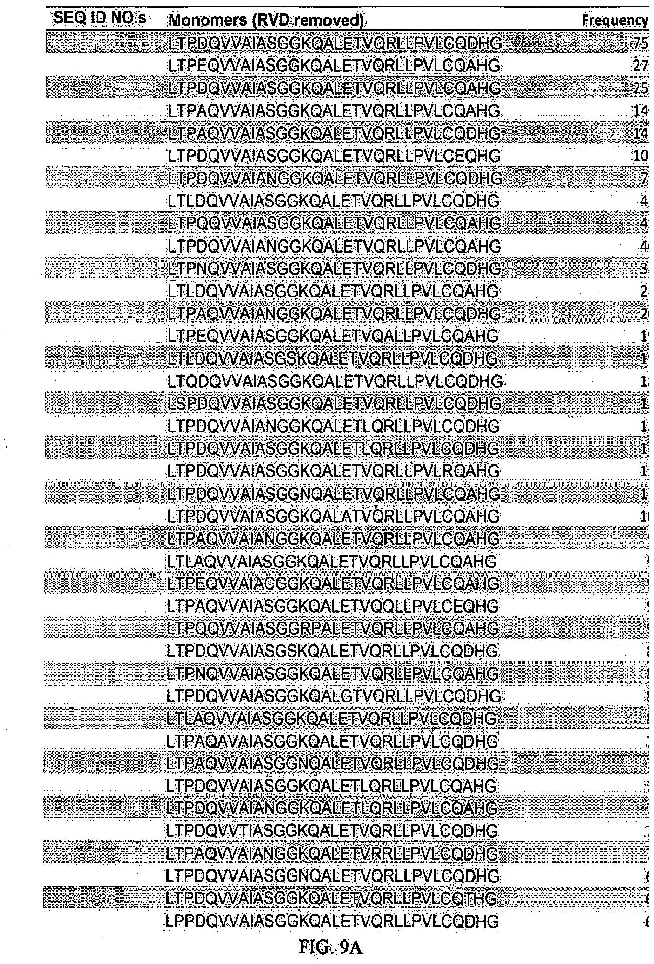

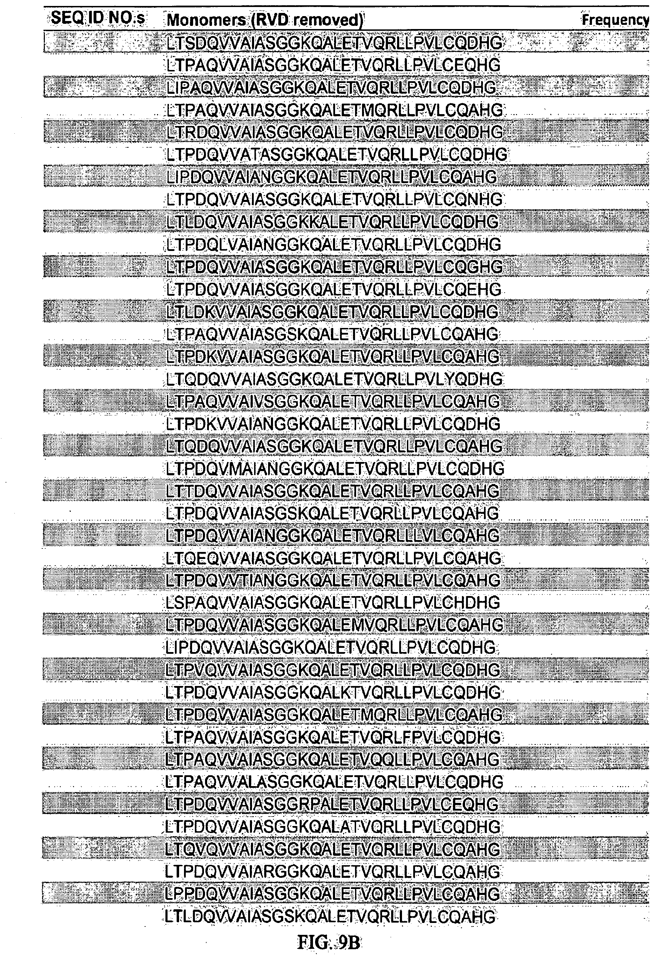

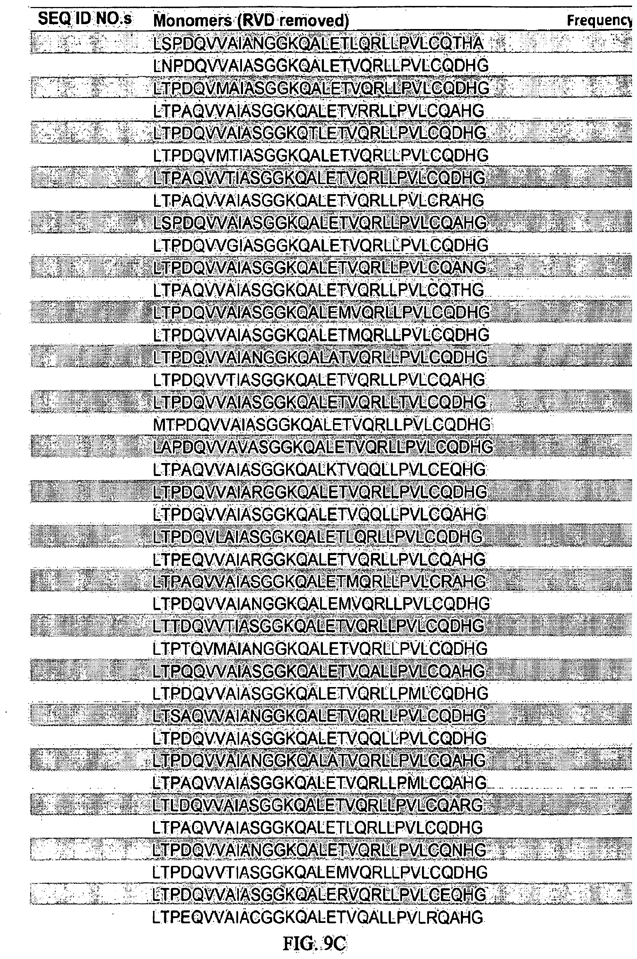

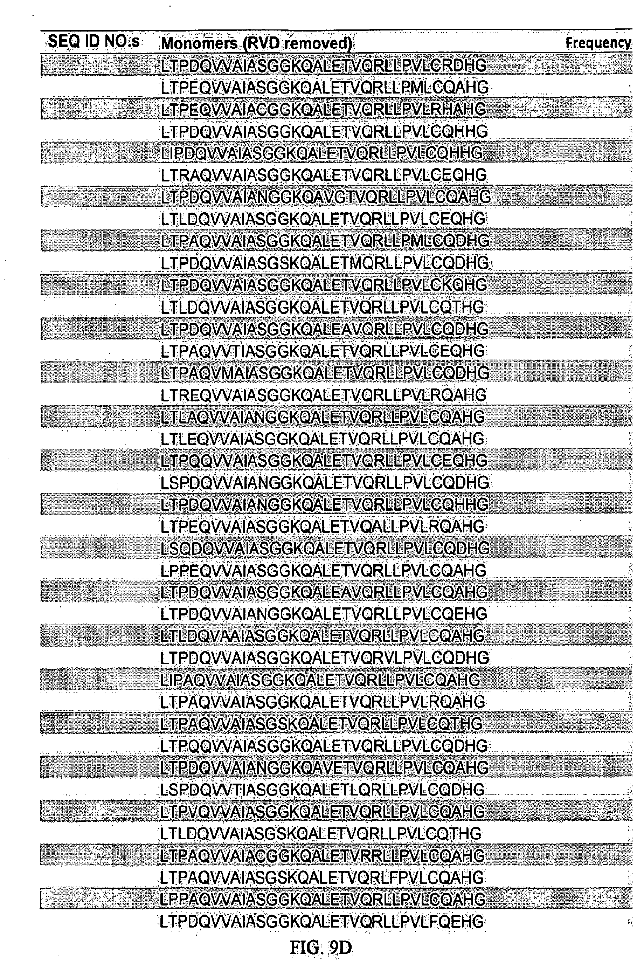

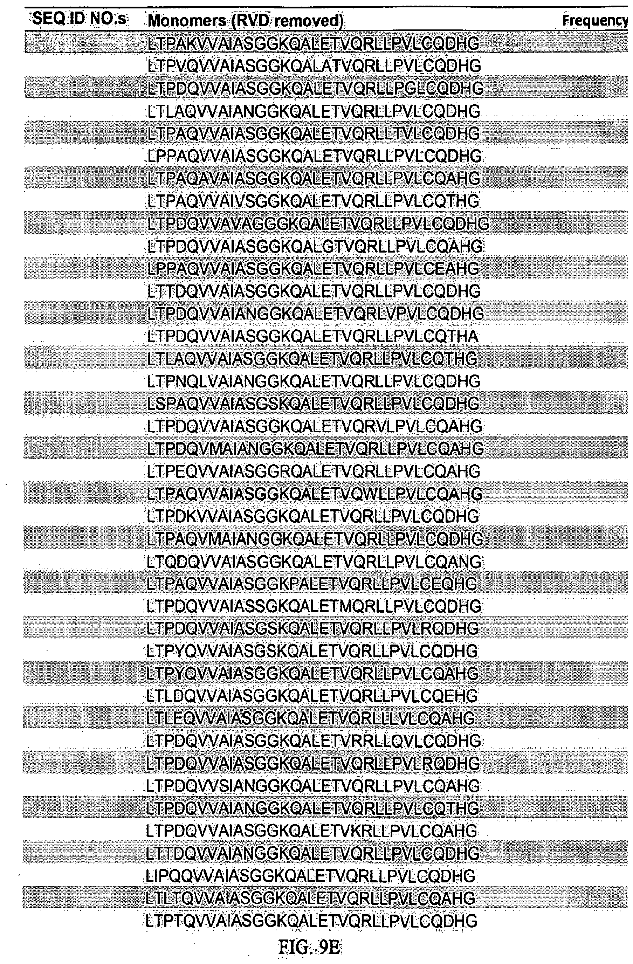

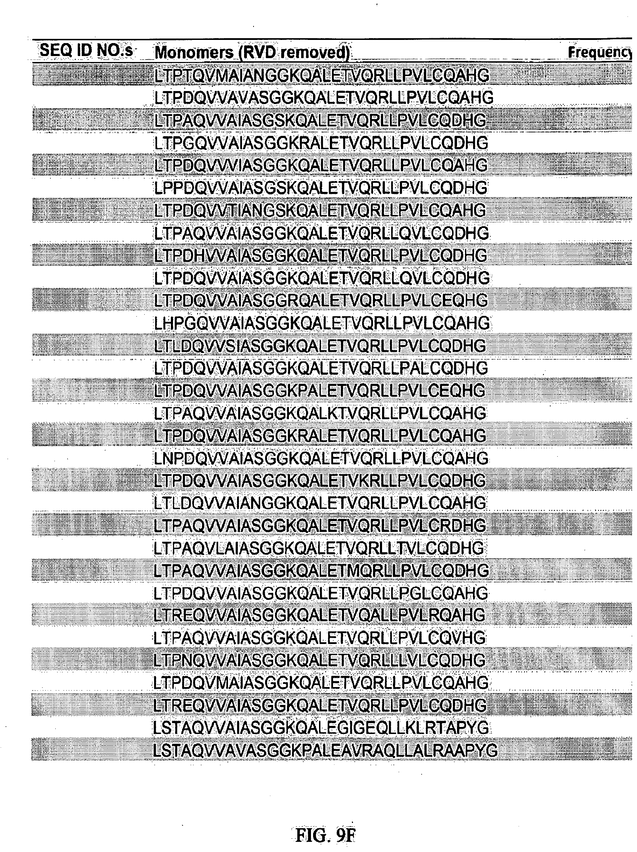

**FIG. 9A-F** shows a table listing monomer sequences (excluding the RVDs at positions 12 and 13) and the frequency with which monomers having a particular sequence occur.

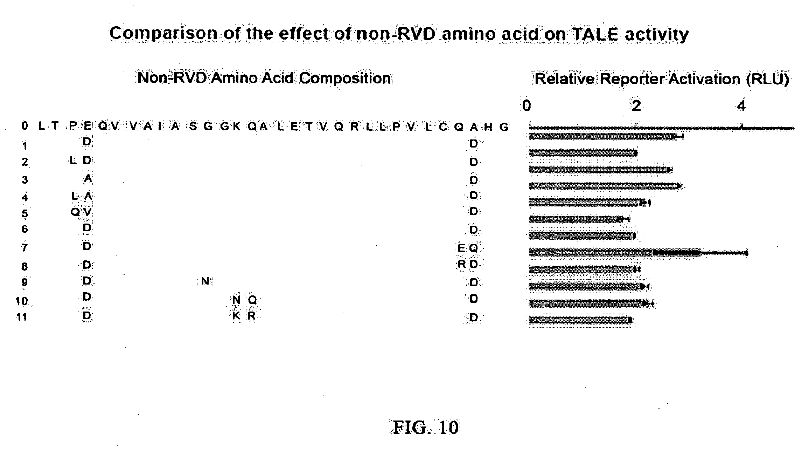

**FIG. 10** shows the comparison of the effect of non-RVD amino acid on TALE activity.

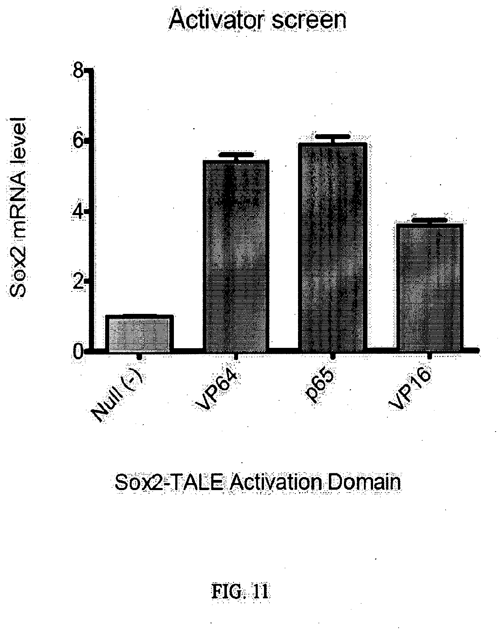

**FIG. 11** shows an activator screen comparing levels of activation between VP64, p65 and VP16.

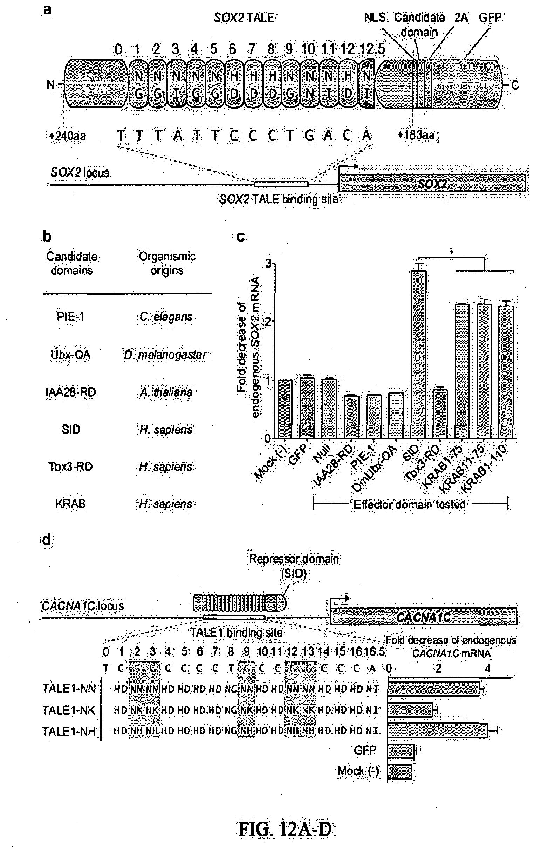

**FIGS. 12A-D** show the development of a TALE transcriptional repressor architecture. (**a**) Design of *SOX2* TALE for TALE repressor screening. A TALE targeting a 14bp sequence within the *SOX2* locus of the human genome was synthesized. (**b**) List of all repressors screened and their host origin (left). Eight different candidate repressor domains were fused to the C-term of the *SOX2* TALE. (c) The fold decrease of endogenous SOX2 mRNA is measured using qRTPCR by dividing the *SOX2* mRNA levels in mock transfected cells by *SOX2* mRNA levels in cells transfected with each candidate TALE repressor. (d) Transcriptional repression of endogenous CACNA1C. TALEs using NN, NK, and NH as the G-targeting RVD were constructed to target a 18 bp target site within the human *CACNA1C* locus. Each TALE is fused to the SID repression domain. NLS, nuclear localization signal; KRAB, Krüppel-associated box; SID, mSin interaction domain. All results are collected from three independent experiments in HEK 293FT cells. Error bars indicate s.e.m.; *n* = 3. * *p* < 0.05, Student's t test.

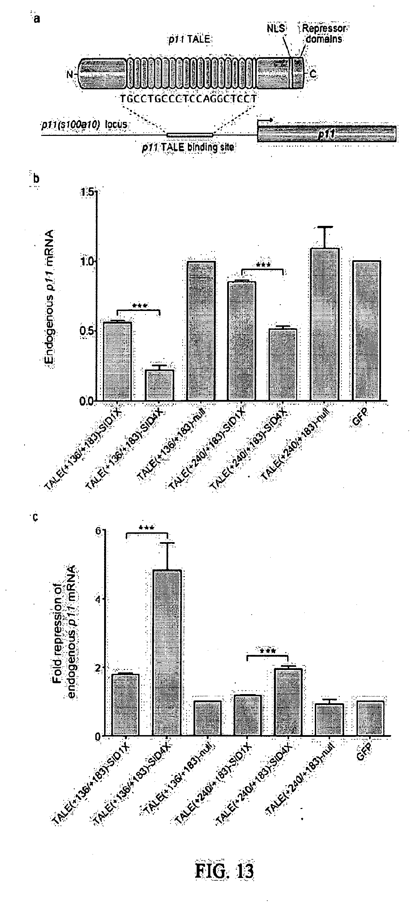

**FIGS. 13A-C** shows the optimization of TALE transcriptional repressor architecture using SID and SID4X. (a) Design of *p11* TALE for testing of TALE repressor architecture. A TALE targeting a 20bp sequence (*p11* TALE binding site) within the *p11* (*s100a10*) locus of the mouse (*Mus musculus*) genome was synthesized. (**b**) Transcriptional repression of endogenous mouse *p11* mRNA. TALEs targeting the mouse *p11* locus harboring two different truncations of the wild type TALE architecture were fused to different repressor domains as indicated on the *x*-axis. The value in the bracket indicate the number of amino acids at the N- and C- termini of the TALE DNA binding domain flanking the DNA binding repeats, followed by the repressor domain used in the construct. The endogenous p11 mRNA levels were measured using qRT-PCR and normalized to the level in the negative control cells transfected with a GFP-encoding construct. (**c**) Fold of transcriptional repression of endogenous mouse *p11.* The fold decrease of endogenous *p11* mRNA is measured using qRT-PCR through dividing the *p11* mRNA levels in cells transfected with a negative control GFP construct by *p11* mRNA levels in cells transfected with each candidate TALE repressors. The labeling of the constructs along the *x*-axis is the same as previous panel. NLS, nuclear localization signal; SID, mSin interaction domain; SID4X, an optimized four-time tandem repeats of SID domain linked by short peptide linkers. All results are collected from three independent experiments in Neuro2A cells. Error bars indicate s.e.m.; *n* = 3. *** *p* < 0.001, Student's t test.

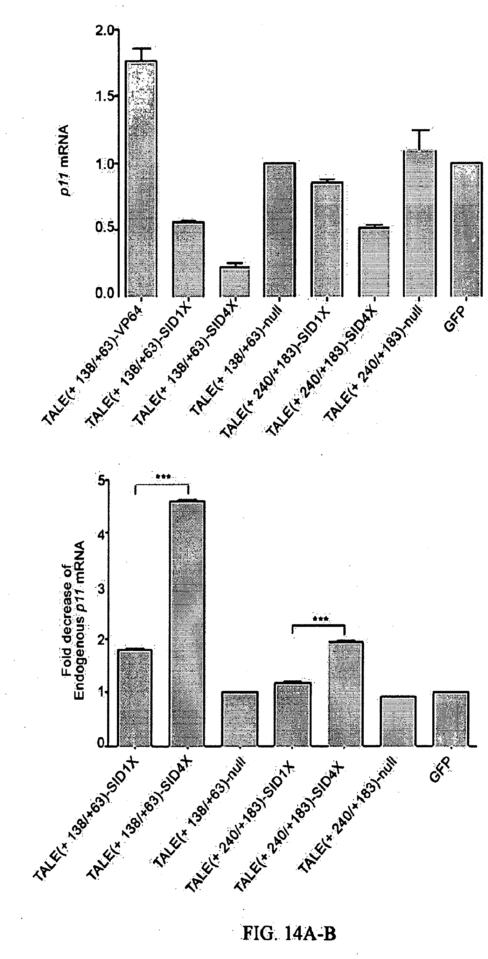

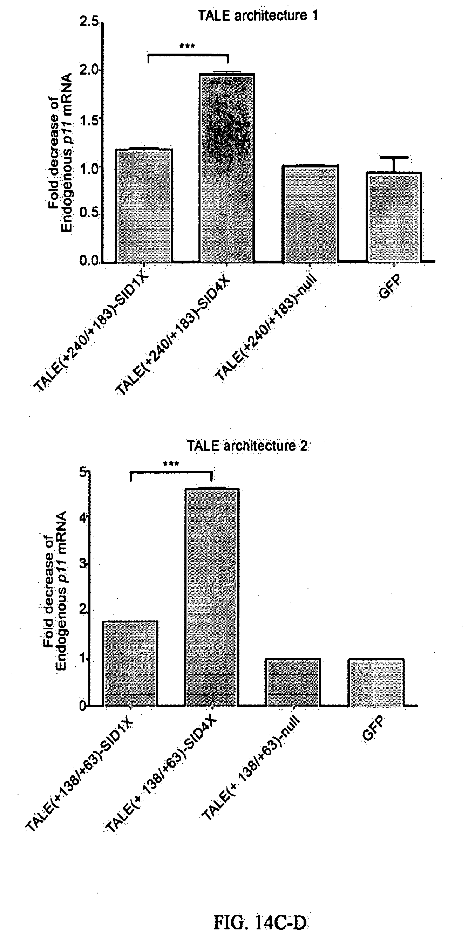

**FIG. 14** shows a comparison of two different types of TALE architecture.

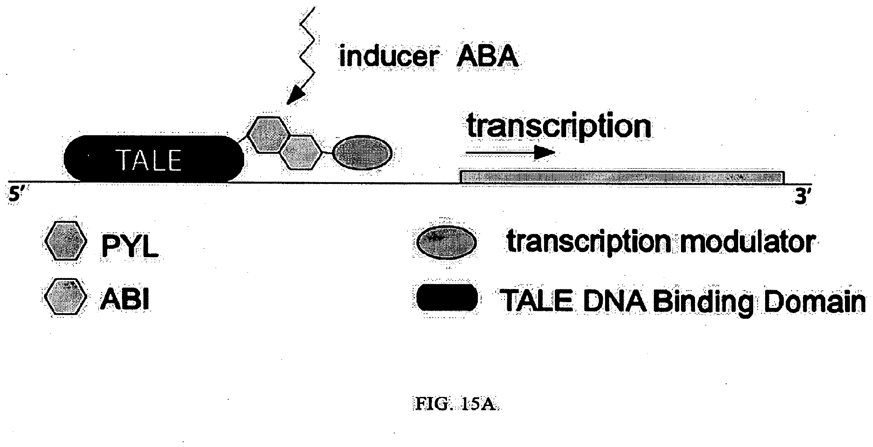

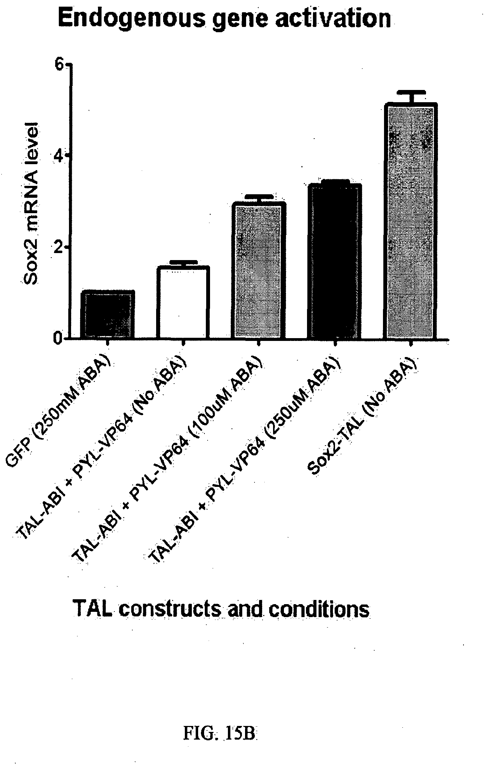

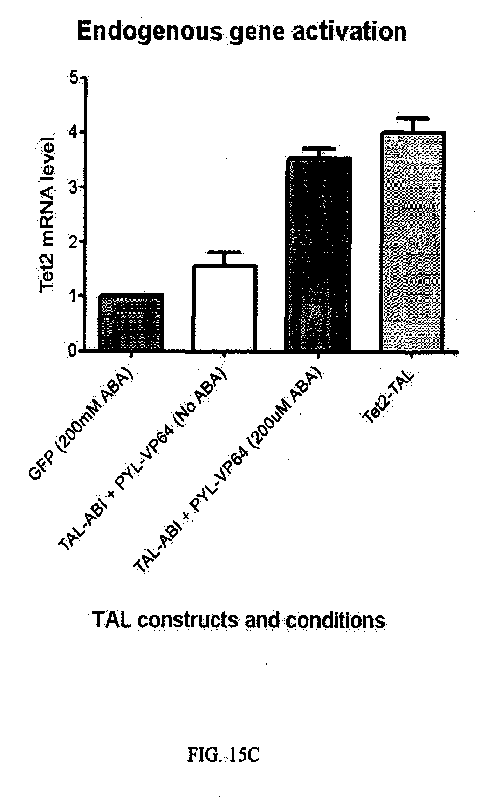

**FIGS. 15A-C** show a chemically inducible TALE ABA inducible system. ABI (ABA insensitive 1) and PYL (PYL protein: pyrabactin resistance (PYR)/PYR1-like (PYL)) are domains from two proteins listed below that will dimerize upon binding of plant hormone Abscisic Acid (ABA). This plant hormone is a small molecule chemical that Applicants used in Applicants' inducible TALE system. In this system, the TALE DNA-binding polypeptide is fused to the ABI domain, whereas the VP64 activation domain or SID repressor domain or any effector domains are linked to the PYL domain. Thus, upon the induction by the presence of ABA molecule, the two interacting domains, ABI and PYL, will dimerize and allow the TALE to be linked to the effector domains to perform its activity in regulating target gene expression.

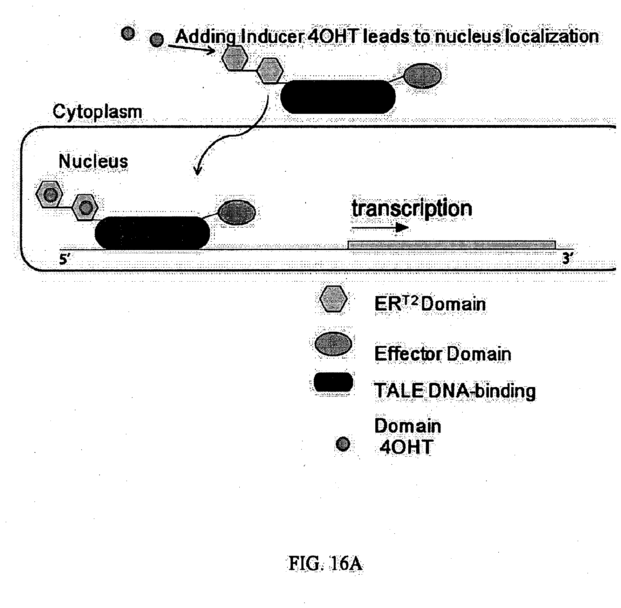

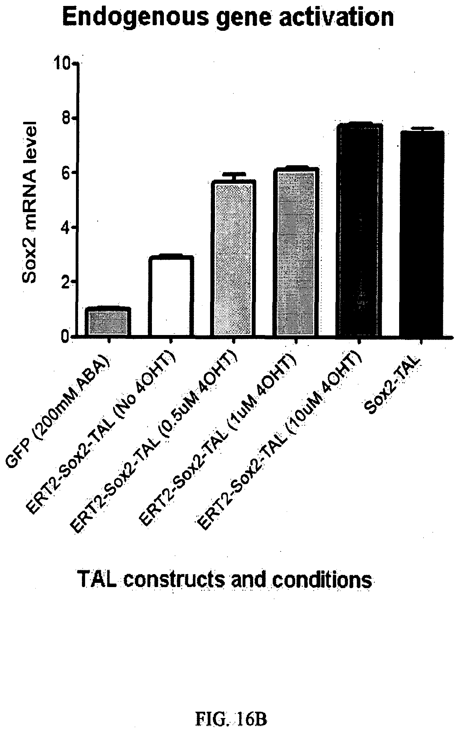

**FIGS. 16A-B** show a chemically inducible TALE 4OHT inducible system.

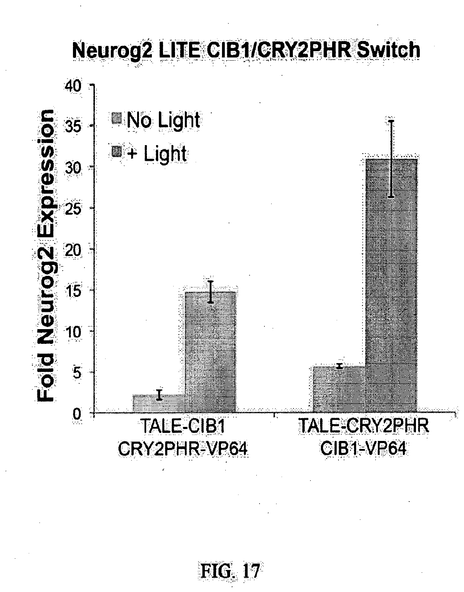

**FIG. 17** depicts an effect of cryptochrome2 heterodimer orientation on LITE functionality.

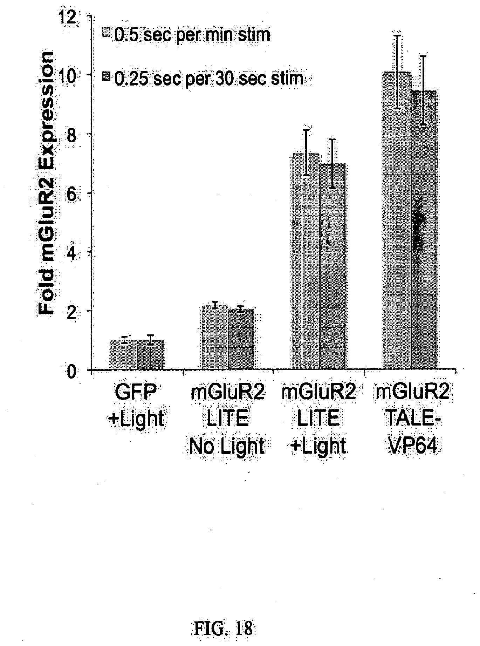

**FIG. 18** depicts mGlur2 LITE activity in mouse cortical neuron culture.

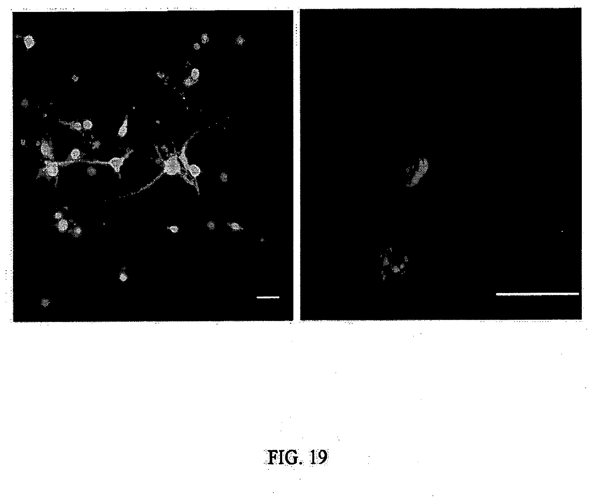

**FIG. 19** depicts transduction of primary mouse neurons with LITE AAV vectors.

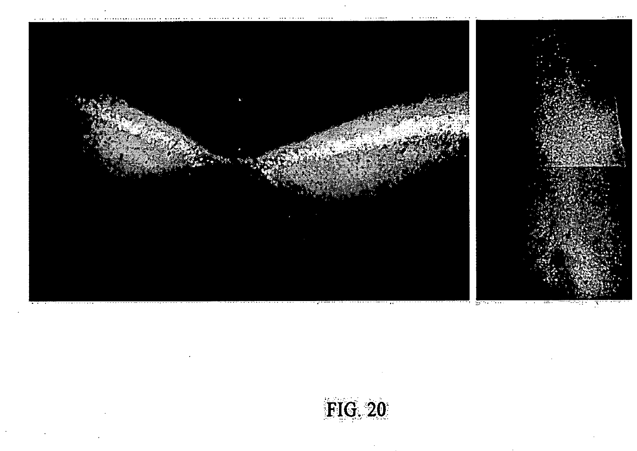

**FIG. 20** depicts expression of LITE component in vivo.

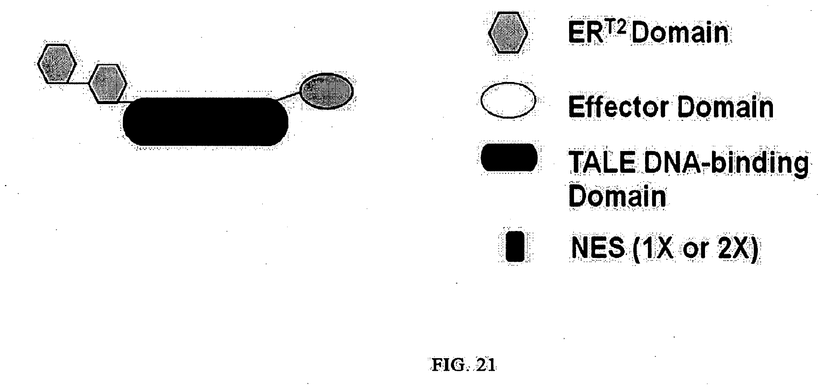

**FIG. 21** depicts an improved design of the construct where the specific NES peptide sequence used is LDLASLIL.

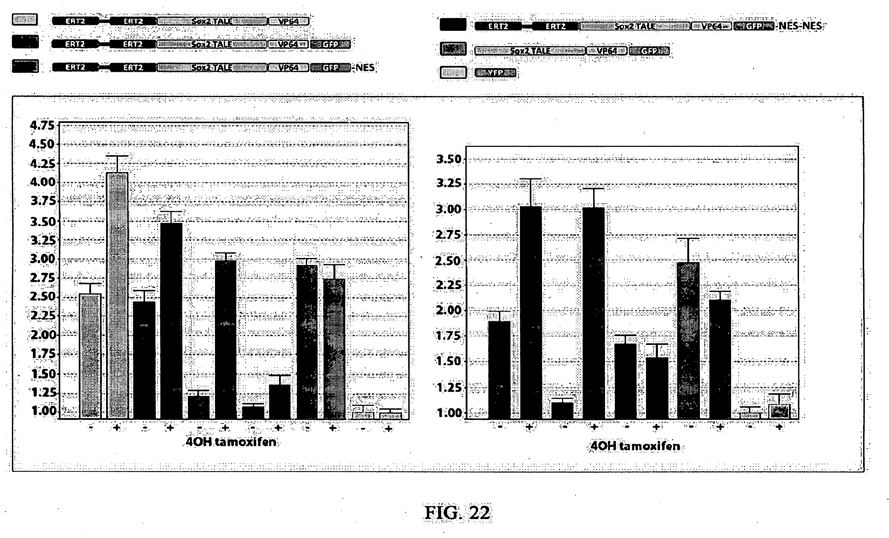

**FIG. 22** depicts Sox2 mRNA levels in the absence and presence of 4OH tamoxifen.

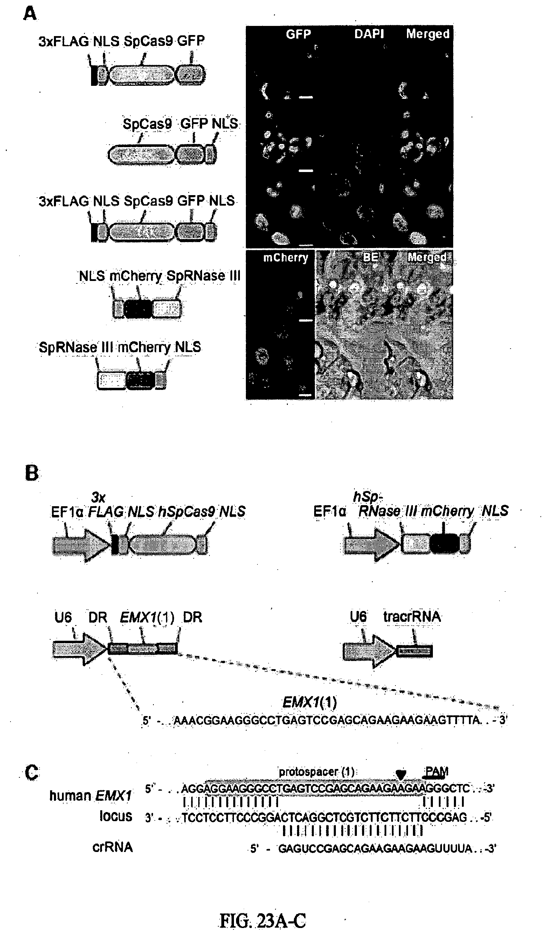

**FIGS. 23A-E** (disclosure) depict a Type II CRISPR locus from *Streptococcus pyogenes* SF370 can be reconstituted in mammalian cells to facilitate targeted DSBs of DNA. (**A**) Engineering of SpCas9 and SpRNase III with NLSs enables import into the mammalian nucleus. (**B**) Mammalian expression of SpCas9 and SpRNase III are driven by the EFla promoter, whereas tracrRNA and pre-crRNA array (DR-Spacer-DR) are driven by the U6 promoter. A protospacer (blue highlight) from the human *EMX1* locus with PAM is used as template for the spacer in the pre-crRNA array. (**C**) Schematic representation of base pairing between target locus and *EMX1*-targeting crRNA. Red arrow indicates putative cleavage site. (**D**) SURVEYOR assay for SpCas9-mediated indels. (**E**) An example chromatogram showing a micro-deletion, as well as representative sequences of mutated alleles identified from 187 clonal amplicons. Red dashes, deleted bases; red bases, insertions or mutations. Scale bar = 10µm.

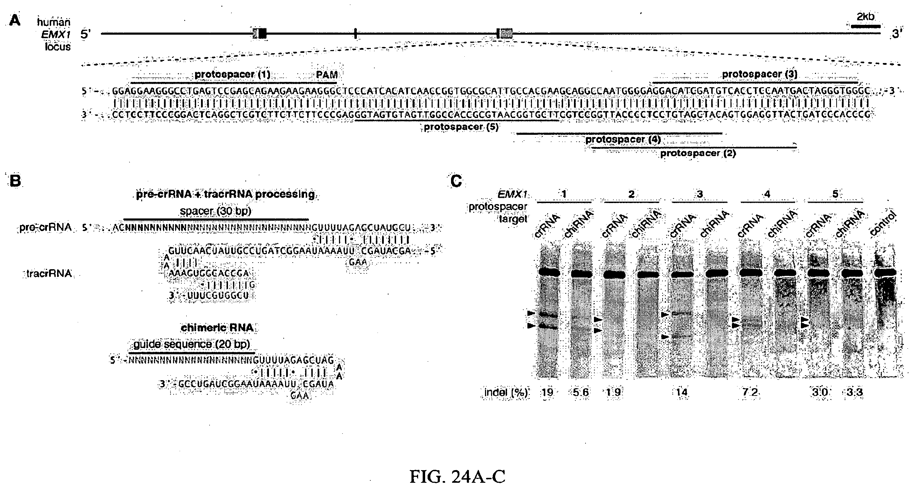

**FIGS. 24A-C** (disclosure) depict a SpCas9 can be reprogrammed to target multiple genomic loci in mammalian cells. (**A**) Schematic of the human *EMX1* locus showing the location of five protospacers, indicated by blue lines with corresponding PAM in magenta. (**B**) Schematic of the pre-crRNA:tracrRNA complex (top) showing hybridization between the direct repeat (gray) region of the pre-crRNA and tracrRNA. Schematic of a chimeric RNA design (M. Jinek et al., A programmable dual-RNA-guided DNA endonuclease in adaptive bacterial immunity. Science 337, 816 (Aug 17, 2012)) (bottom). tracrRNA sequence is shown in red and the 20bp spacer sequence in blue. (**C**) SURVEYOR assay comparing the efficacy of Cas9-mediated cleavage at five protospacers in the human *EMX1* locus. Each protospacer is targeted using either processed pre-crRNA:tracrRNA complex (crRNA) or chimeric RNA (chiRNA).

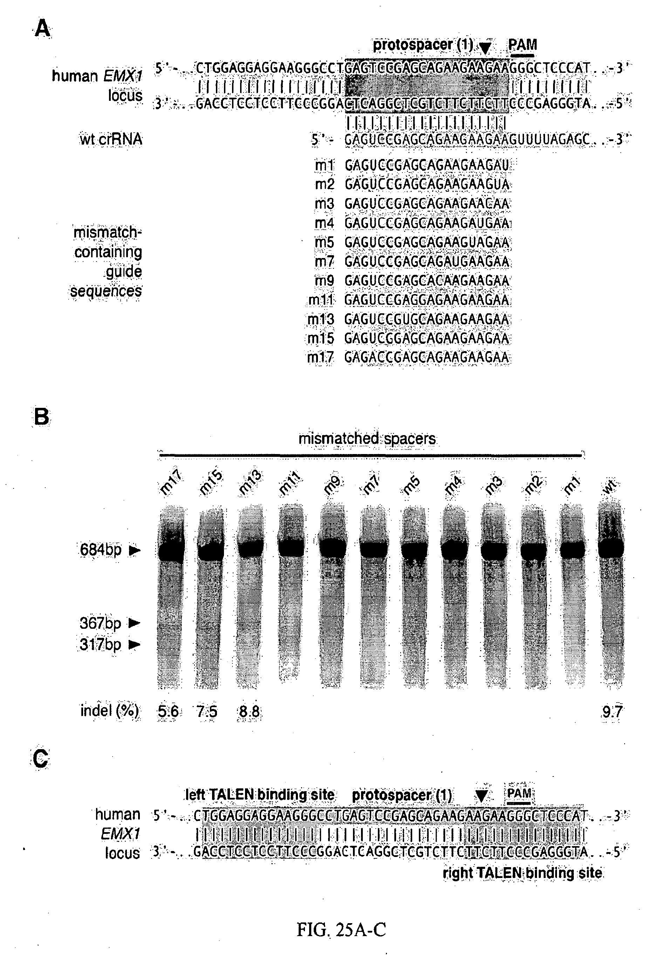

**FIGS. 25A-D** (disclosure) depict an evaluation of the SpCas9 specificity and comparison of efficiency with TALENs. (**A**) *EMX1*-targeting chimeric crRNAs with single point mutations were generated to evaluate the effects of spacer-protospacer mismatches. (**B**) SURVEYOR assay comparing the cleavage efficiency of different mutant chimeric RNAs. (**C**) Schematic showing the design of TALENs targeting *EMX1.* (**D**) SURVEYOR gel comparing the efficiency of TALEN and SpCas9 (*N* = 3).

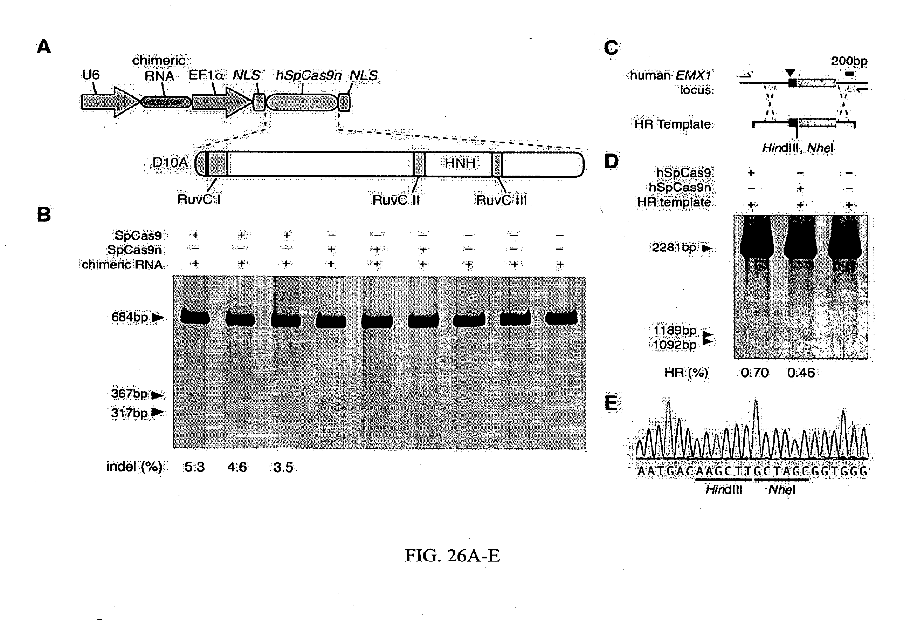

**FIGS. 26A-G** (disclosure) depict applications of Cas9 for homologous recombination and multiplex genome engineering. (**A**) Mutation of the RuvC I domain converts Cas9 into a nicking enzyme (SpCas9n) (**B**) Co-expression of *EMX1*-targeting chimeric RNA with SpCas9 leads to indels, whereas SpCas9n does not (*N* = 3). (**C**) Schematic representation of the recombination strategy. A repair template is designed to insert restriction sites into *EMX1* locus. Primers used to amplify the modified region are shown as red arrows. (**D**) Restriction fragments length polymorphism gel analysis. Arrows indicate fragments generated by *Hin*dIII digestion. (**E**) Example chromatogram showing successful recombination. (**F**) SpCas9 can facilitate multiplex genome modification using a crRNA array containing two spacers targeting *EMX1* and *PVALB.* Schematic showing the design of the crRNA array (top). Both spacers mediate efficient protospacer cleavage (bottom). (**G**) SpCas9 can be used to achieve precise genomic deletion. Two spacers targeting *EMX1* (top) mediated a 118bp genomic deletion (bottom).

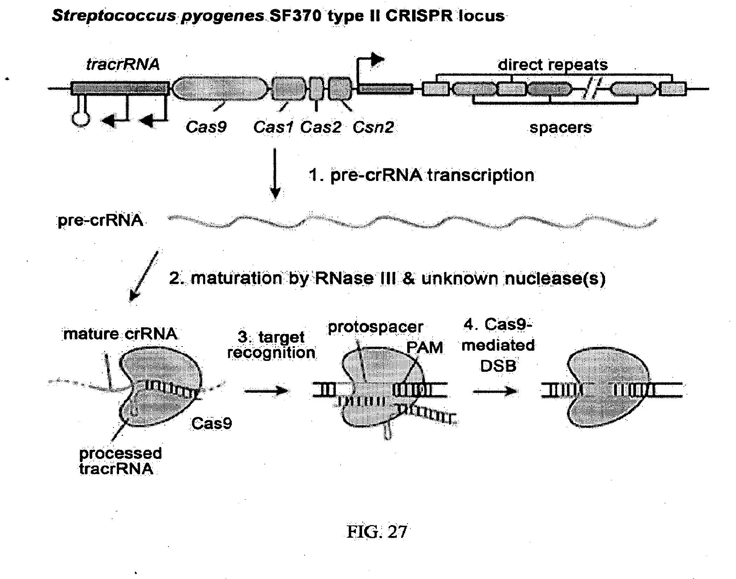

**FIG. 27** (disclosure) depicts a schematic of the type II CRISPR-mediated DNA double-strand break. The type II CRISPR locus from *Streptococcus pyogenes* SF370 contains a cluster of four genes, *Cas9, Cas1, Cas2,* and *Csn1,* as well as two non-coding RNA elements, tracrRNA and a characteristic array of repetitive sequences (direct repeats) interspaced by short stretches of non-repetitive sequences (spacers, 30bp each) (*15-18, 30, 31*). Each spacer is typically derived from foreign genetic material (protospacer), and directs the specificity of CRISPR-mediated nucleic acid cleavage. In the target nucleic acid, each protospacer is associated with a protospacer adjacent motif (PAM) whose recognition is specific to individual CRISPR systems (*22*, *23*). The Type II CRISPR system carries out targeted DNA double-strand break (DSB) in sequential steps (M. Jinek et al., Science 337, 816 (Aug 17, 2012); Gasiunas, R. et al. Proc Natl Acad Sci U S A 109, E2579 (Sep 25, 2012); J. E. Garneau et al., Nature 468, 67 (Nov 4, 2010); R. Sapranauskas et al., Nucleic Acids Res 39, 9275 (Nov, 2011); A. H. Magadan et al. PLoS One 7, e40913 (2012)). First, the pre-crRNA array and tracrRNA are transcribed from the CRISPR locus. Second, tracrRNA hybridizes to the direct repeats of pre-crRNA and associates with Cas9 as a duplex, which mediates the processing of the pre-crRNA into mature crRNAs containing individual,truncated spacer sequences. Third, the mature crRNA:tracrRNA duplex directs Cas9 to the DNA target consisting of the protospacer and the requisite PAM via heteroduplex formation between the spacer region of the crRNA and the protospacer DNA. Finally, Cas9 mediates cleavage of target DNA upstream of PAM to create a DSB within the protospacer.

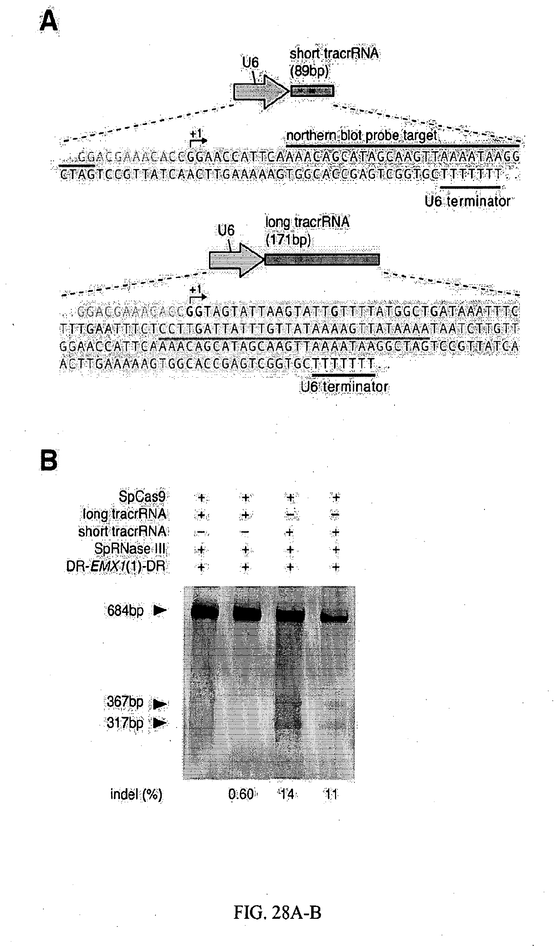

**FIGS. 28A-C** (disclosure) depict a comparison of different tracrRNA transcripts for Cas9-mediated gene targeting. (**A**) Schematic showing the design and sequences of two tracrRNA transcripts tested (short and long). Each transcript is driven by a U6 promoter. Transcription start site is marked as +1 and transcription terminator is as indicated. Blue line indicates the region whose reverse-complement sequence is used to generate northern blot probes for tracrRNA detection. (**B**) SURVEYOR assay comparing the efficiency of hSpCas9-mediated cleavage of the *EMX1* locus. Two biological replicas are shown for each tracrRNA transcript. (**C**) Northern blot analysis of total RNA extracted from 293FT cells transfected with U6 expression constructs carrying long or short tracrRNA, as well as SpCas9 and DR-*EMX1*(1)-DR. Left and right panels are from 293FT cells transfected without or with SpRNase III respectively. U6 indicate loading control blotted with a probe targeting human U6 snRNA. Transfection of the short tracrRNA expression construct led to abundant levels of the processed form of tracrRNA (∼75bp) (E. Deltcheva et al., Nature 471, 602 (Mar 31, 2011)). Very low amounts of long tracrRNA are detected on the Northern blot. As a result of these experiments, Applicants chose to use short tracrRNA for application in mammalian cells.

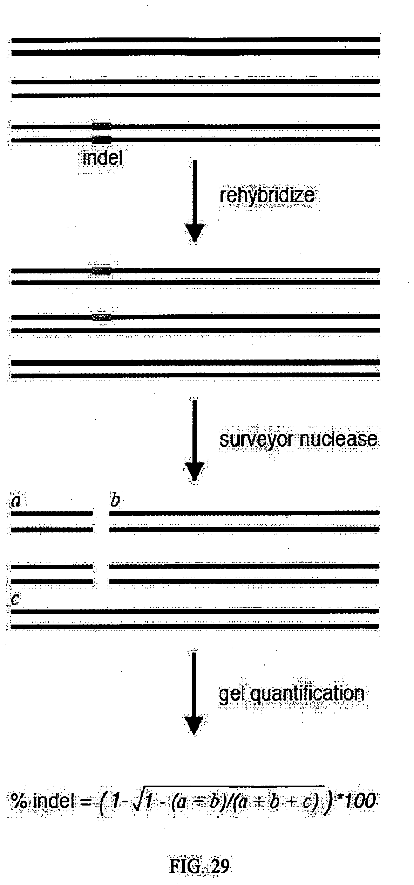

**FIG. 29** (disclosure) depicts a SURVEYOR assay for detection of double strand break-induced micro insertions and deletions (D. Y. Guschin et al. Methods Mol Biol 649, 247 (2010)). Schematic of the SURVEYOR assay used to determine Cas9-mediated cleavage efficiency. First, genomic PCR (gPCR) is used to amplify the Cas9 target region from a heterogeneous population of modified and unmodified cells, and the gPCR products are reannealed slowly to generate heteroduplexes. The reannealed heteroduplexes are cleaved by SURVEYOR nuclease, whereas homoduplexes are left intact. Cas9-mediated cleavage efficiency (% indel) is calculated based on the fraction of cleaved DNA.

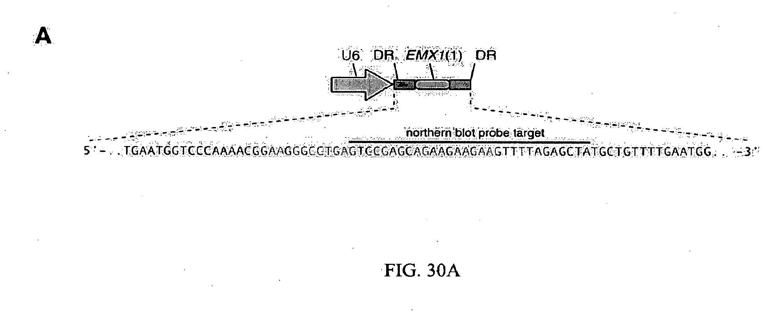

**FIG. 30A-B** (disclosure) depict a Northern blot analysis of crRNA processing in mammalian cells. (A) Schematic showing the expression vector for a single spacer flanked by two direct repeats (DR-*EMX1(1)-DR*). The 30bp spacer targeting the human *EMX1* locus protospacer 1 (Table 1) is shown in blue and direct repeats are in shown in gray. Orange line indicates the region whose reversecomplement sequence is used to generate northern blot probes for *EMX1*(1) crRNA detection. (**B**) Northern blot analysis of total RNA extracted from 293FT cells transfected with U6 expression constructs carrying DR-*EMX1*(1)-DR. Left and right panels are from 293FT cells transfected without or with SpRNase III respectively. DR-*EMX1*(1)-DR was processed into mature crRNAs only in the presence of SpCas9 and short tracrRNA, and was not dependent on the presence of SpRNase III. The mature crRNA detected from transfected 293FT total RNA is ∼33bp and is shorter than the 39-42bp mature crRNA from *S. pyogenes* (E. Deltcheva et al., Nature 471, 602 (Mar 31, 2011)), suggesting that the processed mature crRNA in human 293FT cells is likely different from the bacterial mature crRNA in *S. pyogenes.*

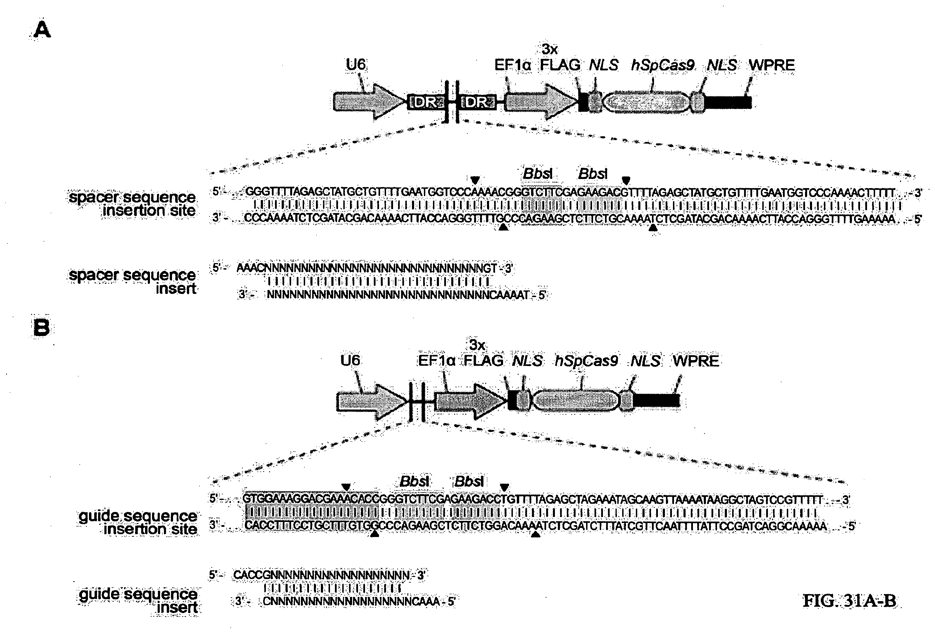

**FIG. 31A-B** (disclosure) depict a bicistronic expression vectors for pre-crRNA array or chimeric crRNA with Cas9. (**A**) Schematic showing the design of an expression vector for the pre-crRNA array. Spacers can be inserted between two *Bbs*I sites using annealed oligonucleotides. Sequence design for the oligonucleotides are shown below with the appropriate ligation adapters indicated. (**B**) Schematic of the expression vector for chimeric crRNA. The guide sequence can be inserted between two *Bbs*I sites using annealed oligonucleotides. The vector already contains the partial direct repeat (gray) and partial tracrRNA (red) sequences. WPRE, Woodchuck hepatitis virus posttranscriptional regulatory element.

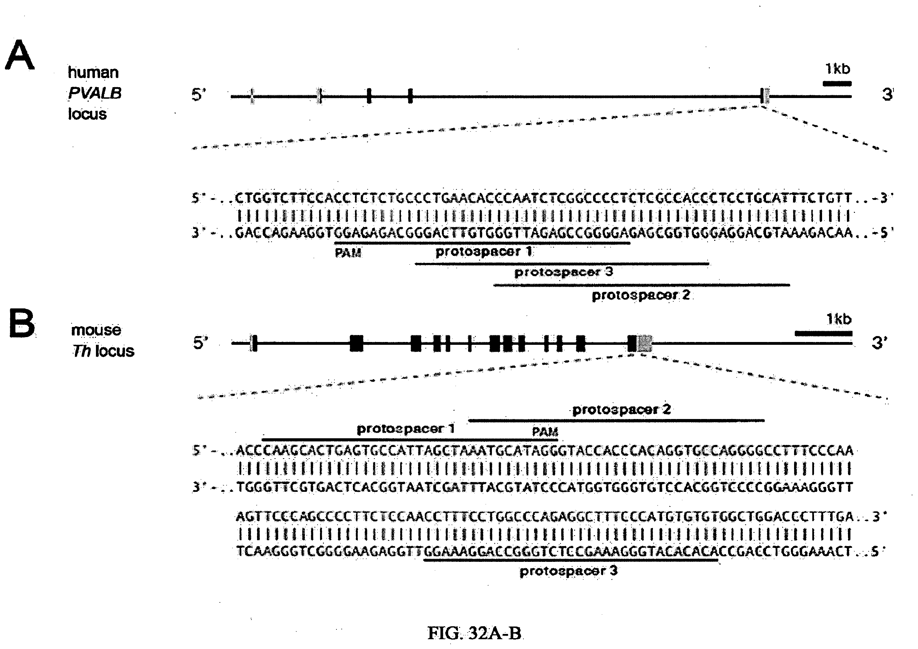

**FIGS. 32A-B** (disclosure) depict a selection of protospacers in the human *PVALB* and mouse *Th* loci. Schematic of the human *PVALB* (**A**) and mouse *Th* (**B**) loci and the location of the three protospacers within the last exon of the *PVALB* and *Th* genes, respectively. The 30bp protospacers are indicated by black lines and the adjacent PAM sequences are indicated by the magenta bar. Protospacers on the sense and anti-sense strands are indicated above and below the DNA sequences respectively.

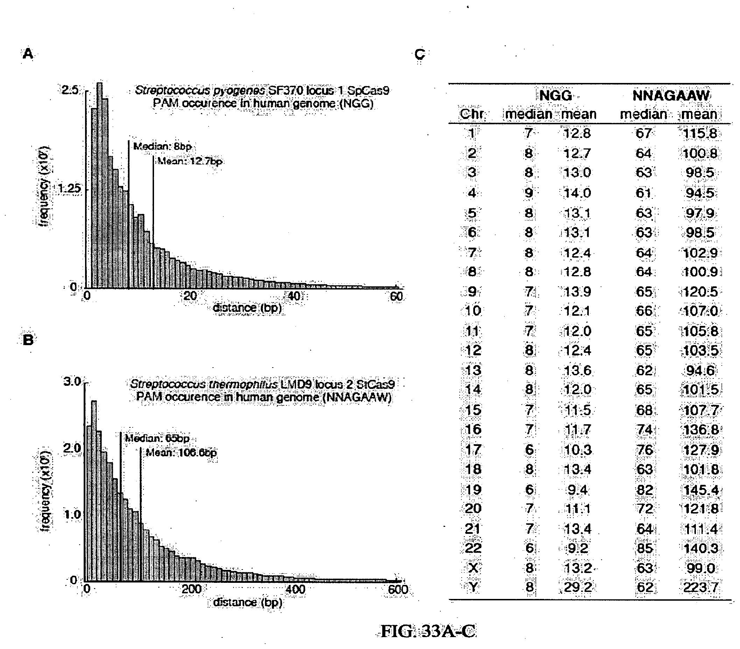

**FIGS. 33A-C** (disclosure) depict occurrences of PAM sequences in the human genome. Histograms of distances between adjacent *Streptococcus pyogenes* SF370 locus 1 PAM (NGG) (**A**) and *Streptococcus thermophiles* LMD9 locus 1 PAM (NNAGAAW) (**B**) in the human genome. (**C**) Distances for each PAM by chromosome. Chr, chromosome. Putative targets were identified using both the plus and minus strands of human chromosomal sequences. Given that there may be chromatin, DNA methylation-, RNA structure, and other factors that may limit the cleavage activity at some protospacer targets, it is important to note that the actual targeting ability might be less than the result of this computational analysis.

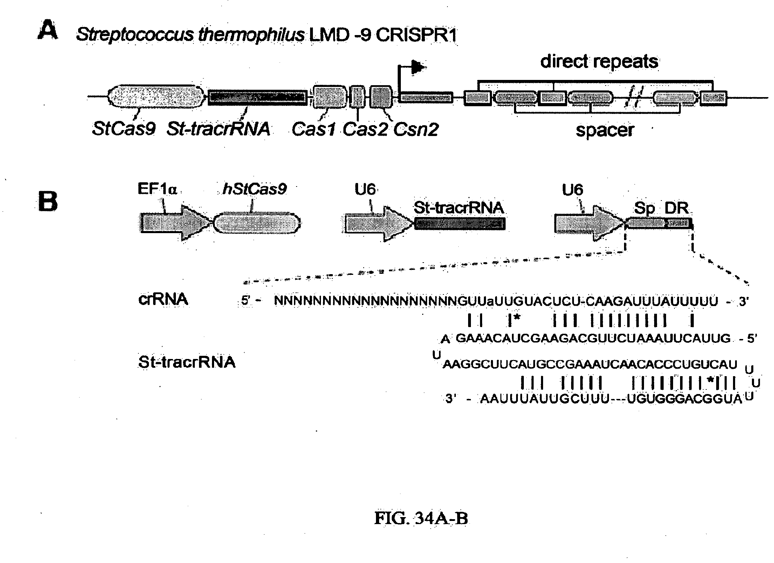

**FIGS. 34A-D** (disclosure) depict type II CRISPR from *Streptococcus thermophilus* LMD-9 can also function in eukaryotic cells. (**A**) Schematic of CRISPR locus 2 from *Streptococcus thermophilus* LMD-9. (**B**) Design of the expression system for the S. *thermphilus* CRISPR system. Human codon-optimized *hStCas9* is expressed using a constitutive EFla promoter. Mature versions of tracrRNA and crRNA are expressed using the U6 promoter to ensure precise transcription initiation. Sequences for the mature crRNA and tracrRNA are shown. A single based indicated by the lower case "a" in the crRNA sequence was used to remove the polyU sequence, which serves as a RNA Pol III transcriptional terminator. (**C**) Schematic showing protospacer and corresponding PAM sequences targets in the human *EMX1* locus. Two protospacer sequences are highlighted and their corresponding PAM sequences satisfying the NNAGAAW motif are indicated by magenta lines. Both protospacers are targeting the anti-sense strand. (**D**) SURVEYOR assay showing StCas9-mediated cleavage in the target locus. RNA guide spacers 1 and 2 induced 14% and 6.4% respectively. Statistical analysis of cleavage activity across biological replica at these two protospacer sites can be found in Table 1.

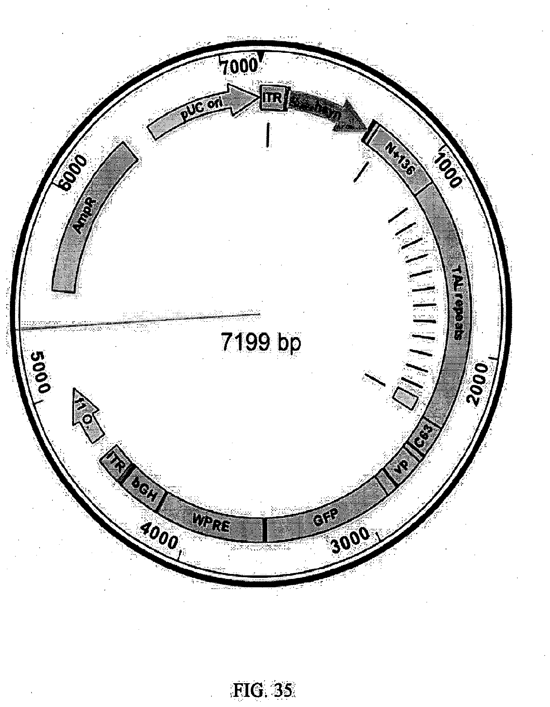

**FIG. 35** depicts an example of an AAV-promoter-TALE-effector construct, where hSyn = human synapsin 1 promoter, N+136 = TALE N-term, AA +136 truncation, C63 = TALE C-term, AA +63 truncation, vp = VP64 effector domain, GFP = green fluorescent protein, WPRE = Woodchuck Hepatitis Virus Posttranscriptional Regulatory Element, bGH = bovine growth hormone polyA, ITR = AAV inverted terminal repeat and AmpR = ampicillin resistance gene.

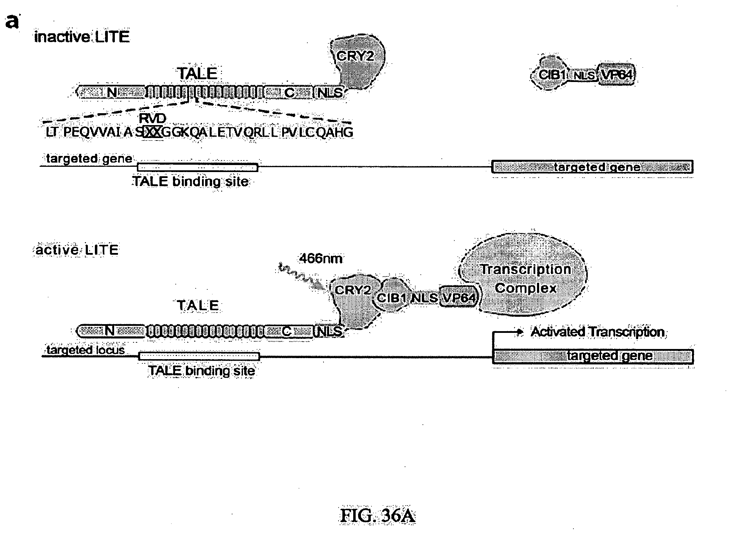

**FIG. 36A-C** depict design and optimization of the LITE system. (**a**) A TALE DNA-binding domain is fused to CRY2 and a transcriptional effector domain is fused to CIB1. In the inactive state, TALE-CRY2 binds the promoter region of the target gene while CIB1-effector remains unbound in the nucleus. The VP64 transcriptional activator is shown above. Upon illumination with blue light, TALE-CRY2 and CIB1-effector rapidly dimerize, recruiting CIB1-effector to the target promoter. The effector in turn modulates transcription of the target gene. (**b**) Light-dependent upregulation of the endogenous target *Neurog2* mRNA with LITEs containing functional truncations of its light-sensitive binding partners. LITE-transfected Neuro-2a cells were stimulated for 24 h with 466 nm light at an intensity of 5mW/cm² and a duty cycle of 7% (1 s pulses at 0.066 Hz). (c) Time course of light-dependent *Neurog2* upregulation by TALE-CRY2PHR and CIB1-VP64 LITEs. LITE-transfected Neuro-2a cells were stimulated with 466 nm light at an intensity of 5 mW/cm² and a duty cycle of 7% (1 s pulses at 0.066 Hz) and decrease of *Neurog2* mRNA levels after 6 h of light stimulation. All *Neurog2* mRNA levels were measured relative to expressing GFP control cells (mean ± s.e.m.; *n* = 3-4) (*, *p* < 0.05; and ***, *p* < 0.001).

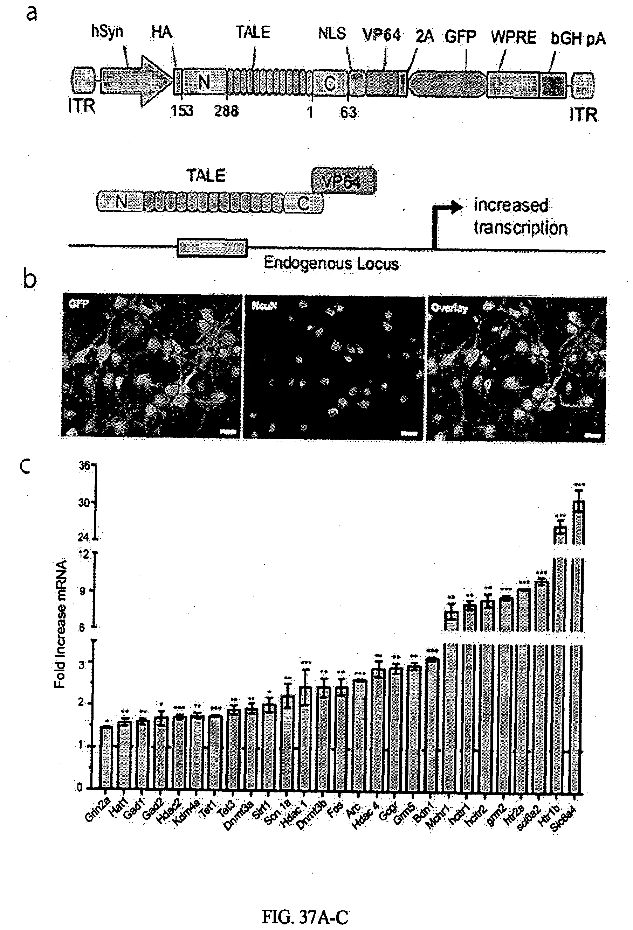

**FIG. 37A-F** depict *in vitro* and *in vivo* AAV-mediated TALE delivery targeting endogenous loci in neurons. (**a**) General schematic of constitutive TALE transcriptional activator packaged into AAV. Effector domain VP64 highlighted. hSyn: human synapsin promoter; 2A: foot-and-mouth disease-derived 2A peptide; WPRE: woodchuck hepatitis post-transcriptional response element; bGH pA: bovine growth hormone poly-A signal, (**b**) Representative images showing transduction with AAV-TALE-VP64 construct from (a) in primary cortical neurons. Cells were stained for GFP and neuronal marker NeuN. Scale bars = 25 µm. (**c**) AAV-TALE-VP64 constructs targeting a variety of endogenous loci were screened for transcriptional activation in primary cortical neurons (*, p < 0.05; **, p < 0.01; ***, p < 0.001). (**d**) Efficient delivery of TALE-VP64 by AAV into the ILC of mice. Scale bar = 100 µm. (Cg1 = cingulate cortex, PLC = prelimbic cortex, ILC = infralimbic cortex). (**e**) Higher magnification image of efficient transduction of neurons in ILC. (**f**) *Grm2* mRNA upregulation by TALE-VP64 *in vivo* in ILC (mean ± s.e.m.; n = 3 animals per condition), measured using a 300µm tissue punch.

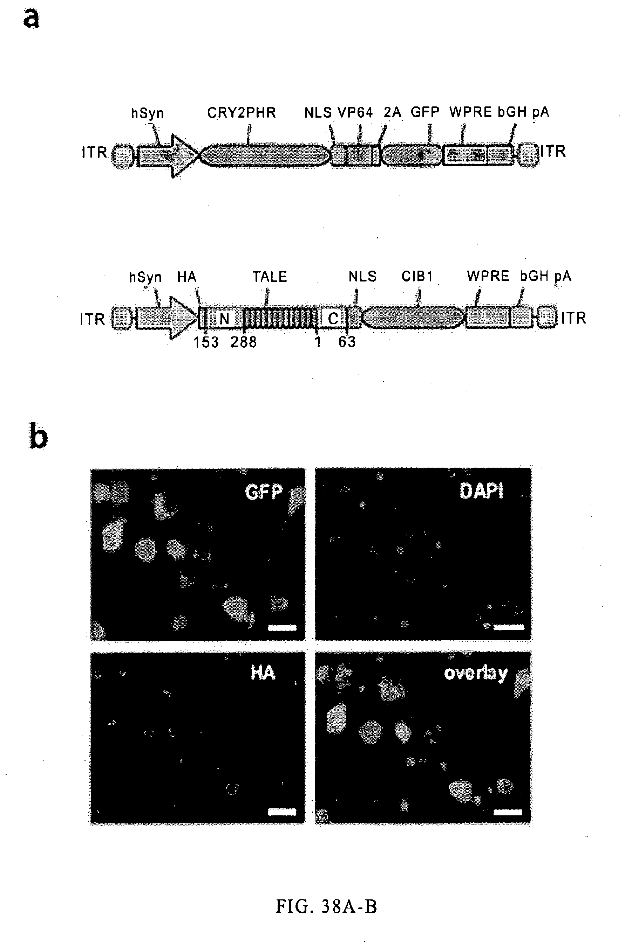

**FIGS. 38A-I** depict LITE-mediated optogenetic modulation of endogenous transcription in primary neurons and *in vivo.* (**a**) AAV-LITE activator construct with switched CRY2PHR and CIB1 architecture, (**b**) Representative images showing co-transduction of AAV-delivered LITE constructs in primary neurons. Cells were stained for GFP, HA-tag, and DAPI. (Scale bars = 25 µm). (**c**) Light-induced activation of *Grm2* expression in primary neurons after 24 h of stimulation with 0.8% duty cycle pulsed 466 nm light (250 ms pulses at 0.033Hz or 500 ms pulses at 0.016Hz; 5mW/cm²). (**d**) Upregulation of *Grm2* mRNA in primary cortical neurons with and without light stimulation at 4 h and 24 h time points. Expression levels are shown relative to neurons transduced with GFP only. (**e**) Quantification of mGluR2 protein levels in GFP only control transductions, unstimulated neurons with LITEs, and light-stimulated neurons with LITEs. A representative western blot is shown with β-tubulin-III as a loading control, (**f**) Schematic showing transduction of ILC with the LITE system, the optical fiber implant, and the 0.35 mm diameter brain punch used for tissue isolation, (**g**) Representative images of ILC co-transduced with both LITE components. Stains are shown for HA-tag (red), GFP (green), and DAPI (blue). (Scale bar = 25 µm). (**h**) Light-induced activation of endogenous *Grm2* expression using LITEs transduced into ILC. **, *p* < 0.05; data generated from 4 different mice for each experimental condition. (**i**) Fold increases and light induction of *Neurog2* expression using LITE1.0 and optimized LITE 2.0. LITE2.0 provides minimal background while maintaining a high level of activation. NLS_{α-importin} and NLS_{SV40}, nuclear localization signal from α-importin and simian virus 40 respectively; GS, Gly-Ser linker; NLS*, mutated NLS where the indicated residues have been substituted with Ala to prevent nuclear localization activity; Δ318-334; deletion of a higher plant helix-loop-helix transcription factor homology region.

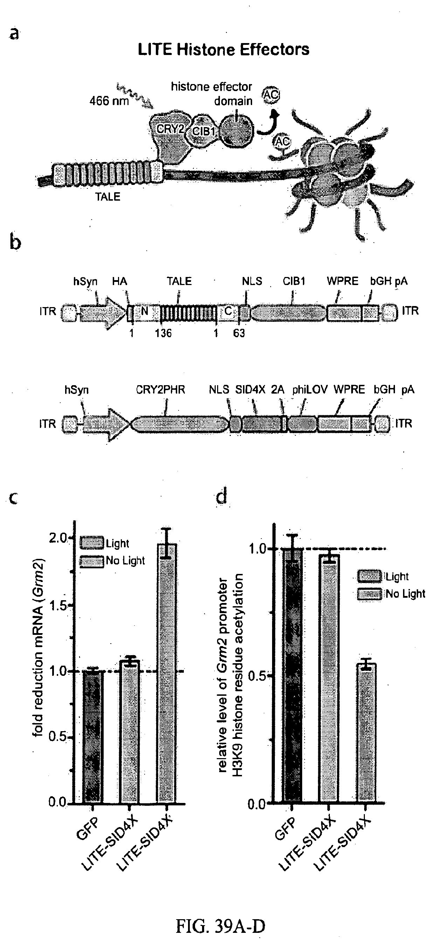

**FIG. 39A-H** depict TALE- and LITE-mediated epigenetic modifications (**a**) Schematic of LITE epigenetic modifiers (epiLITE). (**b**) Schematic of engineered epigenetic transcriptional repressor SID4X within an AAV vector. phiLOV2.1 (330bp) was used as a fluorescent marker rather than GFP (800bp) to ensure efficient AAV packaging. (**c**) epiLITE-mediated repression of endogenous *Grm2* expression in primary cortical neurons with and without light stimulation. Fold down regulation is shown relative to neurons transduced with GFP alone. (**d**) epiLITE-mediated decrease in H3K9 histone residue acetylation at the *Grm2* promoter with and without light-stimulation. (**e**, **f**) Fold reduction of *Grm2* mRNA by epiTALE-methyltransferases (epiTALE-KYP, -TgSET8, and -NUE), and corresponding enrichment of histone methylation marks H3K9mel, H4K20me3, and H3K27me3 at the *Grm2* promoter, (**g**, **h**) Fold reduction of *Grm2* mRNA by epiTALE histone deacetylases (epiTALE-HDAC8, -RPD3, - Sir2a, and -Sin3a), and corresponding decreases in histone residue acetylation marks H4K8Ac and H3K9Ac at the *Grm2* promoter. Values shown in all panels are mean ± s.e.m., *n* = 3-4.

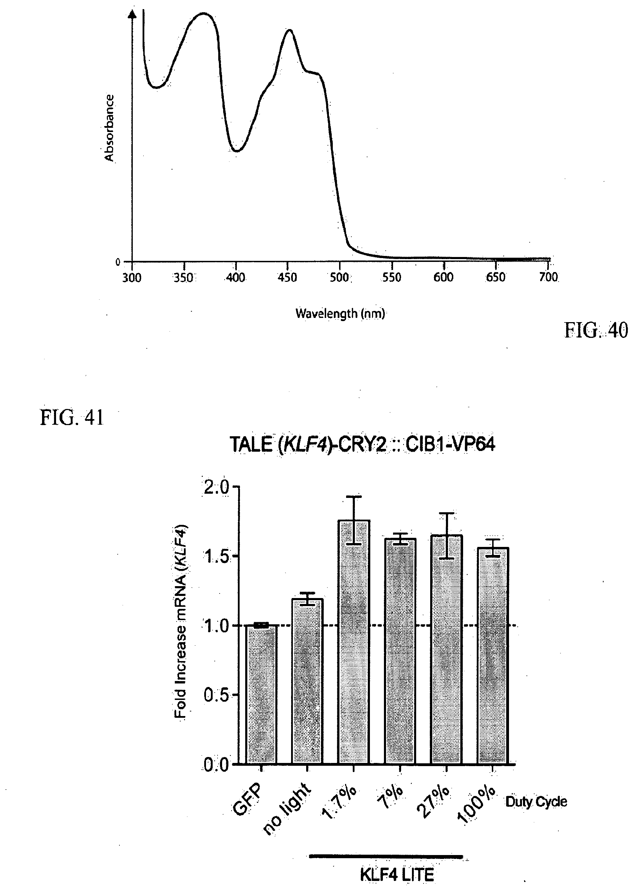

**FIG. 40** depicts an illustration of the absorption spectrum of CRY2 *in vitro.* Cryptochrome 2 was optimally activated by 350-475 nm light¹. A sharp drop in absorption and activation was seen for wavelengths greater than 480 nm. Spectrum was adapted from Banerjee, R. et al. The Signaling State of Arabidopsis Cryptochrome 2 Contains Flavin Semiquinone. Journal of Biological Chemistry 282, 14916-14922, doi:10.1074/jbc.M700616200 (2007).
**FIG. 41** depicts an impact of illumination duty cycle on LITE-mediated gene expression. Varying duty cycles (illumination as percentage of total time) were used to stimulate 293FT cells expressing LITEs targeting the *KLF4* gene, in order to investigate the effect of duty cycle on LITE activity. *KLF4* expression levels were compared to cells expressing GFP only. Stimulation parameters were: 466 nm, 5 mW/cm² for 24 h. Pulses were performed at 0.067 Hz with the following durations: 1.7% = 0.25 s pulse, 7% = 1 s pulse, 27% = 4 s pulse, 100% = constant illumination, (mean ± s.e.m.; n = 3-4).

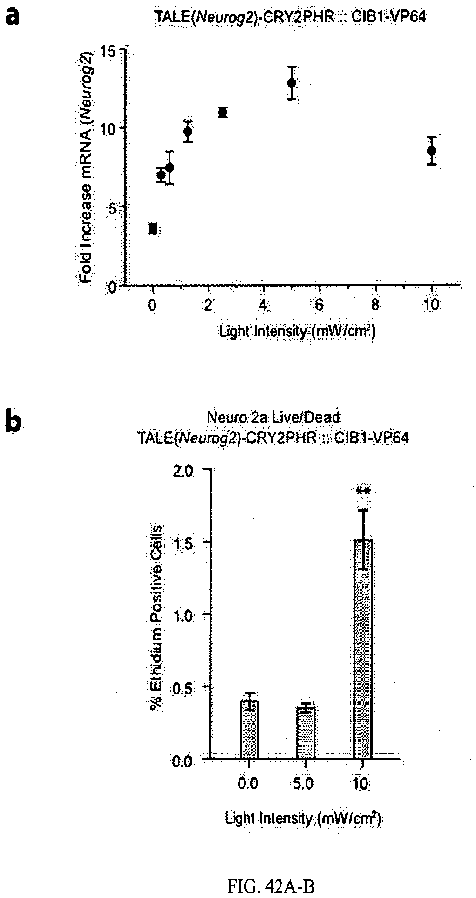

**FIGS. 42A-B** depict an impact of light intensity on LITE-mediated gene expression and cell survival. (**a**) The transcriptional activity of CRY2PHR :: CIB1 LITE was found to vary according to the intensity of 466 nm blue light. Neuro 2a cells were stimulated for 24 h hours at a 7% duty cycle (Is pulses at 0.066Hz) (**b**) Light-induced toxicity measured as the percentage of cells positive for red-fluorescent ethidium homodimer-1 versus calcein-positive cells. All *Neurog2* mRNA levels were measured relative to cells expressing GFP only (mean ± s.e.m.; n = 3-4).

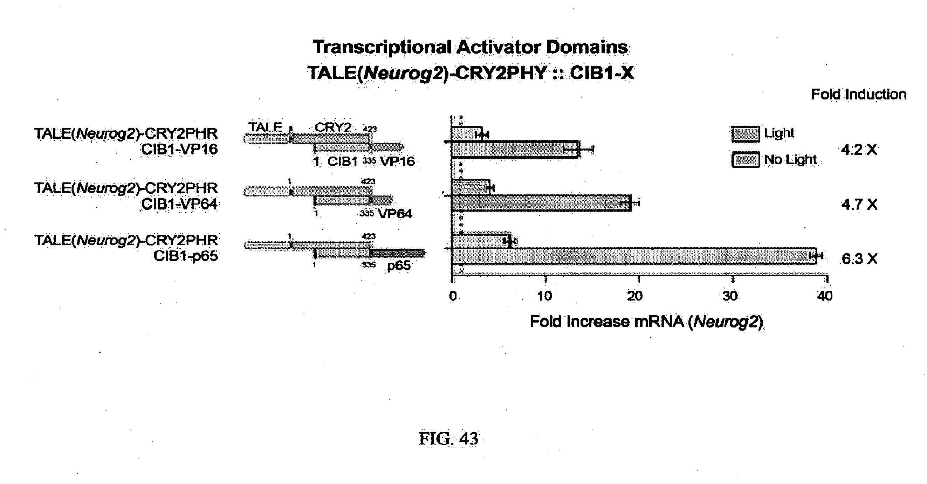

**FIG. 43** depicts an impact of transcriptional activation domains on LITE-mediated gene expression. *Neurog2* up-regulation with and without light by LITEs using different transcriptional activation domains (VP16, VP64, and p65). Neuro-2a cells transfected with LITE were stimulated for 24 h with 466 nm light at an intensity of 5mW/cm² and a duty cycle of 7% (1 s pulses at 0.066 Hz). (mean ± s.e.m.; n = 3-4)

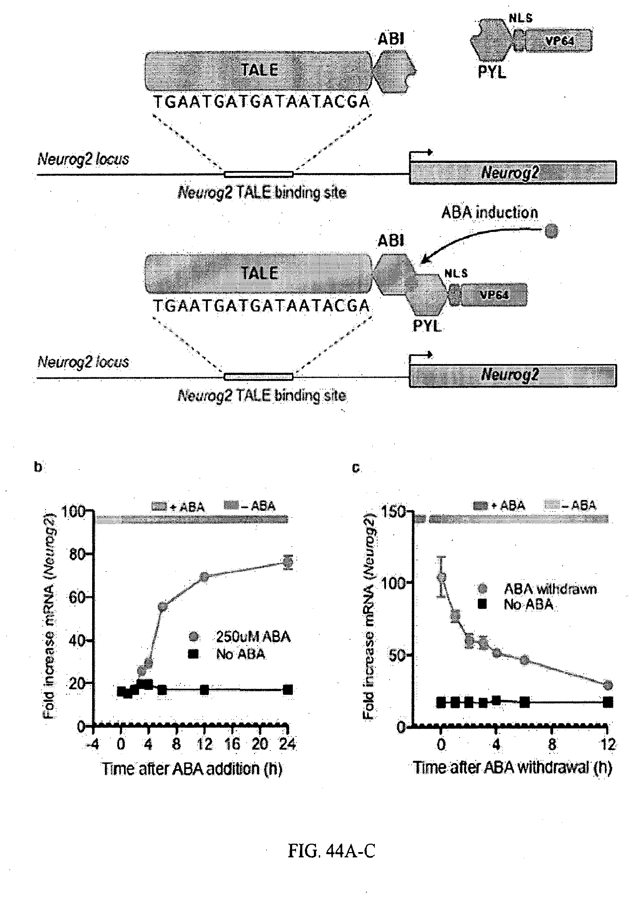

**FIGS. 44A-C** depict chemical induction of endogenous gene transcription. (**a**) Schematic showing the design of a chemical inducible two hybrid TALE system based on the abscisic acid (ABA) receptor system. ABI and PYL dimerize upon the addition of ABA and dissociates when ABA is withdrawn. (**b**) Time course of ABA-dependent *Neurog2* up-regulation. 250 µM of ABA was added to HEK 293FT cells expressing TALE(*Neurog2*)-ABI and PYL-VP64. Fold mRNA increase was measured at the indicated time points after the addition of ABA. (**c**) Decrease of *Neurog2* mRNA levels after 24 h of ABA stimulation. All *Neurog2* mRNA levels were measured relative to expressing GFP control cells (mean ± s.e.m.; *n* = 3-4).

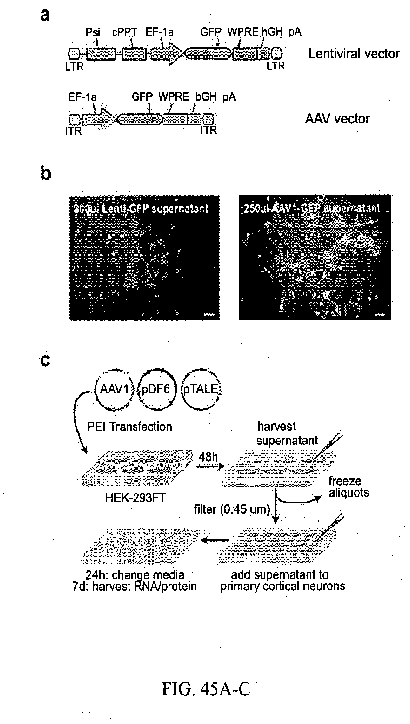

**FIGS. 45A-C** depict **AAV supernatant production.** (**a**) Lentiviral and AAV vectors carrying GFP were used to test transduction efficiency. (**b**) Primary embryonic cortical neurons were transduced with 300 and 250 µL supernatant derived from the same number of AAV or lentivirus-transfected 293FT cells. Representative images of GFP expression were collected at 7 d.p.i. Scale bars = 50 µm. (**c**) The depicted process was developed for the production of AAV supernatant and subsequent transduction of primary neurons. 293FT cells were transfected with an AAV vector carrying the gene of interest, the AAV1 serotype packaging vector (pAAV1), and helper plasmid (pDF6) using PEI. 48 h later, the supernatant was harvested and filtered through a 0.45 µm PVDF membrane. Primary neurons were then transduced with supernatant and remaining aliquots were stored at -80°C. Stable levels of AAV construct expression were reached after 5-6 days. AAV supernatant production following this process can be used for production of up to 96 different viral constructs in 96-well format (employed for TALE screen in neurons shown in **FIG. 37C**).

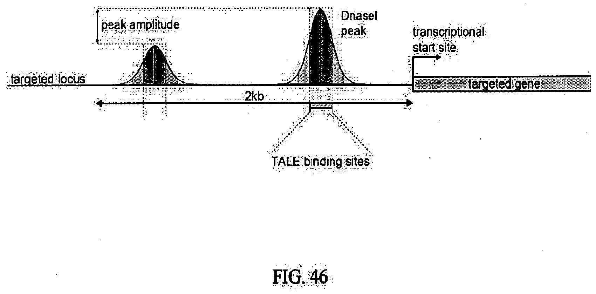

**FIG. 46** depicts selection of TALE target sites guided by DNaseI-sensitive chromatin regions. High DNaseI sensitivity based on mouse cortical tissue data from ENCODE (http://genome.ucsc.edu) was used to identify open chromatin regions. The peak with the highest amplitude within the region 2 kb upstream of the transcriptional start site was selected for targeting. TALE binding targets were then picked within a 200 bp region at the center of the peak.

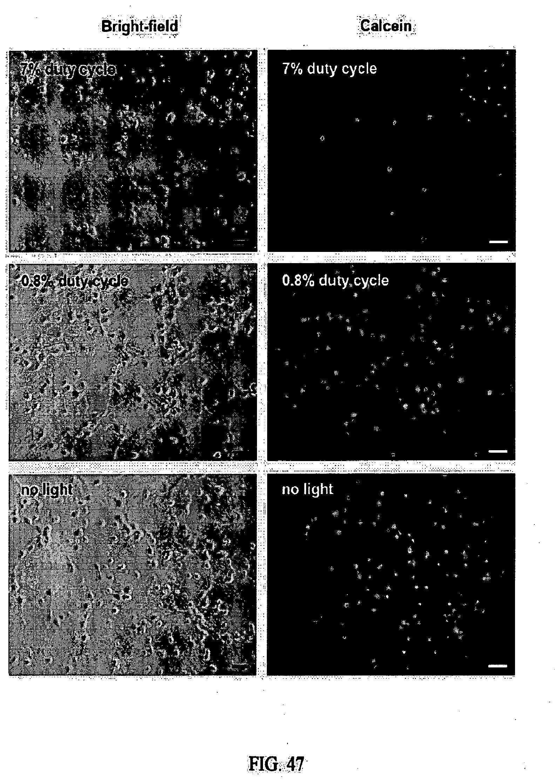

**FIG. 47** depicts an impact of light duty cycle on primary neuron health. The effect of light stimulation on primary cortical neuron health was compared for duty cycles of 7%, 0.8%, and no light conditions. Calcein was used to evaluate neuron viability. Bright-field images were captured to show morphology and cell integrity. Primary cortical neurons were stimulated with the indicated duty cycle for 24 h with 5 mW/cm² of 466 nm light. Representative images, scale bar = 50 µm. Pulses were performed in the following manner: 7% duty cycle = 1 s pulse at 0.067 Hz, 0.8% duty cycle = 0.5 s pulse at 0.0167 Hz.

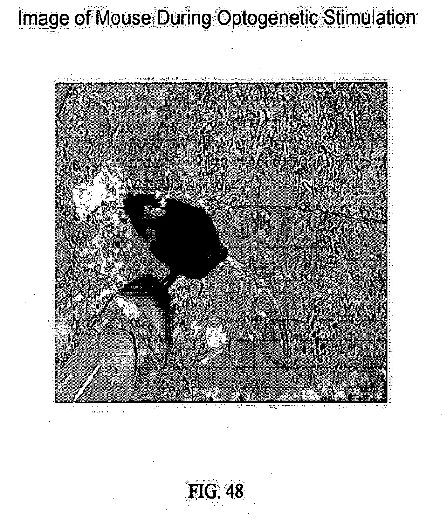

**FIG. 48** depicts an image of a mouse during optogenetic stimulation. An awake, freely behaving, LITE-injected mouse is pictured with a stereotactically implanted cannula and optical fiber.

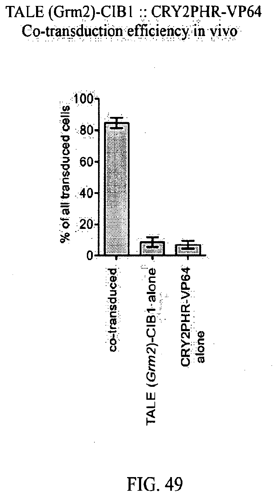

**FIG. 49** depicts co-transduction efficiency of LITE components by AAV1/2 in mouse infralimbic cortex. Cells transduced by TALE(*Grm2*)-CIB1 alone, CRY2PHR-VP64 alone, or co-transduced were calculated as a percentage of all transduced cells.

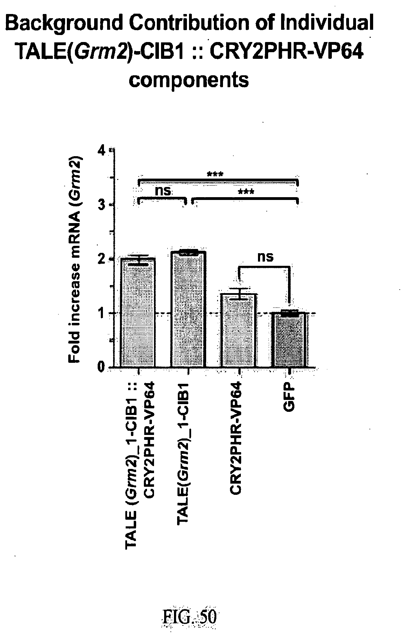

**FIG. 50** depicts a contribution of individual LITE components to baseline transcription modulation. *Grm2* mRNA levels were determined in primary neurons transfected with individual LITE components. Primary neurons expressing *Grm2* TALE_1-CIB1 alone led to a similar increase in *Grm2* mRNA levels as unstimulated cells expressing the complete LITE system. (mean ± s.e.m.; *n* = 3-4).

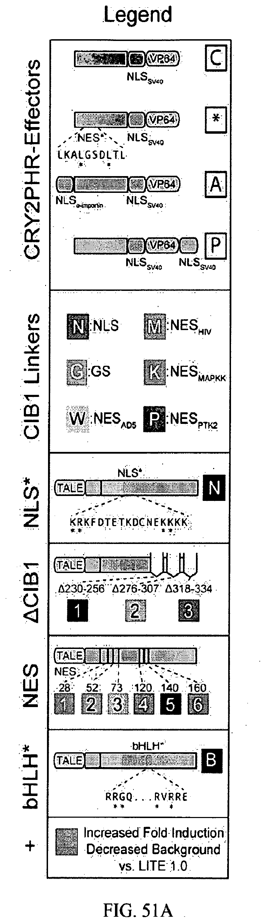

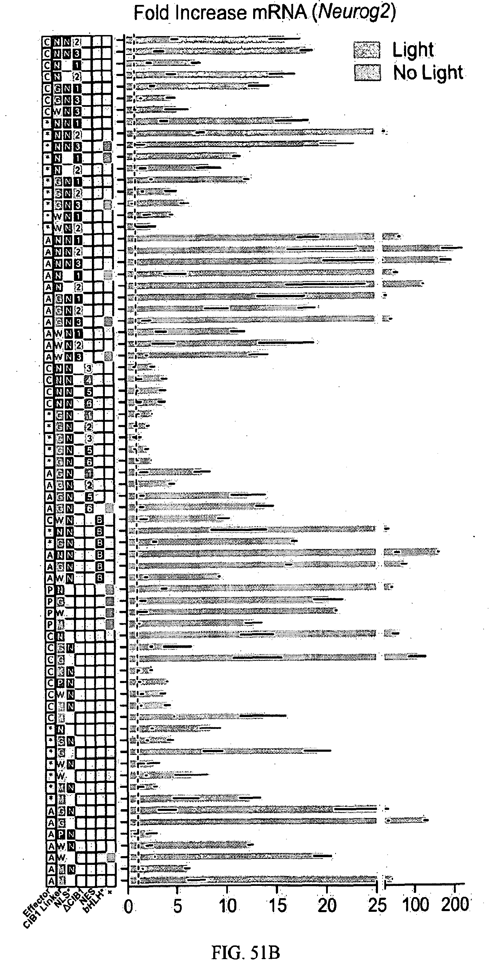

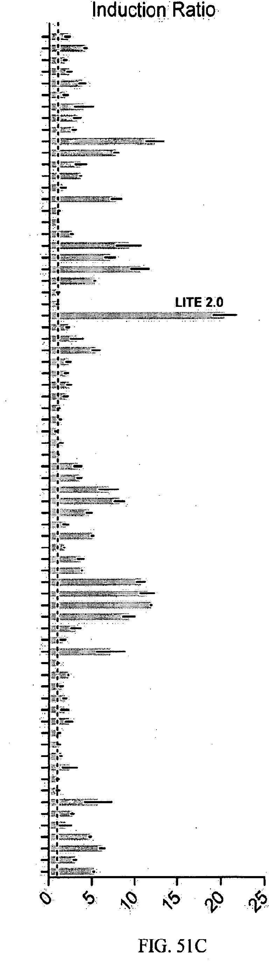

**FIG. 51** depicts effects of LITE Component Engineering on Activation, Background Signal, and Fold Induction. Protein modifications were employed to find LITE components resulting in reduced background transcriptional activation while improving induction ratio by light. Protein alterations are discussed in detail below. In brief, nuclear localization signals and mutations in an endogenous nuclear export signal were used to improve nuclear import of the CRY2PHR-VP64 component. Several variations of CIB1 intended to either reduce nuclear localization or CIB1 transcriptional activation were pursued in order to reduce the contribution of the TALE-CIB1 component to background activity. The results of all combinations of CRY2PHR-VP64 and TALE-CIB1 which were tested are shown above. The table to the left of the bar graphs indicates the particular combination of domains/mutations used for each condition. Each row of the table and bar graphs contains the component details, Light/No light activity, and induction ratio by light for the particular CRY2PHR/CIB1 combination. Combinations that resulted in both decreased background and increased fold induction compared to LITE 1.0 are highlighted in green in the table column marked "+" (t-test p<0.05). **CRY2PHR-VP64 Constructs:** Three new constructs were designed with the goal of improving CRY2PHR-VP64 nuclear import. First, the mutations L70A and L74A within a predicted endogenous nuclear export sequence of CRY2PHR were induced to limit nuclear export of the protein (referred to as '*' in the Effector column). Second, the α-importin nuclear localization sequence was fused to the N-terminus of CRY2PHR-VP64 (referred to as 'A' in the Effector column). Third, the SV40 nuclear localization sequence was fused to the C-terminus of CRY2PHR-VP64 (referred to as 'P' in the Effector column). **TALE-CIB1 Linkers:** The SV40 NLS linker between TALE and CIB1 used in LITE 1.0 was replaced with one of several linkers designed to increase nuclear export of the TALE-CIB1 protein (The symbols used in the CIB1 Linker column are shown in parentheses): a flexible glycine-serine linker (G), an adenovirus type 5 E1B nuclear export sequence (W), an HIV nuclear export sequence (M), a MAPKK nuclear export sequence (K), and a PTK2 nuclear export sequence (P). **NLS*** **Endogenous CIB1 Nuclear Localization Sequence Mutation:** A nuclear localization signal exists within the wild type CIB1 sequence. This signal was mutated in NLS* constructs at K92A, R93A, K105A, and K106A in order to diminish TALE-CIB1 nuclear localization (referred to as 'N' in the NLS* column). Δ**CIB1 Transcription Factor Homology Deletions:** In an effort to eliminate possible basal CIB1 transcriptional activation, deletion constructs were designed in which regions of high homology to basic helix-loop-helix transcription factors in higher plants were removed. These deleted regions consisted of Δaa230-256, Δaa276-307, Δaa308-334 (referred to as '1' '2' and '3' in the ΔCIB1 column). In each case, the deleted region was replaced with a 3 residue GGS link. **NES Insertions into CIB1:** One strategy to facilitate light-dependent nuclear import of TALE-CIB1 was to insert an NES in CIB1 at its dimerization interface with CRY2PHR such that the signal would be concealed upon binding with CRY2PHR. To this end, an NES was inserted at different positions within the known CRY2 interaction domain CIBN (aa 1-170). The positions are as follows (The symbols used in the NES column are shown in parentheses): aa28 (1), aa52 (2), aa73 (3), aa120 (4), aa140 (5), aa160 (6). ***bHLH basic Helix-Loop-Helix Mutation:** To reduce direct CIB1-DNA interactions, several basic residues of the basic helix-loop-helix region in CIB1 were mutated. The following mutations are present in all *bHLH constructs (referred to as 'B' in the *bHLH column of FIG. 51): R175A, G176A, R187A, and R189A.

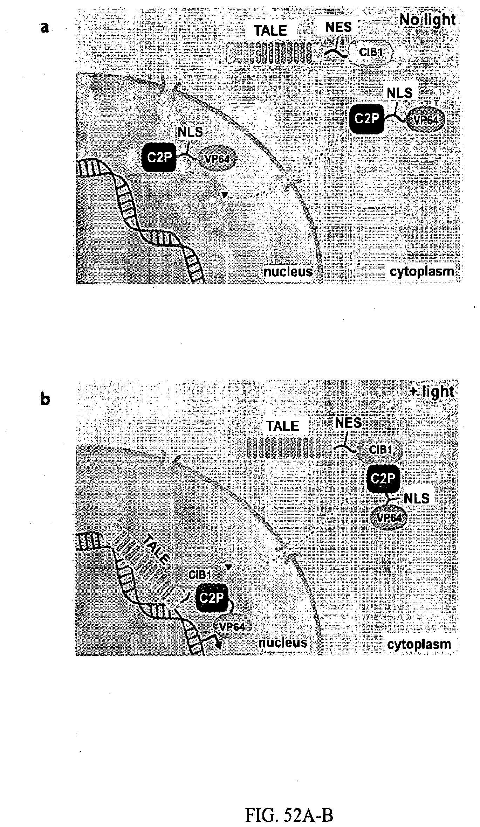

**FIG. 52** depicts an illustration of light mediated co-dependent nuclear import of TALE-CIB1 (a) In the absence of light, the TALE-CIB1 LITE component resides in the cytoplasm due to the absence of a nuclear localization signal, NLS (or the addition of a weak nuclear export signal, NES). The CRY2PHR-VP64 component containing a NLS on the other hand is actively imported into the nucleus on its own. (b) In the presence of blue light, TALE-CIB1 binds to CRY2PHR. The strong NLS present in CRY2PHR-VP64 now mediates nuclear import of the complex of both LITE components, enabling them to activate transcription at the targeted locus.

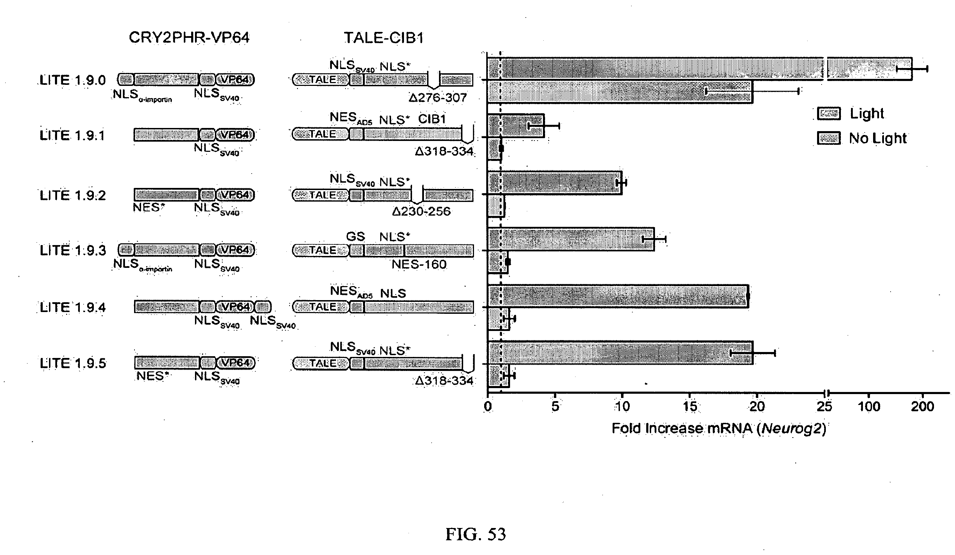

**FIG. 53** depicts notable LITE 1.9 combinations. In addition to the LITE 2.0 constructs, several CRY2PHR-VP64 :: TALE-CIB1 combinations from the engineered LITE component screen were of particular note. LITE 1.9.0, which combined the α-importin NLS effector construct with a mutated endogenous NLS and Δ276-307 TALE-CIB1 construct, exhibited an induction ratio greater than 9 and an absolute light activation of more than 180. LITE 1.9.1, which combined the unmodified CRY2PHR-VP64 with a mutated NLS, Δ318-334, AD5 NES TALE-CIB 1 construct, achieved an induction ratio of 4 with a background activation of 1.06. A selection of other LITE 1.9 combinations with background activations lower than 2 and induction ratios ranging from 7 to 12 were also highlighted.

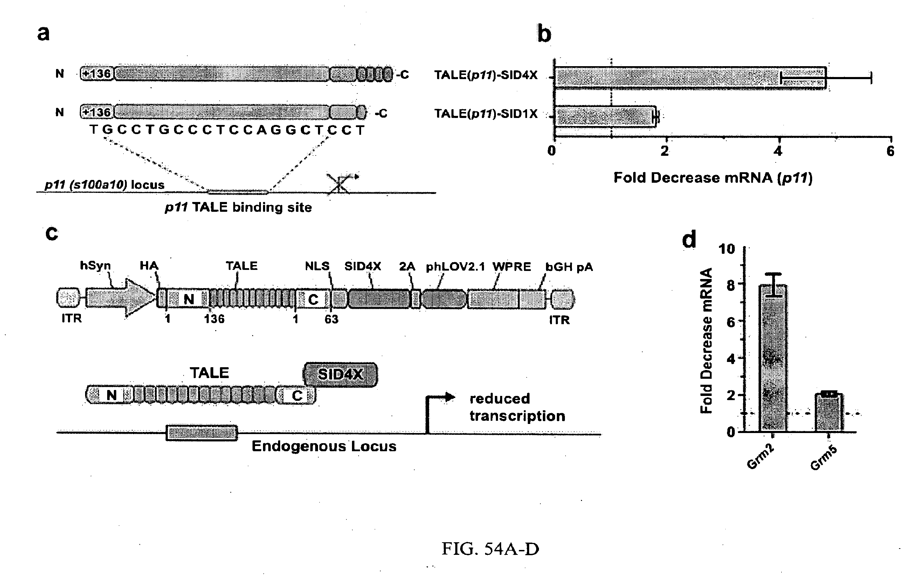

**FIGS. 54A-D** depict TALE SID4X repressor characterization and application in neurons, a) A synthetic repressor was constructed by concatenating 4 SID domains (SID4X). To identify the optimal TALE-repressor architecture, SID or SID4X was fused to a TALE designed to target the mouse *p11* gene. (**b**) Fold decrease in *p11* mRNA was assayed using qRT-PCR. (c) General schematic of constitutive TALE transcriptional repressor packaged into AAV. Effector domain SID4X is highlighted. hSyn: human synapsin promoter; 2A: foot-and-mouth disease-derived 2A peptide; WPRE: woodchuck hepatitis post-transcriptional response element; bGH pA: bovine growth hormone poly-A signal. phiLOV2.1 (330bp) was chosen as a shorter fluorescent marker to ensure efficient AAV packaging, (**d**) 2 TALEs targeting the endogenous mouse loci *Grm5,* and *Grm2* were fused to SID4X and virally transduced into primary neurons. The target gene down-regulation via SID4X is shown for each TALE relative to levels in neurons expressing GFP only. (mean ± s.e.m.; *n* = 3-4).

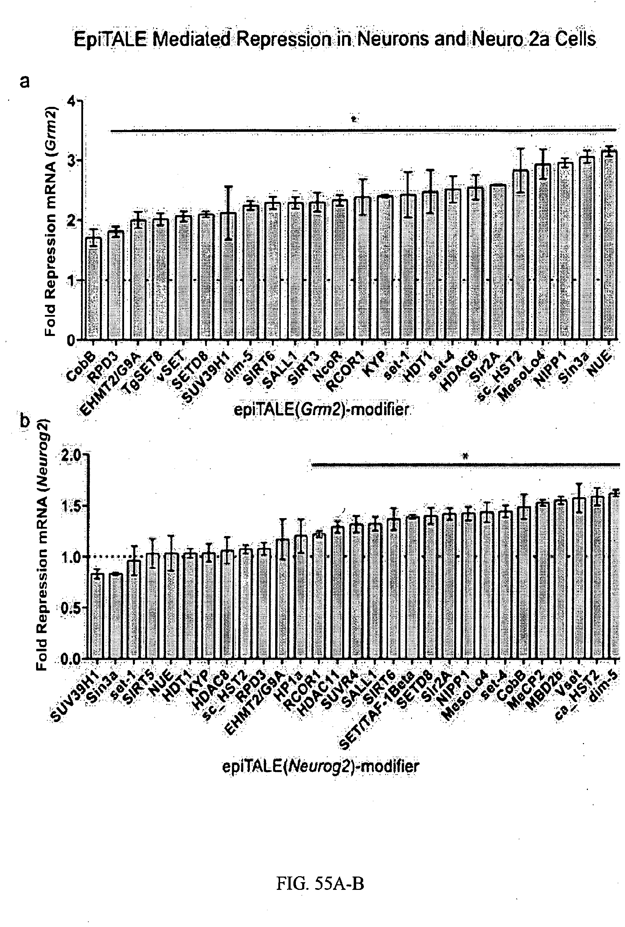

**FIGS. 55A-B** depict a diverse set of epiTALEs mediate transcriptional repression in neurons and Neuro2a cells **a**) A total of 24 *Grm2* targeting TALEs fused to different histone effector domains were transduced into primary cortical mouse neurons using AAV. *Grm2* mRNA levels were measured using RT-qPCR relative to neurons transduced with GFP only. * denotes repression with p < 0.05. **b**) A total of 32 epiTALEs were transfected into Neuro2A cells. 20 of them mediated significant repression of the targeted Neurog2 locus (* = p<0.05).

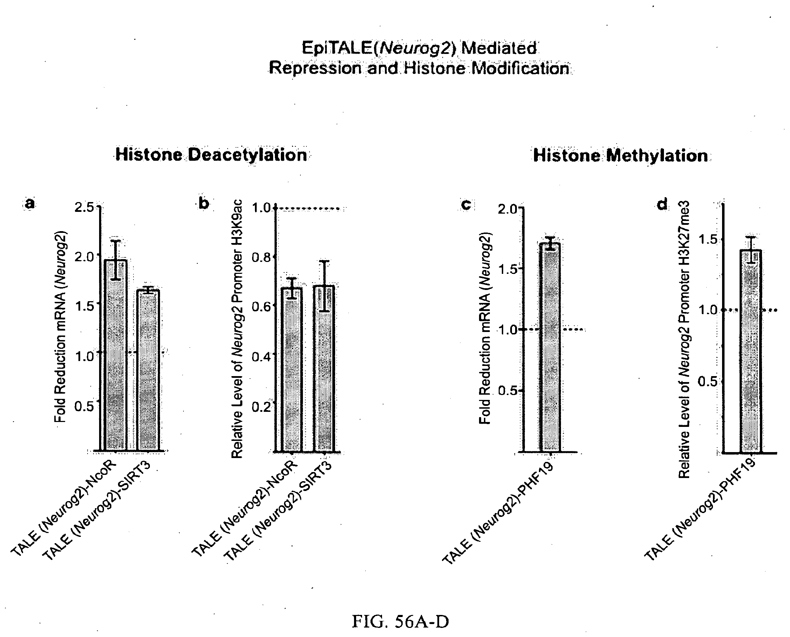

**FIGS. 56A-D** depict epiTALEs mediating transcriptional repression along with histone modifications in Neuro 2A cells (**a**) TALEs fused to histone deacetylating epigenetic effectors NcoR and SIRT3 targeting the murine *Neurog2* locus in Neuro 2A cells were assayed for repressive activity on *Neurog2* transcript levels, (**b**) ChIP RT-qPCR showing a reduction in H3K9 acetylation at the *Neurog2* promoter for NcoR and SIRT3 epiTALEs. (**c**) The epigenetic effector PHF19 with known histone methyltransferase binding activity was fused to a TALE targeting *Neurog2* mediated repression of *Neurog2* mRNA levels. (**d**) ChIP RT-qPCR showing an increase in H3K27me3 levels at the *Neurog2* promoter for the PHF19 epiTALE.

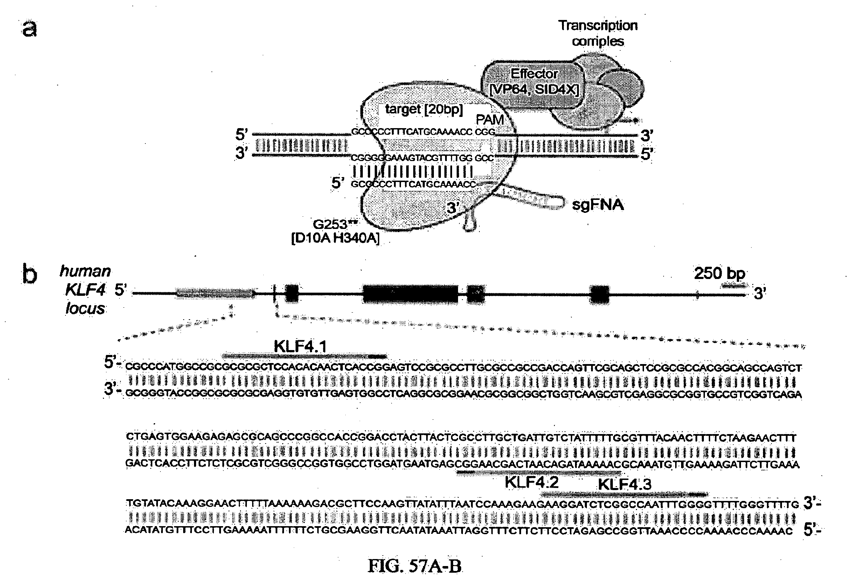

**FIGS. 57A-G** (disclosure) depict RNA-guided DNA binding protein Cas9 can be used to target transcription effector domains to specific genomic loci. (**a**) The RNA-guided nuclease Cas9 from the type II *Streptococcus pyogenes* CRISPR/Cas system can be converted into a nucleolytically-inactive RNA-guided DNA binding protein (Cas9**) by introducing two alanine substitutions (D10A and H840A). Schematic showing that a synthetic guide RNA (sgRNA) can direct Cas9**-effector fusion to a specific locus in the human genome. The sgRNA contains a 20bp guide sequence at the 5' end which specifies the target sequence. On the target genomic DNA, the 20bp target site needs to be followed by a 5'-NGG PAM motif. (**b**, **c**) Schematics showing the sgRNA target sites in the human *KLF4* and *SOX2* loci respectively. Each target site is indicated by the blue bar and the corresponding PAM sequence is indicated by the magenta bar. (**d**, **e**) Schematics of the Cas9**-VP64 transcription activator and SID4X-Cas9** transcription repressor constructs. (**f**, **g**) Cas9**-VP64 and SID4X-Cas9** mediated activation of *KLF4* and repression of *SOX2* respectively. All mRNA levels were measured relative to GFP mock transfected control cells (mean ± s.e.m.; *n* = 3).

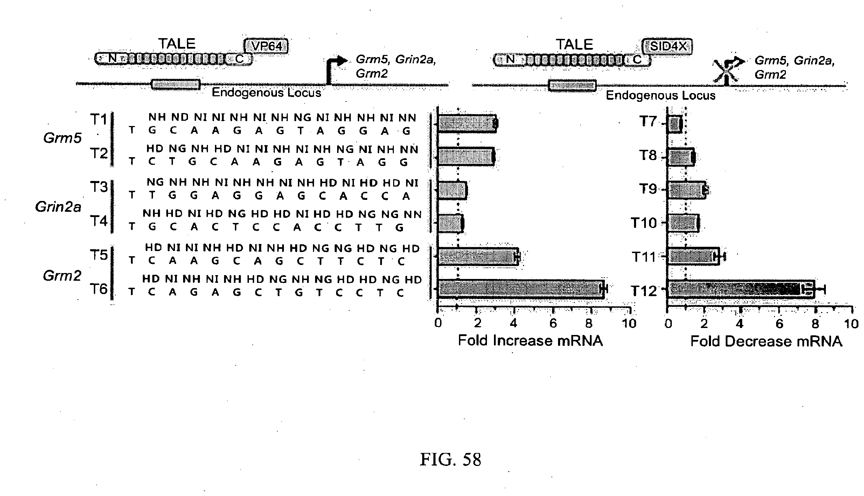

**FIG. 58** depicts 6 TALEs which were designed, with two TALEs targeting each of the endogenous mouse loci *Grm5*, *Grin2a,* and *Grm2.* TALEs were fused to the transcriptional activator domain VP64 or the repressor domain SID4X and virally transduced into primary neurons. Both the target gene upregulation via VP64 and downregulation via SID4X are shown for each TALE relative to levels in neurons expressing GFP only.

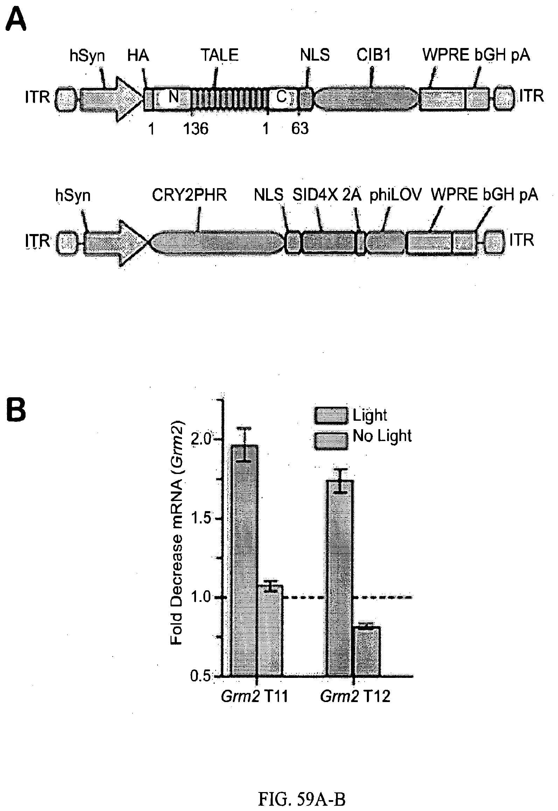

**FIGS. 59A-B** depict (**A**) LITE repressor construct highlighting SID4X repressor domain. (**B**) Light-induced repression of endogenous *Grm2* expression in primary cortical neurons using *Grm2* T1-LITE and *Grm2* T2-LITE. Fold downregulation is shown relative to neurons transduced with GFP only (mean ± s.e.m.; n = 3-4 for all subpanels).

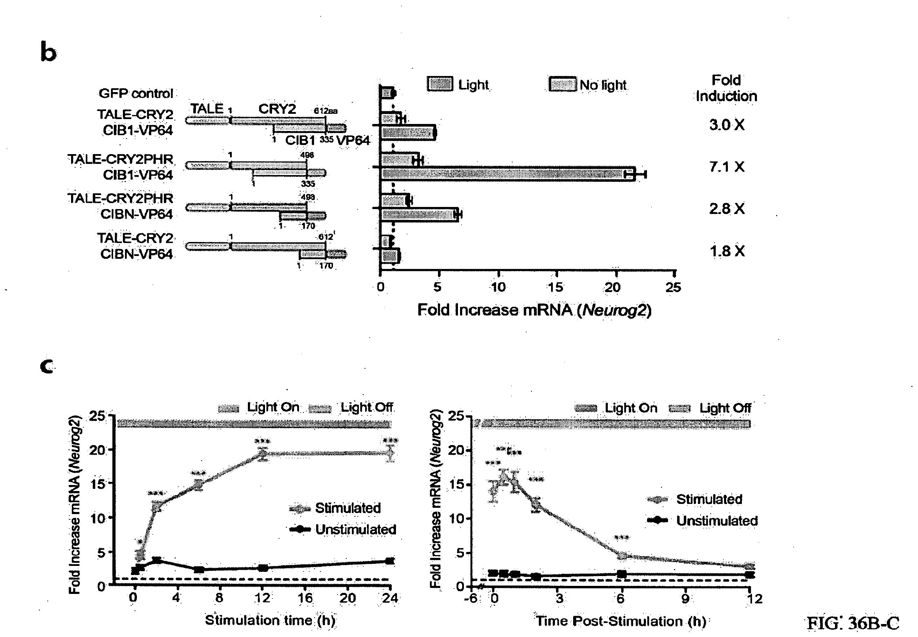

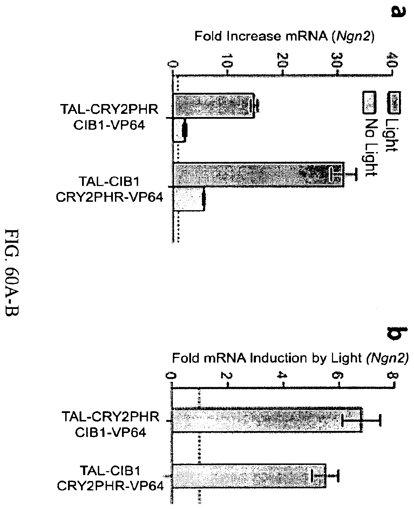

**FIGS. 60A-B** depict exchanging CRY2PHR and CIB1 components. (A) TALE-CIB1::CRY2PHR-VP64 was able to activate *Ngn2* at higher levels than TALE-CRY2PHR::CIB1-VP64. (**B**) Fold activation ratios (light versus no light) ratios of *Ngn2* LITEs show similar efficiency for both designs. Stimulation parameters were the same as those used in FIG. 36B.

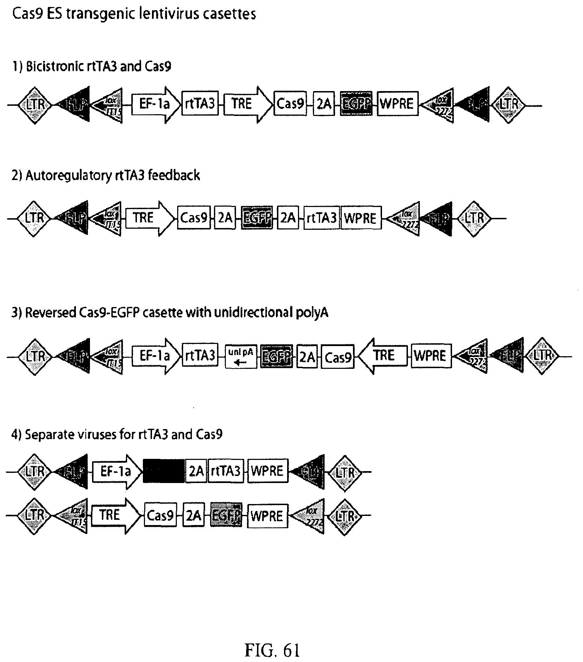

**FIG. 61** (disclosure) depicts Tet Cas9 vector designs for inducible Cas9.

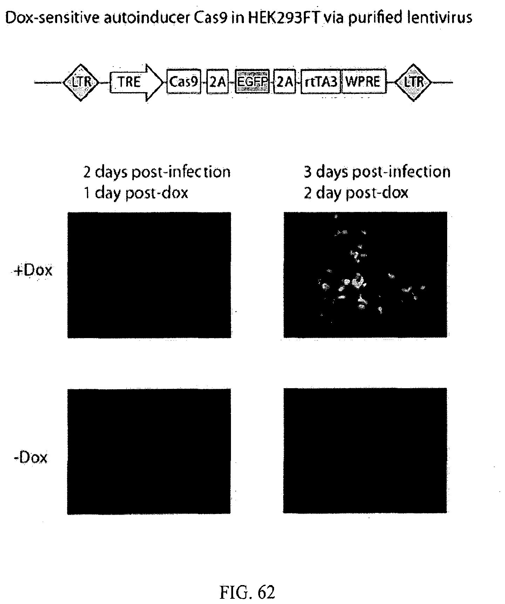

**FIG. 62** (disclosure) depicts a vector and EGFP expression in 293FT cells after Doxycycline induction of Cas9 and EGFP.

### DETAILED DESCRIPTION OF THE INVENTION AND OF THE DISCLOSURE

The term "nucleic acid" or "nucleic acid sequence" refers to a deoxyribonucleic or ribonucleic oligonucleotide in either single- or double-stranded form. The term encompasses nucleic acids, *i.e.,* oligonucleotides, containing known analogues of natural nucleotides. The term also encompasses nucleic-acid-like structures with synthetic backbones, see, e.g., Eckstein, 1991; Baserga et al., 1992; Milligan, 1993; WO 97/03211; WO 96/39154; Mata, 1997; Strauss-Soukup, 1997; and Samstag, 1996.

As used herein, "recombinant" refers to a polynucleotide synthesized or otherwise manipulated *in vitro* (e.g., "recombinant polynucleotide"), to methods of using recombinant polynucleotides to produce gene products in cells or other biological systems, or to a polypeptide ("recombinant protein") encoded by a recombinant polynucleotide. "Recombinant means" encompasses the ligation of nucleic acids having various coding regions or domains or promoter sequences from different sources into an expression cassette or vector for expression of, e.g., inducible or constitutive expression of polypeptide coding sequences in the vectors of invention.

The term "heterologous" when used with reference to a nucleic acid, indicates that the nucleic acid is in a cell or a virus where it is not normally found in nature; or, comprises two or more subsequences that are not found in the same relationship to each other as normally found in nature, or is recombinantly engineered so that its level of expression, or physical relationship to other nucleic acids or other molecules in a cell, or structure, is not normally found in nature. A similar term used in this context is "exogenous". For instance, a heterologous nucleic acid is typically recombinantly produced, having two or more sequences from unrelated genes arranged in a manner not found in nature; e.g., a human gene operably linked to a promoter sequence inserted into an adenovirus-based vector of the invention. As an example, a heterologous nucleic acid of interest may encode an immunogenic gene product, wherein the adenovirus is administered therapeutically or prophylactically as a carrier or drug-vaccine composition. Heterologous sequences may comprise various combinations of promoters and sequences, examples of which are described in detail herein.

A "therapeutic ligand" may be a substance which may bind to a receptor of a target cell with therapeutic effects.

A "therapeutic effect" may be a consequence of a medical treatment of any kind, the results of which are judged by one of skill in the field to be desirable and beneficial. The "therapeutic effect" may be a behavioral or physiologic change which occurs as a response to the medical treatment. The result may be expected, unexpected, or even an unintended consequence of the medical treatment. A "therapeutic effect" may include, for example, a reduction of symptoms in a subject suffering from infection by a pathogen.

A "target cell" may be a cell in which an alteration in its activity may induce a desired result or response. As used herein, a cell may be an in vitro cell. The cell may be an isolated cell which may not be capable of developing into a complete organism.

A "ligand" may be any substance that binds to and forms a complex with a biomolecule to serve a biological purpose. As used herein, "ligand" may also refer to an "antigen" or "immunogen". As used herein "antigen" and "immunogen" are used interchangeably.

"Expression" of a gene or nucleic acid encompasses not only cellular gene expression, but also the transcription and translation of nucleic acid(s) in cloning systems and in any other context.

As used herein, a "vector" is a tool that allows or facilitates the transfer of an entity from one environment to another. By way of example, some vectors used in recombinant DNA techniques allow entities, such as a segment of DNA (such as a heterologous DNA segment, such as a heterologous cDNA segment), to be transferred into a target cell. The present invention comprehends recombinant vectors that may include viral vectors, bacterial vectors, protozoan vectors, DNA vectors, or recombinants thereof.

With respect to exogenous DNA for expression in a vector (e.g., encoding an epitope of interest and/or an antigen and/or a therapeutic) and documents providing such exogenous DNA, as well as with respect to the expression of transcription and/or translation factors for enhancing expression of nucleic acid molecules, and as to terms such as "epitope of interest", "therapeutic", "immune response", "immunological response", "protective immune response", "immunological composition", "immunogenic composition", and "vaccine composition", *inter alia,* reference is made to U.S. Patent No. 5,990,091 issued November 23, 1999, and WO 98/00166 and WO 99/60164, and the documents cited therein and the documents of record in the prosecution of that patent and those PCT applications. Thus, U.S. Patent No. 5,990,091 and WO 98/00166 and WO 99/60164 and documents cited therein and documents of record in the prosecution of that patent and those PCT applications, and other documents cited herein, may be consulted in the practice of this invention; and, all exogenous nucleic acid molecules, promoters, and vectors cited therein may be used in the practice of this invention. In this regard, mention is also made of U.S. Patents Nos. 6,706,693; 6,716,823; 6,348,450; U.S. Patent Application Serial Nos. 10/424,409; 10/052,323; 10/116,963; 10/346,021; and WO 99/08713, published February 25, 1999, from PCT/US98/16739.

Aspects of the invention or the disclosure comprehend the TALE (invention) and CRISPR-Cas (disclosure) systems of the invention being delivered into an organism or a cell or to a locus of interest via a delivery system. One means of delivery is via a vector, wherein the vector is a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided. In some embodiments, one or more of the viral or plasmid vectors may be delivered via nanoparticles, exosomes, microvesciles, or a gene-gun.

As used herein, the terms "drug composition" and "drug", "vaccinal composition", "vaccine", "vaccine composition", "therapeutic composition" and "therapeutic-immunologic composition" cover any composition that induces protection against an antigen or pathogen. In some embodiments, the protection may be due to an inhibition or prevention of infection by a pathogen. In other embodiments, the protection may be induced by an immune response against the antigen(s) of interest, or which efficaciously protects against the antigen; for instance, after administration or injection into the subject, elicits a protective immune response against the targeted antigen or immunogen or provides efficacious protection against the antigen or immunogen expressed from the inventive adenovirus vectors of the invention. The term "pharmaceutical composition" means any composition that is delivered to a subject. In some embodiments, the composition may be delivered to inhibit or prevent infection by a pathogen.

A "therapeutically effective amount" is an amount or concentration of the recombinant vector encoding the gene of interest, that, when administered to a subject, produces a therapeutic response or an immune response to the gene product of interest.

The term "viral vector" as used herein includes but is not limited to retroviruses, adenoviruses, adeno-associated viruses, alphaviruses, and herpes simplex virus.

The present disclosure comprehends spatiotemporal control of endogenous or exogenous gene expression using a form of energy. The form of energy may include but is not limited to electromagnetic radiation, sound energy, chemical energy and thermal energy. In the invention, the form of energy is electromagnetic radiation, preferably, light energy. Previous approaches to control expression of endogenous genes, such as transcription activators linked to DNA binding zinc finger proteins provided no mechanism for temporal or spatial control. The capacity for photoactivation of the system described herein allows the induction of gene expression modulation to begin at a precise time within a localized population of cells.

Aspects of control as detailed in this application relate to at least one or more switch(es). The term "switch" as used herein refers to a system or a set of components that act in a coordinated manner to affect a change, encompassing all aspects of biological function such as activation, repression, enhancement or termination of that function. In one aspect the term switch encompasses genetic switches which comprise the basic components of gene regulatory proteins and the specific DNA sequences that these proteins recognize. In one aspect, switches relate to inducible and repressible systems used in gene regulation. In general, an inducible system may be off unless there is the presence of some molecule (called an inducer) that allows for gene expression. The molecule is said to "induce expression". The manner by which this happens is dependent on the control mechanisms as well as differences in cell type. A repressible system is on except in the presence of some molecule (called a corepressor) that suppresses gene expression. The molecule is said to "repress expression". The manner by which this happens is dependent on the control mechanisms as well as differences in cell type. The term "inducible" as used herein may encompass all aspects of a switch irrespective of the molecular mechanism involved. Accordingly a switch as comprehended by the disclosure may include but is not limited to antibiotic based inducible systems, electromagnetic energy based inducible systems, small molecule based inducible systems, nuclear receptor based inducible systems and hormone based inducible systems. In preferred embodiments the switch may be a tetracycline (Tet)/DOX inducible system, a light inducible systems, a Abscisic acid (ABA) inducible system, a cumate repressor/operator system, a 4OHT/estrogen inducible system, an ecdysone-based inducible systems or a FKBP12/FRAP (FKBP12-rapamycin complex) inducible system.

In one aspect of the disclosure at least one switch may be associated with a TALE or CRISPR-Cas system wherein the activity of the TALE or CRISPR-Cas system is controlled by contact with at least one inducer energy source as to the switch. The term "contact" as used herein for aspects of the invention refers to any associative relationship between the switch and the inducer energy source, which may be a physical interaction with a component (as in molecules or proteins which bind together) or being in the path or being struck by energy emitted by the energy source (as in the case of absorption or reflection of light, heat or sound). In some aspects of the invention the contact of the switch with the inducer energy source is brought about by application of the inducer energy source. The disclosure also comprehends contact via passive feedback systems. This includes but is not limited to any passive regulation mechanism by which the TALE or CRISPR-Cas system activity is controlled by contact with an inducer energy source that is already present and hence does not need to be applied. For example this energy source may be a molecule or protein already existent in the cell or in the cellular environment. Interactions which bring about contact passively may include but are not limited to receptor/ligand binding, receptor/chemical ligand binding, receptor/protein binding, antibody/protein binding, protein dimerization, protein heterodimerization, protein multimerization, nuclear receptor/ligand binding, post-translational modifications such as phosphorylation, dephosphorylation, ubiquitination or deubiquitination.

Two key molecular tools were leveraged in the design of the photoresponsive transcription activator-like (TAL) effector system. First, the DNA binding specificity of engineered TAL effectors is utilized to localize the complex to a particular region in the genome. Second, light-induced protein dimerization is used to attract an activating or repressing domain to the region specified by the TAL effector, resulting in modulation of the downstream gene.

Inducible effectors are contemplated for *in vitro* or *in vivo* application in which temporally or spatially specific gene expression control is desired. *In vitro* examples: temporally precise induction/suppression of developmental genes to elucidate the timing of developmental cues, spatially controlled induction of cell fate reprogramming factors for the generation of cell-type patterned tissues. *In vivo* examples: combined temporal and spatial control of gene expression within specific brain regions.

In a preferred embodiment of the invention, the inducible effector is a Light Inducible Transcriptional Effector (LITE). The modularity of the LITE system allows for any number of effector domains to be employed for transcriptional modulation. In a particularly advantageous embodiment, transcription activator like effector (TALE) and the activation domain VP64 are utilized in the present invention.

LITEs are designed to modulate or alter expression of individual endogenous genes in a temporally and spatially precise manner. Each LITE may comprise a two component system consisting of a customized DNA-binding transcription activator like effector (TALE) protein, a light-responsive cryptochrome heterodimer from Arabadopsis thaliana, and a transcriptional activation/repression domain. The TALE is designed to bind to the promoter sequence of the gene of interest. The TALE protein is fused to one half of the cryptochrome heterodimer (cryptochrome-2 or CIB1), while the remaining cryptochrome partner is fused to a transcriptional effector domain. Effector domains may be either activators, such as VP16, VP64, or p65, or repressors, such as KRAB, EnR, or SID. In a LITE's unstimulated state, the TALE-cryptochrome2 protein localizes to the promoter of the gene of interest, but is not bound to the CIB1-effector protein. Upon stimulation of a LITE with blue spectrum light, cryptochrome-2 becomes activated, undergoes a conformational change, and reveals its binding domain. CIB1, in turn, binds to cryptochrome-2 resulting in localization of the effector domain to the promoter region of the gene of interest and initiating gene overexpression or silencing.

Activator and repressor domains may selected on the basis of species, strength, mechanism, duration, size, or any number of other parameters. Preferred effector domains include, but are not limited to, a transposase domain, integrase domain, recombinase domain, resolvase domain, invertase domain, protease domain, DNA methyltransferase domain, DNA demethylase domain, histone acetylase domain, histone deacetylases domain, nuclease domain, repressor domain, activator domain, nuclear-localization signal domains, transcription-protein recruiting domain, cellular uptake activity associated domain, nucleic acid binding domain or antibody presentation domain.

Gene targeting in a LITE or in any other inducible effector may be achieved via the specificity of customized TALE DNA binding proteins. A target sequence in the promoter region of the gene of interest is selected and a TALE customized to this sequence is designed. The central portion of the TALE consists of tandem repeats 34 amino acids in length. Although the sequences of these repeats are nearly identical, the 12th and 13th amino acids (termed repeat variable diresidues) of each repeat vary, determining the nucleotide-binding specificity of each repeat. Thus, by synthesizing a construct with the appropriate ordering of TALE monomer repeats, a DNA binding protein specific to the target promoter sequence is created.

In advantageous embodiments of the invention, the methods provided herein use isolated, non-naturally occurring, recombinant or engineered DNA binding proteins that comprise TALE monomers or TALE monomers or half monomers as a part of their organizational structure that enable the targeting of nucleic acid sequences with improved efficiency and expanded specificity.

Naturally occurring TALEs or "wild type TALEs" are nucleic acid binding proteins secreted by numerous species of proteobacteria. TALE polypeptides contain a nucleic acid binding domain composed of tandem repeats of highly conserved monomer polypeptides that are predominantly 33, 34 or 35 amino acids in length and that differ from each other mainly in amino acid positions 12 and 13. In advantageous embodiments the nucleic acid is DNA. As used herein, the term "polypeptide monomers", "TALE monomers" or "monomers" will be used to refer to the highly conserved repetitive polypeptide sequences within the TALE nucleic acid binding domain and the term "repeat variable di-residues" or "RVD" will be used to refer to the highly variable amino acids at positions 12 and 13 of the polypeptide monomers. A general representation of a TALE monomer which is comprised within the DNA binding domain is X_{1- 11}-(X₁₂X₁₃)-X_{14-33 or 34 or 35}, where the subscript indicates the amino acid position and X represents any amino acid. X₁₂X₁₃ indicate the RVDs. In some polypeptide monomers, the variable amino acid at position 13 is missing or absent and in such monomers, the RVD consists of a single amino acid. In such cases the RVD may be alternatively represented as X*, where X represents X₁₂ and (*) indicates that X₁₃ is absent. The DNA binding domain comprises several repeats of TALE monomers and this may be represented as (X₁₋₁₁-(X₁₂X₁₃)-X_{14-33 or 34 or 35})_{z}, where in an advantageous embodiment, z is at least 5 to 40. In a further advantageous embodiment, z is at least 10 to 26.

The TALE monomers have a nucleotide binding affinity that is determined by the identity of the amino acids in its RVD. For example, polypeptide monomers with an RVD of NI preferentially bind to adenine (A), monomers with an RVD of NG preferentially bind to thymine (T), monomers with an RVD of HD preferentially bind to cytosine (C) and monomers with an RVD of NN preferentially bind to both adenine (A) and guanine (G). In yet another embodiment of the invention, monomers with an RVD of IG preferentially bind to T. Thus, the number and order of the polypeptide monomer repeats in the nucleic acid binding domain of a TALE determines its nucleic acid target specificity. In still further embodiments of the invention, monomers with an RVD of NS recognize all four base pairs and may bind to A, T, G or C. The structure and function of TALEs is further described in, for example, Moscou et al., Science 326:1501 (2009); Boch et al., Science 326:1509-1512 (2009); and Zhang et al., Nature Biotechnology 29:149-153 (2011).

The polypeptides used in methods of the invention are isolated, non-naturally occurring, recombinant or engineered nucleic acid-binding proteins that have nucleic acid or DNA binding regions containing polypeptide monomer repeats that are designed to target specific nucleic acid sequences.

As described herein, polypeptide monomers having an RVD of HN or NH preferentially bind to guanine and thereby allow the generation of TALE polypeptides with high binding specificity for guanine containing target nucleic acid sequences. In a preferred embodiment of the invention, polypeptide monomers having RVDs RN, NN, NK, SN, NH, KN, HN, NQ, HH, RG, KH, RH and SS preferentially bind to guanine. In a much more advantageous embodiment of the invention, polypeptide monomers having RVDs RN, NK, NQ, HH, KH, RH, SS and SN preferentially bind to guanine and thereby allow the generation of TALE polypeptides with high binding specificity for guanine containing target nucleic acid sequences. In an even more advantageous embodiment of the invention, polypeptide monomers having RVDs HH, KH, NH, NK, NQ, RH, RN and SS preferentially bind to guanine and thereby allow the generation of TALE polypeptides with high binding specificity for guanine containing target nucleic acid sequences. In a further advantageous embodiment, the RVDs that have high binding specificity for guanine are RN, NH RH and KH. Furthermore, polypeptide monomers having an RVD of NV preferentially bind to adenine and guanine. In more preferred embodiments of the invention, monomers having RVDs of H*, HA, KA, N*, NA, NC, NS, RA, and S* bind to adenine, guanine, cytosine and thymine with comparable affinity.

In even more advantageous embodiments of the invention the RVDs that have a specificity for adenine are NI, RI, KI, HI, and SI. In more preferred embodiments of the invention, the RVDs that have a specificity for adenine are HN, SI and RI, most preferably the RVD for adenine specificity is SI. In even more preferred embodiments of the invention the RVDs that have a specificity for thymine are NG, HG, RG and KG. In further advantageous embodiments of the invention, the RVDs that have a specificity for thymine are KG, HG and RG, most preferably the RVD for thymine specificity is KG or RG. In even more preferred embodiments of the invention the RVDs that have a specificity for cytosine are HD, ND, KD, RD, HH, YG and SD. In a further advantageous embodiment of the invention, the RVDs that have a specificity for cytosine are SD and RD. Refer to **FIG. 7B** for representative RVDs and the nucleotides they target to be incorporated into the most preferred embodiments of the invention. In a further advantageous embodiment the variant TALE monomers may comprise any of the RVDs that exhibit specificity for a nucleotide as depicted in **FIG. 7A****.** All such TALE monomers allow for the generation of degenerative TALE polypeptides able to bind to a repertoire of related, but not identical, target nucleic acid sequences. In still further embodiments of the invention, the RVD NT may bind to G and A. In yet further embodiments of the invention, the RVD NP may bind to A, T and C. In more advantageous embodiments of the invention, at least one selected RVD may be NI, HD, NG, NN, KN, RN, NH, NQ, SS, SN, NK, KH, RH, HH, KI, HI, RI, SI, KG, HG, RG, SD, ND, KD, RD, YG, HN, NV, NS, HA, S*, N*, KA, H*, RA, NA or NC.

The predetermined N-terminal to C-terminal order of the one or more polypeptide monomers of the nucleic acid or DNA binding domain determines the corresponding predetermined target nucleic acid sequence to which the polypeptides of the invention will bind. As used herein the monomers and at least one or more half monomers are "specifically ordered to target" the genomic locus or gene of interest. In plant genomes, the natural TALE-binding sites always begin with a thymine (T), which may be specified by a cryptic signal within the non-repetitive N-terminus of the TALE polypeptide; in some cases this region may be referred to as repeat 0. In animal genomes, TALE binding sites do not necessarily have to begin with a thymine (T) and polypeptides of the invention may target DNA sequences that begin with T, A, G or C. The tandem repeat of TALE monomers always ends with a half-length repeat or a stretch of sequence that may share identity with only the first 20 amino acids of a repetitive full length TALE monomer and this half repeat may be referred to as a half-monomer (**FIG. 8**). Therefore, it follows that the length of the nucleic acid or DNA being targeted is equal to the number of full monomers plus two.

For example, nucleic acid binding domains may be engineered to contain 5, 6, 7, 8, 9, 10, 11, 12, 13, 14, 15, 16, 17, 18, 19, 20, 21, 22, 23, 24, 25, or more polypeptide monomers arranged in a N-terminal to C-terminal direction to bind to a predetermined 5, 6, 7, 8, 9, 10, 11, 12, 13, 14, 15, 16, 17, 18, 19, 20, 21, 22, 23, 24, 25 nucleotide length nucleic acid sequence. In more advantageous embodiments of the invention, nucleic acid binding domains may be engineered to contain 5, 6, 7, 8, 9, 10, 11, 12, 13, 14, 15, 16, 17, 18, 19, 20, 21, 22, 23, 24, 25, 26 or more full length polypeptide monomers that are specifically ordered or arranged to target nucleic acid sequences of length 7, 8, 9, 10, 11, 12, 13, 14, 15, 16, 17, 18, 19, 20, 21, 22, 23, 24, 25, 26, 27 and 28 nucleotides, respectively. In certain embodiments the polypeptide monomers are contiguous. In some embodiments, half-monomers may be used in the place of one or more monomers, particularly if they are present at the C-terminus of the TALE polypeptide.

Polypeptide monomers are generally 33, 34 or 35 amino acids in length. With the exception of the RVD, the amino acid sequences of polypeptide monomers are highly conserved or as described herein, the amino acids in a polypeptide monomer, with the exception of the RVD, exhibit patterns that effect TALE activity, the identification of which may be used in preferred embodiments of the invention. Representative combinations of amino acids in the monomer sequence, excluding the RVD, are shown by the Applicants to have an effect on TALE activity (**FIG. 10**). In more preferred embodiments of the invention, when the DNA binding domain comprises (X₁₋₁₁-X₁₂X₁₃-X_{14-33 or 34 or 35})_{z}, wherein X₁₋₁₁ is a chain of 11 contiguous amino acids, wherein X₁₂X₁₃ is a repeat variable diresidue (RVD), wherein X_{14-33 or 34 or 35} is a chain of 21, 22 or 23 contiguous amino acids, wherein z is at least 5 to 26, then the preferred combinations of amino acids are [LTLD] or [LTLA] or [LTQV] at X₁₋₄, or [EQHG] or [RDHG] at positions X₃₀₋₃₃ or X₃₁₋₃₄ or X₃₂₋₃₅. Furthermore, other amino acid combinations of interest in the monomers are [LTPD] at X₁₋₄ and [NQALE] at X₁₆₋₂₀ and [DHG] at X₃₂₋₃₄ when the monomer is 34 amino acids in length. When the monomer is 33 or 35 amino acids long, then the corresponding shift occurs in the positions of the contiguous amino acids [NQALE] and [DHG]; preferably, embodiments of the invention may have [NQALE] at X₁₅₋₁₉ or X₁₇₋₂₁ and [DHG] at X₃₁₋₃₃ or X₃₃₋₃₅.

In still further embodiments of the invention, amino acid combinations of interest in the monomers, are [LTPD] at X₁₋₄ and [KRALE] at X₁₆₋₂₀ and [AHG] at X₃₂₋₃₄ or [LTPE] at X₁₋₄ and [KRALE] at X₁₆₋₂₀ and [DHG] at X₃₂₋₃₄ when the monomer is 34 amino acids in length. When the monomer is 33 or 35 amino acids long, then the corresponding shift occurs in the positions of the contiguous amino acids [KRALE], [AHG] and [DHG]. In preferred embodiments, the positions of the contiguous amino acids may be ([LTPD] at X₁₋₄ and [KRALE] at X₁₅₋₁₉ and [AHG] at X₃₁₋₃₃) or ([LTPE] at X₁₄ and [KRALE] at X₁₅₋₁₉ and [DHG] at X₃₁₋₃₃) or ([LTPD] at X₁₋₄ and [KRALE] at X₁₇₋₂₁ and [AHG] at X₃₃₋₃₅) or ([LTPE] at X₁₋₄ and [KRALE] at X₁₇₋₂₁ and [DHG] at X₃₃₋₃₅). In still further embodiments of the invention, contiguous amino acids [NGKQALE] are present at positions X₁₄₋₂₀ or X₁₃₋₁₉ or X₁₅₋₂₁. These representative positions put forward various embodiments of the invention and provide guidance to identify additional amino acids of interest or combinations of amino acids of interest in all the TALE monomers described herein (**FIGs .9A-F** and **10**).

Provided below are exemplary amino acid sequences of conserved portions of polypeptide monomers. The position of the RVD in each sequence is represented by XX or by X* (wherein (*) indicates that the RVD is a single amino acid and residue 13 (X₁₃) is absent).

L T P A Q V V A I A S X X G G K Q A L E T V Q R L L P V L C Q D H G

L T P A Q V V A I A S X * G G K Q A L E T V Q R L L P V L C Q D H G

L T P D Q V V A I A N X X G G K Q A L A T V Q R L L P V L C Q D H G

L T P D Q V V A I A N X X G G K Q A L E T L Q R L L P V L C Q D H G

L T P D Q V V A I A N X X G G K Q A L E T V Q R L L P V L C Q D H G

L T P D Q V V A I A S X X G G K Q A L A T V Q R L L P V L C Q D H G

L T P D Q V V A I A S X X G G K Q A L E T V Q R L L P V L C Q D H G

L T P D Q V V A I A S X X G G K Q A L E T V Q R V L P V L C Q D H G

L T P E Q V V A I A S X X G G K Q A L E T V Q R L L P V L C Q A H G

L T P Y Q V V A I A S X X G S K Q A L E T V Q R L L P V L C Q D H G

L T R E Q V V A I A S X X G G K Q A L E T V Q R L L P V L C Q D H G

L S T A Q V V A I A S X X G G K Q A L E G I G E Q L L K L R T A P Y G

L S T A Q V V A V A S X X G G K P A L E A V R A Q L L A L R A A P Y G

A further listing of TALE monomers excluding the RVDs which may be denoted in a sequence (X₁₋₁₁-X₁₄₋₃₄ or X₁₋₁₁-X₁₄₋₃₅), wherein X is any amino acid and the subscript is the amino acid position is provided in **FIG. 9A-F**. The frequency with which each monomer occurs is also indicated.

As described in Zhang et al., Nature Biotechnology 29:149-153 (2011), TALE polypeptide binding efficiency may be increased by including amino acid sequences from the "capping regions" that are directly N-terminal or C-terminal of the DNA binding region of naturally occurring TALEs into the engineered TALEs at positions N-terminal or C-terminal of the engineered TALE DNA binding region. Thus, in certain embodiments, the TALE polypeptides described herein further comprise an N-terminal capping region and/or a C-terminal capping region.

An exemplary amino acid sequence of a N-terminal capping region is:

M D P I R S R T P S P A R E L L S G P Q P D G V Q P T A D R G V S P

P A G G P L D G L P A R R T M S R T R L P S P P A P S P A F S A D S

F S D L L R Q F D P S L F N T S L F D S L P P F G A H H T E A A T G

E W D E V Q S G L R A A D A P P P T M R V A V T A A R P P R A K P A

P R R R A A Q P S D A S P A A Q V D L R T L G Y S Q Q Q Q E K I K P

K V R S T V A Q H H E A L V G H G F T H A H I V A L S Q H P A A L G

T V A V K Y Q D M I A A L P E A T H E A I V G V G K Q W S G A R A L

E A L L T V A G E L R G P P L Q L D T G Q L L K I A K R G G V T A V

E A V H A W R N A L T G A P L N

An exemplary amino acid sequence of a C-terminal capping region is:

R P A L E S I V A Q L S R P D P A L A A L T N D H L V A L A C L G

G R P A L D A V K K G L P H A P A L I K R T N R R I P E R T S H R

V A D H A Q V V R V L G F F Q C H S H P A Q A F D D A M T Q F G M

S R H G L L Q L F R R V G V T E L E A R S G T L P P A S Q R W D R

I L Q A S G M K R A K P S P T S T Q T P D Q A S L H A F A D S L E

R D L D A P S P M H E G D Q T R A S

As used herein the predetermined "N-terminus" to "C terminus" orientation of the N-terminal capping region, the DNA binding domain comprising the repeat TALE monomers and the C-terminal capping region provide structural basis for the organization of different domains in the d-TALEs or polypeptides of the invention.

The entire N-terminal and/or C-terminal capping regions are not necessary to enhance the binding activity of the DNA binding region. Therefore, in certain embodiments, fragments of the N-terminal and/or C-terminal capping regions are included in the TALE polypeptides described herein.

In certain embodiments, the TALE polypeptides described herein contain a N-terminal capping region fragment that included at least 10, 20, 30, 40, 50, 54, 60, 70, 80, 87, 90, 94, 100, 102, 110, 117, 120, 130, 140, 147, 150, 160, 170, 180, 190, 200, 210, 220, 230, 240, 250, 260 or 270 amino acids of an N-terminal capping region. In certain embodiments, the N-terminal capping region fragment amino acids are of the C-terminus (the DNA-binding region proximal end) of an N-terminal capping region. As described in Zhang et al., Nature Biotechnology 29:149-153 (2011), N-terminal capping region fragments that include the C-terminal 240 amino acids enhance binding activity equal to the full length capping region, while fragments that include the C-terminal 147 amino acids retain greater than 80% of the efficacy of the full length capping region, and fragments that include the C-terminal 117 amino acids retain greater than 50% of the activity of the full-length capping region.

In some embodiments, the TALE polypeptides described herein contain a C-terminal capping region fragment that included at least 6, 10, 20, 30, 37, 40, 50, 60, 68, 70, 80, 90, 100, 110, 120, 127, 130, 140, 150, 155, 160, 170, 180 amino acids of a C-terminal capping region. In certain embodiments, the C-terminal capping region fragment amino acids are of the N-terminus (the DNA-binding region proximal end) of a C-terminal capping region. As described in Zhang et al., Nature Biotechnology 29:149-153 (2011), C-terminal capping region fragments that include the C-terminal 68 amino acids enhance binding activity equal to the full length capping region, while fragments that include the C-terminal 20 amino acids retain greater than 50% of the efficacy of the full length capping region.

In certain embodiments, the capping regions of the TALE polypeptides described herein do not need to have identical sequences to the capping region sequences provided herein. Thus, in some embodiments, the capping region of the TALE polypeptides described herein have sequences that are at least 50%, 60%, 70%, 80%, 85%, 90%, 91%, 92%, 93%, 94%, 95%, 96%, 97%, 98% or 99% identical or share identity to the capping region amino acid sequences provided herein. Sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. In some preferred embodiments, the capping region of the TALE polypeptides described herein have sequences that are at least 95% dentical or share identity to the capping region amino acid sequences provided herein.

Sequence homologies may be generated by any of a number of computer programs known in the art, which include but are not limited to BLAST or FASTA. Suitable computer program for carrying out alignments like the GCG Wisconsin Bestfit package may also be used. Once the software has produced an optimal alignment, it is possible to calculate % homology, preferably % sequence identity. The software typically does this as part of the sequence comparison and generates a numerical result.

In advantageous embodiments described herein, the TALE polypeptides of the invention include a nucleic acid binding domain linked to the one or more effector domains. The terms "effector domain" or "regulatory and functional domain" refer to a polypeptide sequence that has an activity other than binding to the nucleic acid sequence recognized by the nucleic acid binding domain. By combining a nucleic acid binding domain with one or more effector domains, the polypeptides of the invention may be used to target the one or more functions or activities mediated by the effector domain to a particular target DNA sequence to which the nucleic acid binding domain specifically binds. The terms "effector domain" and "functional domain" are used interchangeably throughout this application.

In some embodiments of the TALE polypeptides described herein, the activity mediated by the effector domain is a biological activity. For example, in some embodiments the effector domain is a transcriptional inhibitor (i.e., a repressor domain), such as an mSin interaction domain (SID). SID4X domain or a Krüppel-associated box (KRAB) or fragments of the KRAB domain. In some embodiments the effector domain is an enhancer of transcription (i.e. an activation domain), such as the VP16, VP64 or p65 activation domain. A graphical comparison of the effect these different activation domains have on Sox2 mRNA level is provided in **FIG. 11****.**

As used herein, VP16 is a herpesvirus protein. It is a very strong transcriptional activator that specifically activates viral immediate early gene expression. The VP16 activation domain is rich in acidic residues and has been regarded as a classic acidic activation domain (AAD). As used herein, VP64 activation doamin is a tetrameric repeat of VP16's minimal activation domain. As used herein, p65 is one of two proteins that the NF-kappa B transcription factor complex is composed of. The other protein is p50. The p65 activation domain is a part of the p65 subunit is a potent transcriptional activator even in the absence of p50. In certain embodiments, the effector domain is a mammalian protein or biologically active fragment thereof. Such effector domains are referred to as "mammalian effector domains."

In some embodiments, the nucleic acid binding is linked, for example, with an effector domain or functional domain that includes but is not limited to transposase domain, integrase domain, recombinase domain, resolvase domain, invertase domain, protease domain, DNA methyltransferase domain, DNA hydroxylmethylase domain, DNA demethylase domain, histone acetylase domain, histone deacetylases domain, nuclease domain, repressor domain, activator domain, nuclear-localization signal domains, transcription-regulatory protein (or transcription complex recruiting) domain, cellular uptake activity associated domain, nucleic acid binding domain, antibody presentation domain, histone modifying enzymes, recruiter of histone modifying enzymes; inhibitor of histone modifying enzymes, histone methyltransferase, histone demethylase, histone kinase, histone phosphatase, histone ribosylase, histone deribosylase, histone ubiquitinase, histone deubiquitinase, histone biotinase and histone tail protease.

In some embodiments, the effector domain is a protein domain which exhibits activities which include but are not limited to transposase activity, integrase activity, recombinase activity, resolvase activity, invertase activity, protease activity, DNA methyltransferase activity, DNA demethylase activity, histone acetylase activity, histone deacetylase activity, nuclease activity, nuclear-localization signaling activity, transcriptional repressor activity, transcriptional activator activity, transcription factor recruiting activity, or cellular uptake signaling activity. Other preferred embodiments of the invention may include any combination the activities described herein.

As described in Zhang et al., Nature Biotechnology 29:149-153 (2011), a TALE polypeptide having a nucleic acid binding domain and an effector domain may be used to target the effector domain's activity to a genomic position having a predetermined nucleic acid sequence recognized by the nucleic acid binding domain. In some embodiments of the invention described herein, TALE polypeptides are designed and used for targeting gene regulatory activity, such as transcriptional or translational modifier activity, to a regulatory, coding, and/or intergenic region, such as enhancer and/or repressor activity, that may affect transcription upstream and downstream of coding regions, and may be used to enhance or repress gene expression. For example, TALEs polypeptide may comprise effector domains having DNA-binding domains from transcription factors, effector domains from transcription factors (activators, repressors, co-activators, co-repressors), silencers, nuclear hormone receptors, and/or chromatin associated proteins and their modifiers (e.g., methylases, kinases, phosphatases, acetylases and deacetylases). In a preferred embodiment, the TALE polypeptide may comprise a nuclease domain. In a more preferred embodiment the nuclease domain is a non-specific FokI endonucleases catalytic domain.

In a further embodiment, useful domains for regulating gene expression may also be obtained from the gene products of oncogenes. In yet further advantageous embodiments of the invention, effector domains having integrase or transposase activity may be used to promote integration of exogenous nucleic acid sequence into specific nucleic acid sequence regions, eliminate (knock-out) specific endogenous nucleic acid sequence, and/or modify epigenetic signals and consequent gene regulation, such as by promoting DNA methyltransferase, DNA demethylase, histone acetylase and histone deacetylase activity. In other embodiments, effector domains having nuclease activity may be used to alter genome structure by nicking or digesting target sequences to which the polypeptides of the invention specifically bind, and may allow introduction of exogenous genes at those sites. In still further embodiments, effector domains having invertase activity may be used to alter genome structure by swapping the orientation of a DNA fragment.

In particularly advantageous embodiments, the polypeptides used in the methods of the invention may be used to target transcriptional activity. As used herein, the term "transcription factor" refers to a protein or polypeptide that binds specific DNA sequences associated with a genomic locus or gene of interest to control transcription. Transcription factors may promote (as an activator) or block (as a repressor) the recruitment of RNA polymerase to a gene of interest. Transcription factors may perform their function alone or as a part of a larger protein complex. Mechanisms of gene regulation used by transcription factors include but are not limited to a) stabilization or destabilization of RNA polymerase binding, b) acetylation or deacetylation of histone proteins and c) recruitment of co-activator or co-repressor proteins. Furthermore, transcription factors play roles in biological activities that include but are not limited to basal transcription, enhancement of transcription, development, response to intercellular signaling, response to environmental cues, cell-cycle control and pathogenesis. With regards to information on transcriptional factors, mention is made of Latchman and DS (1997) Int. J. Biochem. Cell Biol. 29 (12): 1305-12; Lee TI, Young RA (2000) Annu. Rev. Genet. 34: 77-137and Mitchell PJ, Tjian R (1989) Science 245 (4916): 371-8.

Light responsiveness of a LITE is achieved via the activation and binding of cryptochrome-2 and CIB1. As mentioned above, blue light stimulation induces an activating conformational change in cryptochrome-2, resulting in recruitment of its binding partner CIB1. This binding is fast and reversible, achieving saturation in <15 sec following pulsed stimulation and returning to baseline <15 min after the end of stimulation. These rapid binding kinetics result in a LITE system temporally bound only by the speed of transcription/translation and transcript/protein degradation, rather than uptake and clearance of inducing agents. Crytochrome-2 activation is also highly sensitive, allowing for the use of low light intensity stimulation and mitigating the risks of phototoxicity. Further, in a context such as the intact mammalian brain, variable light intensity may be used to control the size of a LITE stimulated region, allowing for greater precision than vector delivery alone may offer.

The modularity of the LITE system allows for any number of effector domains to be employed for transcriptional modulation. Thus, activator and repressor domains may be selected on the basis of species, strength, mechanism, duration, size, or any number of other parameters.

Applicants next present two prototypical manifestations of the LITE system. The first example is a LITE designed to activate transcription of the mouse gene NEUROG2. The sequence TGAATGATGATAATACGA, located in the upstream promoter region of mouse NEUROG2, was selected as the target and a TALE was designed and synthesized to match this sequence. The TALE sequence was linked to the sequence for cryptochrome-2 via a nuclear localization signal (amino acids: SPKKKRKVEAS) to facilitate transport of the protein from the cytosol to the nuclear space. A second vector was synthesized comprising the CIB1 domain linked to the transcriptional activator domain VP64 using the same nuclear localization signal. This second vector, also a GFP sequence, is separated from the CIB1-VP64 fusion sequence by a 2A translational skip signal. Expression of each construct was driven by a ubiquitous, constitutive promoter (CMV or EF1-α). Mouse neuroblastoma cells from the Neuro 2A cell line were co-transfected with the two vectors. After incubation to allow for vector expression, samples were stimulated by periodic pulsed blue light from an array of 488 nm LEDs. Unstimulated co-tranfected samples and samples transfected only with the fluorescent reporter YFP were used as controls. At the end of each experiment, mRNA was purified from the samples analyzed via qPCR.

Truncated versions of cryptochrome-2 and CIB1 were cloned and tested in combination with the full-length versions of cryptochrome-2 and CIB1 in order to determine the effectiveness of each heterodimer pair. The combination of the CRY2PHR domain, consisting of the conserved photoresponsive region of the cryptochrome-2 protein, and the full-length version of CIB1 resulted in the highest upregulation of Neurog2 mRNA levels (-22 fold over YFP samples and ∼7 fold over unstimulated co-transfected samples). The combination of full-length cryptochrome-2 (CRY2) with full-length CIB1 resulted in a lower absolute activation level (-4.6 fold over YFP), but also a lower baseline activation (-1.6 fold over YFP for unstimulated co-transfected samples). These cryptochrome protein pairings may be selected for particular uses depending on absolute level of induction required and the necessity to minimize baseline "leakiness" of the LITE system.

Speed of activation and reversibility are critical design parameters for the LITE system. To characterize the kinetics of the LITE system, constructs consisting of the Neurog2 TALE-CRY2PHR and CIB1-VP64 version of the system were tested to determine its activation and inactivation speed. Samples were stimulated for as little as 0.5 h to as long as 24 h before extraction. Upregulation of Neurog2 expression was observed at the shortest, 0.5 h, time point (-5 fold vs YFP samples). Neurog2 expression peaked at 12 h of stimulation (-19 fold vs YFP samples). Inactivation kinetics were analyzed by stimulating co-transfected samples for 6 h, at which time stimulation was stopped, and samples were kept in culture for 0 to 12 h to allow for mRNA degradation. Neurog2 mRNA levels peaked at 0.5h after the end of stimulation (-16 fold vs. YFP samples), after which the levels degraded with an -3 h half-life before returning to near baseline levels by 12 h.

The second prototypical example is a LITE designed to activate transcription of the human gene KLF4. The sequence TTCTTACTTATAAC, located in the upstream promoter region of human KLF4, was selected as the target and a TALE was designed and synthesized to match this sequence. The TALE sequence was linked to the sequence for CRY2PHR via a nuclear localization signal (amino acids: SPKKKRKVEAS). The identical CIB1-VP64 activator protein described above was also used in this manifestation of the LITE system. Human embryonal kidney cells from the HEK293FT cell line were co-transfected with the two vectors. After incubation to allow for vector expression, samples were stimulated by periodic pulsed blue light from an array of 488 nm LEDs. Unstimulated co-tranfected samples and samples transfected only with the fluorescent reporter YFP were used as controls. At the end of each experiment, mRNA was purified from the samples analyzed via qPCR.

The light-intensity response of the LITE system was tested by stimulating samples with increased light power (0-9 mW/cm²). Upregulation of KLF4 mRNA levels was observed for stimulation as low as 0.2 mW/cm². KLF4 upregulation became saturated at 5 mW/cm² (2.3 fold vs. YFP samples). Cell viability tests were also performed for powers up to 9 mW/cm² and showed >98% cell viability. Similarly, the KLF4 LITE response to varying duty cycles of stimulation was tested (1.6-100%). No difference in KLF4 activation was observed between different duty cycles indicating that a stimulation paradigm of as low as 0.25 sec every 15 sec should result in maximal activation.

The disclosure contemplates energy sources such as electromagnetic radiation, sound energy or thermal energy. Advantageously, the electromagnetic radiation is a component of visible light. In a preferred embodiment, the light is a blue light with a wavelength of about 450 to about 495 nm. In an especially preferred embodiment, the wavelength is about 488 nm. In another preferred embodiment, the light stimulation is via pulses. The light power may range from about 0-9 mW/cm². In a preferred embodiment, a stimulation paradigm of as low as 0.25 sec every 15 sec should result in maximal activation.

The invention particularly relates to inducible methods of perturbing a genomic or epigenomic locus or altering expression of a genomic locus of interest in a cell wherein the genomic or epigenomic locus may be contacted with a non-naturally occurring or engineered composition comprising a deoxyribonucleic acid (DNA) binding polypeptide.

The cells of the present invention may be a prokaryotic cell or a eukaryotic cell, advantageously an animal cell, more advantageously a mammalian cell.

This polypeptide of the disclosure may include a DNA binding domain comprising at least five or more Transcription activator-like effector (TALE) monomers and at least one or more half-monomers specifically ordered to target the genomic locus of interest or at least one or more effector domains linked to a chemical sensitive protein or fragment thereof. The chemical or energy sensitive protein or fragment thereof may undergo a conformational change upon induction by the binding of a chemical source allowing it to bind an interacting partner. The polypeptide may also include a DNA binding domain comprising at least one or more variant TALE monomers or half-monomers specifically ordered to target the genomic locus of interest or at least one or more effector domains linked to the interacting partner, wherein the chemical or energy sensitive protein or fragment thereof may bind to the interacting partner upon induction by the chemical source. The method may also include applying the chemical source and determining that the expression of the genomic locus is altered.

There are several different designs of this chemical inducible system: 1. ABI-PYL based system inducible by Abscisic Acid (ABA) (see, e.g., http://stke.sciencemag.org/cgi/content/abstract/sigtrans;4/164/rs2), 2. FKBP-FRB based system inducible by rapamycin (or related chemicals based on rapamycin) (see, e.g., http://www.nature.com/nmeth/journal/v2/n6/full/nmeth763.html), 3. GID1-GAI based system inducible by Gibberellin (GA) (see, e.g., http://www.nature.com/nchembio/journal/v8/n5/full/nchembio.922.html).

Another system contemplated by the present disclosure is a chemical inducible system based on change in sub-cellular localization. Applicants also developed a system in which the polypeptide include a DNA binding domain comprising at least five or more Transcription activator-like effector (TALE) monomers and at least one or more half-monomers specifically ordered to target the genomic locus of interest linked to at least one or more effector domains are further linker to a chemical or energy sensitive protein. This protein will lead to a change in the sub-cellular localization of the entire polypeptide (i.e. transportation of the entire polypeptide from cytoplasm into the nucleus of the cells) upon the binding of a chemical or energy transfer to the chemical or energy sensitive protein. This transportation of the entire polypeptide from one sub-cellular compartments or organelles, in which its activity is sequestered due to lack of substrate for the effector domain, into another one in which the substrate is present would allow the entire polypeptide to come in contact with its desired substrate (i.e. genomic DNA in the mammalian nucleus) and result in activation or repression of target gene expression.

This type of system could also be used to induce the cleavage of a genomic locus of interest in a cell when the effector domain is a nuclease.

The designs for this chemical inducible system is an estrogen receptor (ER) based system inducible by 4-hydroxytamoxifen (4OHT) (see, e.g., http://www.pnas.org/content/104/3/1027.abstract). A mutated ligand-binding domain of the estrogen receptor called ERT2 translocates into the nucleus of cells upon binding of 4-hydroxytamoxifen. Two tandem ERT2 domains were linked together with a flexible peptide linker and then fused to the TALE protein targeting a specific sequence in the mammalian genome and linked to one or more effector domains. This polypeptide will be in the cytoplasm of cells in the absence of 4OHT, which renders the TALE protein linked to the effector domains inactive. In the presence of 4OHT, the binding of 4OHT to the tandem ERT2 domain will induce the transportation of the entire peptide into nucleus of cells, allowing the TALE protein linked to the effector domains become active.

In another embodiment of the estrogen receptor (ER) based system inducible by 4-hydroxytamoxifen (4OHT), the present disclosure may comprise a nuclear exporting signal (NES). Advantageously, the NES may have the sequence of LDLASLIL. In further embodiments of the disclosure any naturally occurring or engineered derivative of any nuclear receptor, thyroid hormone receptor, retinoic acid receptor, estrogren receptor, estrogen-related receptor, glucocorticoid receptor, progesterone receptor, androgen receptor may be used in inducible systems analogous to the ER based inducible system.

Another inducible system is based on the design using Transient receptor potential (TRP) ion channel based system inducible by energy, heat or radio-wave (see, e.g., http://www.sciencemag.org/content/336/6081/604). These TRP family proteins respond to different stimuli, including light and heat. When this protein is activated by light or heat, the ion channel will open and allow the entering of ions such as calcium into the plasma membrane. This inflex of ions will bind to intracellular ion interacting partners linked to a polypeptide include TALE protein and one or more effector domains, and the binding will induce the change of sub-cellular localization of the polypeptide, leading to the entire polypeptide entering the nucleus of cells. Once inside the nucleus, the TALE protein linked to the effector domains will be active and modulating target gene expression in cells.

This type of system could also be used to induce the cleavage of a genomic locus of interest in a cell when the effector domain is a nuclease. The light could be generated with a laser or other forms of energy sources. The heat could be generated by raise of temperature results from an energy source, or from nano-particles that release heat after absorbing energy from an energy source delivered in the form of radio-wave.

While light activation may be an advantageous embodiment, sometimes it may be disadvantageous especially for *in vivo* applications in which the light may not penetrate the skin or other organs. In this instance, other methods of energy activation are contemplated, in particular, electric field energy and/or ultrasound which have a similar effect. If necessary, the proteins pairings of the LITE system may be altered and/or modified for maximal effect by another energy source.

Electric field energy is preferably administered substantially as described in the art, using one or more electric pulses of from about 1 Volt/cm to about 10 kVolts/cm under *in vivo* conditions. Instead of or in addition to the pulses, the electric field may be delivered in a continuous manner. The electric pulse may be applied for between 1 µs and 500 milliseconds, preferably between 1 µs and 100 milliseconds. The electric field may be applied continuously or in a pulsed manner for 5 about minutes.

As used herein, 'electric field energy' is the electrical energy to which a cell is exposed. Preferably the electric field has a strength of from about 1 Volt/cm to about 10 kVolts/cm or more under *in vivo* conditions (see WO97/49450).

As used herein, the term "electric field" includes one or more pulses at variable capacitance and voltage and including exponential and/or square wave and/or modulated wave and/or modulated square wave forms. References to electric fields and electricity should be taken to include reference the presence of an electric potential difference in the environment of a cell. Such an environment may be set up by way of static electricity, alternating current (AC), direct current (DC), etc, as known in the art. The electric field may be uniform, non-uniform or otherwise, and may vary in strength and/or direction in a time dependent manner.

Single or multiple applications of electric field, as well as single or multiple applications of ultrasound are also possible, in any order and in any combination. The ultrasound and/or the electric field may be delivered as single or multiple continuous applications, or as pulses (pulsatile delivery).

Electroporation has been used in both *in vitro* and *in vivo* procedures to introduce foreign material into living cells. With *in vitro* applications, a sample of live cells is first mixed with the agent of interest and placed between electrodes such as parallel plates. Then, the electrodes apply an electrical field to the cell/implant mixture. Examples of systems that perform *in vitro* electroporation include the Electro Cell Manipulator ECM600 product, and the Electro Square Porator T820, both made by the BTX Division of Genetronics, Inc (see U.S. Pat. No 5,869,326).

The known electroporation techniques (both *in vitro* and *in vivo*) function by applying a brief high voltage pulse to electrodes positioned around the treatment region. The electric field generated between the electrodes causes the cell membranes to temporarily become porous, whereupon molecules of the agent of interest enter the cells. In known electroporation applications, this electric field comprises a single square wave pulse on the order of 1000 V/cm, of about 100 .mu.s duration. Such a pulse may be generated, for example, in known applications of the Electro Square Porator T820.

Preferably, the electric field has a strength of from about 1 V/cm to about 10 kV/cm under *in vitro* conditions. Thus, the electric field may have a strength of 1 V/cm, 2 V/cm, 3 V/cm, 4 V/cm, 5 V/cm, 6 V/cm, 7 V/cm, 8 V/cm, 9 V/cm, 10 V/cm, 20 V/cm, 50 V/cm, 100 V/cm, 200 V/cm, 300 V/cm, 400 V/cm, 500 V/cm, 600 V/cm, 700 V/cm, 800 V/cm, 900 V/cm, 1 kV/cm, 2 kV/cm, 5 kV/cm, 10 kV/cm, 20 kV/cm, 50 kV/cm or more. More preferably from about 0.5 kV/cm to about 4.0 kV/cm under *in vitro* conditions. Preferably the electric field has a strength of from about 1 V/cm to about 10 kV/cm under *in vivo* conditions. However, the electric field strengths may be lowered where the number of pulses delivered to the target site are increased. Thus, pulsatile delivery of electric fields at lower field strengths is envisaged.

Preferably the application of the electric field is in the form of multiple pulses such as double pulses of the same strength and capacitance or sequential pulses of varying strength and/or capacitance. As used herein, the term "pulse" includes one or more electric pulses at variable capacitance and voltage and including exponential and/or square wave and/or modulated wave/square wave forms.

Preferably the electric pulse is delivered as a waveform selected from an exponential wave form, a square wave form, a modulated wave form and a modulated square wave form.

A preferred embodiment employs direct current at low voltage. Thus, Applicants disclose the use of an electric field which is applied to the cell, tissue or tissue mass at a field strength of between 1V/cm and 20V/cm, for a period of 100 milliseconds or more, preferably 15 minutes or more.

Ultrasound is advantageously administered at a power level of from about 0.05 W/cm² to about 100 W/cm². Diagnostic or therapeutic ultrasound may be used, or combinations thereof.

As used herein, the term "ultrasound" refers to a form of energy which consists of mechanical vibrations the frequencies of which are so high they are above the range of human hearing. Lower frequency limit of the ultrasonic spectrum may generally be taken as about 20 kHz. Most diagnostic applications of ultrasound employ frequencies in the range 1 and 15 MHz' (From Ultrasonics in Clinical Diagnosis, P. N. T. Wells, ed., 2nd. Edition, Publ. Churchill Livingstone [Edinburgh, London & NY, 1977]).

Ultrasound has been used in both diagnostic and therapeutic applications. When used as a diagnostic tool ("diagnostic ultrasound"), ultrasound is typically used in an energy density range of up to about 100 mW/cm² (FDA recommendation), although energy densities of up to 750 mW/cm² have been used. In physiotherapy, ultrasound is typically used as an energy source in a range up to about 3 to 4 W/cm² (WHO recommendation). In other therapeutic applications, higher intensities of ultrasound may be employed, for example, HIFU at 100 W/cm up to 1 kW/cm² (or even higher) for short periods of time. The term "ultrasound" as used in this specification is intended to encompass diagnostic, therapeutic and focused ultrasound.

Focused ultrasound (FUS) allows thermal energy to be delivered without an invasive probe (see Morocz et al 1998 Journal of Magnetic Resonance Imaging Vol.8, No. 1, pp. 136-142. Another form of focused ultrasound is high intensity focused ultrasound (HIFU) which is reviewed by Moussatov et al in Ultrasonics (1998) Vol.36, No.8, pp.893-900 and TranHuuHue et al in Acustica (1997) Vol.83, No.6, pp.1103-1106.

Preferably, a combination of diagnostic ultrasound and a therapeutic ultrasound is employed. This combination is not intended to be limiting, however, and the skilled reader will appreciate that any variety of combinations of ultrasound may be used. Additionally, the energy density, frequency of ultrasound, and period of exposure may be varied.

Preferably the exposure to an ultrasound energy source is at a power density of from about 0.05 to about 100 Wcm⁻². Even more preferably, the exposure to an ultrasound energy source is at a power density of from about 1 to about 15 Wcm⁻².

Preferably the exposure to an ultrasound energy source is at a frequency of from about 0.015 to about 10.0 MHz. More preferably the exposure to an ultrasound energy source is at a frequency of from about 0.02 to about 5.0 MHz or about 6.0 MHz. Most preferably, the ultrasound is applied at a frequency of 3 MHz.

Preferably the exposure is for periods of from about 10 milliseconds to about 60 minutes. Preferably the exposure is for periods of from about 1 second to about 5 minutes. More preferably, the ultrasound is applied for about 2 minutes. Depending on the particular target cell to be disrupted, however, the exposure may be for a longer duration, for example, for 15 minutes.

Advantageously, the target tissue is exposed to an ultrasound energy source at an acoustic power density of from about 0.05 Wcm⁻² to about 10 Wcm⁻² with a frequency ranging from about 0.015 to about 10 MHz (see WO 98/52609). However, alternatives are also possible, for example, exposure to an ultrasound energy source at an acoustic power density of above 100 Wcm⁻², but for reduced periods of time, for example, 1000 Wcm⁻² for periods in the millisecond range or less.

Preferably the application of the ultrasound is in the form of multiple pulses; thus, both continuous wave and pulsed wave (pulsatile delivery of ultrasound) may be employed in any combination. For example, continuous wave ultrasound may be applied, followed by pulsed wave ultrasound, or vice versa. This may be repeated any number of times, in any order and combination. The pulsed wave ultrasound may be applied against a background of continuous wave ultrasound, and any number of pulses may be used in any number of groups.

Preferably, the ultrasound may comprise pulsed wave ultrasound. In a highly preferred embodiment, the ultrasound is applied at a power density of 0.7 Wcm⁻² or 1.25 Wcm⁻² as a continuous wave. Higher power densities may be employed if pulsed wave ultrasound is used.

Use of ultrasound is advantageous as, like light, it may be focused accurately on a target. Moreover, ultrasound is advantageous as it may be focused more deeply into tissues unlike light. It is therefore better suited to whole-tissue penetration (such as but not limited to a lobe of the liver) or whole organ (such as but not limited to the entire liver or an entire muscle, such as the heart) therapy. Another important advantage is that ultrasound is a non-invasive stimulus which is used in a wide variety of diagnostic and therapeutic applications. By way of example, ultrasound is well known in medical imaging techniques and, additionally, in orthopedic therapy. Furthermore, instruments suitable for the application of ultrasound to a subject vertebrate are widely available and their use is well known in the art.

The rapid transcriptional response and endogenous targeting of LITEs make for an ideal system for the study of transcriptional dynamics. For example, LITEs may be used to study the dynamics of mRNA splice variant production upon induced expression of a target gene. On the other end of the transcription cycle, mRNA degradation studies are often performed in response to a strong extracellular stimulus, causing expression level changes in a plethora of genes. LITEs may be utilized to reversibly induce transcription of an endogenous target, after which point stimulation may be stopped and the degradation kinetics of the unique target may be tracked.

The temporal precision of LITEs may provide the power to time genetic regulation in concert with experimental interventions. For example, targets with suspected involvement in long-term potentiation (LTP) may be modulated in organotypic or dissociated neuronal cultures, but only during stimulus to induce LTP, so as to avoid interfering with the normal development of the cells. Similarly, in cellular models exhibiting disease phenotypes, targets suspected to be involved in the effectiveness of a particular therapy may be modulated only during treatment. Conversely, genetic targets may be modulated only during a pathological stimulus. Any number of experiments in which timing of genetic cues to external experimental stimuli is of relevance may potentially benefit from the utility of LITE modulation.

The *in vivo* context offers equally rich opportunities for the use of LITEs to control gene expression. As mentioned above, photoinducibility provides the potential for previously unachievable spatial precision. Taking advantage of the development of optrode technology, a stimulating fiber optic lead may be placed in a precise brain region. Stimulation region size may then be tuned by light intensity. This may be done in conjunction with the delivery of LITEs via viral vectors or the molecular sleds of US Provisional Patent application no. 61/671,615, or, if transgenic LITE animals were to be made available, may eliminate the use of viruses while still allowing for the modulation of gene expression in precise brain regions. LITEs may be used in a transparent organism, such as an immobilized zebrafish, to allow for extremely precise laser induced local gene expression changes.

The present disclosure also contemplates a multiplex genome engineering using CRISPR/Cas sytems. Functional elucidation of causal genetic variants and elements requires precise genome editing technologies. The type II prokaryotic CRISPR (clustered regularly interspaced short palindromic repeats) adaptive immune system has been shown to facilitate RNA-guided site-specific DNA cleavage. Applicants engineered two different type II CRISPR systems and demonstrate that Cas9 nucleases can be directed by short RNAs to induce precise cleavage at endogenous genomic loci in human and mouse cells. Cas9 can also be converted into a nicking enzyme to facilitate homology-directed repair with minimal mutagenic activity. Finally, multiple guide sequences can be encoded into a single CRISPR array to enable simultaneous editing of several sites within the mammalian genome, demonstrating easy programmability and wide applicability of the CRISPR technology.

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In some embodiments of the disclosure, one or more elements of a CRISPR system is derived from a type I, type II, or type III CRISPR system. In some embodiments, one or more elements of a CRISPR system is derived from a particular organism comprising an endogenous CRISPR system, such as *Streptococcus pyogenes.* In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, all or a portion of the tracr sequence may also form part of a CRISPR complex, such as by hybridization to all or a portion of a tracr mate sequence that is operably linked to the guide sequence. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments of the disclosure, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments of the disclosure, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments of the disclosure, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments of the disclosure, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Csel, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In some embodiments of the disclosure, the unmodified CRISPR enzyme has DNA cleavage activity, such as Cas9. In some embodiments of the disclosure, the CRISPR enzyme directs cleavage of one or both strands at the location of a target sequence, such as within the target sequence and/or within the complement of the target sequence. In some embodiments of the disclosure, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments of the disclosure, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from *S. pyogenes* converts Cas9 from a nuclease that cleaves both strands to a nickase (cleaves a single strand). Other examples of mutations that render Cas9 a nickase include, without limitation, H840A, N854A, and N863A. As a further example, two or more catalytic domains of Cas9 (RuvC I, RuvC II, and RuvC III) may be mutated to produce a mutated Cas9 substantially lacking all DNA cleavage activity. In some embodiments of the disclosure, a D10A mutation is combined with one or more of H840A, N854A, or N863A mutations to produce a Cas9 enzyme substantially lacking all DNA cleavage activity. In some embodiments of the disclosure, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form.

In some embodiments of the disclosure, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000"Nucl. Acids Res. 28:292 (2000). Computer algorithms for codon optimizing a particular sequence for expression in a particular host cell are also available, such as Gene Forge (Aptagen; Jacobus, PA), are also available. In some embodiments of the disclosure, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments of the disclosure, a vector encodes a CRISPR enzyme comprising one or more nuclear localization sequences (NLSs), such as about or more than about 1, 2, 3, 4, 5, 6, 7, 8, 9, 10, or more NLSs. In some embodiments of the disclosure, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGQYFAKPRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

In another embodiment of the present disclosure, the invention relates to an inducible CRISPR which may comprise an inducible Cas9.

According to the disclosure, the CRISPR system may be encoded within a vector system which may comprise one or more vectors which may comprise I. a first regulatory element operably linked to a CRISPR/Cas system chimeric RNA (chiRNA) polynucleotide sequence, wherein the polynucleotide sequence may comprise (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme which may comprise at least one or more nuclear localization sequences, wherein (a), (b) and (c) are arranged in a 5' to 3'orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex may comprise the CRISPR enzyme complexed with (1) the guide sequence that is hybridized to the target sequence, and (2) the tracr mate sequence that is hybridized to the tracr sequence, wherein the enzyme coding sequence encoding the CRISPR enzyme further encodes a heterologous functional domain.

In an advantageous embodiment of the disclosure, the inducible Cas9 may be prepared in a lentivirus. For example, FIG. 61 depicts Tet Cas9 vector designs and FIG. 62 depicts a vector and EGFP expression in 293FT cells. In particular, an inducible tetracycline system is contemplated for an inducible CRISPR..The vector may be designed as described in Markusic et al., Nucleic Acids Research, 2005, Vol. 33, No. 6 e63. The tetracycline-dependent transcriptional regulatory system is based on the Escherichia coli Tn10 Tetracycline resistance operator consisting of the tetracycline repressor protein (TetR) and a specific DNA-binding site, the tetracycline operator sequence (TetO). In the absence of tetracycline, TetR dimerizes and binds to the TetO. Tetracycline or doxycycline (a tetracycline derivative) can bind and induce a conformational change in the TetR leading to its disassociation from the TetO. In an advantageous embodiment of the disclosure, the vector may be a single Tet-On lentiviral vector with autoregulated rtTA expression for regulated expression of the CRISPR complex. Tetracycline or doxycycline may be contemplated for activating the inducible CRISPR complex.

In another embodiment of the disclosure, a cumate gene-switch system is contemplated for an inducible CRISPR. A similar system as described in Mullick et al., BMC Biotechnology 2006, 6:43 doi:10.1186/1472-6750-6-43. The inducible cumate system involves regulatory mechanisms of bacterial operons (cmt and cym) to regulate gene expression in mammalian cells using three different strategies. In the repressor configuration, regulation is mediated by the binding of the repressor (CymR) to the operator site (CuO), placed downstream of a strong constitutive promoter. Addition of cumate, a small molecule, relieves the repression. In the transactivator configuration, a chimaeric transactivator (cTA) protein, formed by the fusion of CymR with the activation domain of VP16, is able to activate transcription when bound to multiple copies of CuO, placed upstream of the CMV minimal promoter. Cumate addition abrogates DNA binding and therefore transactivation by cTA. The disclosure also contemplates a reverse cumate activator (rcTA), which activates transcription in the presence rather than the absence of cumate. CymR may be used as a repressor that reversibly blocks expression from a strong promoter, such as CMV. Certain aspects of the Cumate repressor/operator system are further described in US patent No. 7745592.

Other inducible systems are contemplated such as, but not limited to, regulation by heavy-metals [Mayo KE et al., Cell 1982, 29:99-108; Searle PF et al., Mol Cell Biol 1985, 5:1480-1489 and Brinster RL et al., Nature (London) 1982, 296:39-42], steroid hormones [Hynes NE et al., Proc Natl Acad Sci USA 1981, 78:2038-2042; Klock G et al., Nature (London) 1987, 329:734-736 and Lee F et al., Nature (London) 1981, 294:228-232.], heat shock [Nouer L: Heat Shock Response. Boca Raton, FL: CRC; 1991] and other reagents have been developed [Mullick A, Massie B: Transcription, translation and the control of gene expression. In Encyclopedia of Cell Technology Edited by: Speir RE. Wiley; 2000:1140-1164 and Fussenegger M, . Biotechnol Prog 2001, 17:1-51]. However, there are limitations with these inducible mammalian promoters such as "leakiness" of the "off" state and pleiotropic effects of inducers (heat shock, heavy metals, glucocorticoids etc.). The use of insect hormones (ecdysone) has been proposed in an attempt to reduce the interference with cellular processes in mammalian cells [No D et al., Proc Natl Acad Sci USA 1996, 93:3346-3351]. Another elegant system uses rapamycin as the inducer [Rivera VM et al., Nat Med 1996, 2:1028-1032] but the role of rapamycin as an immunosuppressant was a major limitation to its use in vivo and therefore it was necessary to find a biologically inert compound [Saez E et al., Proc Natl Acad Sci USA 2000, 97:14512-14517] for the control of gene expression.

The present disclosure also encompasses nucleic acid encoding the polypeptides of the present invention. The nucleic acid may comprise a promoter, advantageously human Synapsin I promoter (hSyn). In a particularly advantageous embodiment, the nucleic acid may be packaged into an adeno associated viral vector (AAV).

Also contemplated by the present disclosure are recombinant vectors and recombinant adenoviruses that may comprise subviral particles from more than one adenovirus serotype. For example, it is known that adenovirus vectors may display an altered tropism for specific tissues or cell types (Havenga, M.J.E. et al., 2002), and therefore, mixing and matching of different adenoviral capsids, i.e., fiber, or penton proteins from various adenoviral serotypes may be advantageous. Modification of the adenoviral capsids, including fiber and penton may result in an adenoviral vector with a tropism that is different from the unmodified adenovirus. Adenovirus vectors that are modified and optimized in their ability to infect target cells may allow for a significant reduction in the therapeutic or prophylactic dose, resulting in reduced local and disseminated toxicity.

Viral vector gene delivery systems are commonly used in gene transfer and gene therapy applications. Different viral vector systems have their own unique advantages and disadvantages. Viral vectors that may be used to express the pathogen-derived ligand of the present invention include but are not limited to adenoviral vectors, adeno-associated viral vectors, alphavirus vectors, herpes simplex viral vectors, and retroviral vectors, described in more detail below.

Additional general features of adenoviruses are such that the biology of the adenovirus is characterized in detail; the adenovirus is not associated with severe human pathology; the adenovirus is extremely efficient in introducing its DNA into the host cell; the adenovirus may infect a wide variety of cells and has a broad host range; the adenovirus may be produced in large quantities with relative ease; and the adenovirus may be rendered replication defective and/or non-replicating by deletions in the early region 1 ("E1") of the viral genome.

Adenovirus is a non-enveloped DNA virus. The genome of adenovirus is a linear double-stranded DNA molecule of approximately 36,000 base pairs ("bp") with a 55-kDa terminal protein covalently bound to the 5'-terminus of each strand. The adenovirus DNA contains identical inverted terminal repeats ("ITRs") of about 100 bp, with the exact length depending on the serotype. The viral origins of replication are located within the ITRs exactly at the genome ends. DNA synthesis occurs in two stages. First, replication proceeds by strand displacement, generating a daughter duplex molecule and a parental displaced strand. The displaced strand is single stranded and may form a "panhandle" intermediate, which allows replication initiation and generation of a daughter duplex molecule. Alternatively, replication may proceed from both ends of the genome simultaneously, obviating the requirement to form the panhandle structure.

During the productive infection cycle, the viral genes are expressed in two phases: the early phase, which is the period up to viral DNA replication, and the late phase, which coincides with the initiation of viral DNA replication. During the early phase, only the early gene products, encoded by regions E1, E2, E3 and E4, are expressed, which carry out a number of functions that prepare the cell for synthesis of viral structural proteins (Berk, A.J., 1986). During the late phase, the late viral gene products are expressed in addition to the early gene products and host cell DNA and protein synthesis are shut off. Consequently, the cell becomes dedicated to the production of viral DNA and of viral structural proteins (Tooze, J., 1981).

The E1 region of adenovirus is the first region of adenovirus expressed after infection of the target cell. This region consists of two transcriptional units, the E1A and E1B genes, both of which are required for oncogenic transformation of primary (embryonal) rodent cultures. The main functions of the E1A gene products are to induce quiescent cells to enter the cell cycle and resume cellular DNA synthesis, and to transcriptionally activate the E1B gene and the other early regions (E2, E3 and E4) of the viral genome. Transfection of primary cells with the E1A gene alone may induce unlimited proliferation (immortalization), but does not result in complete transformation. However, expression of E1A, in most cases, results in induction of programmed cell death (apoptosis), and only occasionally is immortalization obtained (Jochemsen et al., 1987). Co-expression of the E1B gene is required to prevent induction of apoptosis and for complete morphological transformation to occur. In established immortal cell lines, high-level expression of E1A may cause complete transformation in the absence of E1B (Roberts, B.E. et al., 1985).

The E1B encoded proteins assist E1A in redirecting the cellular functions to allow viral replication. The E1B 55 kD and E4 33 kD proteins, which form a complex that is essentially localized in the nucleus, function in inhibiting the synthesis of host proteins and in facilitating the expression of viral genes. Their main influence is to establish selective transport of viral mRNAs from the nucleus to the cytoplasm, concomitantly with the onset of the late phase of infection. The E1B 21 kD protein is important for correct temporal control of the productive infection cycle, thereby preventing premature death of the host cell before the virus life cycle has been completed. Mutant viruses incapable of expressing the E1B 21 kD gene product exhibit a shortened infection cycle that is accompanied by excessive degradation of host cell chromosomal DNA (deg-phenotype) and in an enhanced cytopathic effect (cyt-phenotype; Telling et al., 1994). The deg and cyt phenotypes are suppressed when in addition the E1A gene is mutated, indicating that these phenotypes are a function of E1A (White, E. et al., 1988). Furthermore, the E1B 21 kDa protein slows down the rate by which E1A switches on the other viral genes. It is not yet known by which mechanisms EIB 21 kD quenches these E1A dependent functions.

In contrast to, for example, retroviruses, adenoviruses do not efficiently integrate into the host cell's genome, are able to infect non-dividing cells, and are able to efficiently transfer recombinant genes *in vivo* (Brody et al., 1994). These features make adenoviruses attractive candidates for *in vivo* gene transfer of, for example, an antigen or immunogen of interest into cells, tissues or subjects in need thereof.

Adenovirus vectors containing multiple deletions are preferred to both increase the carrying capacity of the vector and reduce the likelihood of recombination to generate replication competent adenovirus (RCA). Where the adenovirus contains multiple deletions, it is not necessary that each of the deletions, if present alone, would result in a replication defective and/or non-replicating adenovirus. As long as one of the deletions renders the adenovirus replication defective or non-replicating, the additional deletions may be included for other purposes, e.g., to increase the carrying capacity of the adenovirus genome for heterologous nucleotide sequences. Preferably, more than one of the deletions prevents the expression of a functional protein and renders the adenovirus replication defective and/or non-replicating and/or attenuated. More preferably, all of the deletions are deletions that would render the adenovirus replication-defective and/or non-replicating and/or attenuated. However, the invention also encompasses adenovirus and adenovirus vectors that are replication competent and/or wild-type, *i.e.* comprises all of the adenoviral genes necessary for infection and replication in a subject.

Embodiments of the invention employing adenovirus recombinants may include E1-defective or deleted, or E3-defective or deleted, or E4-defective or deleted or adenovirus vectors comprising deletions of E1 and E3, or E1 and E4, or E3 and E4, or E1, E3, and E4 deleted, or the "gutless" adenovirus vector in which all viral genes are deleted. The adenovirus vectors may comprise mutations in E1, E3, or E4 genes, or deletions in these or all adenoviral genes. The E1 mutation raises the safety margin of the vector because E1-defective adenovirus mutants are said to be replication-defective and/or non-replicating in non-permissive cells, and are, at the very least, highly attenuated. The E3 mutation enhances the immunogenicity of the antigen by disrupting the mechanism whereby adenovirus down-regulates MHC class I molecules. The E4 mutation reduces the immunogenicity of the adenovirus vector by suppressing the late gene expression, thus may allow repeated re-vaccination utilizing the same vector. The present invention comprehends adenovirus vectors of any serotype or serogroup that are deleted or mutated in E1, or E3, or E4, or E1 and E3, or E1 and E4. Deletion or mutation of these adenoviral genes result in impaired or substantially complete loss of activity of these proteins.

The "gutless" adenovirus vector is another type of vector in the adenovirus vector family. Its replication requires a helper virus and a special human 293 cell line expressing both E1a and Cre, a condition that does not exist in a natural environment; the vector is deprived of all viral genes, thus the vector as a vaccine carrier is non-immunogenic and may be inoculated multiple times for re-vaccination. The "gutless" adenovirus vector also contains 36 kb space for accommodating antigen or immunogen(s) of interest, thus allowing co-delivery of a large number of antigen or immunogens into cells.

Adeno-associated virus (AAV) is a single-stranded DNA parvovirus which is endogenous to the human population. Although capable of productive infection in cells from a variety of species, AAV is a dependovirus, requiring helper functions from either adenovirus or herpes virus for its own replication. In the absence of helper functions from either of these helper viruses, AAV will infect cells, uncoat in the nucleus, and integrate its genome into the host chromosome, but will not replicate or produce new viral particles.

The genome of AAV has been cloned into bacterial plasmids and is well characterized. The viral genome consists of 4682 bases which include two terminal repeats of 145 bases each. These terminal repeats serve as origins of DNA replication for the virus. Some investigators have also proposed that they have enhancer functions. The rest of the genome is divided into two functional domains. The left portion of the genome codes for the rep functions which regulate viral DNA replication and vital gene expression. The right side of the vital genome contains the cap genes that encode the structural capsid proteins VP1, VP2 and VP3. The proteins encoded by both the rep and cap genes function in trans during productive AAV replication.

AAV is considered an ideal candidate for use as a transducing vector, and it has been used in this manner. Such AAV transducing vectors comprise sufficient cis-acting functions to replicate in the presence of adenovirus or herpes virus helper functions provided in trans. Recombinant AAV (rAAV) have been constructed in a number of laboratories and have been used to carry exogenous genes into cells of a variety of lineages. In these vectors, the AAV cap and/or rep genes are deleted from the viral genome and replaced with a DNA segment of choice. Current vectors may accommodate up to 4300 bases of inserted DNA.

To produce rAAV, plasmids containing the desired vital construct are transfected into adenovirus-infected cells. In addition, a second helper plasmid is cotransfected into these cells to provide the AAV rep and cap genes which are obligatory for replication and packaging of the recombinant viral construct. Under these conditions, the rep and cap proteins of AAV act in trans to stimulate replication and packaging of the rAAV construct. Three days after transfection, rAAV is harvested from the cells along with adenovirus. The contaminating adenovirus is then inactivated by heat treatment.

Herpes Simplex Virus 1 (HSV-1) is an enveloped, double-stranded DNA virus with a genome of 153 kb encoding more than 80 genes. Its wide host range is due to the binding of viral envelope glycoproteins to the extracellular heparin sulphate molecules found in cell membranes (WuDunn & Spear, 1989). Internalization of the virus then requires envelope glycoprotein gD and fibroblast growth factor receptor (Kaner, 1990). HSV is able to infect cells lytically or may establish latency. HSV vectors have been used to infect a wide variety of cell types (Lowenstein, 1994; Huard, 1995; Miyanohara, 1992; Liu, 1996; Goya, 1998).

There are two types of HSV vectors, called the recombinant HSV vectors and the amplicon vectors. Recombinant HSV vectors are generated by the insertion of transcription units directly into the HSV genome, through homologous recombination events. The amplicon vectors are based on plasmids bearing the transcription unit of choice, an origin of replication, and a packaging signal.

HSV vectors have the obvious advantages of a large capacity for insertion of foreign genes, the capacity to establish latency in neurons, a wide host range, and the ability to confer transgene expression to the CNS for up to 18 months (Carpenter & Stevens, 1996).

Retroviruses are enveloped single-stranded RNA viruses, which have been widely used in gene transfer protocols. Retroviruses have a diploid genome of about 7-10 kb, composed of four gene regions termed *gag*, *pro*, *pol* and *env.* These gene regions encode for structural capsid proteins, viral protease, integrase and viral reverse transcriptase, and envelope glycoproteins, respectively. The genome also has a packaging signal and *cis*-acting sequences, termed long-terminal repeats (LTRs), at each end, which have a role in transcriptional control and integration.

The most commonly used retroviral vectors are based on the Moloney murine leukaemia virus (Mo-MLV) and have varying cellular tropisms, depending on the receptor binding surface domain of the envelope glycoprotein.

Recombinant retroviral vectors are deleted from all retroviral genes, which are replaced with marker or therapeutic genes, or both. To propagate recombinant retroviruses, it is necessary to provide the viral genes, *gag*, *pol* and *env* in *trans.*

Lentiviruses are complex retroviruses that have the ability to infect and express their genes in both mitotic and post-mitotic cells. The most commonly known lentivirus is the human immunodeficiency virus (HIV), which uses the envelope glycoproteins of other viruses to target a broad range of cell types.

Alphaviruses, including the prototype Sindbis virus (SIN), Semliki Forest virus (SFV), and Venezuelan equine encephalitis virus (VEE), constitute a group of enveloped viruses containing plus-stranded RNA genomes within icosahedral capsids.

The viral vectors of the present invention are useful for the delivery of nucleic acids expressing antigens or immunogens to cells both *in vitro* and *in vivo.* In particular, the inventive vectors may be advantageously employed to deliver or transfer nucleic acids to cells, more preferably mammalian cells. Nucleic acids of interest include nucleic acids encoding peptides and proteins, preferably therapeutic (e.g., for medical or veterinary uses) or immunogenic (e.g., for vaccines) peptides or proteins.

Preferably, the codons encoding the antigen or immunogen of interest are "optimized" codons, i.e., the codons are those that appear frequently in, e.g.., highly expressed genes in the subject's species, instead of those codons that are frequently used by, for example, an influenza virus. Such codon usage provides for efficient expression of the antigen or immunogen in animal cells. In other embodiments, for example, when the antigen or immunogen of interest is expressed in bacteria, yeast or another expression system, the codon usage pattern is altered to represent the codon bias for highly expressed genes in the organism in which the antigen or immunogen is being expressed. Codon usage patterns are known in the literature for highly expressed genes of many species (e.g., Nakamura et al., 1996; Wang et al., 1998; McEwan et al. 1998).

As a further alternative, the viral vectors may be used to infect a cell in culture to express a desired gene product, e.g., to produce a protein or peptide of interest. Preferably, the protein or peptide is secreted into the medium and may be purified therefrom using routine techniques known in the art. Signal peptide sequences that direct extracellular secretion of proteins are known in the art and nucleotide sequences encoding the same may be operably linked to the nucleotide sequence encoding the peptide or protein of interest by routine techniques known in the art. Alternatively, the cells may be lysed and the expressed recombinant protein may be purified from the cell lysate. Preferably, the cell is an animal cell, more preferably a mammalian cell. Also preferred are cells that are competent for transduction by particular viral vectors of interest. Such cells include PER.C6 cells, 911 cells, and HEK293 cells.

A culture medium for culturing host cells includes a medium commonly used for tissue culture, such as M199-earle base, Eagle MEM (E-MEM), Dulbecco MEM (DMEM), SC-UCM102, UP-SFM (GIBCO BRL), EX-CELL302 (Nichirei), EX-CELL293-S (Nichirei), TFBM-01 (Nichirei), ASF104, among others. Suitable culture media for specific cell types may be found at the American Type Culture Collection (ATCC) or the European Collection of Cell Cultures (ECACC). Culture media may be supplemented with amino acids such as L-glutamine, salts, anti-fungal or anti-bacterial agents such as Fungizone®, penicillin-streptomycin, animal serum, and the like. The cell culture medium may optionally be serum-free.

The present invention also relates to cell lines or hon-human transgenic animals expressing or overexpressing a system as claimed. Preferably the cell line or animal expresses or overexpresses one or more systems as claimed.

The transgenic animal is typically a vertebrate, more preferably a rodent, such as a rat or a mouse, but also includes other non-human mammals such asgoat, pig or cow etc.

Such non-human transgenic animals are useful as animal models of disease and in screening assays for new useful compounds. By specifically expressing one or more polypeptides, as defined above, the effect of such polypeptides on the development of disease may be studied. Furthermore, therapies including gene therapy and various drugs may be tested on transgenic animals. Methods for the production of transgenic animals are known in the art. For example, there are several possible routes for the introduction of genes into embryos. These include (i) direct transfection or retroviral infection of embryonic stem cells followed by introduction of these cells into an embryo at the blastocyst stage of development; (ii) retroviral infection of early embryos; and (iii) direct microinjection of DNA into zygotes or early embryo cells. The gene and/or transgene may also include genetic regulatory elements and/or structural elements known in the art. A type of target cell for transgene introduction is the embryonic stem cell (ES). ES cells may be obtained from pre-implantation embryos cultured *in vitro* and fused with embryos (Evans et al., 1981, Nature 292:154-156; Bradley et al., 1984, Nature 309:255-258; Gossler et al., 1986, Proc. Natl. Acad. Sci. USA 83:9065-9069; and Robertson et al., 1986 Nature 322:445-448). Transgenes may be efficiently introduced into the ES cells by a variety of standard techniques such as DNA transfection, microinjection, or by retrovirus-mediated transduction. The resultant transformed ES cells may thereafter be combined with blastocysts from a non-human animal. The introduced ES cells thereafter colonize the embryo and contribute to the germ line of the resulting chimeric animal (Jaenisch, 1988, Science 240: 1468-1474).

LITEs may also offer valuable temporal precision in vivo. LITEs may be used to alter gene expression during a particular stage of development, for example, by repressing a particular apoptosis gene only during a particular stage of C elegans growth. LITEs may be used to time a genetic cue to a particular experimental window. For example, genes implicated in learning may be overexpressed or repressed only during the learning stimulus in a precise region of the intact rodent or primate brain. Further, LITEs may be used to induce gene expression changes only during particular stages of disease development. For example, an oncogene may be overexpressed only once a tumor reaches a particular size or metastatic stage. Conversely, proteins suspected in the development of Alzheimer's may be knocked down only at defined time points in the animal's life and within a particular brain region. Although these examples do not exhaustively list the potential applications of the LITE system, they highlight some of the areas in which LITEs may be a powerful technology.

Therapeutic or diagnostic compositions of the disclosure are administered to an individual in amounts sufficient to treat or diagnose disorders. The effective amount may vary according to a variety of factors such as the individual's condition, weight, sex and age. Other factors include the mode of administration.

The pharmaceutical compositions may be provided to the individual by a variety of routes such as subcutaneous, topical, oral and intramuscular.

Compounds identified according to the methods disclosed herein may be used alone at appropriate dosages. Alternatively, co-administration or sequential administration of other agents may be desirable.

The present disclosure also has the objective of providing suitable topical, oral, systemic and parenteral pharmaceutical formulations for use in the novel methods of treatment of the present disclosure. The compositions containing compounds identified according to this invention as the active ingredient may be administered in a wide variety of therapeutic dosage forms in conventional vehicles for administration. For example, the compounds may be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts.

Advantageously, compounds of the present disclosure may be administered in a single daily dose, or the total daily dosage may be administered in divided doses of two, three or four times daily. Furthermore, compounds for the present disclosure may be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

For combination treatment with more than one active agent, where the active agents are in separate dosage formulations, the active agents may be administered concurrently, or they each may be administered at separately staggered times.

The dosage regimen utilizing the compounds of the present disclosure is selected in accordance with a variety of factors including type, species, age, weight, sex and medical condition of the patient; the severity of the condition to be treated; the route of administration; the renal, hepatic and cardiovascular function of the one patient; and the particular compound thereof employed. A physician of ordinary skill may readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

Although the present invention and its advantages have been described in detail, it should be understood that various changes, substitutions and alterations may be made herein without departing from the scope of the invention as defined in the appended claims.

The present invention will be further illustrated in the following Examples which are given for illustration purposes only and are not intended to limit the invention in any way.

### Examples

### Example 1

The ability to directly modulate gene expression from the endogenous mammalian genome is critical for elucidating normal gene function and disease mechanism. Advances that further refine the spatial and temporal control of gene expression within cell populations have the potential to expand the utility of gene modulation. Applicants previously developed transcription activator-like effectors (TALEs) from Xanthamonas oryze to enable the rapid design and construction of site-specific DNA binding proteins. Applicants developed a set of molecular tools for enabling light-regulated gene expression in the endogenous mammalian genome. The system consists of engineered artificial transcription factors linked to light-sensitive dimerizing protein domains from Arabidopsis thaliana. The system responds to light in the range of 450nm - 500nm and is capable of inducing a significant increase in the expression of pluripotency factors after stimulation with light at an intensity of 6.2 mW/cm² in mammalian cells. Applicants are developing tools for the targeting of a wide range of genes. Applicants believe that a toolbox for the light-mediated control of gene expression would complement the existing optogenetic methods and may in the future help elucidate the timing-, cell type- and concentrationdependent role of specific genes in the brain.

The ability to directly modulate gene expression from the endogenous mammalian genome is critical for elucidating normal gene function and disease mechanisms. Applicants present the development of a set of molecular tools for enabling light-regulated gene expression in the endogenous mammalian genome. This system consists of a transcription activator like effector (TALE) and the activation domain VP64 linked to the light-sensitive dimerizing protein domains cryptochrome 2 (CRY2) and CIB1 from Arabidopsis thaliana. Applicants show that blue-light stimulation of HEK293FT and Neuro-2a cells transfected with these LITE constructs designed to target the promoter region of KLF4 and Neurog2 results in a significant increase in target expression, demonstrating the functionality of TALE-based optical gene expression modulation technology.

**FIG. 1** shows a schematic depicting the need for spatial and temporal precision.

**FIG. 2** shows transcription activator like effectors (TALEs). TALEs consist of 34 aa repeats at the core of their sequence. Each repeat corresponds to a base in the target DNA that is bound by the TALE. Repeats differ only by 2 variable amino acids at positions 12 and 13. The code of this correspondence has been elucidated (Boch, J et al., Science, 2009 and Moscou, M et al. , Science, 2009) and is shown in this figure. Applicants developed a method for the synthesis of designer TALEs incorporating this code and capable of binding a sequence of choice within the genome (Zhang, F et al., Nature Biotechnology, 2011).

**FIG. 3** depicts a design of a LITE: TALE/Cryptochrome transcriptional activation. Each LITE is a two-component system which may comprise a TALE fused to CRY2 and the cryptochrome binding partner CIB1 fused to VP64, a transcription activor. In the inactive state, the TALE localizes its fused CRY2 domain to the promoter region of the gene of interest. At this point, CIB1 is unable to bind CRY2, leaving the CIB1-VP64 unbound in the nuclear space. Upon stimulation with 488 nm (blue) light, CRY2 undergoes a conformational change, revealing its CIB1 binding site (Liu, H et al. , Science, 2008). Rapid binding of CIB1 results in recruitment of the fused VP64 domain, which induces transcription of the target gene.

**FIG. 4** depicts effects of cryptochrome dimer truncations on LITE activity. Truncations known to alter the activity of CRY2 and CIB1 () were compared against the full length proteins. A LITE targeted to the promoter of Neurog2 was tested in Neuro-2a cells for each combination of domains. Following stimulation with 488 nm light, transcript levels of Neurog2 were quantified using qPCR for stimulated and unstimulated samples.

**FIG. 5** depicts a light-intensity dependent response of KLF4 LITE.

**FIG. 6** depicts activation kinetics of Neurog2 LITE and inactivation kinetics of Neurog2 LITE.

### Example 2

Normal gene expression is a dynamic process with carefully orchestrated temporal and spatial components, the precision of which are necessary for normal development, homeostasis, and advancement of the organism. In turn, the dysregulation of required gene expression patterns, either by increased, decreased, or altered function of a gene or set of genes, has been linked to a wide array of pathologies. Technologies capable of modulating gene expression in a spatiotemporally precise fashion will enable the elucidation of the genetic cues responsible for normal biological processes and disease mechanisms. To address this technological need, Applicants developed light-inducible transcriptional effectors (LITEs), which provide light-mediated control of endogenous gene expression.

Inducible gene expression systems have typically been designed to allow for chemically inducible activation of an inserted open reading frame or shRNA sequence, resulting in gene overexpression or repression, respectively. Disadvantages of using open reading frames for overexpression include loss of splice variation and limitation of gene size. Gene repression via RNA interference, despite its transformative power in human biology, may be hindered by complicated off-target effects. Certain inducible systems including estrogen, ecdysone, and FKBP12/FRAP based systems are known to activate off-target endogenous genes. The potentially deleterious effects of long-term antibiotic treatment may complicate the use of tetracycline transactivator (TET) based systems. *In vivo*, the temporal precision of these chemically inducible systems is dependent upon the kinetics of inducing agent uptake and elimination. Further, because inducing agents are generally delivered systemically, the spatial precision of such systems is bounded by the precision of exogenous vector delivery.

In response to these limitations, LITEs are designed to modulate expression of individual endogenous genes in a temporally and spatially precise manner. Each LITE is a two component system consisting of a customized DNA-binding transcription activator like effector (TALE) protein, a light-responsive crytochrome heterodimer from Arabadopsis thaliana, and a transcriptional activation/repression domain. The TALE is designed to bind to the promoter sequence of the gene of interest. The TALE protein is fused to one half of the cryptochrome heterodimer (cryptochrome-2 or CIB1), while the remaining cryptochrome partner is fused to a transcriptional effector domain. Effector domains may be either activators, such as VP16, VP64, or p65, or repressors, such as KRAB, EnR, or SID. In a LITE's unstimulated state, the TALE-cryptochrome2 protein localizes to the promoter of the gene of interest, but is not bound to the CIB1-effector protein. Upon stimulation of a LITE with blue spectrum light, cryptochrome-2 becomes activated, undergoes a conformational change, and reveals its binding domain. CIB1, in turn, binds to cryptochrome-2 resulting in localization of the effector domain to the promoter region of the gene of interest and initiating gene overexpression or silencing.

Gene targeting in a LITE is achieved via the specificity of customized TALE DNA binding proteins. A target sequence in the promoter region of the gene of interest is selected and a TALE customized to this sequence is designed. The central portion of the TALE consists of tandem repeats 34 amino acids in length. Although the sequences of these repeats are nearly identical, the 12th and 13th amino acids (termed repeat variable diresidues) of each repeat vary, determining the nucleotide-binding specificity of each repeat. Thus, by synthesizing a construct with the appropriate ordering of TALE monomer repeats, a DNA binding protein specific to the target promoter sequence is created.

Light responsiveness of a LITE is achieved via the activation and binding of cryptochrome-2 and CIB1. As mentioned above, blue light stimulation induces an activating conformational change in cryptochrome-2, resulting in recruitment of its binding partner CIB1. This binding is fast and reversible, achieving saturation in <15 sec following pulsed stimulation and returning to baseline <15 min after the end of stimulation. These rapid binding kinetics result in a LITE system temporally bound only by the speed of transcription/translation and transcript/protein degradation, rather than uptake and clearance of inducing agents. Crytochrome-2 activation is also highly sensitive, allowing for the use of low light intensity stimulation and mitigating the risks of phototoxicity. Further, in a context such as the intact mammalian brain, variable light intensity may be used to control the size of a LITE stimulated region, allowing for greater precision than vector delivery alone may offer.

The modularity of the LITE system allows for any number of effector domains to be employed for transcriptional modulation. Thus, activator and repressor domains may be selected on the basis of species, strength, mechanism, duration, size, or any number of other parameters.

Applicants next present two prototypical manifestations of the LITE system. The first example is a LITE designed to activate transcription of the mouse gene NEUROG2. The sequence TGAATGATGATAATACGA, located in the upstream promoter region of mouse NEUROG2, was selected as the target and a TALE was designed and synthesized to match this sequence. The TALE sequence was linked to the sequence for cryptochrome-2 via a nuclear localization signal (amino acids: SPKKKRKVEAS) to facilitate transport of the protein from the cytosol to the nuclear space. A second vector was synthesized comprising the CIB1 domain linked to the transcriptional activator domain VP64 using the same nuclear localization signal. This second vector, also a GFP sequence, is separated from the CIB1-VP64 fusion sequence by a 2A translational skip signal. Expression of each construct was driven by a ubiquitous, constitutive promoter (CMV or EF1-α). Mouse neuroblastoma cells from the Neuro 2A cell line were co-transfected with the two vectors. After incubation to allow for vector expression, samples were stimulated by periodic pulsed blue light from an array of 488 nm LEDs. Unstimulated co-tranfected samples and samples transfected only with the fluorescent reporter YFP were used as controls. At the end of each experiment, mRNA was purified from the samples analyzed via qPCR.

Truncated versions of cryptochrome-2 and CIB1 were cloned and tested in combination with the full-length versions of cryptochrome-2 and CIB1 in order to determine the effectiveness of each heterodimer pair. The combination of the CRY2PHR domain, consisting of the conserved photoresponsive region of the cryptochrome-2 protein, and the full-length version of CIB1 resulted in the highest upregulation of Neurog2 mRNA levels (∼22 fold over YFP samples and ∼7 fold over unstimulated co-transfected samples). The combination of full-length cryptochrome-2 (CRY2) with full-length CIB1 resulted in a lower absolute activation level (∼4.6 fold over YFP), but also a lower baseline activation (∼1.6 fold over YFP for unstimulated co-transfected samples). These cryptochrome protein pairings may be selected for particular uses depending on absolute level of induction required and the necessity to minimize baseline "leakiness" of the LITE system.

Speed of activation and reversibility are critical design parameters for the LITE system. To characterize the kinetics of the LITE system, constructs consisting of the Neurog2 TALE-CRY2PHR and CIB1-VP64 version of the system were tested to determine its activation and inactivation speed. Samples were stimulated for as little as 0.5 h to as long as 24 h before extraction. Upregulation of Neurog2 expression was observed at the shortest, 0.5 h, time point (∼5 fold vs YFP samples). Neurog2 expression peaked at 12 h of stimulation (∼19 fold vs YFP samples). Inactivation kinetics were analyzed by stimulating co-transfected samples for 6 h, at which time stimulation was stopped, and samples were kept in culture for 0 to 12 h to allow for mRNA degradation. Neurog2 mRNA levels peaked at 0.5h after the end of stimulation (∼16 fold vs. YFP samples), after which the levels degraded with an ∼3 h half-life before returning to near baseline levels by 12 h.

The second prototypical example is a LITE designed to activate transcription of the human gene KLF4. The sequence TTCTTACTTATAAC, located in the upstream promoter region of human KLF4, was selected as the target and a TALE was designed and synthesized to match this sequence. The TALE sequence was linked to the sequence for CRY2PHR via a nuclear localization signal (amino acids: SPKKKRKVEAS). The identical CIB1-VP64 activator protein described above was also used in this manifestation of the LITE system. Human embryonal kidney cells from the HEK293FT cell line were co-transfected with the two vectors. After incubation to allow for vector expression, samples were stimulated by periodic pulsed blue light from an array of 488 nm LEDs. Unstimulated co-tranfected samples and samples transfected only with the fluorescent reporter YFP were used as controls. At the end of each experiment, mRNA was purified from the samples analyzed via qPCR.

The light-intensity response of the LITE system was tested by stimulating samples with increased light power (0-9 mW/cm²). Upregulation of KLF4 mRNA levels was observed for stimulation as low as 0.2 mW/cm². KLF4 upregulation became saturated at 5 mW/cm² (2.3 fold vs. YFP samples). Cell viability tests were also performed for powers up to 9 mW/cm² and showed >98% cell viability. Similarly, the KLF4 LITE response to varying duty cycles of stimulation was tested (1.6-100%). No difference in KLF4 activation was observed between different duty cycles indicating that a stimulation paradigm of as low as 0.25 sec every 15 sec should result in maximal activation.

There are potential applications for which LITEs represent an advantageous choice for gene expression control. There exist a number of *in vitro* applications for which LITEs are particularly attractive. In all these cases, LITEs have the advantage of inducing endogenous gene expression with the potential for correct splice variant expression.

Because LITE activation is photoinducible, spatially defined light patterns, created via masking or rasterized laser scanning, may be used to alter expression levels in a confined subset of cells. For example, by overexpressing or silencing an intercellular signaling molecule only in a spatially constrained set of cells, the response of nearby cells relative to their distance from the stimulation site may help elucidate the spatial characteristics of cell non-autonomous processes. Additionally, recent advances in cell reprogramming biology have shown that overexpression of sets of transcription factors may be utilized to transform one cell type, such as fibroblasts, into another cell type, such as neurons or cardiomyocytes. Further, the correct spatial distribution of cell types within tissues is critical for proper organotypic function. Overexpression of reprogramming factors using LITEs may be employed to reprogram multiple cell lineages in a spatially precise manner for tissue engineering applications.

The rapid transcriptional response and endogenous targeting of LITEs make for an ideal system for the study of transcriptional dynamics. For example, LITEs may be used to study the dynamics of mRNA splice variant production upon induced expression of a target gene. On the other end of the transcription cycle, mRNA degradation studies are often performed in response to a strong extracellular stimulus, causing expression level changes in a plethora of genes. LITEs may be utilized to reversibly induce transcription of an endogenous target, after which point stimulation may be stopped and the degradation kinetics of the unique target may be tracked.

The temporal precision of LITEs may provide the power to time genetic regulation in concert with experimental interventions. For example, targets with suspected involvement in long-term potentiation (LTP) may be modulated in organotypic or dissociated neuronal cultures, but only during stimulus to induce LTP, so as to avoid interfering with the normal development of the cells. Similarly, in cellular models exhibiting disease phenotypes, targets suspected to be involved in the effectiveness of a particular therapy may be modulated only during treatment. Conversely, genetic targets may be modulated only during a pathological stimulus. Any number of experiments in which timing of genetic cues to external experimental stimuli is of relevance may potentially benefit from the utility of LITE modulation.

The *in vivo* context offers equally rich opportunities for the use of LITEs to control gene expression. As mentioned above, photoinducibility provides the potential for previously unachievable spatial precision. Taking advantage of the development of optrode technology, a stimulating fiber optic lead may be placed in a precise brain region. Stimulation region size may then be tuned by light intensity. This may be done in conjunction with the delivery of LITEs via viral vectors, or, if transgenic LITE animals were to be made available, may eliminate the use of viruses while still allowing for the modulation of gene expression in precise brain regions. LITEs may be used in a transparent organism, such as an immobilized zebrafish, to allow for extremely precise laser induced local gene expression changes.

LITEs may also offer valuable temporal precision *in vivo.* LITEs may be used to alter gene expression during a particular stage of development, for example, by repressing a particular apoptosis gene only during a particular stage of C elegans growth. LITEs may be used to time a genetic cue to a particular experimental window. For example, genes implicated in learning may be overexpressed or repressed only during the learning stimulus in a precise region of the intact rodent or primate brain. Further, LITEs may be used to induce gene expression changes only during particular stages of disease development. For example, an oncogene may be overexpressed only once a tumor reaches a particular size or metastatic stage. Conversely, proteins suspected in the development of Alzheimer's may be knocked down only at defined time points in the animal's life and within a particular brain region. Although these examples do not exhaustively list the potential applications of the LITE system, they highlight some of the areas in which LITEs may be a powerful technology.

### Example 3

### Development of mammalian TALE transcriptional repressors

Applicants developed mammalian TALE repressor architectures to enable researchers to suppress transcription of endogenous genes. TALE repressors have the potential to suppress the expression of genes as well as non-coding transcripts such as microRNAs, rendering them a highly desirable tool for testing the causal role of specific genetic elements. In order to identify a suitable repression domain for use with TALEs in mammalian cells, a TALE targeting the promoter of the human *SOX2* gene was used to evaluate the transcriptional repression activity of a collection of candidate repression domains (**FIG. 12a**). Repression domains across a range of eukaryotic host species were selected to increase the chance of finding a potent synthetic repressor, including the PIE-1 repression domain (PIE-1) (Batchelder, C. et al. Transcriptional repression by the Caenorhabditis elegans germ-line protein PIE-1. Genes Dev. 13, 202-212 (1999)) from *Caenorhabditis elegans*, the QA domain within the Ubx gene (Ubx-QA) (Tour, E., Hittinger, C.T. & McGinnis, W. Evolutionarily conserved domains required for activation and repression functions of the Drosophila Hox protein Ultrabithorax. Development 132, 5271-5281 (2005)) from *Drosophila melanogaster*, the IAA28 repression domain (IAA28-RD)(4) from *Arabidopsis thaliana,* the mSin interaction domain (SID) (Ayer, D.E., Laherty, C.D., Lawrence, Q.A., Armstrong, A.P. & Eisenman, R.N. Mad proteins contain a dominant transcription repression domain. Mol. Cell. Biol. 16, 5772-5781 (1996)), Tbx3 repression domain (Tbx3-RD), and the Krüppel-associated box (KRAB) (Margolin, J.F. et al. Kruppel-associated boxes are potent transcriptional repression domains. Proc. Natl. Acad. Sci. U S A 91, 4509-4513 (1994)) repression domain from *Homo Sapiens.* Since different truncations of KRAB have been known to exhibit varying levels of transcriptional repression (Margolin, J.F. et al. Kruppel-associated boxes are potent transcriptional repression domains. Proc. Natl. Acad. Sci. U S A 91, 4509-4513 (1994)), three different truncations of KRAB were tested (**FIG. 12c**). These candidate TALE repressors were expressed in HEK 293FTcells and it was found that TALEs carrying two widely used mammalian transcriptional repression domains, the SID (Ayer, D.E., Laherty, C.D., Lawrence, Q.A., Armstrong, A.P. & Eisenman, R.N. Mad proteins contain a dominant transcription repression domain. Mol. Cell. Biol. 16, 5772-5781 (1996)) and KRAB (Margolin, J.F. et al. Kruppel-associated boxes are potent transcriptional repression domains. Proc. Natl. Acad. Sci. U S A 91, 4509-4513 (1994)) domains, were able to repress endogenous *SOX2* expression, while the other domains had little effect on transcriptional activity (**FIG. 12c**). To control for potential perturbation of SOX2 transcription due to TALE binding, expression of the SOX2-targeting TALE DNA binding domain alone without any effector domain had no effect (similar to mock or expression of GFP) on the transcriptional activity of SOX2 (**FIG. 12c**, Null condition). Since the SID domain was able to achieve 26% more transcriptional repression of the endogenous *SOX2* locus than the KRAB domain (**FIG. 12c**), it was decided to use the SID domain for subsequent studies.

To further test the effectiveness of the SID repressor domain for down regulating endogenous transcription, SID was combined with CACNA1C-target TALEs from the previous experiment (**FIG. 12d**). Using qRT-PCR, it was found that replacement of the VP64 domain on CACNA1C-targeting TALEs with SID was able to repress CACNA1C transcription. The NH-containing TALE repressor was able to achieve a similar level of transcriptional repression as the NN-containing TALE (∼4 fold repression), while the TALE repressor using NK was significantly less active (∼2 fold repression) (**FIG. 12d**). These data demonstrate that SID is indeed a suitable repression domain, while also further supporting NH as a more suitable G-targeting RVD than NK.

TALEs may be easily customized to recognize specific sequences on the endogenous genome. Here, a series of screens were conducted to address two important limitations of the TALE toolbox. Together, the identification of a more stringent G-specific RVD with uncompromised activity strength as well as a robust TALE repressor architecture further expands the utility of TALEs for probing mammalian transcription and genome function.

After identifying SID (mSin interaction domain) as a robust novel repressor domain to be used with TALEs, more active repression domain architecture based on SID domain for use with TALEs in mammalian cells were further designed and verified. This domain is called SID4X, which is a tandem repeat of four SID domains linked by short peptide linkers. For testing different TALE repressor architectures, a TALE targeting the promoter of the mouse (*Mus musculus) p11* (*s100a10*) gene was used to evaluate the transcriptional repression activity of a series of candidate TALE repressor architectures (**FIG. 13a**). Since different truncations of TALE are known to exhibit varying levels of transcriptional activation activity, two different truncations of TALE fused to SID or SID4X domain were tested, one version with 136 and 183 amino acids at N- and C- termini flanking the DNA binding tandem repeats, with another one retaining 240 and 183 amino acids at N- and C-termini (**FIG. 13b****, c**). The candidate TALE repressors were expressed in mouse Neuro2A cells and it was found that TALEs carrying both SID and SID4X domains were able to repress endogenous *p11* expression up to 4.8 folds, while the GFP-encoding negative control construct had no effect on transcriptional of target gene (**FIG. 13b****, c**). To control for potential perturbation of *p11* transcription due to TALE binding, expression of the *p11*-targeting TALE DNA binding domain (with the same N- and C- termini truncations as the tested constructs) without any effector domain had no effect on the transcriptional activity of endogenous *p11* **(****FIG. 13b, c**, null constructs).

Because the constructs harboring SID4X domain were able to achieve 167% and 66% more transcriptional repression of the endogenous *p11* locus than the SID domain depending on the truncations of TALE DNA binding domain (**FIG. 13c**), it was concluded that a truncated TALE DNA binding domain, bearing 136 and 183 amino acids at N- and C- termini respectively, fused to the SID4X domain is a potent TALE repressor architecture that enables down-regulation of target gene expression and is more active than the previous design employing SID domain.

The mSin interaction domain (SID) and SID4X domain were codon optimized for mammalian expression and synthesized with flanking *Nhe*I and *Xba*I restriction sites (Genscript). Truncation variants of the TALE DNA binding domains are PCR amplified and fused to the SID or the SID4X domain using *Nhe*I and *Xba*I restriction sites. To control for any effect on transcription resulting from TALE binding, expression vectors carrying the TALE DNA binding domain alone using PCR cloning were constructed. The coding regions of all constructs were completely verified using Sanger sequencing. A comparison of two different types of TALE architecture is seen in **FIG. 14****.**

### Example 4

### Development of mammalian TALE transcriptional activators and nucleases

Customized TALEs may be used for a wide variety of genome engineering applications, including transcriptional modulation and genome editing. Here, Applicants describe a toolbox for rapid construction of custom TALE transcription factors (TALE-TFs) and nucleases (TALENs) using a hierarchical ligation procedure. This toolbox facilitates affordable and rapid construction of custom TALE-TFs and TALENs within 1 week and may be easily scaled up to construct TALEs for multiple targets in parallel. Applicants also provide details for testing the activity in mammalian cells of custom TALE-TFs and TALENs using quantitative reverse-transcription PCR and Surveyor nuclease, respectively. The TALE toolbox will enable a broad range of biological applications.
TALEs are natural bacterial effector proteins used by *Xanthomonas sp.* to modulate gene transcription in host plants to facilitate bacterial colonization (Boch, J. & Bonas, U. Xanthomonas AvrBs3 family-type III effectors: discovery and function. Annu. Rev. Phytopathol. 48, 419-436 (2010) and Bogdanove, A.J., Schornack, S. & Lahaye, T. TAL effectors: finding plant genes for disease and defense. Curr. Opin. Plant Biol. 13, 394-401 (2010)). The central region of the protein contains tandem repeats of 34-aa sequences (termed monomers) that are required for DNA recognition and binding (Romer, P. et al. Plant pathogen recognition mediated by promoter activation of the pepper Bs3 resistance gene. Science 318, 645-648 (2007); Kay, S., Hahn, S., Marois, E., Hause, G. & Bonas, U. A bacterial effector acts as a plant transcription factor and induces a cell size regulator. Science 318, 648-651 (2007); Kay, S., Hahn, S., Marois, E., Wieduwild, R. & Bonas, U. Detailed analysis of the DNA recognition motifs of the Xanthomonas type III effectors AvrBs3 and AvrBs3Deltarep16. Plant J. 59, 859-871 (2009) and Romer, P. et al. Recognition of AvrBs3-like proteins is mediated by specific binding to promoters of matching pepper Bs3 alleles. Plant Physiol. 150, 1697-1712 (2009).) (**FIG. 8**). Naturally occurring TALEs have been found to have a variable number of monomers, ranging from 1.5 to 33.5 (Boch, J. & Bonas, U. Xanthomonas AvrBs3 family-type III effectors: discovery and function. Annu. Rev. Phytopathol. 48, 419-436 (2010)). Although the sequence of each monomer is highly conserved, they differ primarily in two positions termed the repeat variable diresidues (RVDs, 12th and 13th positions). Recent reports have found that the identity of these two residues determines the nucleotide-binding specificity of each TALE repeat and that a simple cipher specifies the target base of each RVD (NI = A, HD = C, NG = T, NN = G or A) (Boch, J. et al. Breaking the code of DNA binding specificity of TAL-type III effectors. Science 326, 1509-1512 (2009) and Moscou, M.J. & Bogdanove, A.J. A simple cipher governs DNA recognition by TAL effectors. Science 326, 1501 (2009)). Thus, each monomer targets one nucleotide and the linear sequence of monomers in a TALE specifies the target DNA sequence in the 5' to 3' orientation. The natural TALE-binding sites within plant genomes always begin with a thymine (Boch, J. et al. Breaking the code of DNA binding specificity of TAL-type III effectors. Science 326, 1509-1512 (2009) and Moscou, M.J. & Bogdanove, A.J. A simple cipher governs DNA recognition by TAL effectors. Science 326, 1501 (2009)), which is presumably specified by a cryptic signal within the nonrepetitive N terminus of TALEs. The tandem repeat DNA-binding domain always ends with a half-length repeat (0.5 repeat, **FIG. 8**). Therefore, the length of the DNA sequence being targeted is equal to the number of full repeat monomers plus two.

In plants, pathogens are often host-specific. For example, Fusarium oxysporum f. sp. lycopersici causes tomato wilt but attacks only tomato, and F. oxysporum f. dianthii Puccinia graminis f. sp. tritici attacks only wheat. Plants have existing and induced defenses to resist most pathogens. Mutations and recombination events across plant generations lead to genetic variability that gives rise to susceptibility, especially as pathogens reproduce with more frequency than plants. In plants there can be non-host resistance, e.g., the host and pathogen are incompatible. There can also be Horizontal Resistance, e.g., partial resistance against all races of a pathogen, typically controlled by many genes and Vertical Resistance, e.g., complete resistance to some races of a pathogen but not to other races, typically controlled by a few genes. In a Gene-for-Gene level, plants and pathogens evolve together, and the genetic changes in one balance changes in other. Accordingly, using Natural Variability, breeders combine most useful genes for Yield, Quality, Uniformity, Hardiness, Resistance. The sources of resistance genes include native or foreign Varieties, Heirloom Varieties, Wild Plant Relatives, and Induced Mutations, e.g., treating plant material with mutagenic agents. Using the present invention, plant breeders are provided with a new tool to induce mutations. Accordingly, one skilled in the art can analyze the genome of sources of resistance genes, and in Varieties having desired characteristics or traits employ the present invention to induce the rise of resistance genes, with more precision than previous mutagenic agents and hence accelerate and improve plant breeding programs.

Applicants have further improved the TALE assembly system with a few optimizations, including maximizing the dissimilarity of ligation adaptors to minimize misligations and combining separate digest and ligation steps into single Golden Gate (Engler, C., Kandzia, R. & Marillonnet, S. A one pot, one step, precision cloning method with high throughput capability. PLoS ONE 3, e3647 (2008); Engler, C., Gruetzner, R., Kandzia, R. & Marillonnet, S. Golden gate shuffling: a one-pot DNA shuffling method based on type IIs restriction enzymes. PLoS ONE 4, e5553 (2009) and Weber, E., Engler, C., Gruetzner, R., Werner, S. & Marillonnet, S. A modular cloning system for standardized assembly of multigene constructs. PLoS ONE 6, e16765 (2011)) reactions. Briefly, each nucleotide-specific monomer sequence is amplified with ligation adaptors that uniquely specify the monomer position within the TALE tandem repeats. Once this monomer library is produced, it may conveniently be reused for the assembly of many TALEs. For each TALE desired, the appropriate monomers are first ligated into hexamers, which are then amplified via PCR. Then, a second Golden Gate digestion-ligation with the appropriate TALE cloning backbone (**FIG. 8**) yields a fully assembled, sequence-specific TALE. The backbone contains a *ccdB* negative selection cassette flanked by the TALE N and C termini, which is replaced by the tandem repeat DNA-binding domain when the TALE has been successfully constructed. *ccdB* selects against cells transformed with an empty backbone, thereby yielding clones with tandem repeats inserted (Cermak, T. et al. Efficient design and assembly of custom TALEN and other TAL effector-based constructs for DNA targeting. Nucleic Acids Res. 39, e82 (2011)).

Assemblies of monomeric DNA-binding domains may be inserted into the appropriate TALE-TF or TALEN cloning backbones to construct customized TALE-TFs and TALENs. TALE-TFs are constructed by replacing the natural activation domain within the TALE C terminus with the synthetic transcription activation domain VP64 (Zhang, F. et al. Efficient construction of sequence-specific TAL effectors for modulating mammalian transcription. Nat. Biotechnol. 29, 149-153 (2011); **FIG. 8**). By targeting a binding site upstream of the transcription start site, TALE-TFs recruit the transcription complex in a site-specific manner and initiate gene transcription. TALENs are constructed by fusing a C-terminal truncation (+63 aa) of the TALE DNA-binding domain (Miller, J.C. et al. A TALE nuclease architecture for efficient genome editing. Nat. Biotechnol. 29, 143-148 (2011)) with the nonspecific FokI endonuclease catalytic domain (**FIG. 14**). The +63-aa C-terminal truncation has also been shown to function as the minimal C terminus sufficient for transcriptional modulation (Zhang, F. et al. Efficient construction of sequence-specific TAL effectors for modulating mammalian transcription. Nat. Biotechnol. 29, 149-153 (2011)). TALENs form dimers through binding to two target sequences separated by -17 bases. Between the pair of binding sites, the FokI catalytic domains dimerize and function as molecular scissors by introducing double-strand breaks (DSBs; **FIG. 8**). Normally, DSBs are repaired by the nonhomologous end-joining (Huertas, P. DNA resection in eukaryotes: deciding how to fix the break. Nat. Struct. Mol. Biol. 17, 11-16 (2010)) pathway (NHEJ), resulting in small deletions and functional gene knockout. Alternatively, TALEN-mediated DSBs may stimulate homologous recombination, enabling site-specific insertion of an exogenous donor DNA template (Miller, J.C. et al. A TALE nuclease architecture for efficient genome editing. Nat. Biotechnol. 29, 143-148 (2011) and Hockemeyer, D. et al. Genetic engineering of human pluripotent cells using TALE nucleases. Nat. Biotechnol. 29, 731-734 (2011)).

Along with the TALE-TFs being constructed with the VP64 activation domain, other embodiments of the invention relate to TALE polypeptides being constructed with the VP16 and p65 activation domains. A graphical comparison of the effect these different activation domains have on Sox2 mRNA level is provided in **FIG. 11****.**

### Example 5

**FIG. 17** depicts an effect of cryptochrome2 heterodimer orientation on LITE functionality. Two versions of the Neurogenin 2 (Neurog2) LITE were synthesized to investigate the effects of cryptochrome 2 photolyase homology region (CRY2PHR)/calcium and integrin-binding protein 1 (CIB1) dimer orientation. In one version, the CIB1 domain was fused to the C-terminus of the TALE (Neurog2) domain, while the CRY2PHR domain was fused to the N-terminus of the VP64 domain. In the converse version, the CRY2PHR domain was fused to the C-terminus of the TALE (Neurog2) domain, while the CIB1 domain was fused to the N-terminus of the VP64 domain. Each set of plasmids were transfected in Neuro2a cells and stimulated (466 nm, 5 mW/cm², 1 sec pulse per 15 sec, 12 h) before harvesting for qPCR analysis. Stimulated LITE and unstimulated LITE Neurog2 expression levels were normalized to Neurog2 levels from stimulated GFP control samples. The TALE-CRY2PHR/CIB1-VP64 LITE exhibited elevated basal activity and higher light induced Neurog2 expression, and suggested its suitability for situations in which higher absolute activation is required. Although the relative light inducible activity of the TALE-CIB1/CRY2PHR-VP64 LITE was lower that its counterpart, the lower basal activity suggested its utility in applications requiring minimal baseline activation. Further, the TALE-CIB1 construct was smaller in size, compared to the TALE-CRY2PHR construct, a potential advantage for applications such as viral packaging.

**FIG. 18** depicts metabotropic glutamate receptor 2 (mGlur2) LITE activity in mouse cortical neuron culture. A mGluR2 targeting LITE was constructed via the plasmids pAAV-human Synapsin I promoter (hSyn)-HA-TALE(mGluR2)-CIB1 and pAAV-hSyn-CRY2PHR-VP64-2A-GFP. These fusion constructs were then packaged into adeno associated viral vectors (AAV). Additionally, AAV carrying hSyn-TALE-VP64-2A-GFP and GFP only were produced. Embryonic mouse (E16) cortical cultures were plated on Poly-L-lysine coated 24 well plates. After 5 days in vitro neural cultures were co-trasnduced with a mixture of TALE(mGluR2)-CIB1 and CRY2PHR-VP64 AAV stocks. Control samples were transduced with either TALE(mGluR2)-VP64 AAV or GFP AAV. 6 days after AAV transduction, experimental samples were stimulated using either of two light pulsing paradigms: 0.5 s per min and 0.25 sec per 30 sec. Neurons were stimulated for 24 h and harvested for qPCR analysis. All mGluR2 expression levels were normalized to the respective stimulated GFP control. The data suggested that the LITE system could be used to induce the light-dependent activation of a target gene in primary neuron cultures in vitro.

**FIG. 19** depicts transduction of primary mouse neurons with LITE AAV vectors. Primary mouse cortical neuron cultures were co-transduced at 5 days in vitro with AAV vectors encoding hSyn-CRY2PHR-VP64-2A-GFP and hSyn-HA-TALE-CIB1, the two components of the LITE system. Left panel: at 6 days after transduction, neural cultures exhibited high expression of GFP from the hSyn-CRY2PHR-VP64-2A-GFP vector. Right panel: Co-transduced neuron cultures were fixed and stained with an antibody specific to the HA epitope on the N-terminus of the TALE domain in hSyn-HA-TALE-CIB1. Red signal indicated HA expression, with particularly strong nuclear signal (DNA stained by DAPI in blue channel). Together these images suggested that the expression of each LITE component could be achieved in primary mouse neuron cultures. (scale bars=50 um).

**FIG. 20** depicts expression of a LITE component in vivo. An AAV vector of seratype 1/2 carrying hSyn-CRY2PHR-VP64 was produced via transfection of HEK293FT cells and purified via heparin column binding. The vector was concentrated for injection into the intact mouse brain. 1 uL of purified AAV stock was injected into the hippocampus and infralimbic cortex of an 8 week old male C57BL/6 mouse by steroeotaxic surgery and injection. 7 days after in vivo transduction, the mouse was euthanized and the brain tissue was fixed by paraformaldehyde perfusion. Slices of the brain were prepared on a vibratome and mounted for imaging. Strong and widespread GFP signals in the hippocampus and infralimbic cortex suggested efficient transduction and high expression of the LITE component CRY2PHR-VP64.

### Example 6

### Improved design by using NES element

Estrogen receptor T2 (ERT2) has a leakage issue. The ERT2 domain would enter the nucleus even in the absence of 4-Hydroxytestosterone (4OHT), leading to a background level of activation of target gene by TAL. NES (nuclear exporting signal) is a peptide signal that targets a protein to the cytoplasm of a living cell. By adding NES to an existing construct, Applicants aim to prevent the entering of ERT2-TAL protein into nucleus in the absence of 4OHT, lowering the background activation level due to the "leakage" of the ERT2 domain.

**FIG. 21** depicts an improved design of the construct where the specific NES peptide sequence used is LDLASLIL.

FIG. 22 depicts Sox2 mRNA levels in the absence and presence of 4OH tamoxifen. Y-axis is Sox2 mRNA level as measured by qRT-PCR. X-axis is a panel of different construct designs described on top. Plus and minus signs indicate the presence or absence of 0.5uM 4OHT.

### Example 7 (disclosure)

### Multiplex Genome Engineering Using CRISPR/Cas Systems

Functional elucidation of causal genetic variants and elements requires precise genome editing technologies. The type II prokaryotic CRISPR (clustered regularly interspaced short palindromic repeats) adaptive immune system has been shown to facilitate RNA-guided site-specific DNA cleavage. Applicants engineered two different type II CRISPR systems and demonstrate that Cas9 nucleases can be directed by short RNAs to induce precise cleavage at endogenous genomic loci in human and mouse cells. Cas9 can also be converted into a nicking enzyme to facilitate homology-directed repair with minimal mutagenic activity. Finally, multiple guide sequences can be encoded into a single CRISPR array to enable simultaneous editing of several sites within the mammalian genome, demonstrating easy programmability and wide applicability of the CRISPR technology.

Prokaryotic CRISPR adaptive immune systems can be reconstituted and engineered to mediate multiplex genome editing in mammalian cells.

Precise and efficient genome targeting technologies are needed to enable systematic reverse engineering of causal genetic variations by allowing selective perturbation of individual genetic elements. Although genome-editing technologies such as designer zinc fingers (ZFs) (M. H. Porteus, D. Baltimore, Chimeric nucleases stimulate gene targeting in human cells. Science 300, 763 (May 2, 2003); J. C. Miller et al., An improved zinc-finger nuclease architecture for highly specific genome editing. Nat Biotechnol 25, 778 (Jul, 2007); J. D. Sander et al., Selection-free zinc-finger-nuclease engineering by context-dependent assembly (CoDA). Nat Methods 8, 67 (Jan, 2011) and A. J. Wood et al., Targeted genome editing across species using ZFNs and TALENs. Science 333, 307 (Jul 15, 2011)), transcription activator-like effectors (TALEs) (A. J. Wood et al., Targeted genome editing across species using ZFNs and TALENs. Science 333, 307 (Jul 15, 2011); M. Christian et al., Targeting DNA double-strand breaks with TAL effector nucleases. Genetics 186, 757 (Oct, 2010); F. Zhang et al., Efficient construction of sequence-specific TAL effectors for modulating mammalian transcription. Nat Biotechnol 29, 149 (Feb, 2011); J. C. Miller et al., A TALE nuclease architecture for efficient genome editing. Nat Biotechnol 29, 143 (Feb, 2011); D. Reyon et al., FLASH assembly of TALENs for high-throughput genome editing. Nat Biotechnol 30, 460 (May, 2012); J. Boch et al., Breaking the code of DNA binding specificity of TAL-type III effectors. Science 326, 1509 (Dec 11, 2009) and M. J. Moscou, A. J. Bogdanove, A simple cipher governs DNA recognition by TAL effectors. Science 326, 1501 (Dec 11, 2009)), and homing meganucleases (B. L. Stoddard, Homing endonuclease structure and function. Quarterly reviews of biophysics 38, 49 (Feb, 2005)) have begun to enable targeted genome modifications, there remains a need for new technologies that are scalable, affordable, and easy to engineer. Here, Applicants report the development of a new class of precision genome engineering tools based on the RNA-guided Cas9 nuclease (M. Jinek et al., A programmable dual-RNA-guided DNA endonuclease in adaptive bacterial immunity. Science 337, 816 (Aug 17, 2012); G. Gasiunas, R. Barrangou, P. Horvath, V. Siksnys, Cas9-crRNA ribonucleoprotein complex mediates specific DNA cleavage for adaptive immunity in bacteria. Proc Natl Acad Sci U S A 109, E2579 (Sep 25, 2012) and J. E. Garneau et al., The CRISPR/Cas bacterial immune system cleaves bacteriophage and plasmid DNA. Nature 468, 67 (Nov 4, 2010)) from the type II prokaryotic CRISPR adaptive immune system (H. Deveau, J. E. Garneau, S. Moineau, CRISPR/Cas system and its role in phage-bacteria interactions. Annual review of microbiology 64, 475 (2010); P. Horvath, R. Barrangou, CRISPR/Cas, the immune system of bacteria and archaea. Science 327, 167 (Jan 8, 2010); K. S. Makarova et al., Evolution and classification of the CRISPR-Cas systems. Nat Rev Microbiol 9, 467 (Jun, 2011) and D. Bhaya, M. Davison, R. Barrangou, CRISPR-Cas systems in bacteria and archaea: versatile small RNAs for adaptive defense and regulation. Annu Rev Genet 45, 273 (2011)).

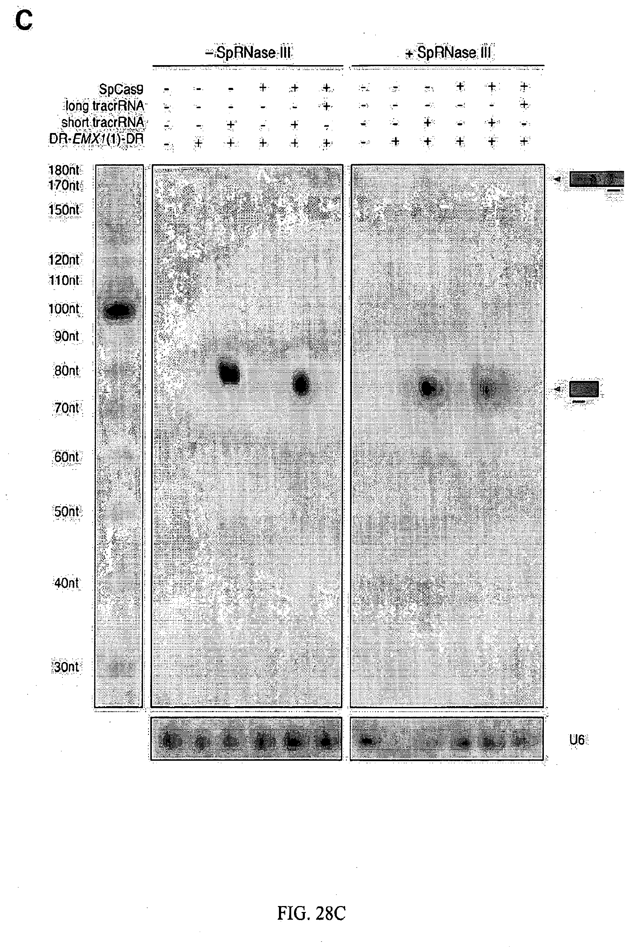

The *Streptococcus pyogenes* SF370 type II CRISPR locus consists of four genes, including the Cas9 nuclease, as well as two non-coding RNAs: tracrRNA and a pre-crRNA array containing nuclease guide sequences (spacers) interspaced by identical direct repeats (DRs) (FIG. 27) (E. Deltcheva et al., CRISPR RNA maturation by trans-encoded small RNA and host factor RNase III. Nature 471, 602 (Mar 31, 2011)). Applicants sought to harness this prokaryotic RNA-programmable nuclease system to introduce targeted double stranded breaks (DSBs) in mammalian chromosomes through heterologous expression of the key components. It has been previously shown that expression of tracrRNA, pre-crRNA, host factor RNase III, and Cas9 nuclease are necessary and sufficient for cleavage of DNA *in vitro* (M. Jinek et al., A programmable dual-RNA-guided DNA endonuclease in adaptive bacterial immunity. Science 337, 816 (Aug 17, 2012) and G. Gasiunas, R. Barrangou, P. Horvath, V. Siksnys, Cas9-crRNA ribonucleoprotein complex mediates specific DNA cleavage for adaptive immunity in bacteria. Proc Natl Acad Sci U S A 109, E2579 (Sep 25, 2012)) and in prokaryotic cells (R. Sapranauskas et al., The Streptococcus thermophilus CRISPR/Cas system provides immunity in Escherichia coli. Nucleic Acids Res 39, 9275 (Nov, 2011) and A. H. Magadan, M. E. Dupuis, M. Villion, S. Moineau, Cleavage of phage DNA by the Streptococcus thermophilus CRISPR3-Cas system. PLoS One 7, e40913 (2012)). Applicants codon optimized the *S. pyogenes Cas9 (SpCas9)* and *RNase III (SpRNase III)* and attached nuclear localization signals (NLS) to ensure nuclear compartmentalization in mammalian cells. Expression of these constructs in human 293FT cells revealed that two NLSs are required for targeting SpCas9 to the nucleus (FIG. 23A). To reconstitute the non-coding RNA components of CRISPR, Applicants expressed an 89-nucleotide (nt) tracrRNA (FIG. 28) under the RNA polymerase III U6 promoter (FIG. 23B). Similarly, Applicants used the U6 promoter to drive the expression of a pre-crRNA array comprising a single guide spacer flanked by DRs (FIG. 23B). Applicants designed an initial spacer to target a 30-basepair (bp) site (protospacer) in the human *EMX1* locus that precedes an NGG, the requisite protospacer adjacent motif (PAM) (FIG. 23C and FIG. 27) (H. Deveau et al., Phage response to CRISPR-encoded resistance in Streptococcus thermophilus. J Bacteriol 190, 1390 (Feb, 2008) and F. J. Mojica, C. Diez-Villasenor, J. Garcia-Martinez, C. Almendros, Short motif sequences determine the targets of the prokaryotic CRISPR defence system. Microbiology 155, 733 (Mar, 2009)).

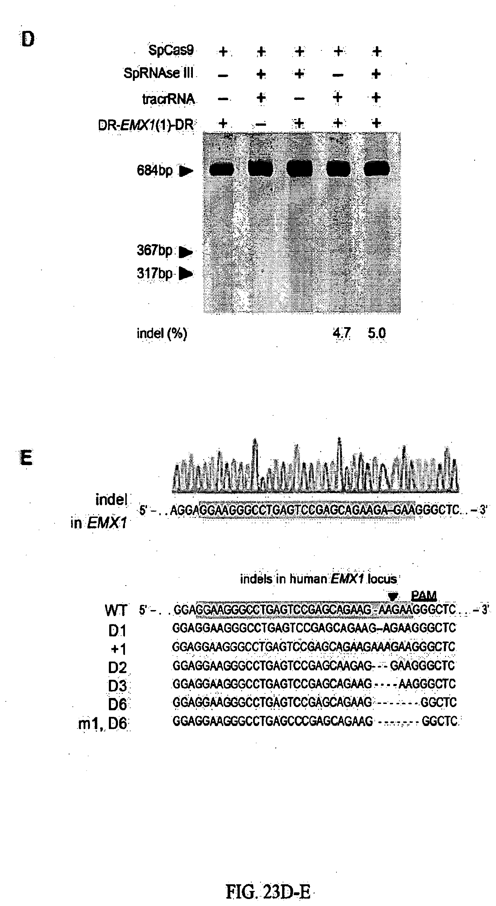

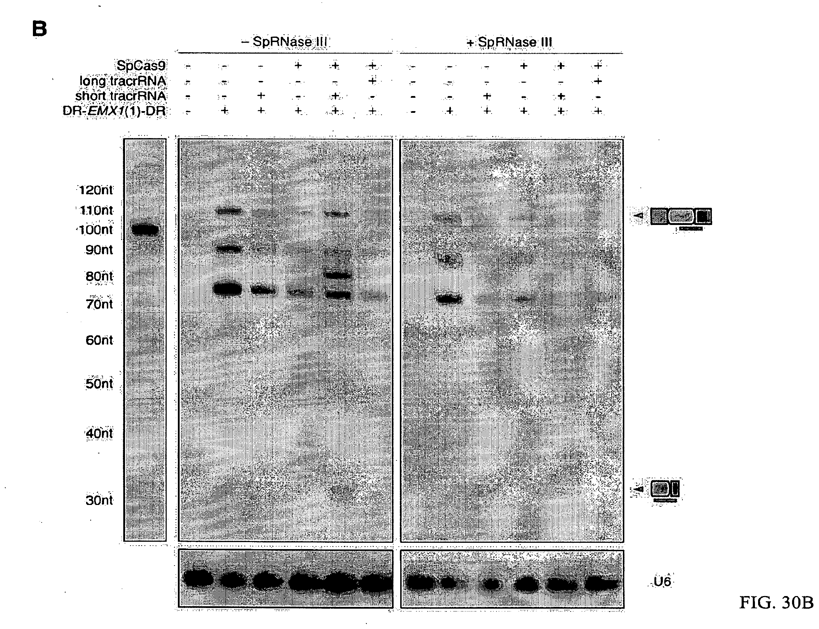

To test whether heterologous expression of the CRISPR system (SpCas9, SpRNase III, tracrRNA, and pre-crRNA) can achieve targeted cleavage of mammalian chromosomes, Applicants transfected 293FT cells with different combinations of CRISPR components. Since DSBs in mammalian DNA are partially repaired by the indel-forming non-homologous end joining (NHEJ) pathway, Applicants used the SURVEYOR assay (FIG. 29) to detect endogenous target cleavage (FIG.23D and FIG.28B). Co-transfection of all four required CRISPR components resulted in efficient cleavage of the protospacer (FIG. 23D and FIG. 28B), which is subsequently verified by Sanger sequencing (FIG.23E). Interestingly, SpRNase III was not necessary for cleavage of the protospacer (FIG. 23D), and the 89-nt tracrRNA is processed in its absence (FIG. 28C). Similarly, maturation of pre-crRNA does not require RNase III (FIG. 23D and FIG. 30), suggesting that there may be endogenous mammalian RNases that assist in pre-crRNA maturation (M. Jinek, J. A. Doudna, A three-dimensional view of the molecular machinery of RNA interference. Nature 457, 405 (Jan 22, 2009); C. D. Malone, G. J. Hannon, Small RNAs as guardians of the genome. Cell 136, 656 (Feb 20, 2009) and G. Meister, T. Tuschl, Mechanisms of gene silencing by double-stranded RNA. Nature 431, 343 (Sep 16, 2004)). Removing any of the remaining RNA or Cas9 components abolished the genome cleavage activity of the CRISPR system (FIG. 23D). These results define a minimal three-component system for efficient CRISPR-mediated genome modification in mammalian cells.

Next, Applicants explored the generalizability of CRISPR-mediated cleavage in eukaryotic cells by targeting additional protospacers within the *EMX1* locus (FIG. 24A). To improve co-delivery, Applicants designed an expression vector to drive both pre-crRNA and SpCas9 (FIG. 31). In parallel, Applicants adapted a chimeric crRNA-tracrRNA hybrid (FIG. 24B, top) design recently validated *in vitro* (M. Jinek et al., A programmable dual-RNA-guided DNA endonuclease in adaptive bacterial immunity. Science 337, 816 (Aug 17, 2012)), where a mature crRNA is fused to a partial tracrRNA via a synthetic stem-loop to mimic the natural crRNA:tracrRNA duplex (FIG. 24B, bottom). Applicants observed cleavage of all protospacer targets when SpCas9 is co-expressed with pre-crRNA (DR-spacer-DR) and tracrRNA. However, not all chimeric RNA designs could facilitate cleavage of their genomic targets (FIG. 24C, Table 1). Applicants then tested targeting of additional genomic loci in both human and mouse cells by designing pre-crRNAs and chimeric RNAs targeting the human *PVALB* and the mouse *Th* loci (FIG. 32). Applicants achieved efficient modification at all three mouse *Th* and one *PVALB* targets using the crRNA:tracrRNA design, thus demonstrating the broad applicability of the CRISPR system in modifying different loci across multiple organisms (Table 1). For the same protospacer targets, cleavage efficiencies of chimeric RNAs were either lower than those of crRNA:tracrRNA duplexes or undetectable. This may be due to differences in the expression and stability of RNAs, degradation by endogenous RNAi machinery, or secondary structures leading to inefficient Cas9 loading or target recognition.

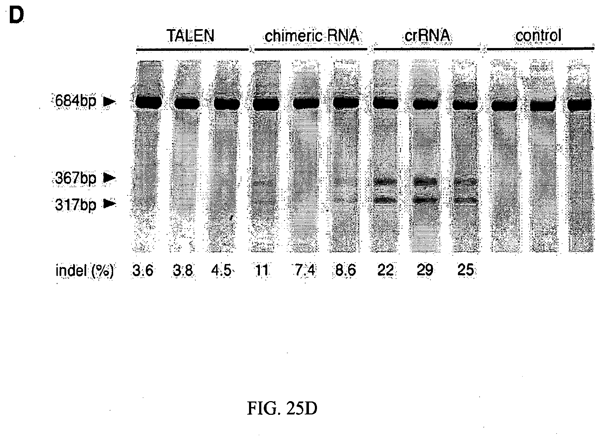

Effective genome editing requires that nucleases target specific genomic loci with both high precision and efficiency. To investigate the specificity of CRISPR-mediated cleavage, Applicants analyzed single-nucleotide mismatches between the spacer and its mammalian protospacer target (FIG. 25A). Applicants observed that single-base mismatch up to 12-bp 5' of the PAM completely abolished genomic cleavage by SpCas9, whereas spacers with mutations farther upstream retained activity against the protospacer target (FIG. 25B). This is consistent with previous bacterial and *in vitro* studies of Cas9 specificity (M. Jinek et al., A programmable dual-RNA-guided DNA endonuclease in adaptive bacterial immunity. Science 337, 816 (Aug 17, 2012) and R. Sapranauskas et al., The Streptococcus thermophilus CRISPR/Cas system provides immunity in Escherichia coli. Nucleic Acids Res 39, 9275 (Nov, 2011)). Furthermore, CRISPR is able to mediate genomic cleavage as efficiently as a pair of TALE nucleases (TALEN) targeting the same *EMX1* protospacer (FIG. 25, C and D).

Targeted modification of genomes ideally avoids mutations arising from the error-prone NHEJ mechanism. The wild-type SpCas9 is able to mediate site-specific DSBs, which can be repaired through either NHEJ or homology-directed repair (HDR). Applicants engineered an aspartate-to-alanine substitution (D10A) in the RuvC I domain of SpCas9 to convert the nuclease into a DNA nickase (SpCas9n, FIG. 26A) (M. Jinek et al., A programmable dual-RNA-guided DNA endonuclease in adaptive bacterial immunity. Science 337, 816 (Aug 17, 2012); G. Gasiunas, R. Barrangou, P. Horvath, V. Siksnys, Cas9-crRNA ribonucleoprotein complex mediates specific DNA cleavage for adaptive immunity in bacteria. Proc Natl Acad Sci U S A 109, E2579 (Sep 25, 2012) and R. Sapranauskas et al., The Streptococcus thermophilus CRISPR/Cas system provides immunity in Escherichia coli. Nucleic Acids Res 39, 9275 (Nov, 2011)), because nicked genomic DNA is typically repaired either seamlessly or through high-fidelity HDR. SURVEYOR (FIG. 26B) and sequencing of 327 amplicons did not detect any indels induced by SpCas9n. However, it is worth noting that nicked DNA can in rare cases be processed via a DSB intermediate and result in a NHEJ event (M. T. Certo et al., Tracking genome engineering outcome at individual DNA breakpoints. Nat Methods 8, 671 (Aug, 2011)). Applicants then tested Cas9-mediated HDR at the same *EMX1* locus with a homology repair template to introduce a pair of restriction sites near the protospacer (FIG. 26C). SpCas9 and SpCas9n catalyzed integration of the repair template into *EMX1* locus at similar levels (FIG. 26D), which Applicants further verified via Sanger sequencing (FIG. 26E). These results demonstrate the utility of CRISPR for facilitating targeted genomic insertions. Given the 14-bp (12-bp from the seed sequence and 2-bp from PAM) target specificity (FIG. 25B) of the wild type SpCas9, the use of a nickase may reduce off-target mutations.

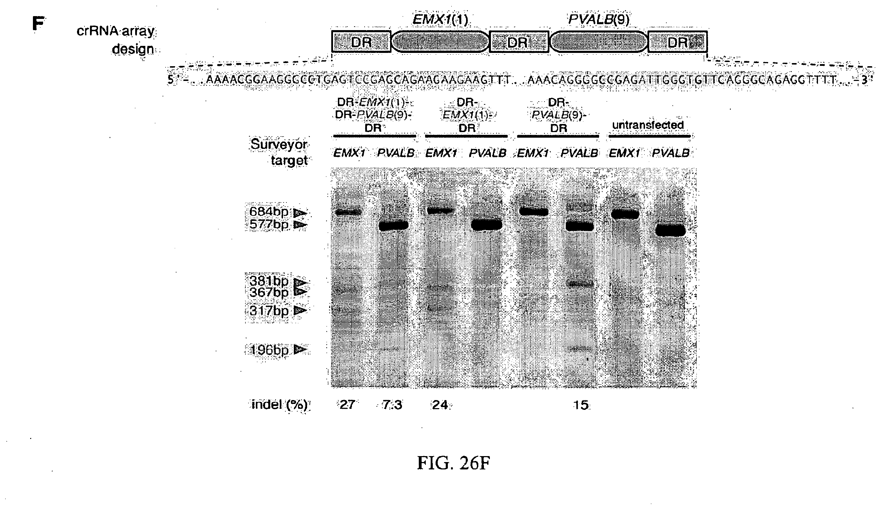

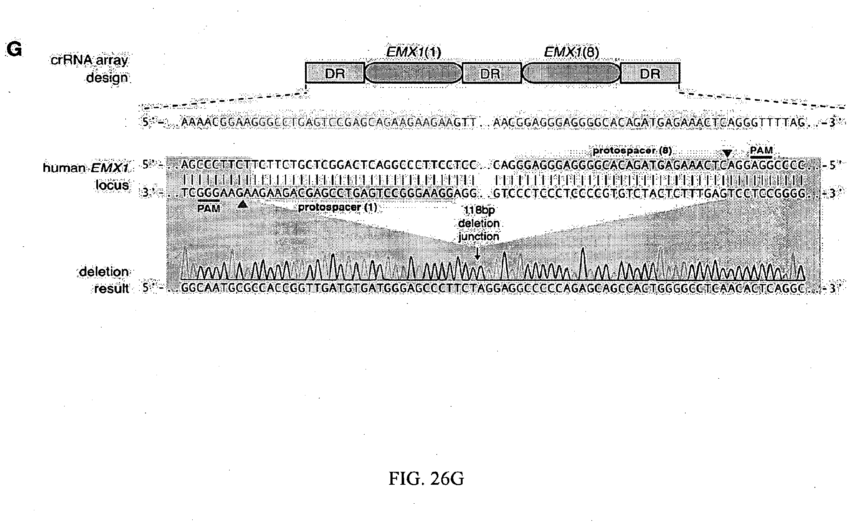

Finally, the natural architecture of CRISPR loci with arrayed spacers (FIG. 27) suggests the possibility of multiplexed genome engineering. Using a single CRISPR array encoding a pair of *EMX1-* and *PVALB*-targeting spacers, Applicants detected efficient cleavage at both loci (FIG. 26F). Applicants further tested targeted deletion of larger genomic regions through concurrent DSBs using spacers against two targets within *EMX1* spaced by 119-bp, and observed a 1.6% deletion efficacy (3 out of 182 amplicons; FIG. 26G), thus demonstrating the CRISPR system can mediate multiplexed editing within a single genome.

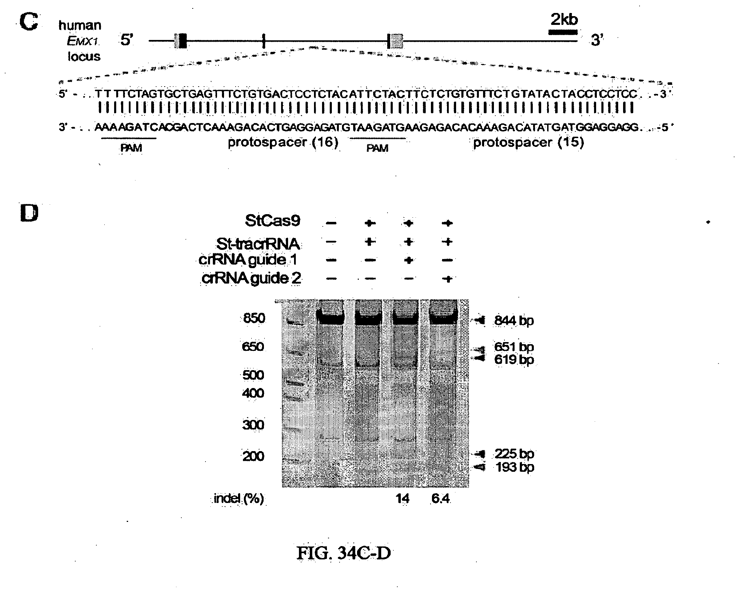

The ability to use RNA to program sequence-specific DNA cleavage defines a new class of genome engineering tools. Here, Applicants have shown that the *S. pyogenes* CRISPR system can be heterologously reconstituted in mammalian cells to facilitate efficient genome editing; an accompanying study has independently confirmed high efficiency CRISPR-mediated genome targeting in several human cell lines (Mali et al.). However, several aspects of the CRISPR system can be further improved to increase its efficiency and versatility. The requirement for an NGG PAM restricts the *S. pyogenes* CRISPR target space to every 8-bp on average in the human genome (FIG. 33), not accounting for potential constraints posed by crRNA secondary structure or genomic accessibility due to chromatin and DNA methylation states. Some of these restrictions may be overcome by exploiting the family of Cas9 enzymes and its differing PAM requirements (H. Deveau et al., Phage response to CRISPR-encoded resistance in Streptococcus thermophilus. J Bacteriol 190, 1390 (Feb, 2008) and F. J. Mojica, C. Diez-Villasenor, J. Garcia-Martinez, C. Almendros, Short motif sequences determine the targets of the prokaryotic CRISPR defence system. Microbiology 155, 733 (Mar, 2009)) across the microbial diversity (K. S. Makarova et al., Evolution and classification of the CRISPR-Cas systems. Nat Rev Microbiol 9, 467 (Jun, 2011)). Indeed, other CRISPR loci are likely to be transplantable into mammalian cells; for example, the *Streptococcus thermophilus* LMD-9 CRISPR1 can also mediate mammalian genome cleavage (FIG. 34). Finally, the ability to carry out multiplex genome editing in mammalian cells enables powerful applications across basic science, biotechnology, and medicine (P. A. Carr, G. M. Church, Genome engineering. Nat Biotechnol 27, 1151 (Dec, 2009)).

### Example 8 (disclosure)

### Multiplex Genome Engineering Using CRISPR/Cas Systems: Supplementary Material

*Cell culture and transfection.* Human embryonic kidney (HEK) cell line 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100µg/mL streptomycin at 37°C with 5% CO2 incubation. Mouse neuro2A-(N2A) cell line (ATCC) was maintained with DMEM supplemented with 5% fetal bovine serum (HyClone), 2mM GlutaMAX (Life Technologies), 100U/mL penicillin, and 100µg/mL streptomycin at 37°C with 5% CO₂.

293FT or N2A cells were seeded into 24-well plates (Corning) one day prior to transfection at a density of 200,000 cells per well. Cells were transfected using Lipofectamine 2000 (Life Technologies) following the manufacturer's recommended protocol. For each well of a 24-well plate a total of 800ng plasmids was used.

*Suveryor assay and sequencing analysis for genome modification.* 293FT or N2A cells were transfected with plasmid DNA as described above. Cells were incubated at 37°C for 72 hours post transfection before genomic DNA extraction. Genomic DNA was extracted using the QuickExtract DNA extraction kit (Epicentre) following the manufacturer's protocol. Briefly, cells were resuspended in QuickExtract solution and incubated at 65°C for 15 minutes and 98°C for 10 minutes.

Genomic region surrounding the CRISPR target site for each gene was PCR amplified, and products were purified using QiaQuick Spin Column (Qiagen) following manufacturer's protocol. A total of 400ng of the purified PCR products were mixed with 2µl 10X Taq polymerase PCR buffer (Enzymatics) and ultrapure water to a final volume of 20µl, and subjected to a re-annealing process to enable heteroduplex formation: 95°C for 10min, 95C to 85°C ramping at - 2°C/s, 85°C to 25°C at - 0.25°C/s, and 25°C hold for 1 minute. After reannealing, products were treated with SURVEYOR nuclease and SURVEYOR enhancer S (Transgenomics) following the manufacturer's recommended protocol, and analyzed on 4-20 Novex TBE poly-acrylamide gels (Life Technologies). Gels were stained with SYBR Gold DNA stain (Life Technologies) for 30 minutes and imaged with a Gel Doc gel imaging system (Biorad). Quantification was based on relative band intensities.

*Restriction fragment length polymorphism assay for detection of homologous recombination.* HEK 293FT and N2A cells were transfected with plasmid DNA, and incubated at 37°C for 72 hours before genomic DNA extraction as described above. The target genomic region was PCR amplified using primers outside the homology arms of the homologous recombination (HR) template. PCR products were separated on a 1% agarose gel and extracted with MinElute GelExtraction Kit (Qiagen). Purified products were digested with HindIII (Fermentas) and analyzed on a 6% Novex TBE poly-acrylamide gel (Life Technologies).

*RNA extraction and purification.* HEK 293FT cells were maintained and transfected as stated previously. Cells were harvested by trypsinization followed by washing in phosphate buffered saline (PBS). Total cell RNA was extracted with TRI reagent (Sigma) following manufacturer's protocol. Extracted total RNA was quantified using Naonodrop (Thermo Scientific) and normalized to same concentration.

*Northern blot analysis of crRNA and tracrRNA expression in mammalian cells.* RNAs were mixed with equal volumes of 2X loading buffer (Ambion), heated to 95°C for 5 min, chilled on ice for 1 min and then loaded onto 8% denaturing polyacrylamide gels (SequaGel, National Diagnostics) after pre-running the gel for at least 30 minutes. The samples were electrophoresed for 1.5 hours at 40W limit. Afterwards, the RNA was transferred to Hybond N+ membrane (GE Healthcare) at 300 mA in a semi-dry transfer apparatus (Bio-rad) at room temperature for 1.5 hours. The RNA was crosslinked to the membrane using autocrosslink button on Stratagene UV Crosslinker the Stratalinker (Stratagene). The membrane was pre-hybridized in ULTRAhyb-Oligo Hybridization Buffer (Ambion) for 30 min with rotation at 42°C and then probes were added and hybridized overnight. Probes were ordered from IDT and labeled with [gamma-32P] ATP (Perkin Elmer) with T4 polynucleotide kinase (New England Biolabs). The membrane was washed once with pre-warmed (42°C) 2xSSC, 0.5% SDS for 1 min followed by two 30 minute washes at 42°C. The membrane was exposed to phosphor screen for one hour or overnight at room temperature and then scanned with phosphorimager (Typhoon).

Table 1. Protospacer sequences and modification efficiencies of mammalian genomic targets. Protospacer targets designed based on Streptococcus pyogenes type II CRISPR and Streptococcus thermophilus CRISPR1 loci with their requisite PAMs against three different genes in human and mouse genomes. Cells were transfected with Cas9 and either precrRNA/ tracrRNA or chimeric RNA. Cells were analyzed 72 hours after transfection. Percent indels are calculated based on SURVEYOR assay results from indicated cell lines, N = 3 for all protospacer targets, errors are S.E.M. N.D., not detectable using the SURVEYOR assay; N.T., not tested in this study.

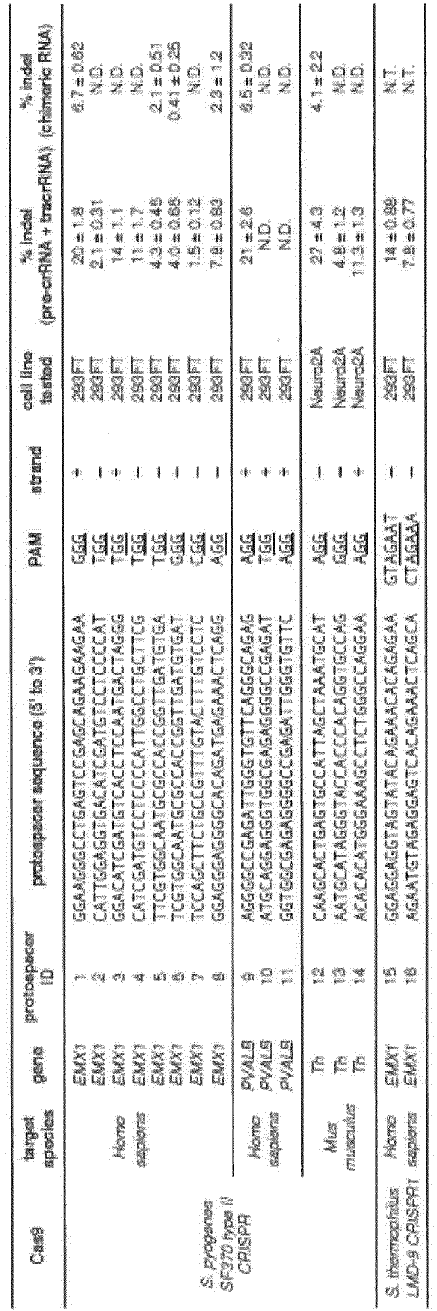

**Table 2. Sequences for primers and probes used for SURVEYOR assay, RFLP assay, genomic sequencing, and Northern blot.**

| **Primer name** | **Assay** | **Genomic Target** | **Primer sequence** |
|---|---|---|---|
| Sp-EMX1-F | SURVEYOR assay, sequencing | *EMX1* | AAAACCACCCTTCTCTCTGGC |
| Sp-EMX1-R | SURVEYOR assay, sequencing | *EMX1* | GGAGATTGGAGACACGGAGAG |
| Sp-PVALB-F | SURVEYOR assay, sequencing | *PVALB* | CTGGAAAGCCAATGCCTGAC |
| Sp-PVALB-R | SURVEYOR assay, sequencing | *PVALB* | GGCAGCAAACTCCTTGTCCT |
| Sp-Th-F | SURVEYOR assay, sequencing | *Th* | GTGCTTTGCAGAGGCCTACC |
| Sp-Th-R | SURVEYOR assay, sequencing | *Th* | CCTGGAGCGCATGCAGTAGT |
| St-EMX1-F | SURVEYOR assay, sequencing | *EMX1* | ACCTTCTGTGTTTCCACCATTC |
| St-EMX1-R | SURVEYOR assay, sequencing | *EMX1* | TTGGGGAGTGCACAGACTTC |
| Sp-EMX1-RFLP-F | RFLP, sequencing | *EMX1* | GGCTCCCTGGGTTCAAAGTA |
| Sp-EMX1-RFLP-R | RFLP, sequencing | *EMX1* | AGAGGGGTCTGGATGTCGTAA |
| Pb_EMX1_sp1 | Northern Blot Probe | Not applicable | TAGCTCTAAAACTTCTTCTTCTGCTCGGAC |
| Pb_tracrRNA | Northern Blot Probe | Not applicable | CTAGCCTTATTTTAACTTGCTATGCTGTTT |

### Supplementary Sequences

> U6-short tracrRNA (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGAACCATTCAAAACAGCATAGCAAGTTAA
AATAAGGCTAGTCCGTTATCAACTTGAAAAAGTGGCACCGAGTCGGTGCTTTTTTT
> U6-long tracrRNA (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGTAGTATTAAGTATTGTTTTATGGCTGATA
AATTTCTTTGAATTTCTCCTTGATTATTTGTTATAAAAGTTATAAAATAATCTTGTTG
GAACCATTCAAAACAGCATAGCAAGTTAAAATAAGGCTAGTCCGTTATCAACTTGA
AAAAGTGGCACCGAGTCGGTGCTTTTTTT
> U6-DR-BbsI backbone-DR (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGGTTTTAGAGCTATGCTGTTTTGAATGGTC
CCAAAACGGGTCTTCGAGAAGACGTTTTAGAGCTATGCTGTTTTGAATGGTCCCAAA
AC
> U6-chimeric RNA-BbsI backbone (Streptococcus pyogenes SF370) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGGTCTTCGAGAAGACCTGTTTTAGAGCTA
GAAATAGCAAGTTAAAATAAGGCTAGTCCG
> 3xFLAG-NLS-SpCas9-NLS 
ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGACAAGAAGTACAGCATCGGCCTGGACATCGGCACCAACTC
TGTGGGCTGGGCCGTGATCACCGACGAGTACAAGGTGCCCAGCAAGAAATTCAAGG
TGCTGGGCAACACCGACCGGCACAGCATCAAGAAGAACCTGATCGGAGCCCTGCTG
TTCGACAGCGGCGAAACAGCCGAGGCCACCCGGCTGAAGAGAACCGCCAGAAGAA
GATACACCAGACGGAAGAACCGGATCTGCTATCTGCAAGAGATCTTCAGCAACGAG
ATGGCCAAGGTGGACGACAGCTTCTTCCACAGACTGGAAGAGTCCTTCCTGGTGGA
AGAGGATAAGAAGCACGAGCGGCACCCCATCTTCGGCAACATCGTGGACGAGGTGG
CCTACCACGAGAAGTACCCCACCATCTACCACCTGAGAAAGAAACTGGTGGACAGC
ACCGACAAGGCCGACCTGCGGCTGATCTATCTGGCCCTGGCCCACATGATCAAGTTC
CGGGGCCACTTCCTGATCGAGGGCGACCTGAACCCCGACAACAGCGACGTGGACAA
GCTGTTCATCCAGCTGGTGCAGACCTACAACCAGCTGTTCGAGGAAAACCCCATCAA
CGCCAGCGGCGTGGACGCCAAGGCCATCCTGTCTGCCAGACTGAGCAAGAGCAGAC
GGCTGGAAAATCTGATCGCCCAGCTGCCCGGCGAGAAGAAGAATGGCCTGTTCGGC
AACCTGATTGCCCTGAGCCTGGGCCTGACCCCCAACTTCAAGAGCAACTTCGACCTG
GCCGAGGATGCCAAACTGCAGCTGAGCAAGGACACCTACGACGACGACCTGGACAA
CCTGCTGGCCCAGATCGGCGACCAGTACGCCGACCTGTTTCTGGCCGCCAAGAACCT
GTCCGACGCCATCCTGCTGAGCGACATCCTGAGAGTGAACACCGAGATCACCAAGG
CCCCCCTGAGCGCCTCTATGATCAAGAGATACGACGAGCACCACCAGGACCTGACC
CTGCTGAAAGCTCTCGTGCGGCAGCAGCTGCCTGAGAAGTACAAAGAGATTTTCTTC
GACCAGAGCAAGAACGGCTACGCCGGCTACATTGACGGCGGAGCCAGCCAGGAAG
AGTTCTACAAGTTCATCAAGCCCATCCTGGAAAAGATGGACGGCACCGAGGAACTG
CTCGTGAAGCTGAACAGAGAGGACCTGCTGCGGAAGCAGCGGACCTTCGACAACGG
CAGCATCCCCCACCAGATCCACCTGGGAGAGCTGCACGCCATTCTGCGGCGGCAGG
AAGATTTTTACCCATTCCTGAAGGACAACCGGGAAAAGATCGAGAAGATCCTGACC
TTCCGCATCCCCTACTACGTGGGCCCTCTGGCCAGGGGAAACAGCAGATTCGCCTGG
ATGACCAGAAAGAGCGAGGAAACCATCACCCCCTGGAACTTCGAGGAAGTGGTGGA
CAAGGGCGCTTCCGCCCAGAGCTTCATCGAGCGGATGACCAACTTCGATAAGAACC
TGCCCAACGAGAAGGTGCTGCCCAAGCACAGCCTGCTGTACGAGTACTTCACCGTGT
ATAACGAGCTGACCAAAGTGAAATACGTGACCGAGGGAATGAGAAAGCCCGCCTTC
 
CTGAGCGGCGAGCAGAAAAAGGCCATCGTGGACCTGCTGTTCAAGACCAACCGGAA
AGTGACCGTGAAGCAGCTGAAAGAGGACTACTTCAAGAAAATCGAGTGCTTCGACT
CCGTGGAAATCTCCGGCGTGGAAGATCGGTTCAACGCCTCCCTGGGCACATACCAC
GATCTGCTGAAAATTATCAAGGACAAGGACTTCCTGGACAATGAGGAAAACGAGGA
CATTCTGGAAGATATCGTGCTGACCCTGACACTGTTTGAGGACAGAGAGATGATCGA
GGAACGGCTGAAAACCTATGCCCACCTGTTCGACGACAAAGTGATGAAGCAGCTGA
AGCGGCGGAGATACACCGGCTGGGGCAGGCTGAGCCGGAAGCTGATCAACGGCATC
CGGGACAAGCAGTCCGGCAAGACAATCCTGGATTTCCTGAAGTCCGACGGCTTCGC
CAACAGAAACTTCATGCAGCTGATCCACGACGACAGCCTGACCTTTAAAGAGGACA
TCCAGAAAGCCCAGGTGTCCGGCCAGGGCGATAGCCTGCACGAGCACATTGCCAAT
CTGGCCGGCAGCCCCGCCATTAAGAAGGGCATCCTGCAGACAGTGAAGGTGGTGGA
CGAGCTCGTGAAAGTGATGGGCCGGCACAAGCCCGAGAACATCGTGATCGAAATGG
CCAGAGAGAACCAGACCACCCAGAAGGGACAGAAGAACAGCCGCGAGAGAATGAA
GCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATG
TGGACCATATCGTGCCTCAGAGCTTTCTGAAGGACGACTCCATCGACAACAAGGTGC
TGACCAGAAGCGACAAGAACCGGGGCAAGAGCGACAACGTGCCCTCCGAAGAGGT
CGTGAAGAAGATGAAGAACTACTGGCGGCAGCTGCTGAACGCCAAGCTGATTACCC
AGAGAAAGTTCGACAATCTGACCAAGGCCGAGAGAGGCGGCCTGAGCGAACTGGAT
AAGGCCGGCTTCATCAAGAGACAGCTGGTGGAAACCCGGCAGATCACAAAGCACGT
GGCACAGATCCTGGACTCCCGGATGAACACTAAGTACGACGAGAATGACAAGCTGA
TCCGGGAAGTGAAAGTGATCACCCTGAAGTCCAAGCTGGTGTCCGATTTCCGGAAG
GATTTCCAGTTTTACAAAGTGCGCGAGATCAACAACTACCACCACGCCCACGACGCC
TACCTGAACGCCGTCGTGGGAACCGCCCTGATCAAAAAGTACCCTAAGCTGGAAAG
CGAGTTCGTGTACGGCGACTACAAGGTGTACGACGTGCGGAAGATGATCGCCAAGA
GCGAGCAGGAAATCGGCAAGGCTACCGCCAAGTACTTCTTCTACAGCAACATCATG
AACTTTTTCAAGACCGAGATTACCCTGGCCAACGGCGAGATCCGGAAGCGGCCTCT
GATCGAGACAAACGGCGAAACCGGGGAGATCGTGTGGGATAAGGGCCGGGATTTTG
CCACCGTGCGGAAAGTGCTGAGCATGCCCCAAGTGAATATCGTGAAAAAGACCGAG
GTGCAGACAGGCGGCTTCAGCAAAGAGTCTATCCTGCCCAAGAGGAACAGCGATAA
 
GCTGATCGCCAGAAAGAAGGACTGGGACCCTAAGAAGTACGGCGGCTTCGACAGCC
CCACCGTGGCCTATTCTGTGCTGGTGGTGGCCAAAGTGGAAAAGGGCAAGTCCAAG
AAACTGAAGAGTGTGAAAGAGCTGCTGGGGATCACCATCATGGAAAGAAGCAGCTT
CGAGAAGAATCCCATCGACTTTCTGGAAGCCAAGGGCTACAAAGAAGTGAAAAAGG
ACCTGATCATCAAGCTGCCTAAGTACTCCCTGTTCGAGCTGGAAAACGGCCGGAAG
AGAATGCTGGCCTCTGCCGGCGAACTGCAGAAGGGAAACGAACTGGCCCTGCCCTC
CAAATATGTGAACTTCCTGTACCTGGCCAGCCACTATGAGAAGCTGAAGGGCTCCCC
CGAGGATAATGAGCAGAAACAGCTGTTTGTGGAACAGCACAAGCACTACCTGGACG
AGATCATCGAGCAGATCAGCGAGTTCTCCAAGAGAGTGATCCTGGCCGACGCTAAT
CTGGACAAAGTGCTGTCCGCCTACAACAAGCACCGGGATAAGCCCATCAGAGAGCA
GGCCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGTGC
TGGACGCCACCCTGATCCACCAGAGCATCACCGGCCTGTACGAGACACGGATCGAC
CTGTCTCAGCTGGGAGGCGACAAGCGTCCTGCTGCTACTAAGAAAGCTGGTCAAGCT
AAGAAAAAGAAA
> SpRNase3-mCherry-NLS 
ATGAAGCAGCTGGAGGAGTTACTTTCTACCTCTTTCGACATCCAGTTTAAT
GACCTGACCCTGCTGGAAACCGCCTTCACTCACACCTCCTACGCGAATGAGCACCGC
CTACTGAATGTGAGCCACAACGAGCGCCTGGAGTTTCTGGGGGATGCTGTCTTACAG
CTGATCATCTCTGAATATCTGTTTGCCAAATACCCTAAGAAAACCGAAGGGGACATG
TCAAAGCTGCGCTCCATGATAGTCAGGGAAGAGAGCCTGGCGGGCTTTAGTCGTTTT
TGCTCATTCGACGCTTATATCAAGCTGGGAAAAGGCGAAGAGAAGTCCGGCGGCAG
GAGGCGCGATACAATTCTGGGCGATCTCTTTGAAGCGTTTCTGGGCGCACTTCTACT
GGACAAAGGGATCGACGCAGTCCGCCGCTTTCTGAAACAAGTGATGATCCCTCAGG
TCGAAAAGGGAAACTTCGAGAGAGTGAAGGACTATAAAACATGTTTGCAGGAATTT
CTCCAGACCAAGGGAGATGTAGCAATAGATTATCAGGTAATAAGTGAGAAAGGACC
AGCTCACGCCAAACAATTCGAAGTTAGCATCGTTGTTAATGGCGCAGTGTTGTCGAA
GGGCTTGGGTAAATCAAAAAAACTGGCCGAGCAGGACGCTGCTAAAAACGCCCTCG
CTCAGCTCAGCGAGGTAGGATCCGTGAGCAAGGGCGAGGAGGATAACATGGCCATC
ATCAAGGAGTTCATGCGCTTCAAGGTGCACATGGAGGGCTCCGTGAACGGCCACGA
GTTCGAGATCGAGGGCGAGGGCGAGGGCCGCCCCTACGAGGGCACCCAGACCGCCA
 
AGCTGAAGGTGACCAAGGGTGGCCCCCTGCCCTTCGCCTGGGACATCCTGTCCCCTC
AGTTCATGTACGGCTCCAAGGCCTACGTGAAGCACCCCGCCGACATCCCCGACTACT
TGAAGCTGTCCTTCCCCGAGGGCTTCAAGTGGGAGCGCGTGATGAACTTCGAGGAC
GGCGGCGTGGTGACCGTGACCCAGGACTCCTCCCTGCAGGACGGCGAGTTCATCTA
CAAGGTGAAGCTGCGCGGCACCAACTTCCCCTCCGACGGCCCCGTAATGCAGAAGA
AGACCATGGGCTGGGAGGCCTCCTCCGAGCGGATGTACCCCGAGGACGGCGCCCTG
AAGGGCGAGATCAAGCAGAGGCTGAAGCTGAAGGACGGCGGCCACTACGACGCTG
AGGTCAAGACCACCTACAAGGCCAAGAAGCCCGTGCAGCTGCCCGGCGCCTACAAC
GTCAACATCAAGTTGGACATCACCTCCCACAACGAGGACTACACCATCGTGGAACA
GTACGAACGCGCCGAGGGCCGCCACTCCACCGGCGGCATGGACGAGCTGTACAAGA
AGCGTCCTGCTGCTACTAAGAAAGCTGGTCAAGCTAAGAAAAAGAAA
> 3xFLAG-NLS-SpCas9n-NLS (the D10A nickase mutation is underlined) 
ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGACAAGAAGTACAGCATCGGCCTGGCCATCGGCACCAACTC
TGTGGGCTGGGCCGTGATCACCGACGAGTACAAGGTGCCCAGCAAGAAATTCAAGG
TGCTGGGCAACACCGACCGGCACAGCATCAAGAAGAACCTGATCGGAGCCCTGCTG
TTCGACAGCGGCGAAACAGCCGAGGCCACCCGGCTGAAGAGAACCGCCAGAAGAA
GATACACCAGACGGAAGAACCGGATCTGCTATCTGCAAGAGATCTTCAGCAACGAG
ATGGCCAAGGTGGACGACAGCTTCTTCCACAGACTGGAAGAGTCCTTCCTGGTGGA
AGAGGATAAGAAGCACGAGCGGCACCCCATCTTCGGCAACATCGTGGACGAGGTGG
CCTACCACGAGAAGTACCCCACCATCTACCACCTGAGAAAGAAACTGGTGGACAGC
ACCGACAAGGCCGACCTGCGGCTGATCTATCTGGCCCTGGCCCACATGATCAAGTTC
CGGGGCCACTTCCTGATCGAGGGCGACCTGAACCCCGACAACAGCGACGTGGACAA
GCTGTTCATCCAGCTGGTGCAGACCTACAACCAGCTGTTCGAGGAAAACCCCATCAA
CGCCAGCGGCGTGGACGCCAAGGCCATCCTGTCTGCCAGACTGAGCAAGAGCAGAC
GGCTGGAAAATCTGATCGCCCAGCTGCCCGGCGAGAAGAAGAATGGCCTGTTCGGC
AACCTGATTGCCCTGAGCCTGGGCCTGACCCCCAACTTCAAGAGCAACTTCGACCTG
GCCGAGGATGCCAAACTGCAGCTGAGCAAGGACACCTACGACGACGACCTGGACAA
CCTGCTGGCCCAGATCGGCGACCAGTACGCCGACCTGTTTCTGGCCGCCAAGAACCT
GTCCGACGCCATCCTGCTGAGCGACATCCTGAGAGTGAACACCGAGATCACCAAGG
 
CCCCCCTGAGCGCCTCTATGATCAAGAGATACGACGAGCACCACCAGGACCTGACC
CTGCTGAAAGCTCTCGTGCGGCAGCAGCTGCCTGAGAAGTACAAAGAGATTTTCTTC
GACCAGAGCAAGAACGGCTACGCCGGCTACATTGACGGCGGAGCCAGCCAGGAAG
AGTTCTACAAGTTCATCAAGCCCATCCTGGAAAAGATGGACGGCACCGAGGAACTG
CTCGTGAAGCTGAACAGAGAGGACCTGCTGCGGAAGCAGCGGACCTTCGACAACGG
CAGCATCCCCCACCAGATCCACCTGGGAGAGCTGCACGCCATTCTGCGGCGGCAGG
AAGATTTTTACCCATTCCTGAAGGACAACCGGGAAAAGATCGAGAAGATCCTGACC
TTCCGCATCCCCTACTACGTGGGCCCTCTGGCCAGGGGAAACAGCAGATTCGCCTGG
ATGACCAGAAAGAGCGAGGAAACCATCACCCCCTGGAACTTCGAGGAAGTGGTGGA
CAAGGGCGCTTCCGCCCAGAGCTTCATCGAGCGGATGACCAACTTCGATAAGAACC
TGCCCAACGAGAAGGTGCTGCCCAAGCACAGCCTGCTGTACGAGTACTTCACCGTGT
ATAACGAGCTGACCAAAGTGAAATACGTGACCGAGGGAATGAGAAAGCCCGCCTTC
CTGAGCGGCGAGCAGAAAAAGGCCATCGTGGACCTGCTGTTCAAGACCAACCGGAA
AGTGACCGTGAAGCAGCTGAAAGAGGACTACTTCAAGAAAATCGAGTGCTTCGACT
CCGTGGAAATCTCCGGCGTGGAAGATCGGTTCAACGCCTCCCTGGGCACATACCAC
GATCTGCTGAAAATTATCAAGGACAAGGACTTCCTGGACAATGAGGAAAACGAGGA
CATTCTGGAAGATATCGTGCTGACCCTGACACTGTTTGAGGACAGAGAGATGATCGA
GGAACGGCTGAAAACCTATGCCCACCTGTTCGACGACAAAGTGATGAAGCAGCTGA
AGCGGCGGAGATACACCGGCTGGGGCAGGCTGAGCCGGAAGCTGATCAACGGCATC
CGGGACAAGCAGTCCGGCAAGACAATCCTGGATTTCCTGAAGTCCGACGGCTTCGC
CAACAGAAACTTCATGCAGCTGATCCACGACGACAGCCTGACCTTTAAAGAGGACA
TCCAGAAAGCCCAGGTGTCCGGCCAGGGCGATAGCCTGCACGAGCACATTGCCAAT
CTGGCCGGCAGCCCCGCCATTAAGAAGGGCATCCTGCAGACAGTGAAGGTGGTGGA
CGAGCTCGTGAAAGTGATGGGCCGGCACAAGCCCGAGAACATCGTGATCGAAATGG
CCAGAGAGAACCAGACCACCCAGAAGGGACAGAAGAACAGCCGCGAGAGAATGAA
GCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATG
TGGACCATATCGTGCCTCAGAGCTTTCTGAAGGACGACTCCATCGACAACAAGGTGC
TGACCAGAAGCGACAAGAACCGGGGCAAGAGCGACAACGTGCCCTCCGAAGAGGT
CGTGAAGAAGATGAAGAACTACTGGCGGCAGCTGCTGAACGCCAAGCTGATTACCC
 
AGAGAAAGTTCGACAATCTGACCAAGGCCGAGAGAGGCGGCCTGAGCGAACTGGAT
AAGGCCGGCTTCATCAAGAGACAGCTGGTGGAAACCCGGCAGATCACAAAGCACGT
GGCACAGATCCTGGACTCCCGGATGAACACTAAGTACGACGAGAATGACAAGCTGA
TCCGGGAAGTGAAAGTGATCACCCTGAAGTCCAAGCTGGTGTCCGATTTCCGGAAG
GATTTCCAGTTTTACAAAGTGCGCGAGATCAACAACTACCACCACGCCCACGACGCC
TACCTGAACGCCGTCGTGGGAACCGCCCTGATCAAAAAGTACCCTAAGCTGGAAAG
CGAGTTCGTGTACGGCGACTACAAGGTGTACGACGTGCGGAAGATGATCGCCAAGA
GCGAGCAGGAAATCGGCAAGGCTACCGCCAAGTACTTCTTCTACAGCAACATCATG
AACTTTTTCAAGACCGAGATTACCCTGGCCAACGGCGAGATCCGGAAGCGGCCTCT
GATCGAGACAAACGGCGAAACCGGGGAGATCGTGTGGGATAAGGGCCGGGATTTTG
CCACCGTGCGGAAAGTGCTGAGCATGCCCCAAGTGAATATCGTGAAAAAGACCGAG
GTGCAGACAGGCGGCTTCAGCAAAGAGTCTATCCTGCCCAAGAGGAACAGCGATAA
GCTGATCGCCAGAAAGAAGGACTGGGACCCTAAGAAGTACGGCGGCTTCGACAGCC
CCACCGTGGCCTATTCTGTGCTGGTGGTGGCCAAAGTGGAAAAGGGCAAGTCCAAG
AAACTGAAGAGTGTGAAAGAGCTGCTGGGGATCACCATCATGGAAAGAAGCAGCTT
CGAGAAGAATCCCATCGACTTTCTGGAAGCCAAGGGCTACAAAGAAGTGAAAAAGG
ACCTGATCATCAAGCTGCCTAAGTACTCCCTGTTCGAGCTGGAAAACGGCCGGAAG
AGAATGCTGGCCTCTGCCGGCGAACTGCAGAAGGGAAACGAACTGGCCCTGCCCTC
CAAATATGTGAACTTCCTGTACCTGGCCAGCCACTATGAGAAGCTGAAGGGCTCCCC
CGAGGATAATGAGCAGAAACAGCTGTTTGTGGAACAGCACAAGCACTACCTGGACG
AGATCATCGAGCAGATCAGCGAGTTCTCCAAGAGAGTGATCCTGGCCGACGCTAAT
CTGGACAAAGTGCTGTCCGCCTACAACAAGCACCGGGATAAGCCCATCAGAGAGCA
GGCCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGTGC
TGGACGCCACCCTGATCCACCAGAGCATCACCGGCCTGTACGAGACACGGATCGAC
CTGTCTCAGCTGGGAGGCGACAAGCGTCCTGCTGCTACTAAGAAAGCTGGTCAAGCT
AAGAAAAAGAAA
> hEMX1-HRTemplate-HindIII-NheI 
GAATGCTGCCCTCAGACCCGCTTCCTCCCTGTCCTTGTCTGTCCAAGGAGA
ATGAGGTCTCACTGGTGGATTTCGGACTACCCTGAGGAGCTGGCACCTGAGGACA
AGGCCCCCCACCTGCCCAGCTCCAGCCTCTGATGAGGGGTGGGAGAGAGCTACATG
 
AGGTTGCTAAGAAAGCCTCCCCTGAAGGAGACCACACAGTGTGTGAGGTTGGAGTC
TCTAGCAGCGGGTTCTGTGCCCCCAGGGATAGTCTGGCTGTCCAGGCACTGCTCTTG
ATATAAACACCACCTCCTAGTTATGAAACCATGCCCATTCTGCCTCTCTGTATGGAA
AAGAGCATGGGGCTGGCCCGTGGGGTGGTGTCCACTTTAGGCCCTGTGGGAGATCA
TGGGAACCCACGCAGTGGGTCATAGGCTCTCTCATTTACTACTCACATCCACTCTGT
GAAGAAGCGATTATGATCTCTCCTCTAGAAACTCGTAGAGTCCCATGTCTGCCGGCT
TCCAGAGCCTGCACTCCTCCACCTTGGCTTGGCTTTGCTGGGGCTAGAGGAGCTAGG
ATGCACAGCAGCTCTGTGACCCTTTGTTTGAGAGGAACAGGAAAACCACCCTTCTCT
CTGGCCCACTGTGTCCTCTTCCTGCCCTGCCATCCCCTTCTGTGAATGTTAGACCCAT
GGGAGCAGCTGGTCAGAGGGGACCCCGGCCTGGGGCCCCTAACCCTATGTAGCCTC
AGTCTTCCCATCAGGCTCTCAGCTCAGCCTGAGTGTTGAGGCCCCAGTGGCTGCTCT
GGGGGCCTCCTGAGTTTCTCATCTGTGCCCCTCCCTCCCTGGCCCAGGTGAAGGTGT
GGTTCCAGAACCGGAGGACAAAGTACAAACGGCAGAAGCTGGAGGAGGAAGGGCC
TGAGTCCGAGCAGAAGAAGAAGGGCTCCCATCACATCAACCGGTGGCGCATTGCCA
CGAAGCAGGCCAATGGGGAGGACATCGATGTCACCTCCAATGACaagcttgctagcGGTGG
GCAACCACAAACCCACGAGGGCAGAGTGCTGCTTGCTGCTGGCCAGGCCCCTGCGT
GGGCCCAAGCTGGACTCTGGCCACTCCCTGGCCAGGCTTTGGGGAGGCCTGGAGTC
ATGGCCCCACAGGGCTTGAAGCCCGGGGCCGCCATTGACAGAGGGACAAGCAATGG
GCTGGCTGAGGCCTGGGACCACTTGGCCTTCTCCTCGGAGAGCCTGCCTGCCTGGGC
GGGCCCGCCCGCCACCGCAGCCTCCCAGCTGCTCTCCGTGTCTCCAATCTCCCTTTTG
TTTTGATGCATTTCTGTTTTAATTTATTTTCCAGGCACCACTGTAGTTTAGTGATCCCC
AGTGTCCCCCTTCCCTATGGGAATAATAAAAGTCTCTCTCTTAATGACACGGGCATC
CAGCTCCAGCCCCAGAGCCTGGGGTGGTAGATTCCGGCTCTGAGGGCCAGTGGGGG
CTGGTAGAGCAAACGCGTTCAGGGCCTGGGAGCCTGGGGTGGGGTACTGGTGGAGG
GGGTCAAGGGTAATTCATTAACTCCTCTCTTTTGTTGGGGGACCCTGGTCTCTACCTC
CAGCTCCACAGCAGGAGAAACAGGCTAGACATAGGGAAGGGCCATCCTGTATCTTG
AGGGAGGACAGGCCCAGGTCTTTCTTAACGTATTGAGAGGTGGGAATCAGGCCCAG
GTAGTTCAATGGGAGAGGGAGAGTGCTTCCCTCTGCCTAGAGACTCTGGTGGCTTCT
CCAGTTGAGGAGAAACCAGAGGAAAGGGGAGGATTGGGGTCTGGGGGAGGGAACA
CCATTCACAAAGGCTGACGGTTCCAGTCCGAAGTCGTGGGCCCACCAGGATGCTCA
CCTGTCCTTGGAGAACCGCTGGGCAGGTTGAGACTGCAGAGACAGGGCTTAAGGCT
 
GAGCCTGCAACCAGTCCCCAGTGACTCAGGGCCTCCTCAGCCCAAGAAAGAGCAAC
GTGCCAGGGCCCGCTGAGCTCTTGTGTTCACCTG
> NLS-StCsn1-NLS 
ATGAAAAGGCCGGCGGCCACGAAAAAGGCCGGCCAGGCAAAAAAGAAA
AAGTCCGACCTGGTACTTGGACTGGATATTGGTATCGGTTCGGTGGGAGTCGGAATC
CTCAACAAGGTCACGGGGGAGATCATTCACAAGAACTCGCGGATCTTCCCCGCAGC
TCAGGCTGAGAACAACTTGGTGCGGAGAACG
 
AATAGGCAGGGCAGGCGACTGGCGAGGAGGAAGAAACACAGGAGAGTC
CGATTGAACCGGCTGTTCGAGGAGTCCGGTTTGATCACCGACTTTACGAAAATCTCG
ATTAACCTTAATCCCTATCAGCTTCGGGTGAAAGGCCTGACAGACGAACTTTCGAAT
GAGGAACTTTTCATCGCGCTGAAAAACATGGTCAAGCACAGAGGGATTTCCTACCTC
GATGACGCCTCGGATGACGGAAATTCCTCAGTAGGAGATTATGCACAGATCGTGAA
AGAGAACTCAAAGCAACTGGAAACAAAGACACCGGGGCAGATCCAACTTGAAAGA
TACCAGACATACGGACAGCTCAGAGGAGATTTTACGGTGGAGAAGGACGGTAAAAA
GCACAGACTCATTAACGTATTTCCCACGTCGGCGTACAGATCCGAAGCGCTCCGCAT
CCTTCAGACTCAACAGGAGTTCAACCCGCAAATTACTGATGAGTTCATCAACCGCTA
TTTGGAAATCTTGACCGGAAAGCGCAAGTATTATCATGGGCCGGGTAATGAGAAAT
CCAGAACAGATTACGGCCGATACAGAACTTCGGGGAAACCTTGGATAACATCTTT
GGTATTTTGATTGGAAAGTGCACCTTTTACCCGGACGAGTTTCGAGCGGCCAAGGCG
TCATACACAGCACAAGAGTTTAATCTCTTGAATGATTTGAACAACTTGACGGTCCCC
ACGGAGACAAAGAAGCTCTCCAAAGAGCAAAAGAACCAAATCATCAACTACGTCA
AGAACGAGAAGGCTATGGGGCCAGCGAAGCTGTTCAAGTATATCGCTAAACTTCTC
AGCTGTGATGTGGCGGACATCAAAGGGTACCGAATCGACAAGTCGGGAAAAGCGGA
AATTCACACGTTTGAAGCATATCGAAAGATGAAAACGTTGGAAACACTGGACATTG
AGCAGATGGACCGGGAAACGCTCGACAAACTGGCATACGTGCTCACGTTGAATACT
GAACGAGAGGGAATCCAAGAGGCCCTTGAACATGAGTTCGCCGATGGATCGTTCAG
CCAGAAGCAGGTCGACGAACTTGTGCAATTCCGCAAGGCGAATAGCTCCATCTTCG
GGAAGGGATGGCACAACTTTTCGGTCAAACTCATGATGGAGTTGATCCCAGAACTTT
ATGAGACTTCGGAGGAGCAAATGACGATCTTGACGCGCTTGGGGAAACAGAAAACG
ACAAGCTCATCGAACAAAACTAAGTACATTGATGAGAAATTGCTGACGGAAGAAAT
CTATAATCCGGTAGTAGCGAAATCGGTAAGACAAGCGATCAAAATCGTGAACGCGG
 
CGATCAAGGAATATGGTGACTTTGATAACATCGTAATTGAAATGGCTAGAGAGACG
AACGAAGATGACGAGAAAAAGGCAATCCAGAAGATCCAGAAGGCCAACAAGGATG
AAAAAGATGCAGCGATGCTTAAAGCGGCCAACCAATACAATGGAAAGGCGGAGCT
GCCCCATTCAGTGTTTCACGGTCATAAACAGTTGGCGACCAAGATCCGACTCTGGCA
TCAGCAGGGTGAGCGGTGTCTCTACACCGGAAAGACTATCTCCATCCATGACTTGAT
TAACAATTCGAACCAGTTTGAAGTGGATCATATTCTGCCCCTGTCAATCACCTTTGA
CGACTCGCTTGCGAACAAGGTGCTCGTGTACGCAACGGCAAATCAGGAGAAAGGCC
AGCGGACTCCGTATCAGGCGCTCGACTCAATGGACGATGCGTGGTCATTCCGGGAG
CTGAAGGCGTTCGTACGCGAGAGCAAGACACTGAGCAACAAAAAGAAAGAGTATCT
GCTGACAGAGGAGGACATCTCGAAATTCGATGTCAGGAAGAAGTTCATCGAGCGGA
ATCTTGTCGACACTCGCTACGCTTCCAGAGTAGTACTGAACGCGCTCCAGGAACACT
TTAGAGCGCACAAAATTGACACGAAGGTGTCAGTGGTGAGAGGGCAGTTCACATCC
CAACTCCGCCGACATTGGGGCATCGAAAAGACGCGGGACACATATCACCATCATGC
GGTGGACGCGCTGATTATTGCCGCTTCGTCCCAGTTGAATCTCTGGAAAAAGCAGAA
GAACACGCTGGTGTCGTATTCGGAGGATCAGCTTTTGGACATCGAAACCGGGGAGC
TGATTTCCGACGATGAATACAAAGAATCGGTGTTTAAGGCACCATATCAGCATTTCG
TGGACACGCTGAAGAGCAAAGAGTTTGAGGACAGCATCCTCTTTTCGTACCAAGTG
GACTCGAAGTTTAATCGCAAGATTTCAGACGCCACAATCTACGCGACGAGGCAGGC
GAAGGTGGGCAAAGATAAAGCAGATGAAACCTACGTCCTTGGTAAAATCAAGGACA
TCTACACTCAGGACGGGTACGATGCGTTCATGAAAATCTACAAGAAGGATAAGTCG
AAGTTTCTCATGTACCGCCACGATCCACAGACTTTCGAAAAAGTCATTGAGCCTATT
TTGGAGAACTACCCTAACAAGCAAATCAACGAGAAAGGAAAGAAGTCCCGTGCAA
CCCCTTTCTGAAGTACAAGGAAGAGCACGGTTATATCCGCAAATACTCGAAGAAAG
GAAATGGGCCTGAGATTAAGTCGCTTAAGTATTACGACTCAAAGTTGGGTAACCAC
ATCGACATTACCCCGAAAGACTCCAACAACAAAGTCGTGTTGCAGTCCGTCTCGCCC
TGGCGAGCAGATGTGTATTTTAATAAGACGACCGGCAAATATGAGATCCTTGGACTC
AAATACGCAGACCTTCAATTCGAAAAGGGGACGGGCACTTATAAGATTTCACAAGA
GAAGTACAACGACATCAAGAAAAAGGAAGGGGTCGATTCAGATTCGGAGTTCAAAT
TCACCCTCTACAAAAACGACCTCCTGCTTGTGAAGGACACAGAAACGAAGGAGCAG
CAGCTCTTTCGGTTCCTCTCACGCACGATGCCCAAACAAAAACATTACGTCGAACTT
AAACCTTACGATAAGCAAAAGTTTGAAGGGGGAGAGGCACTGATCAAAGTATTGGG
 
TAACGTAGCCAATAGCGGACAGTGTAAGAAAGGGCTGGGAAAGTCCAATATCTCGA
TCTATAAAGTACGAACAGATGTATTGGGAAACCAGCATATCATCAAAAATGAGGGG
GATAAACCCAAACTCGATTTCAAGCGTCCTGCTGCTACTAAGAAAGCTGGTCAAGCT
AAGAAAAAGAAATAA
> U6-St_tracrRNA(7-97) 
GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGTTACTTAAATCTTGCAGAAGCTACAAAGA
TAAGGCTTCATGCCGAAATCAACACCCTGTCATTTTATGGCAGGGTGTTTTCGTTATT
TAA
>EMX1_TALEN_Left 
ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGTAGATTTGAGAACTTTGGGATATTCACAGCAGCAGCAGGA
AAAGATCAAGCCCAAAGTGAGGTCGACAGTCGCGCAGCATCACGAAGCGCTGGTGG
GTCATGGGTTTACACATGCCCACATCGTAGCCTTGTCGCAGCACCCTGCAGCCCTTG
GCACGGTCGCCGTCAAGTACCAGGACATGATTGCGGCGTTGCCGGAAGCCACACAT
GAGGCGATCGTCGGTGTGGGGAAACAGTGGAGCGGAGCCCGAGCGCTTGAGGCCCT
GTTGACGGTCGCGGGAGAGCTGAGAGGGCCTCCCCTTCAGCTGGACACGGGCCAGT
TGCTGAAGATCGCGAAGCGGGGAGGAGTCACGGCGGTCGAGGCGGTGCACGCGTGG
CGCAATGCGCTCACGGGAGCACCCCTCAACCTGACCCCAGAGCAGGTCGTGGCAAT
TGCGAGCAACCACGGGGGAAAGCAGGCACTCGAAACCGTCCAGAGGTTGCTGCCTG
TGCTGTGCCAAGCGCACGGACTTACGCCAGAGCAGGTCGTGGCAATTGCGAGCAAC
CACGGGGGAAAGCAGGCACTCGAAACCGTCCAGAGGTTGCTGCCTGTGCTGTGCCA
AGCGCACGGACTAACCCCAGAGCAGGTCGTGGCAATTGCGAGCAACATCGGGGGAA
AGCAGGCACTCGAAACCGTCCAGAGGTTGCTGCCTGTGCTGTGCCAAGCGCACGGG
TTGACCCCAGAGCAGGTCGTGGCAATTGCGAGCAACCACGGGGGAAAGCAGGCACT
CGAAACCGTCCAGAGGTTGCTGCCTGTGCTGTGCCAAGCGCACGGCCTGACCCCAG
AGCAGGTCGTGGCAATTGCGAGCAACCACGGGGGAAAGCAGGCACTCGAAACCGTC
 
CAGAGGTTGCTGCCTGTGCTGTGCCAAGCGCACGGACTGACACCAGAGCAGGTCGT
GGCAATTGCGAGCAACATCGGGGGAAAGCAGGCACTCGAAACCGTCCAGAGGTTGC
TGCCTGTGCTGTGCCAAGCGCACGGACTTACACCCGAACAAGTCGTGGCAATTGCG
AGCAACCACGGGGGAAAGCAGGCACTCGAAACCGTCCAGAGGTTGCTGCCTGTGCT
GTGCCAAGCGCACGGACTTACGCCAGAGCAGGTCGTGGCAATTGCGAGCAACCACG
GGGGAAAGCAGGCACTCGAAACCGTCCAGAGGTTGCTGCCTGTGCTGTGCCAAGCG
CACGGACTAACCCCAGAGCAGGTCGTGGCAATTGCGAGCAACATCGGGGGAAAGCA
GGCACTCGAAACCGTCCAGAGGTTGCTGCCTGTGCTGTGCCAAGCGCACGGGTTGA
CCCCAGAGCAGGTCGTGGCAATTGCGAGCAACATCGGGGGAAAGCAGGCACTCGAA
ACCGTCCAGAGGTTGCTGCCTGTGCTGTGCCAAGCGCACGGCCTGACCCCAGAGCA
GGTCGTGGCAATTGCGAGCAACCACGGGGGAAAGCAGGCACTCGAAACCGTCCAGA
GGTTGCTGCCTGTGCTGTGCCAAGCGCACGGACTGACACCAGAGCAGGTCGTGGCA
ATTGCGAGCAACCACGGGGGAAAGCAGGCACTCGAAACCGTCCAGAGGTTGCTGCC
TGTGCTGTGCCAAGCGCACGGACTCACGCCTGAGCAGGTAGTGGCTATTGCATCCAA
CAACGGGGGCAGACCCGCACTGGAGTCAATCGTGGCCCAGCTTTCGAGGCCGGACC
CCGCGCTGGCCGCACTCACTAATGATCATCTTGTAGCGCTGGCCTGCCTCGGCGGAC
GACCCGCCTTGGATGCGGTGAAGAAGGGGCTCCCGCACGCGCCTGCATTGATTAAG
CGGACCAACAGAAGGATTCCCGAGAGGACATCACATCGAGTGGCAGGTTCCCAACT
CGTGAAGAGTGAACTTGAGGAGAAAAAGTCGGAGCTGCGGCACAAATTGAAATACG
TACCGCATGAATACATCGAACTTATCGAAATTGCTAGGAACTCGACTCAAGACAGA
ATCCTTGAGATGAAGGTAATGGAGTTCTTTATGAAGGTTTATGGATACCGAGGGAAG
CATCTCGGTGGATCACGAAAACCCGACGGAGCAATCTATACGGTGGGGAGCCCGAT
TGATTACGGAGTGATCGTCGACACGAAAGCCTACAGCGGTGGGTACAATCTTCCCAT
CGGGCAGGCAGATGAGATGCAACGTTATGTCGAAGAAAATCAGACCAGGAACAAA
CACATCAATCCAAATGAGTGGTGGAAAGTGTATCCTTCATCAGTGACCGAGTTTAAG
TTTTTGTTTGTCTCTGGGCATTTCAAAGGCAACTATAAGGCCCAGCTCACACGGTTG
AATCACATTACGAACTGCAATGGTGCGGTTTTGTCCGTAGAGGAACTGCTCATTGGT
GGAGAAATGATCAAAGCGGGAACTCTGACACTGGAAGAAGTCAGACGCAAGTTTAA
CAATGGCGAGATCAATTTCCGCTCA
>EMX1_TALEN_Right

ATGGACTATAAGGACCACGACGGAGACTACAAGGATCATGATATTGATTA
CAAAGACGATGACGATAAGATGGCCCCAAAGAAGAAGCGGAAGGTCGGTATCCAC
GGAGTCCCAGCAGCCGTAGATTTGAGAACTTTGGGATATTCACAGCAGCAGCAGGA
AAAGATCAAGCCCAAAGTGAGGTCGACAGTCGCGCAGCATCACGAAGCGCTGGTGG
GTCATGGGTTTACACATGCCCACATCGTAGCCTTGTCGCAGCACCCTGCAGCCCTTG
GCACGGTCGCCGTCAAGTACCAGGACATGATTGCGGCGTTGCCGGAAGCCACACAT
GAGGCGATCGTCGGTGTGGGGAAACAGTGGAGCGGAGCCCGAGCGCTTGAGGCCCT
GTTGACGGTCGCGGGAGAGCTGAGAGGGCCTCCCCTTCAGCTGGACACGGGCCAGT
TGCTGAAGATCGCGAAGCGGGGAGGAGTCACGGCGGTCGAGGCGGTGCACGCGTGG
CGCAATGCGCTCACGGGAGCACCCCTCAACCTGACCCCAGAGCAGGTCGTGGCAAT
TGCGAGCAACCACGGGGGAAAGCAGGCACTCGAAACCGTCCAGAGGTTGCTGCCTG
TGCTGTGCCAAGCGCACGGACTTACGCCAGAGCAGGTCGTGGCAATTGCGAGCAAC
CACGGGGGAAAGCAGGCACTCGAAACCGTCCAGAGGTTGCTGCCTGTGCTGTGCCA
AGCGCACGGACTAACCCCAGAGCAGGTCGTGGCAATTGCGAGCAACCACGGGGGA
AAGCAGGCACTCGAAACCGTCCAGAGGTTGCTGCCTGTGCTGTGCCAAGCGCACGG
GTTGACCCCAGAGCAGGTCGTGGCAATTGCGAGCAACATCGGGGGAAAGCAGGCAC
TCGAAACCGTCCAGAGGTTGCTGCCTGTGCTGTGCCAAGCGCACGGCCTGACCCCAG
AGCAGGTCGTGGCAATTGCGAGCAACCACGGGGGAAAGCAGGCACTCGAAACCGTC
CAGAGGTTGCTGCCTGTGCTGTGCCAAGCGCACGGACTGACACCAGAGCAGGTCGT
GGCAATTGCGAGCCATGACGGGGGAAAGCAGGCACTCGAAACCGTCCAGAGGTTGC
TGCCTGTGCTGTGCCAAGCGCACGGACTTACACCCGAACAAGTCGTGGCAATTGCG
AGCCATGACGGGGGAAAGCAGGCACTCGAAACCGTCCAGAGGTTGCTGCCTGTGCT
GTGCCAAGCGCACGGACTTACGCCAGAGCAGGTCGTGGCAATTGCGAGCCATGACG
GGGGAAAGCAGGCACTCGAAACCGTCCAGAGGTTGCTGCCTGTGCTGTGCCAAGCG
CACGGACTAACCCCAGAGCAGGTCGTGGCAATTGCGAGCAACGGAGGGGGAAAGC
AGGCACTCGAAACCGTCCAGAGGTTGCTGCCTGTGCTGTGCCAAGCGCACGGGTTG
ACCCCAGAGCAGGTCGTGGCAATTGCGAGCAACGGAGGGGGAAAGCAGGCACTCG
AAACCGTCCAGAGGTTGCTGCCTGTGCTGTGCCAAGCGCACGGCCTGACCCCAGAG
CAGGTCGTGGCAATTGCGAGCCATGACGGGGGAAAGCAGGCACTCGAAACCGTCCA
GAGGTTGCTGCCTGTGCTGTGCCAAGCGCACGGACTGACACCAGAGCAGGTCGTGG
CAATTGCGAGCAACGGAGGGGGAAAGCAGGCACTCGAAACCGTCCAGAGGTTGCTG

CCTGTGCTGTGCCAAGCGCACGGACTCACGCCTGAGCAGGTAGTGGCTATTGCATCC
AACGGAGGGGGCAGACCCGCACTGGAGTCAATCGTGGCCCAGCTTTCGAGGCCGGA
CCCCGCGCTGGCCGCACTCACTAATGATCATCTTGTAGCGCTGGCCTGCCTCGGCGG
ACGACCCGCCTTGGATGCGGTGAAGAAGGGGCTCCCGCACGCGCCTGCATTGATTA
AGCGGACCAACAGAAGGATTCCCGAGAGGAATCACATCGAGTGGCAGGTTCCCAAC
TCGTGAAGAGTGAACTTGAGGAGAAAAAGTCGGAGCTGCGGCACAAATTGAAATAC
GTACCGCATGAATACATCGAACTTATCGAAATTGCTAGGAACTCGACTCAAGACAG
AATCCTTGAGATGAAGGTAATGGAGTTCTTTATGAAGGTTTATGGATACCGAGGGAA
GCATCTCGGTGGATCACGAAAACCCGACGGAGCAATCTATACGGTGGGGAGCCCGA
TTGATTACGGAGTGATCGTCGACACGAAAGCCTACAGCGGTGGGTACAATCTTCCCA
TCGGGCAGGCAGATGAGATGCAACGTTATGTCGAAGAAAATCAGACCAGGAACAAA
CACATCAATCCAAATGAGTGGTGGAAAGTGTATCCTTCATCAGTGACCGAGTTTAAG
TTTTTGTTTGTCTCTGGGCATTTCAAAGGCAACTATAAGGCCCAGCTCACACGGTTG
AATCACATTACGAACTGCAATGGTGCGGTTTTGTCCGTAGAGGAACTGCTCATTGGT
GGAGAAATGATCAAAGCGGGAACTCTGACACTGGAAGAAGTCAGACGCAAGTTTAA
CAATGGCGAGATCAATTTCCGCTCA

### Example 9

### Cloning (construction) of AAV constructs

**construction of AAV-promoter-TALE-effector backbone.** For construction of AAV-promoter-TALE-effector a backbone was cloned by standard subcloning methods. Specifically, the vector contained an antibiotics resistance gene, such as ampicillin resistance and two AAV inverted terminal repeats (itr's) flanking the promoter-TALE-effector insert (sequences, see below). The promoter (hSyn), the effector domain (VP64, SID4X or CIB1 in this example)/ the N- and C-terminal portion of the TALE gene containing a spacer with two typeIIS restriction sites (BsaI in this instance) were subcloned into this vector. To achieve subcloning, each DNA component was amplified using polymerase-chain reaction and then digested with specific restriction enzymes to create matching DNA sticky ends. The vector was similarily digested with DNA restriction enzymes. All DNA fragments were subsequently allowed to anneal at matching ends and fused together using a ligase enzyme.

**Assembly of individual TALEs into AAV-promoter-TALE-effector backbone.** For incorporating different TALE monomer sequences into the AAV-promoter-TALE-effector backbone described above, a strategy based on restriction of individual monomers with type IIS restriction enzymes and ligation of their unique overhangs to form an assembly of 12 to 16 monomers to form the final TALE and ligate it into the AAV-promoter-TALE-effector backbone by using the type IIS sites present in the spacer between the N- and C-term (termed golden gate assembly). This method of TALE monomer assembly has previously been described by us (NE Sanjana, L Cong, Y Zhou, M M Cunniff, G Feng & F Zhang A transcription activator-like effector toolbox for genome engineering Nature Protocols 7, 171-192 (2012) doi:10.1038/nprot.2011.431)

By using the general cloning strategy outlined above, AAV vectors containing different promoters, effector domains and TALE monomer sequences can be easily constructed.

Nucleotide Sequences:
**Left AAV ITR** 
cctgcaggcagctgcgcgctcgctcgctcactgaggccgcccgggcaaagcccgggcgtcgggcgacctttggtcgcc
cggcctcagtgagcgagcgagcgcgcagagagggagtggccaactccatcactaggggttcct
**Right AAV ITR** 
Aggaacccctagtgatggagttggccactccctctctgcgcgctcgctcgctcactgaggccgggcgaccaaaggtcgc
ccgacgcccgggctttgcccgggcggcctcagtgagcgagcgagcgcgcagctgcctgcagg
**hSyn promoter** 
gtgtctagactgcagagggccctgcgtatgagtgcaagtgggttttaggaccaggatgaggcggggtgggggtgcctac
ctgacgaccgaccccgacccactggacaagcacccaacccccattccccaaattgcgcatcccctatcagagaggggggaggggaaaca
ggatgcggcgaggcgcgtgcgcactgccagcttcagcaccgcggacagtgccttcgcccccgcctggcggcgcgcgccaccgccgcc
tcagcactgaaggcgcgctgacgtcactcgccggtcccccgcaaactccccttcccggccaccttggtcgcgtccgcgccgccgccggc
ccagccggaccgcaccacgcgaggcgcgagataggggggcacgggcgcgaccatctgcgctgcggcgccggcgactcagcgctgc
ctcagtctgcggtgggcagcggaggagtcgtgtcgtgcctgagagcgcagtcgagaa
**TALE N-term (+136 AA truncation)** 
GTAGATTTGAGAACTTTGGGATATTCACAGCAGCAGCAGGAAAAGATCAA
GCCCAAAGTGAGGTCGACAGTCGCGCAGCATCACGAAGCGCTGGTGGGTCATGGGT
TTACACATGCCCACATCGTAGCCTTGTCGCAGCACCCTGCAGCCCTTGGCACGGTCG
CCGTCAAGTACCAGGACATGATTGCGGCGTTGCCGGAAGCCACACATGAGGCGATC
GTCGGTGTGGGGAAACAGTGGAGCGGAGCCCGAGCGCTTGAGGCCCTGTTGACGGT
CGCGGGAGAGCTGAGAGGGCCTCCCCTTCAGCTGGACACGGGCCAGTTGCTGAAGA
 
TCGCGAAGCGGGGAGGAGTCACGGCGGTCGAGGCGGTGCACGCGTGGCGCAATGC
GCTCACGGGAGCACCCCTCAAC
**TALE C-term (+63 AA truncation)** 
CGGACCCCGCGCTGGCCGCACTCACTAATGATCATCTTGTAGCGCTGGCC
TGCCTCGGCGGACGACCCGCCTTGGATGCGGTGAAGAAGGGGCTCCCGCACGCGCC
TGCATTGATTAAGCGGACCAACAGAAGGATTCCCGAGAGGACATCACATCGAGTGG
CA
**Ampicillin resistance gene**

atgagtattcaacatttccgtgtcgcccttattcccttttttgcggcattttgccttcctgtttttgctcacccagaaacgctggtga
aagtaaaagatgctgaagatcagttgggtgcacgagtgggttacatcgaactggatctcaacagcggtaagatccttgagagttttcgcccc
gaagaacgttttccaatgatgagcacttttaaagttctgctatgtggcgcggtattatcccgtattgacgccgggcaagagcaactcggtcgcc
gcatacactattctcagaatgacttggttgagtactcaccagtcacagaaaagcatcttacggatggcatgacagtaagagaattatgcagtg
ctgccataaccatgagtgataacactgcggccaacttacttctgacaacgatcggaggaccgaaggagctaaccgcttttttgcacaacatg
ggggatcatgtaactcgccttgatcgttgggaaccggagctgaatgaagccataccaaacgacgagcgtgacaccacgatgcctgtagca
atggcaacaacgttgcgcaaactattaactggcgaactacttactctagcttcccggcaacaattaatagactggatggaggcggataaagtt
gcaggaccacttctgcgctcggcccttccggctggctggtttattgctgataaatctggagccggtgagcgtgggtctcgcggtatcattgca
gcactggggccagatggtaagccctcccgtatcgtagttatctacacgacggggagtcaggcaactatggatgaacgaaatagacagatc
gctgagataggtgcctcactgattaagcattgg

### Example 10

### Optical Control of Endogenous Mammalian Transcription

The ability to directly modulate transcription of the endogenous mammalian genome is critical for elucidating normal gene function and disease mechanisms. Here, Applicants describe the development of Light-Inducible Transcriptional Effectors (LITEs), a two-hybrid system integrating the customizable TALE DNA-binding domain with the light-sensitive cryptochrome 2 protein and its interacting partner CIB1 from *Arabidopsis thaliana.* LITEs can be activated within minutes, mediating reversible bidirectional regulation of endogenous mammalian gene expression as well as targeted epigenetic chromatin modifications. Applicants have applied this system in primary mouse neurons, as well as in the brain of awake, behaving mice *in vivo.* The LITE system establishes a novel mode of optogenetic control of endogenous cellular processes and enables direct testing of the causal roles of genetic and epigenetic regulation.

The dynamic nature of gene expression enables cellular programming, homeostasis, and environmental adaptation in living systems. Dissecting the contributions of genes to cellular and organismic function therefore requires an approach that enables spatially and temporally controlled modulation of gene expression. Microbial and plant-derived light-sensitive proteins have been engineered as optogenetic actuators, enabling the use of light - which provides high spatiotemporal resolution - to control many cellular functions (Deisseroth, K. Optogenetics. Nature methods 8, 26-29, doi:10.1038/nmeth.f.324 (2011); Zhang, F. et al. The microbial opsin family of optogenetic tools. Cell 147, 1446-1457, doi:10.1016/j.cell.2011.12.004 (2011); Levskaya, A., Weiner, O. D., Lim, W. A. & Voigt, C. A. Spatiotemporal control of cell signalling using a light-switchable protein interaction. Nature 461, 997-1001, doi:10.1038/nature08446 (2009); Yazawa, M., Sadaghiani, A. M., Hsueh, B. & Dolmetsch, R. E. Induction of protein-protein interactions in live cells using light. Nature biotechnology 27, 941-945, doi:10.1038/nbt.1569 (2009); Strickland, D. et al. TULIPs: tunable, light-controlled interacting protein tags for cell biology. Nature methods 9, 379-384, doi:10.1038/nmeth.1904 (2012); Kennedy, M. J. et al. Rapid blue-light-mediated induction of protein interactions in living cells. Nature methods 7, 973-975, doi:10.1038/nmeth.1524 (2010); Shimizu-Sato, S., Huq, E., Tepperman, J. M. & Quail, P. H. A light-switchable gene promoter system. Nature biotechnology 20, 1041-1044, doi:10.1038/nbt734 (2002); Ye, H., Daoud-El Baba, M., Peng, R. W. & Fussenegger, M. A synthetic optogenetic transcription device enhances blood-glucose homeostasis in mice. Science 332, 1565-1568, doi:10.1126/science.1203535 (2011); Polstein, L. R. & Gersbach, C. A. Light-inducible spatiotemporal control of gene activation by customizable zinc finger transcription factors. Journal of the American Chemical Society 134, 16480-16483, doi:10.1021/ja3065667 (2012); Bugaj, L. J., Choksi, A. T., Mesuda, C. K., Kane, R. S. & Schaffer, D. V. Optogenetic protein clustering and signaling activation in mammalian cells. Nature methods (2013) and Zhang, F. et al. Multimodal fast optical interrogation of neural circuitry. Nature 446, 633-639, doi:10.1038/nature05744 (2007)). However, versatile and robust technologies to directly modulate endogenous transcriptional regulation using light remain elusive.

Here, Applicants report the development of Light-Inducible Transcriptional Effectors (LITEs), a modular optogenetic system that enables spatiotemporally precise control of endogenous genetic and epigenetic processes in mammalian cells. LITEs combine the programmable DNA-binding domain of transcription activator-like effectors (TALEs) (Boch, J. et al. Breaking the code of DNA binding specificity of TAL-type III effectors. Science 326, 1509-1512, doi:10.1126/science.1178811 (2009) and Moscou, M. J. & Bogdanove, A. J. A simple cipher governs DNA recognition by TAL effectors. Science 326, 1501, doi:10.1126/science.1178817 (2009)) from *Xanthomonas* sp. with the light-inducible heterodimeric proteins cryptochrome 2 (CRY2) and CIB1 from *Arabidopsis thaliana* (Kennedy, M. J. et al. Rapid blue-light-mediated induction of protein interactions in living cells. Nature methods 7, 973-975, doi:10.1038/nmeth.1524 (2010) and Liu, H. et al. Photoexcited CRY2 interacts with CIB1 to regulate transcription and floral initiation in Arabidopsis. Science 322, 1535-1539, doi:10.1126/science.1163927 (2008)). They do not require the introduction of heterologous genetic elements, do not depend on exogenous chemical co-factors, and exhibit fast and reversible dimerization kinetics (Levskaya, A., Weiner, O. D., Lim, W. A. & Voigt, C. A. Spatiotemporal control of cell signalling using a light-switchable protein interaction. Nature 461, 997-1001, doi:10.1038/nature08446 (2009); Yazawa, M., Sadaghiani, A. M., Hsueh, B. & Dolmetsch, R. E. Induction of protein-protein interactions in live cells using light. Nature biotechnology 27, 941-945, doi:10.1038/nbt.1569 (2009), Kennedy, M. J. et al. Rapid blue-light-mediated induction of protein interactions in living cells. Nature methods 7, 973-975, doi:10.1038/nmeth.1524 (2010); Shimizu-Sato, S., Huq, E., Tepperman, J. M. & Quail, P. H. A light-switchable gene promoter system. Nature biotechnology 20, 1041-1044, doi:10.1038/nbt734 (2002) and Liu, H. et al. Photoexcited CRY2 interacts with CIB1 to regulate transcription and floral initiation in Arabidopsis. Science 322, 1535-1539, doi:10.1126/science.1163927 (2008)). Like other optogenetic tools, LITEs can be packaged into viral vectors and genetically targeted to probe specific cell populations. Applicants demonstrate the application of this system in primary neurons as well as in the mouse brain *in vivo.*

The LITE system contains two independent components (**FIG. 36A**): The first component is the genomic anchor and consists of a customized TALE DNA-binding domain fused to the light-sensitive CRY2 protein (TALE-CRY2). The second component consists of CIB1 fused to the desired transcriptional effector domain (CIB1-effector). To ensure effective nuclear targeting, Applicants attached a nuclear localization signal (NLS) to both modules. In the absence of light (inactive state), TALE-CRY2 binds the promoter region of the target gene while CIB1-effector remains free within the nuclear compartment. Illumination with blue light (peak ∼450 nm) triggers a conformational change in CRY2 and subsequently recruits CIB1-effector (VP64 shown in **FIG. 36A**) to the target locus to mediate transcriptional modulation. This modular design allows each LITE component to be independently engineered. For example, the same genomic anchor can be combined with activating or repressing effectors (Beerli, R. R., Segal, D. J., Dreier, B. & Barbas, C. F., 3rd. Toward controlling gene expression at will: specific regulation of the erbB-2/HER-2 promoter by using polydactyl zinc finger proteins constructed from modular building blocks. Proceedings of the National Academy of Sciences of the United States of America 95, 14628-14633 (1998) and Cong, L., Zhou, R., Kuo, Y.-c., Cunniff, M. & Zhang, F. Comprehensive interrogation of natural TALE DNA-binding modules and transcriptional repressor domains. Nat Commun 3, 968) to exert positive and negative transcriptional control over the same endogenous genomic locus.

In order to identify the most effective LITE architecture, Applicants fused TALE and the transcriptional activator VP64 (Beerli, R. R., Segal, D. J., Dreier, B. & Barbas, C. F., 3rd. Toward controlling gene expression at will: specific regulation of the erbB-2/HER-2 promoter by using polydactyl zinc finger proteins constructed from modular building blocks. Proceedings of the National Academy of Sciences of the United States of America 95, 14628-14633 (1998); Zhang, F. et al. Efficient construction of sequence-specific TAL effectors for modulating mammalian transcription. Nat Biotechnol 29, 149-153, doi:10.1038/nbt.1775 (2011); Miller, J. C. et al. A TALE nuclease architecture for efficient genome editing. Nature biotechnology 29, 143-148, doi:10.1038/nbt.1755 (2011) and Hsu, P. D. & Zhang, F. Dissecting neural function using targeted genome engineering technologies. ACS chemical neuroscience 3, 603-610, doi:10.1021/cn300089k (2012).) to different truncations (Kennedy, M. J. et al. Rapid blue-light-mediated induction of protein interactions in living cells. Nature methods 7, 973-975, doi:10.1038/nmeth.1524 (2010)) of CRY2 and CIB1, respectively, and assessed the efficacy of each design by measuring blue light illumination induced transcriptional changes of the neural lineage-specifying transcription factor neurogenin 2 (*Neurog2*) (**FIG. 36B**). Applicants evaluated full-length CRY2 as well as a truncation consisting of the photolyase homology region alone (CRY2PHR, amino acids 1-498) (Kennedy, M. J. et al. Rapid blue-light-mediated induction of protein interactions in living cells. Nature methods 7, 973-975, doi:10.1038/nmeth.1524 (2010)). For CIB1, Applicants tested the full-length protein as well as an N-terminal domain-only fragment (CIBN, amino acids 1-170) (Kennedy, M. J. et al. Rapid blue-light-mediated induction of protein interactions in living cells. Nature methods 7, 973-975, doi:10.1038/nmeth.1524 (2010)). 3 out of 4 initial LITE pairings produced significant light-induced *Neurog2* mRNA upregulation in Neuro 2a cells (p < 0.001, **FIG. 36B**)**.** Of these, TALE-CRY2PHR::CIB1-VP64 yielded the highest absolute light-mediated mRNA increase when normalized to either GFP-only control or unstimulated LITE samples (**FIG. 36B**), and was therefore applied in subsequent experiments.

Having established an effective LITE architecture, Applicants systematically optimized light stimulation parameters, including wavelength (**FIG. 40**), duty cycle (**FIG. 41**), and light intensity (**FIG. 42** and **Example 11**) (Banerjee, R. et al. The signaling state of Arabidopsis cryptochrome 2 contains flavin semiquinone. The Journal of biological chemistry 282, 14916-14922, doi:10.1074/jbc.M700616200 (2007)). Applicants also compared the activation domains VP16 and p65 in addition to VP64 to test the modularity of the LITE CIB1-effector component. All three domains produced a significant light-dependent *Neurog2* mRNA upregulation (p < 0.001, **FIG. 43**). Applicants selected VP64 for subsequent experiments due to its lower basal activity in the absence of light-stimulation.

Manipulation of endogenous gene expression presents various challenges, as the rate of expression depends on many factors, including regulatory elements, mRNA processing, and transcript stability (Moore, M. J. & Proudfoot, N. J. Pre-mRNA processing reaches back to transcription and ahead to translation. Cell 136, 688-700, doi:10.1016/j.cell.2009.02.001 (2009) and Proudfoot, N. J., Furger, A. & Dye, M. J. Integrating mRNA processing with transcription. Cell 108, 501-512 (2002)). Although the interaction between CRY2 and CIB1 occurs on a subsecond timescale (Kennedy, M. J. et al. Rapid blue-light-mediated induction of protein interactions in living cells. Nature methods 7, 973-975, doi:10.1038/nmeth.1524 (2010)), LITE-mediated activation is likely to be limited by the inherent kinetics of transcription. Applicants investigated the on-kinetics of LITE-mediated *Neurog2* expression by measuring mRNA levels during a time course of light stimulation from 30 min to 24 h (**FIG. 36C**). Relative levels of *Neurog2* mRNA increased considerably as early as 30 min after the onset of light stimulation and rose steadily until saturating at 12 h with a roughly 20-fold upregulation compared to GFP-transfected negative controls. Similarly, Applicants assessed the off-kinetics of the system by stimulating cells for 6 h and measuring the level of *Neurog2* transcripts at multiple time points after ceasing illumination (**FIG. 36D**)**.** *Neurog2* mRNA levels briefly increased up to 30 min post-stimulation, an effect that may have resulted from residual CRY2PHR-CIB1 dimerization or from previously recruited RNA polymerases. Thereafter, *Neurog2* expression declined with a half-life of ∼3 h, demonstrating that transcripts return to natural levels in the absence of light stimulation. In contrast, a small-molecule inducible TALE system based on the plant hormone abcisic acid receptor (Liang, F.-S., Ho, W. Q. & Crabtree, G. R. Engineering the ABA Plant Stress Pathway for Regulation of Induced Proximity. Sci. Signal. 4, rs2-, doi:10.1126/scisignal.2001449 (2011)) exhibited slower on- and off-kinetics (**FIG. 44**), potentially limited by drug diffusion, metabolism, or clearance.

Applicants next explored the utility of LITEs for neuronal applications via viral transduction. Applicants developed an adeno-associated virus (AAV)-based vector for the delivery of TALE genes and a simplified process for AAV production (**FIGS. 37A and B****,** **FIG. 45**, and **Example 11**)**.** The ssDNA-based genome of AAV is less susceptible to recombination, providing an advantage over lentiviral vectors (Holkers, M. et al. Differential integrity of TALE nuclease genes following adenoviral and lentiviral vector gene transfer into human cells. Nucleic acids research 41, e63, doi:10.1093/nar/gks1446 (2013)).

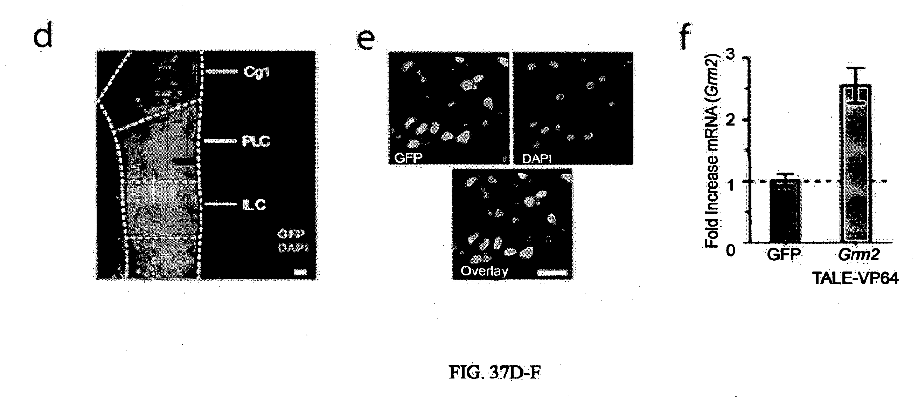

To characterize AAV-mediated TALE delivery for modulating transcription in primary mouse cortical neurons, Applicants constructed a panel of TALE-VP64 transcriptional activators targeting 28 murine loci in all, including genes involved in neurotransmission or neuronal differentiation, ion channel subunits, and genes implicated in neurological diseases. DNase I-sensitive regions in the promoter of each target gene provided a guide for TALE binding sequence selections (**FIG. 46**). Applicants confirmed that TALE activity can be screened efficiently using Applicants' AAV-TALE production process (**FIG. 45**) and found that TALEs chosen in this fashion and delivered into primary neurons using AAV vectors activated a diverse array of gene targets to varying extents (**FIG. 37C**). Moreover, stereotactic delivery of AAV-TALEs mediated robust expression *in vivo* in the mouse prefrontal cortex (**FIG. 37D****, E**). Expression of TALE(*Grm2*)-VP64 in the mouse infralimbic cortex (ILC) induced a 2.5-fold increase in *Grm2* mRNA levels compared to GFP-injected controls (**FIG. 37F**).

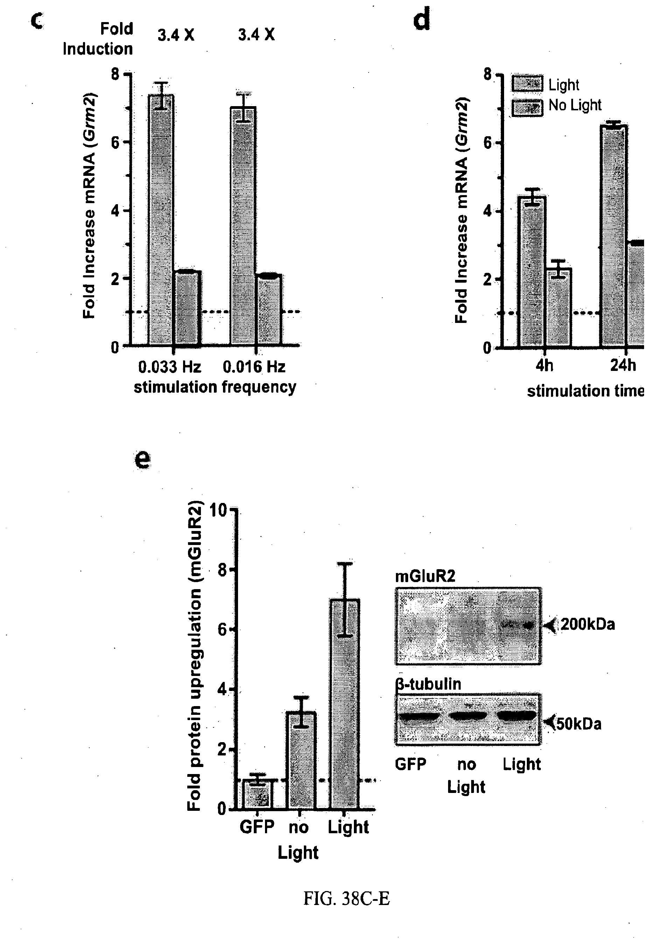

Having delivered TALE activators into cultured primary neurons, Applicants next sought to use AAV as a vector for the delivery of LITE components. To do so, Applicants needed to ensure that the total viral genome size of each recombinant AAV, with the LITE transgenes included, did not exceed the packaging limit of 4.8 kb (Wu, Z., Yang, H. & Colosi, P. Effect of Genome Size on AAV Vector Packaging. Mol Ther 18, 80-86 (2009)). Applicants shortened the TALE N- and C-termini (keeping 136 aa in the N-terminus and 63 aa in the C-terminus) and exchanged the CRY2PHR (1.5kb) and CIB1 (1kb) domains (TALE-CIB1 and CRY2PHR-VP64; **FIG. 38A**). These LITEs were delivered into primary cortical neurons via co-transduction by a combination of two AAV vectors (**FIG. 38B**; delivery efficiencies of 83-92% for individual components with >80% co-transduction efficiency). Applicants tested a *Grm2-*targeted LITE at 2 light pulsing frequencies with a reduced duty cycle of 0.8% to ensure neuron health (**FIG. 47**). Both stimulation conditions achieved a ∼7-fold light-dependent increase in *Grm2* mRNA levels (**FIG. 38C**). Further study verified that substantial target gene expression increases could be attained quickly (4-fold upregulation within 4 h; **FIG. 38D**). In addition, Applicants observed significant upregulation of mGluR2 protein after stimulation, demonstrating that changes effected by LITEs at the mRNA level translate to the protein level (p < 0.01 vs GFP control, p < 0.05 vs no-light condition; **FIG. 38E**).

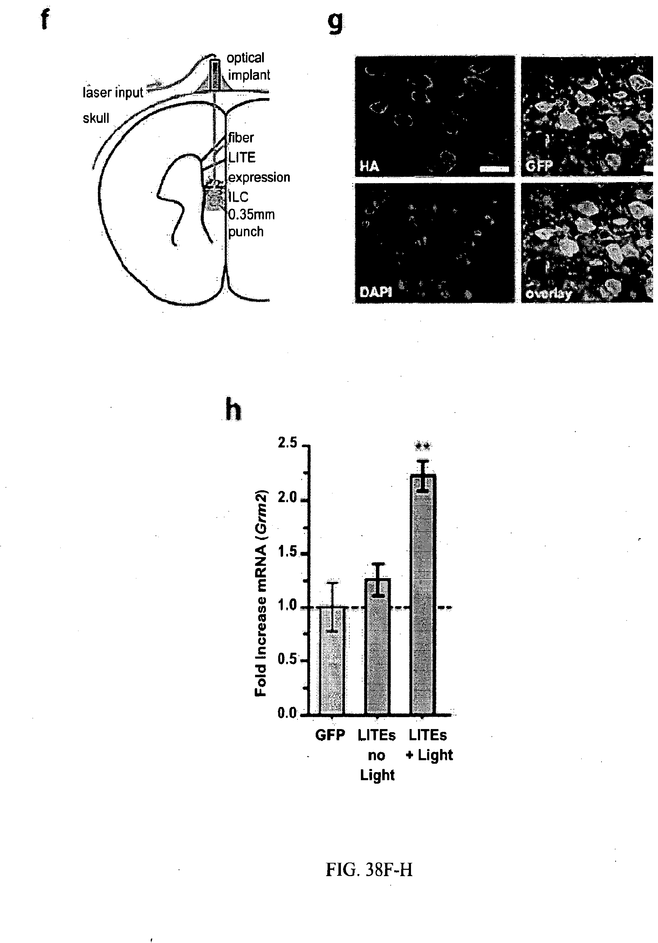

To apply the LITE system *in vivo,* Applicants stereotactically delivered a 1:1 mixture of high concentration AAV vectors (10¹² DNAseI resistant particles/mL) carrying the *Grm2-*targeting TALE-CIB1 and CRY2PHR-VP64 LITE components into ILC of wildtype C57BL/6 mice. To provide optical stimulation of LITE-expressing neurons *in vivo,* Applicants implanted a fiber optic cannula at the injection site (**FIG. 38F** and **FIG. 48**) (Zhang, F. et al. Optogenetic interrogation of neural circuits: technology for probing mammalian brain structures. Nat Protoc 5, 439-456, doi:10.1038/nprot.2009.226 (2010)). Neurons at the injection site were efficiently co-transduced by both viruses, with >80% of transduced cells expressing both TALE(Grm2)-CIB1 and CRY2PHR-VP64 (**FIG. 38G** and **FIG. 49**). 8 days post-surgery, Applicants stimulated the ILC of behaving mice by connecting a solid-state 473 nm laser to the implanted fiber cannula. Following a 12 h stimulation period (5 mW, 0.8% duty cycle using 0.5 s light pulses at 0.0167 Hz), brain tissue from the fiber optic cannula implantation site was analyzed (**FIG. 38H**) for changes in *Grm2* mRNA. Applicants observed a significant increase in *Grm2* mRNA after light stimulation compared with unstimulated ILC (*p* ≤ 0.01). Taken together, these results confirm that LITEs enable optical control of endogenous gene expression in cultured neurons and *in vivo.*

Due to the persistence of basal up-regulation observed in the no-light condition of *in vivo* LITE activators, Applicants undertook another round of optimization, aiming to identify and attenuate the source of the background and improve the efficiency of light-mediated gene induction (light/no-light ratio of gene expression). Neurons expressing only the LITE targeting component TALE-CIB1 produced *Grm2* mRNA increases similar to those found in unstimulated neurons expressing both LITE components (both p < 0.001 versus GFP controls), while the effector component CRY2PHR-VP64 alone did not significantly affect transcription (p > 0.05, **FIG. 50**), implying that the background transcriptional activation caused by LITE could arise solely from the DNA targeting component.

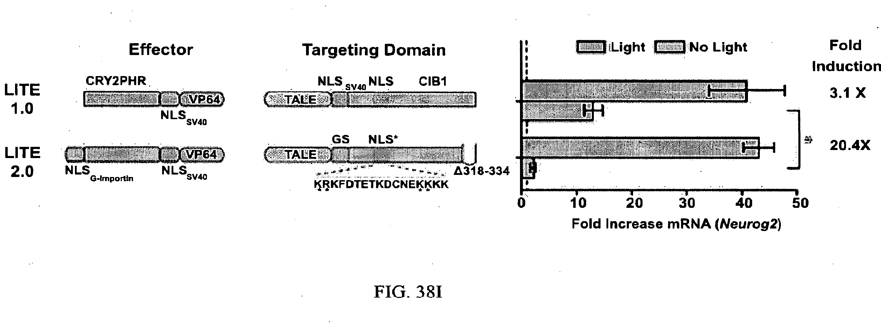

Accordingly, Applicants carried out a comprehensive screen to reduce the basal target up-regulation caused by TALE-CIB 1 (**FIG. 51**). The optimization focused on two strategies: First, CIB1 is a plant transcription factor and may have intrinsic regulatory effects even in mammalian cells (Liu, H. et al. Photoexcited CRY2 Interacts with CIB1 to Regulate Transcription and Floral Initiation in Arabidopsis. Science 322, 1535-1539, doi:10.1126/science.1163927 (2008)). Applicants sought to eliminate these effects by deleting three CIB1 regions conserved amongst the basic helix-loop-helix transcription factors of higher plants (**FIG. 51**). Second, Applicants aimed to prevent TALE-CIB 1 from binding the target locus in the absence of light. To achieve this, Applicants engineered TALE-CIB1 to localize in cytoplasm until light-induced dimerization with the NLS-containing CRY2PHR-VP64 (**FIG. 52**). To test both strategies independently or in combination, Applicants evaluated 73 distinct LITE architectures and identified 12 effector-targeting domain pairs (denoted by the "+" column in **FIG. 51** **and** **FIG. 53**) with both improved light-induction efficiency and reduced overall baseline (fold mRNA increase in the no-light condition compared with the original LITE1.0; p < 0.05). One architecture incorporating both strategies, designated LITE2.0, demonstrated the highest light induction (light/no-light = 20.4) and resulted in greater than 6-fold reduction of background activation compared with the original architecture (**FIG. 38I**)**.** Another - LITE1.9.1 - produced a minimal background mRNA increase (1.06) while maintaining four-fold light induction (**FIG. 53**).

Applicants sought to further expand the range of processes accessible by TALE and LITE modulation. Endogenous transcriptional repression is often mediated by chromatin modifying enzymes such as histone methyltransferases (HMTs) and deacetylases (HDACs). Applicants have previously shown that the mSin3 interaction domain (SID), part of the mSin3-HDAC complex, can be fused with TALE in order to down regulate target genes in 293FT cells (Beerli, R. R., Segal, D. J., Dreier, B. & Barbas, C. F., 3rd. Toward controlling gene expression at will: specific regulation of the erbB-2/HER-2 promoter by using polydactyl zinc finger proteins constructed from modular building blocks. Proceedings of the National Academy of Sciences of the United States of America 95, 14628-14633 (1998) and Cong, L., Zhou, R., Kuo, Y.-c., Cunniff, M. & Zhang, F. Comprehensive interrogation of natural TALE DNA-binding modules and transcriptional repressor domains. Nat Commun 3, 968, doi:http://www.nature.com/ncomms/journal/v3/n7/suppinfo/ncomms1962 S1.html (2012)). Hoping to further improve this TALE repressor, Applicants reasoned that four repeats of SID - analogous to the quadruple VP16 tandem repeat architecture of VP64 (Beerli, R. R., Segal, D. J., Dreier, B. & Barbas, C. F., 3rd. Toward controlling gene expression at will: specific regulation of the erbB-2/HER-2 promoter by using polydactyl zinc finger proteins constructed from modular building blocks. Proceedings of the National Academy of Sciences of the United States of America 95, 14628-14633 (1998)) - might augment its potency to repress gene transcription. Indeed, TALE-SID4X constructs were twice as effective as TALE-SID in 293FT cells (**FIG**. **54A and 54B**) and also mediated efficient gene repression in neurons (**FIG. 54C and 54D**).

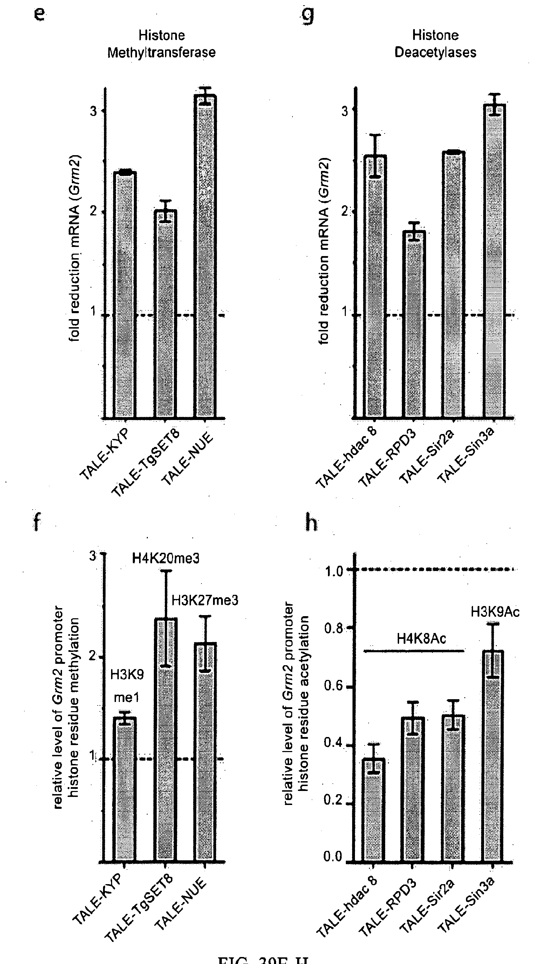

Applicants hypothesized that TALE-mediated targeting of histone effectors to endogenous loci could induce specific epigenetic modifications, enabling the interrogation of epigenetic as well as transcriptional dynamics (**FIG. 39A**). Applicants generated CRY2PHR-SID4X constructs and demonstrated light-mediated transcription repression of *Grm2* in neurons (**FIG. 39B and FIG. 39C**), concomitant with ∼2-fold reduction in H3K9 acetylation at the targeted *Grm2* promoter (**FIG. 39D**). In an effort to expand the diversity of histone residue targets for locus specific histone modification, Applicants derived a set of repressive histone effector domains from the literature (**Table 6**). Drawn from across a wide phylogenetic spectrum, the domains included HDACs, histone methyltransferases (HMTs), and histone acetyltransferase (HAT) inhibitors, as well as HDAC and HMT recruiting proteins. Preference was given to proteins and functional truncations of small size to facilitate efficient AAV packaging. The resulting epigenetic-modifying TALE-histone effector fusion constructs (epiTALEs) were tested in primary neurons and Neuro 2a cells for their ability to repress *Grm2* and *Neurog2* transcription, respectively (**FIG. 39E, FIG. 39F** **and** **FIG. 55**). In primary neurons, 23 out of 24 epiTALEs successfully repressed transcription of *grm2* using the statistical criteria of *p* < 0.05. Similarly, epiTALE expression in Neuro 2a cells led to decreased *Neurog2* expression for 20 of the 32 histone effector domains tested (p < 0.05). A subset of promising epiTALEs were expressed in primary neurons and Neuro 2a cells and relative histone residue mark levels in the targeted endogenous promoter were quantified by ChIP-RT-qPCR (**FIG. 39G**, **FIG. 39H** **and** **FIG. 56**). In primary neurons or Neuro 2a cells, levels of H3K9me1, H4K20me3, H3K27me3, H3K9ac, and H4K8ac were altered by epiTALEs derived from, respectively, KYP (*A. thaliana*), TgSET8 (*T. gondii*)*,* NUE and PHF19 (C. *trachomatis and H. sapiens*), Sin3a, Sirt3 and NcoR, (all *H. sapiens*) and hdac8, RPD3, and Sir2a (*X laevis, S cerevisiae, P falciparum*)*.* These domains provide a ready source of epigenetic effectors to expand the range of transcriptional and epigenetic controls by LITE.

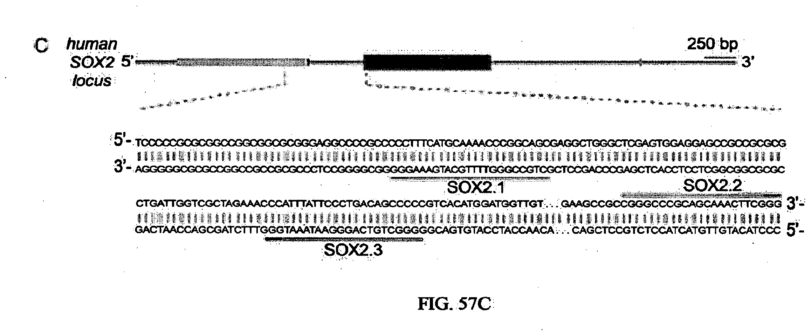

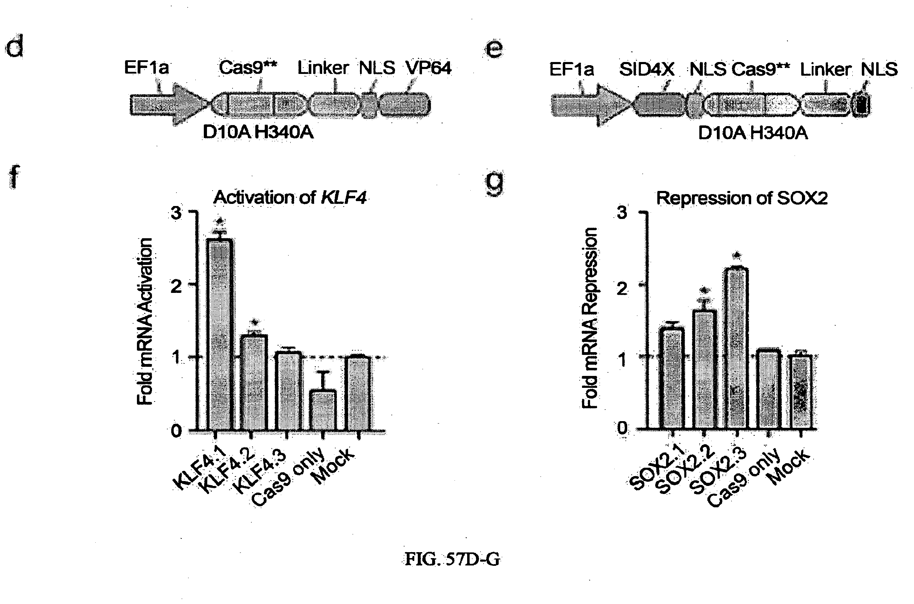

The ability to achieve spatiotemporally precise *in vivo* gene regulation in heterogeneous tissues such as the brain would allow researchers to ask questions about the role of dynamic gene regulation in processes as diverse as development, learning, memory, and disease progression. LITEs can be used to enable temporally precise, spatially targeted, and bimodal control of endogenous gene expression in cell lines, primary neurons, and in the mouse brain *in vivo.* The TALE DNA binding component of LITEs can be customized to target a wide range of genomic loci, and other DNA binding domains such as the RNA-guided Cas9 enzyme (Cong, L. et al. Multiplex genome engineering using CRISPR/Cas systems. Science 339, 819-823 (2013)) may be used in lieu of TALE to enable versatile locus-specific targeting (**FIG. 57**). Novel modes of LITE modulation can also be achieved by replacing the effector module with new functionalities such as epigenetic modifying enzymes (de Groote, M. L., Verschure, P. J. & Rots, M. G. Epigenetic Editing: targeted rewriting of epigenetic marks to modulate expression of selected target genes. Nucleic acids research 40, 10596-10613, doi:10.1093/nar/gks863 (2012)). Therefore the LITE system enables a new set of capabilities for the existing optogenetic toolbox and establishes a highly generalizable and versatile platform for altering endogenous gene regulation using light.

**Methods Summary.** LITE constructs were transfected into in Neuro 2A cells using GenJet. AAV vectors carrying TALE or LITE constructs were used to transduce mouse primary embryonic cortical neurons as well as the mouse brain *in vivo.* RNA was extracted and reverse transcribed and mRNA levels were measured using TaqMan-based RT-qPCR. Light emitting diodes or solid-state lasers were used for light delivery in tissue culture and *in vivo* respectively.

### Design and construction of LITEs. All LITE constructs sequences can be found in Example 11.

**Neuro 2a culture and experiments.** Neuro 2a cells (Sigma-Aldrich) were grown in media containing a 1:1 ratio of OptiMEM (Life Technologies) to high-glucose DMEM with GlutaMax and Sodium Pyruvate (Life Technologies) supplemented with 5% HyClone heat-inactivated FBS (Thermo Scientific), 1% penicillin/streptomycin (Life Technologies), and passaged at 1:5 every 2 days. 120,000 cells were plated in each well of a 24-well plate 18-20 h prior to transfection. 1 h before transfection, media was changed to DMEM supplemented with 5% HyClone heat-inactivated FBS and 1% penicillin/streptomycin. Cells were transfected with 1.0 µg total of construct DNA (at equimolar ratios) per well with 1.5 µL of GenJet (SignaGen Laboratories) transfection reagent according to the manufacturer's instructions. Media was exchanged 24 h and 44 h post-transfection and light stimulation was started at 48 h. Stimulation parameters were: 5 mW/cm2, 466 nm, 7 % duty cycle (1 s light pulse 0.067 Hz) for 24 h unless indicated otherwise in figure legends. RNA was extracted using the RNeasy kit (Qiagen) according to manufacturer's instructions and 1 µg of RNA per sample was reverse-transcribed using qScript (Quanta Biosystems). Relative mRNA levels were measured by quantitative real-time PCR (qRT-PCR) using TaqMan probes specific for the targeted gene as well as GAPDH as an endogenous control (Life Technologies, see **Table 3** for Taqman probe BDs). ΔΔCt analysis was used to obtain fold-changes relative to negative controls transduced with GFP only and subjected to light stimulation. Toxicity experiments were conducted using the LIVE/DEAD assay kit (Life Technologies) according to instructions.

**AAV vector production.** 293FT cells (Life Technologies) were grown in antibiotic-free D10 media (DMEM high glucose with GlutaMax and Sodium Pyruvate, 10% heat-inactivated Hyclone FBS, and 1% 1M HEPES) and passaged daily at 1:2-2.5. The total number of passages was kept below 10 and cells were never grown beyond 85% confluence. The day before transfection, 1x10⁶ cells in 21.5 mL of D10 media were plated onto 15 cm dishes and incubated for 18-22 hours or until ∼80% confluence. For use as a transfection reagent, 1 mg/mL of PEI "Max" (Polysciences) was dissolved in water and the pH of the solution was adjusted to 7.1. For AAV production, 10.4 µg of pDF6 helper plasmid, 8.7 µg of pAAV1 serotype packaging vector, and 5.2 µg of pAAV vector carrying the gene of interest were added to 434 µL of serum-free DMEM and 130 µL of PEI "Max" solution was added to the DMEM-diluted DNA mixture. The DNA/DMEM/PEI cocktail was vortexed and incubated at room temperature for 15 min. After incubation, the transfection mixture was added to 22 mL of complete media, vortexed briefly, and used to replace the media for a 15 cm dish of 293FT cells. For supernatant production, transfection supernatant was harvested at 48 h, filtered through a 0.45 µm PVDF filter (Millipore), distributed into aliquots, and frozen for storage at -80°C.

**Primary cortical neuron culture.** Dissociated cortical neurons were prepared from C57BL/6N mouse embryos on E16 (Charles River Labs). Cortical tissue was dissected in ice-cold HBSS - (50 mL 10x HBSS, 435 mL dH₂O, 0.3 M HEPES pH 7.3, and 1% penicillin/streptomycin). Cortical tissue was washed 3X with 20 mL of ice-cold HBSS and then digested at 37°C for 20 min in 8 mL of HBSS with 240 µL of 2.5% trypsin (Life Technologies). Cortices were then washed 3 times with 20 mL of warm HBSS containing 1 mL FBS. Cortices were gently triturated in 2 ml of HBSS and plated at 150,000 cells/well in poly-D-lysine coated 24-well plates (BD Biosciences). Neurons were maintained in Neurobasal media (Life Technologies), supplemented with IX B27 (Life Technologies), GlutaMax (Life Technologies) and 1% penicillin/streptomycin.

**Primary neuron transduction and light stimulation experiments.** Primary cortical neurons were transduced with 250 µL of AAV1 supernatant on DIV 5. The media and supernatant were replaced with regular complete neurobasal the following day. Neurobasal was exchanged with Minimal Essential Medium (Life Technologies) containing IX B27, GlutaMax (Life Technologies) and 1% penicillin/streptomycin 6 days after AAV transduction to prevent formation of phototoxic products from HEPES and riboflavin contained in Neurobasal during light stimulation.

Light stimulation was started 6 days after AAV transduction (DIV 11) with an intensity of 5 mW/cm², duty cycle of 0.8% (250 ms pulses at 0.033Hz or 500 ms pulses at 0.016Hz), 466 nm blue light for 24 h unless indicated otherwise in figure legends. RNA extraction and reverse transcription were performed using the Cells-to-Ct kit according to the manufacturers instructions (Life. Technologies). Relative mRNA levels were measured by quantitative real-time PCR (qRT-PCR) using TaqMan probes as described above for Neuro 2a cells.

**Immunohistochemistry of primary neurons.** For immunohistochemistry of primary neurons, cells were plated on poly-D-lysine/laminin coated coverslips (BD Biosciences) after harvesting. AAV1-transductions were performed as described above. Neurons were fixed 7 days post-transduction with 4% paraformaldehyde (Sigma Aldrich) for 15 min at RT. Blocking and permeabilization were performed with 10% normal goat serum (Life Technologies) and 0.5% Triton-X100 (Sigma-Aldrich) in DPBS (Life Technologies) for 1 h at room temperature. Neurons were incubated with primary antibodies overnight at 4°C, washed 3X with DPBS and incubated with secondary antibodies for 90 min at RT. For antibody providers and concentrations used, see **Table 4.** Coverslips were finally mounted using Prolong Gold Antifade Reagent with DAPI (Life Technologies) and imaged on an Axio Scope A.1 (Zeiss) with an X-Cite 120Q light source (Lumen Dynamics). Image were acquired using an AxioCam MRm camera and AxioVision 4.8.2.

**Western Blots.** For preparation of total protein lysates, primary cortical neurons were harvested after light stimulation (see above) in ice-cold lysis buffer (RIPA, Cell Signaling; 0.1% SDS, Sigma-Aldrich; and cOmplete ultra protease inhibitor mix, Roche Applied Science). Cell lysates were sonicated for 5 min at 'M' setting in a Bioruptor sonicator (Diagenode) and centrifuged at 21,000 x g for 10 min at 4°C. Protein concentration was determined using the RC DC protein assay (Bio-Rad). 30-40 µg of total protein per lane was separated under nonreducing conditions on 4-15% Tris-HCl gels (Bio-Rad) along with Precision Plus Protein Dual Color Standard (Bio-Rad) After wet electrotransfer to polyvinylidene difluoride membranes (Millipore) and membrane blocking for 45 min in 5% BLOT-QuickBlocker (Millipore) in Tris-buffered saline (TBS, Bio-Rad), western blots were probed with anti-mGluR2 (Abcam, 1:1.000) and anti-α-tubulin (Sigma-Aldrich 1:20,000) overnight at 4°C, followed by washing and antimouse-IgG HRP antibody incubation (Sigma-Aldrich, 1:5,000 - 1:10,000). For further antibody details see **Table 4.** Detection was performed via ECL Western blot substrate (SuperSignal West Femto Kit, Thermo Scientific). Blots were imaged with an AlphaImager (Innotech) system, and quantified using ImageJ software 1.46r.

**Production of concentrated and purified AAV1/2 vectors.** Production of concentrated and purified AAV for stereotactic injection in-vivo was done using the same initial steps outlined above for production of AAV1 supernatant. However, for transfection, equal ratios of AAV1 and AAV2 serotype plasmids were used instead of AAV1 alone. 5 plates were transfected per construct and cells were harvested with a cell-scraper 48 h post transfection. Purification of AAV1/2 particles was performed using HiTrap heparin affinity columns (GE Healthcare) (McClure, C., Cole, K. L., Wulff, P., Klugmann, M. & Murray, A. J. Production and titering of recombinant adeno-associated viral vectors. J Vis Exp, e3348, doi:10.3791/3348 (2011)). Applicants added a second concentration step down to a final volume of 100 µl per construct using an Amicon 500 µl concentration column (100 kDa cutoff, Millipore) to achieve higher viral titers. Titration of AAV was performed by qRT-PCR using a custom Taqman probe for WPRE (Life Technologies). Prior to qRT-PCR, concentrated AAV was treated with DNaseI (New England Biolabs) to achieve a measurement of DNaseI-resistant particles only. Following DNaseI heat-inactivation, the viral envelope was degraded by proteinase K digestion (New England Biolabs). Viral titer was calculated based on a standard curve with known WPRE copy numbers.

**Stereotactic injection of AAV1/2 and optical implant.** All animal procedures were approved by the MIT Committee on Animal Care. Adult (10-14 weeks old) male C57BL/6N mice were anaesthetized by intraperitoneal (i.p.) injection of Ketamine/Xylazine (100 mg/kg Ketamine and 10 mg/kg Xylazine) and pre-emptive analgesia was given (Buprenex, 1 mg/kg, i.p.). Craniotomy was performed according to approved procedures and 1µl of AAV1/2 was injected into ILC at 0.35/1.94/-2.94 (lateral, anterior and inferior coordinates in mm relative to bregma). During the same surgical procedure, an optical cannula with fiber (Doric Lenses) was implanted into ILC unilaterally with the end of the optical fiber located at 0.35/1.94/-2.64 relative to bregma. The cannula was affixed to the skull using Metabond dental cement (Parkell Inc) and Jet denture repair (Lang dental) to build a stable cone around it. The incision was sutured and proper post-operative analgesics were administered for three days following surgery.

**Immunohistochemistry on ILC brain sections.** Mice were injected with a lethal dose of Ketamine/Xylazine anaesthetic and transcardially perfused with PBS and 4% paraformaldehyde (PFA). Brains were additionally fixed in 4% PFA at 4°C overnight and then transferred to 30% sucrose for cryoprotection overnight at room temperature. Brains were then transferred into Tissue-Tek Optimal Cutting Temperature (OCT) Compound (Sakura Finetek) and frozen at -80°C. 18 µm sections were cut on a cryostat (Leica Biosystems) and mounted on Superfrost Plus glass slides (Thermo Fischer). Sections were post-fixed with 4% PFA for 15 min, and immunohistochemistry was performed as described for primary neurons above.

**Light stimulation and mRNA level analysis in ILC.** 8 days post-surgery, awake and freely moving mice were stimulated using a 473 nm laser source (OEM Laser Systems) connected to the optical implant via fiber patch cables and a rotary joint. Stimulation parameters were the same as used on primary neurons: 5 mW (total output), 0.8% duty cycle (500 ms light pulses at 0.016 Hz) for a total of 12 h. Experimental conditions, including transduced constructs and light stimulation are listed in **Table 5.**

After the end of light stimulations, mice were euthanized using CO₂ and the prefrontal cortices (PFC) were quickly dissected on ice and incubated in RNA later (Qiagen) at 4°C overnight. 200 µm sections were cut in RNA later at 4°C on a vibratome (Leica Biosystems). Sections were then frozen on a glass coverslide on dry ice and virally transduced ILC was identified under a fluorescent stereomicroscope (Leica M165 FC). A 0.35 mm diameter punch of ILC, located directly ventrally to the termination of the optical fiber tract, was extracted (Harris uni-core, Ted Pella). The brain punch sample was then homogenized using an RNase-free pellet-pestle grinder (Kimble Chase) in 50 µl Cells-to-Ct RNA lysis buffer and RNA extraction, reverse transcription and qRT-PCR was performed as described for primary neuron samples.

**Chromatin Immunoprecipitation.** Neurons or Neuro2a cells were cultured and transduced or transfected as described above. ChIP samples were prepared as previously described (Blecher-Gonen, R. et al. High-throughput chromatin immunoprecipitation for genome-wide mapping of in vivo protein-DNA interactions and epigenomic states. Nature protocols 8, 539-554 (2013)) with minor adjustments for the cell number and cell type. Cells were harvested in 24-well format, washed in 96-well format, and transferred to microcentrifuge tubes for lysis. Sample cells were directly lysed by water bath sonication with the Biorupter sonication device for 21 minutes using 30s on/off cycles (Diagenode). qPCR was used to assess enrichment of histone marks at the targeted locus.

**Statistical analysis.** All experiments were performed with a minimum of two independent biological replicates. Statistical analysis was performed with Prism (GraphPad) using Student's two-tailed t-test when comparing two conditions, ANOVA with Tukey's post-hoc analysis when comparing multiple samples with each other, and ANOVA with Dunnett's post-hoc analysis when comparing multiple samples to the negative control.

### Example 11

### Supplementary Information to Example 10: Optical Control of Endogenous Mammalian Transcription

**Photostimulation Hardware -** ***in vitro.** In vitro* light stimulation experiments were performed using a custom built LED photostimulation device. All electronic elements were mounted on a custom printed circuit board (ExpressPCB). Blue LEDs with peaks 466 nm (model #: YSL-R542B5C-A11, China Young Sun LED Technology; distributed by SparkFun Electronics as 'LED - Super Bright Blue' COM-00529), were arrayed in groups of three aligned with the wells of a Corning 24-well plate. LED current flow was regulated by a 25 mA DynaOhm driver (LEDdymanics #4006-025). Columns of the LED array were addressed by TTL control (Fairchild Semiconductor PN2222BU-ND) via an Arduino UNO microcontroller board. Light output was modulated via pulse width modulation. Light output was measured from a distance of 80 mm above the array utilizing a Thorlabs PM100D power meter and S120VC photodiode detector. In order to provide space for ventilation and to maximize light field uniformity, an 80 mm tall ventilation spacer was placed between the LED array and the 24-well sample plate. Fans (Evercool EC5015M12CA) were mounted along one wall of the spacer unit, while the opposite wall was fabricated with gaps to allow for increased airflow.

**Quantification of LIVE/DEAD® assay using ImageJ software.** Images of LIVE/DEAD (Life Technologies) stained cells were captured by fluorescence microscopy and processed as follows: Background was subtracted (*Process* → *Subtract Background*)*.* A threshold based on fluorescence area was set to ensure accurate identification of cell state (*Image* → *Adjust* → *Threshold*)*.* A segmentation analysis was performed to enable automated counting of individual cells (*Process* → *Binary* → *Watershed*). Finally, debris signals were filtered and cells were counted (*Analyze* → *Analyze Particles*). Toxicity was determined as the percentage of dead cells.

**Chemically-inducible TALEs.** Neuro2A cells were grown in a medium containing a 1:1 ratio of OptiMEM (Life Technologies) to high-glucose DMEM with GlutaMax and Sodium Pyruvate (Life Technologies) supplemented with 5% HyClone heat-inactivated FBS (Thermo Scientific), 1% penicillin/streptomycin (Life Technologies) and 25mM HEPES (Sigma Aldrich). 150,000 cells were plated in each well of a 24-well plate 18-24 hours prior to transfection. Cells were transfected with 1 µg total of construct DNA (at equimolar ratios) per well and 2 µL of Lipofectamine 2000 (Life Technologies) according to the manufacturer's recommended protocols. Media was exchanged 12 hours post-transfection. For the kinetics test, chemical induction was started 24 hours post-transfection, when abscisic acid (ABA, Sigma Aldrich) was added to fresh media to a final concentration of 250 µM. RNA was extracted using the RNeasy kit (Qiagen) according to manufacturer's instructions and 1 µg of RNA per sample was reverse-transcribed using qScript (Quanta Biosystems). Relative mRNA levels were measured by quantitative real-time PCR (qRT-PCR) using Taqman probes specific for the targeted gene as well as mouse GAPDH as an endogenous control (Life Technologies, see Supplementary Table 2 for Taqman probe IDs). ΔΔCt analysis was used to obtain fold-changes relative to negative controls where cells were subjected to mock transfection with GFP.

**Cas9 transcriptional effectors (disclosure).** HEK 293FT cells were co-transfected with mutant Cas9 fusion protein and a synthetic guide RNA (sgRNA) using Lipofectamine 2000 (Life Technologies) 24 hours after seeding into a 24 well dish. 72 hours post-transfection, total RNA was purified (RNeasy Plus, Qiagen). 1ug of RNA was reverse transcribed into cDNA (qScript, Quanta BioSciences). Quantitative real-time PCR was done according to the manufacturer's protocol (Life Technologies) and performed in triplicate using TaqMan Assays for hKlf4 (Hs00358836_ml), hSox2 (Hs01053049_s1), and the endogenous control GAPDH (Hs02758991_g1).

According to the disclosure, the hSpCas9 activator plasmid was cloned into a lentiviral vector under the expression of the hEFla promoter (pLenti-EF1a-Cas9-NLS-VP64). The hSpCas9 repressor plasmid was cloned into the same vector (pLenti-EFla-SID4x-NLS-Cas9-NLS). Guide sequences (20bp) targeted to the *KLF4* locus are: GCGCGCTCCACACAACTCAC, GCAAAAATAGACAATCAGCA, GAAGGATCTCGGCCAATTTG. Spacer sequences for guide RNAs targeted to the *SOX2* locus are: GCTGCCGGGTTTTGCATGAA, CCGGGCCCGCAGCAAACTTC, GGGGCTGTCAGGGAATAAAT.

**Optogenetic actuators:** Microbial and plant-derived light-sensitive proteins have been engineered as optogenetic actuators, allowing optical control of cellular functions including membrane potential (Deisseroth, K. Optogenetics. Nature methods 8, 26-29, doi:10.1038/nmeth.f.324 (2011); Zhang, F. et al. The microbial opsin family of optogenetic tools. Cell 147, 1446-1457, doi:10.1016/j.cell.2011.12.004 (2011) and Yizhar, O., Fenno, L. E., Davidson, T. J., Mogri, M. & Deisseroth, K. Optogenetics in neural systems. Neuron 71, 9-34, doi:10.1016/j.neuron.2011.06.004 (2011)), intracellular biochemical signaling (Airan, R. D., Thompson, K. R., Fenno, L. E., Bernstein, H. & Deisseroth, K. Temporally precise in vivo control of intracellular signalling. Nature 458, 1025-1029, doi:10.1038/nature07926 (2009))_, protein interactions (Levskaya, A., Weiner, O. D., Lim, W. A. & Voigt, C. A. Spatiotemporal control of cell signalling using a light-switchable protein interaction. Nature 461, 997-1001, doi:10.1038/nature08446 (2009); Yazawa, M., Sadaghiani, A. M., Hsueh, B. & Dolmetsch, R. E. Induction of protein-protein interactions in live cells using light. Nat Biotechnol 27, 941-945, doi:10.1038/nbt.1569 (2009); Strickland, D. et al. TULIPs: tunable, light-controlled interacting protein tags for cell biology. Nature methods 9, 379-384, doi:10.1038/nmeth.1904 (2012) and Kennedy, M. J. et al. Rapid blue-light-mediated induction of protein interactions in living cells. Nature methods 7, 973-975, doi:10.1038/nmeth.1524 (2010)), and heterologous gene expression (Yazawa, M., Sadaghiani, A. M., Hsueh, B. & Dolmetsch, R. E. Induction of protein-protein interactions in live cells using light. Nat Biotechnol 27, 941-945, doi:10.1038/nbt.1569 (2009); Kennedy, M. J. et al. Rapid blue-light-mediated induction of protein interactions in living cells. Nature methods 7, 973-975, doi:10.1038/nmeth.1524 (2010); Shimizu-Sato, S., Huq, E., Tepperman, J. M. & Quail, P. H. A light-switchable gene promoter system. Nat Biotechnol 20, 1041-1044, doi:10.1038/nbt734 (2002); Ye, H., Daoud-El Baba, M., Peng, R. W. & Fussenegger, M. A synthetic optogenetic transcription device enhances blood-glucose homeostasis in mice. Science 332, 1565-1568, doi:10.1126/science. 1203535 (2011); Wang, X., Chen, X. & Yang, Y. Spatiotemporal control of gene expression by a light-switchable transgene system. Nature methods 9, 266-269, doi:10.1038/nmeth.1892 (2012) and Polstein, L. R. & Gersbach, C. A. Light-inducible spatiotemporal control of gene activation by customizable zinc finger transcription factors. J Am Chem Soc 134, 16480-16483, doi:10.1021/ja3065667 (2012)).

**Ambient light exposure:** All cells were cultured at low light levels (<0.01 mW/cm²) at all times except during stimulation. These precautions were taken as ambient light in the room (0.1-0.2 mW/cm²) was found to significantly activate the LITE system (**FIG. 36D**). No special precautions were taken to shield animals from light during *in vivo* experiments - even assuming ideal propagation within the implanted optical fiber, an estimation of light transmission at the fiber terminal due to ambient light was <0.01 mW (based on 200 µm fiber core diameter and 0.22 numerical aperture).

**Optimization of light stimulation parameters in Neuro2A cells:** To minimize near-UV induced cytotoxicity, Applicants selected 466 nm blue LEDs to activate TALE-CRY2, a wavelength slightly red-shifted from the CRY2 absorption maxima of 450 nm but still maintaining over 80% activity (Banerjee, R. et al. The signaling state of Arabidopsis cryptochrome 2 contains flavin semiquinone. J Biol Chem 282, 14916-14922, doi:10.1074/jbc.M700616200 (2007)) (**FIG. 42**). To minimize light exposure, Applicants selected a mild stimulation protocol (1 s light pulses at 0.067 Hz, ∼7% duty cycle). This was based on Applicants' finding that light duty cycle had no significant effect on LITE-mediated transcriptional activation over a wide range of duty cycle parameters (1.7% to 100% duty cycles, **FIG. 41**). Illumination with a range of light intensities from 0 to 10 mW/cm² revealed that *Ngn2* mRNA levels increased as a function of intensity up to 5 mW/cm². However, increases in *Ngn2* mRNA levels declined at 10 mW/cm² (**FIG. 36C**), suggesting that higher intensity light may have detrimental effects on either LITE function or on cell physiology. To better characterize this observation, Applicants performed an ethidium homodimer-1 cytotoxicity assay with a calcein counterstain for living cells and found a significantly higher percentage of ethidium-positive cells at the higher stimulation intensity of 10 mW/cm². Conversely, the ethidium-positive cell count from 5 mW/cm² stimulation was indistinguishable from unstimulated controls. Thus 5 mW/cm² appeared to be optimal for achieving robust LITE activation while maintaining low cytotoxicity.

**Reduction of light-induced toxicity in primary neurons:** Initial application of LITEs in neurons revealed that cultured neurons were much more sensitive to blue light than Neuro 2a cells. Stimulation parameters that Applicants previously optimized for Neuro 2a cells (466 nm, 5 mW/cm² intensity, 7% duty cycle with 1 s light pulse at 0.067 Hz for a total of 24 h) caused >50% toxicity in primary neurons. Applicants therefore tested survival with a lower duty cycle, as Applicants had previously observed that a wide range of duty cycles had little effect on LITE-mediated transcriptional activation (**FIG. 41**). A reduced duty cycle of 0.8% (0.5 s light pulses at 0.0167 Hz) at the same light intensity (5 mW/cm²) was sufficient to maintain a high survival rate that was indistinguishable from that of unstimulated cultures (**FIG. 47**).

**Light propagation and toxicity in *in vivo* experiments:** Previous studies have investigated the propagation efficiency of different wavelengths of light in brain tissue. For 473 nm light (wavelength used in this study), there was a >90% attenuation after passing through 0.35 mm of tissue (Witten, liana B. et al. Recombinase-Driver Rat Lines: Tools, Techniques, and Optogenetic Application to Dopamine-Mediated Reinforcement. Neuron 72, 721-733, doi:http://dx.doi.org/10.1016/j.neuron.2011.10.028 (2011)). An estimated 5 mW/cm² light power density was estimated based on a tissue depth of 0.35 mm of tissue (the diameter of brain punch used in this study) and a total power output of 5 mW. The light stimulation duty cycle used *in vivo* was the same (0.8%, 0.5 s at 0.0167 Hz) as that used for primary neurons (**FIG 47**).

**CRY2 absorption spectrum:** An illustration of the absorption spectrum of CRY2 was shown in **FIG. 42****.** The spectrum showed a sharp drop in absorption above 480 nm (Banerjee, R. et al. The Signaling State of Arabidopsis Cryptochrome 2 Contains Flavin Semiquinone. Journal of Biological Chemistry 282, 14916-14922, doi:10.1074/jbc.M700616200 (2007)). Wavelengths > 500 nm were virtually not absorbed, which could be useful for future multimodal optical control with yellow or red-light sensitive proteins.

**Development of AAV1 supernatant process:** Traditional AAV particle generation required laborious production and purification processes, and made testing many constructs in parallel impractical (Grieger, J. C., Choi, V. W. & Samulski, R. J. Production and characterization of adeno-associated viral vectors. Nat Protoc 1, 1412-1428, doi:10.1038/nprot.2006.207 (2006)). In this study, a simple yet highly effective process of AAV production using filtered supernatant from transfected 293FT cells (**FIG. 43**). Recent reported indicate that AAV particles produced in 293FT cells could be found not only it the cytoplasm but also at considerable amounts in the culture media (Lock M, A. M., Vandenberghe LH, Samanta A, Toelen J, Debyser Z, Wilson JM. Rapid, Simple, and Versatile Manufacturing of Recombinant Adeno-Associated Viral Vectors at Scale. Human Gene Therapy 21, 1259-1271, doi:10.1089/hum.2010.055 (2010)). The ratio of viral particles between the supernatant and cytosol of host cells varied depending on the AAV serotype, and secretion was enhanced if polyethylenimine (PEI) was used to transfect the viral packaging plasmids (Lock M, A. M., Vandenberghe LH, Samanta A, Toelen J, Debyser Z, Wilson JM. Rapid, Simple, and Versatile Manufacturing of Recombinant Adeno-Associated Viral Vectors at Scale. Human Gene Therapy 21, 1259-1271, doi:10.1089/hum.2010.055 (2010)). In the current study, it was found that 2x10⁵ 293FT cells transfected with AAV vectors carrying TALEs (**FIG. 38A**) and packaged using AAV1 serotype were capable of producing 250 µl of AAV1 at a concentration of 5.6 ± 0.24 x 10¹⁰ DNAseI resistant genome copies (gc) per mL. 250 µl of filtered supernatant was able to transduce 150,000 primary cortical neurons at efficiencies of 80-90% (**FIG. 38B** and **FIG. 43**). This process was also successfully adapted to a 96-well format, enabling the production of 125 ul AAV1 supernatant from up to 96 different constructs in parallel. 35ul of supernatant can then be used to transduce one well of primary neurons cultured in 96-well format, enabling the transduction in biological triplicate from a single well.

**Table 3| Product information for all Taqman probes (Life Technologies)**

| **Target** | **Species** | **Probe #** |
|---|---|---|
| *Ngn2* | mouse | Mm00437603_g1 |
| *Grm5 (mGluR5)* | mouse | Mm00690332_m1 |
| *Grm2 (mGluR2)* | mouse | Mm01235831_m1 |
| *Grin2a (NMDAR2A)* | mouse | Mm00433802_m1 |
| *GAPD (GAPDH)* | mouse | 4352932E |
| *KLF4* | human | Hs00358836_m1 |
| *GAPD (GAPDH)* | human | 4352934E |
| *WPRE* | custom | |
| *5-HT1A* | mouse | Mm00434106_s1 |
| *5-HT1B* | mouse | Mm00439377_s1 |
| *5-HTT* | mouse | Mm00439391_m1 |
| *Arc* | mouse | Mm00479619_g1 |
| *BDNF* | mouse | Mm04230607_s1 |
| *c-Fos* | mouse | Mm00487425_m1 |
| *CBP*/*P300* | mouse | Mm01342452_m1 |
| *CREB* | mouse | Mm00501607_m1 |
| *CRHR1* | mouse | Mm00432670_m1 |
| *DNMT1* | mouse | Mm01151063_m1 |
| *DNMT3a* | mouse | Mm00432881_m1 |
| *DNMT3b* | mouse | Mm01240113_m1 |
| *egr-1 (zif-268)* | mouse | Mm00656724_m1 |
| *Gad65* | mouse | Mm00484623_m1 |
| *Gad67* | mouse | Mm00725661_s1 |
| *GR (GCR, NR3C1)* | mouse | Mm00433832_m1 |
| *HAT1* | mouse | Mm00509140_m1 |
| *HCRTR1* | mouse | Mm01185776_m1 |
| *HCRTR2* | mouse | Mm01179312_m1 |
| *HDAC1* | mouse | Mm02391771_g1 |
| *HDAC2* | mouse | Mm00515108_m1 |
| *HDAC4* | mouse | Mm01299557_m1 |
| *JMJD2A* | mouse | Mm00805000_m1 |
| *M1 (CHRM1)* | mouse | Mm00432509_s1 |
| *MCH-R1* | mouse | Mm00653044_ml |
| *NET (SLC6A2)* | mouse | Mm00436661_m1 |
| *NR2B subunit* | mouse | Mm00433820_ml |
| *OXTR* | mouse | Mm01182684_m1 |
| *Scn1a* | mouse | Mm00450580_m1 |
| *SIRT1* | mouse | Mm00490758_m1 |
| *Tet1* | mouse | Mm01169087_m1 |
| *Tet2* | mouse | Mm00524395_m1 |
| *Tet3* | mouse | Mm00805756_m1 |

**Table 4 Clone, product numbers and concentrations for antibodies used in this study Primary Antibodies**

| **Target** | **Host** | **Clone #** | **Manufacturer** | **Product #** | **IsoType** | **Concentration** |
|---|---|---|---|---|---|---|
| **mGluR2** | mouse | mG2Na-s | Abcam | Ab15672 | IgG | 1:1000 |
| **α-tubulin** | mouse | B-5-1-2 | Sigma-Aldrich | T5168 | IgG1 | 1:20000 |
| **NeuN** | mouse | A60 | Millipore | MAB377 | IgG1 | 1:200 |
| **HA (Alexa** | mouse | 6E2 | Cell Signaling | 3444 | IgG1 | 1:100 |
| **Fluor 594 GFP** | chicken | polyclonal | Aves Labs | GFP-1020 | IgY | 1:500 |
| | | | | | | |

| **Target** | **Host** | **Conjugate** | **Manufacturer** | **Product #** | | **Concentration** |
|---|---|---|---|---|---|---|
| **mouse IgG** | goat | HRP | Sigma-Aldrich | A9917 | | 1:5000-10000 |
| **mouse IgG** | goat | Alexa Fluor 594 | Life Technologies | A 11005 | | 1:1000 |
| **chicken IgG** | Goat | Alexa Fluor 488 | Life Technologies | A11039 | | 1:1000 |
| | | | | | | |

| **Target** | **Host** | **Epitope** | **Manufacturer** | **Product #** | **IsoType** | **Concentration** |
|---|---|---|---|---|---|---|
| **H3K9me1** | mouse | 1-18 | Millipore | 17-680 | IgG | 2 µl/IP |
| **H3K9me2** | mouse | 1-18 | Millipore | 17-681 | IgG | 4 µl/IP |
| **H3K9Ac** | rabbit | polyclonal | Millipore | 17-658 | IgG | 3 µg/IP |
| **H4K20me1** | rabbit | 15-24 | Millipore | 17-651 | IgG | 4 µg/IP |
| **H4K8Ac** | rabbit | polyclonal | Millipore | 17-10099 | IgG | 1.5 µl/IP |
| **H4K20me3** | rabbit | 18-22 | Millipore | 17-671 | IgG | 7 µl/IP |
| **H3K27me3** | rabbit | polyclonal | Millipore | 17-622 | IgG | 4 µg/IP |

**Table 5 qPCR primers used for CHIP-qPCR**

| **target** | **Primers** |
|---|---|
| *Grm2* promoter | Forward: CTGTGCTGAAGGATCTGGGG |
| | Reverse: ATGCTGCAGGCATAGGACAA |
| *Neurog2* promoter | Forward: GAGGGGGAGAGGGACTAAAGA |
| | Reverse: GCTCTCCCTCCCCAGCTTA |
| *Myt-1* promoter control | Cell Signaling Technologies SimpleChIP® Mouse MYT-1 Promoter Primers #8985 |
| *RPL30* Intron 2 control | Cell Signaling Technologies SimpleChIP® Mouse RPL30 Intron 2 Primers #7015 |

**Table 6 genomic sequences targeted by TALEs**

| | |
|---|---|
| *5-HT1B* | TATCTGAACTCTCC |
| *5-HTT* | TGTCTGTCTTGCAT |
| *Arc* | TGGCTGTTGCCAGG |
| *BDNF* | TACCTGGAGCTAGC |
| *c-Fos* | TACACAGGATGTCC |
| *DNMT3a* | TTGGCCCTGTGCAG |
| *DNMT3b* | TAGCGCAGCGATCG |
| *gad65* | TATTGCCAAGAGAG |
| *gad67* | TGACTGGAACATAC |
| *GR (GCR, NR3C1 )* | TGATGGACTTGTAT |
| *HAT1* | TGGACCTTCTCCCT |
| *HCRTR1* | TAGGTCTCCTGGAG |
| *HCRTR2* | TGGCTCAGGAACTT |
| *HDAC1* | TTCTCTAAGCTGCC |
| *HDAC2* | TGAGCCCTGGAGGA |
| *HDAC4* | TGCCTAAGATGGAG |
| *JMJD2A* | TGTAGTGAGTGTTC |
| *MCH-R1* | TGTCTAGGTGATGT |
| *NET* | TCTCTGCTAGAAGG |
| *Scn1a* | TCTAGGTCAAGTGT |
| *SIRT1* | TCCTCTGCTCCGCT |
| *tet1* | TCTAGGAGTGTAGC |
| *tet3* | TGCCTGGCTGCTGG |
| *5-HT1B* | TATCTGAACTCTCC |
| *Grm2* | TCAGAGCTGTCCTC |
| *Grm5* | TGCAAGAGTAGGAG |
| *5-HT2A* | TAGTGACTGATTCC |
| *Grin2a* | TTGGAGGAGCACCA |
| *Neurog2* | TGAATGATGATAATAC |

**Table 7 Viral transduction and light stimulation parameters for in vivo LITE-mediated activation of Grm2 in the mouse infralimbic cortex (ILC). Grm2 mRNA levels in the ipsilateral LITE-expressing hemisphere are compared with the contralateral mCherry-expressing control hemisphere for all three experimental conditions shown in FIG. 39J.**

| *Experimental condition* | *ILC Hemisphere (ipsilateral)* | | *ILC Hemisphere (contralateral)* |
|---|---|---|---|
| | **AAV vector** | **Light stimulation** | **AAV vector** |
| GFP | GFP | yes | mCherry |
| LITEs / no Light | TALE-CIB1: :CRY2PHR-VP64 | no | mCherry |
| LITEs / + Light | TALE-CIB1: :CRY2PHR-VP64 | yes | mCherry |

**Table 8 HDAC Recruiter Effector Domains**

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| Sin3a | **MeCP2** | - | - | *R. norvegicus* | 492 | 207-492 (Nan) | 286 | - |
| Sin3a | **MBD2b** | - | - | *H. sapiens* | 262 | 45-262 (Boeke) | 218 | - |
| Sin3a | **Sin3a** | - | - | *H. sapiens* | 1273 | 524-851 (Laherty) | 328 | 627-829: HDAC1 interaction |
| NcoR | **NcoR** | - | - | *H. sapiens* | 2440 | 420-488 (Zhang) | 69 | - |
| NuRD | **SALL1** | - | - | *M. musculus* | 1322 | 1-93 (Lauberth) | 93 | - |
| CoREST | **RCOR1** | - | - | *H. sapiens* | 482 | 81-300 (Gu, Ouyang) | 220 | - |

| | | | | | | | | |
|---|---|---|---|---|---|---|---|---|
| Nan, X. et al. Transcriptional repression by the methyl-CpG-binding protein MeCP2 involves a histone deacetylase complex. Nature 393, 386-389 (1998). Boeke, J., Ammerpohl, O., Kegel, S., Moehren, U. & Renkawitz, R. The minimal repression domain of MBD2b overlaps with the methyl-CpG-binding domain and binds directly to Sin3A. Journal of Biological Chemistry 275, 34963-34967 (2000). Laherty, C. D. et al. Histone deacetylases associated with the mSin3 corepressor mediate mad transcriptional repression. Cell 89, 349-356 (1997). Zhang, J., Kalkum, M., Chait, B. T. & Roeder, R. G. The N-CoR-HDAC3 nuclear receptor corepressor complex inhibits the JNK pathway through the integral subunit GPS2. Molecular cell 9, 611-623 (2002). Lauberth, S. M. & Rauchman, M. A conserved 12-amino acid motif in Sall1 recruits the nucleosome remodeling and deacetylase corepressor complex. Journal of Biological Chemistry 281, 23922-23931 (2006). Gu, H. & Roizman, B. Herpes simplex virus-infected cell protein 0 blocks the silencing of viral DNA by dissociating histone deacetylases from the CoREST,ÄiREST complex. Ouyang, J., Shi, Y., Valin, A., Xuan, Y. & Gill, G. Direct binding of CoREST1 to SUMO-2/3 contributes to gene-specific repression by the LSD1/CoREST1/HDAC complex. Molecular cell 34, 145-154 (2009) | | | | | | | | |

**Table 9 HDAC Effector Domains**

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| HDAC I | **HDAC 8** | - | - | *X. laevis* | 325 | 1-325 | 325 | 1-272: HDAC |
| HDAC1 | **RPD3** | - | - | *S. cerevisiae* | 433 | 19-340 | 322 (Vannie r) | 19-331: HDAC |
| HDAC IV | **MesoL o4** | - | - | *M. loti* | 300 | 1-300 (Gregoretti) | 300 | - |
| HDAC IV | **HDAC 11** | - | - | *H. sapiens* | 347 | 1-347 (Gao) | 347 | 14-326: HDAC |
| HD2 | **HDT1** | - | - | *A. thaliana* | 245 | 1-211 (Wu) | 211 | - |
| SIRT I | **SIRT3** | H3K9Ac | - | *H. sapiens* | 399 | 143-399 (Scher) | 257 | 126-382: SIRT |
| | | H4K16Ac | | | | | | |
| | | H3K56Ac | | | | | | |
| SIRT I | **HST2** | - | - | *C. albicans* | 331 | 1-331 (Hnisz) | 331 | - |
| SIRT I | **CobB** | - | - | *E. coli (K12)* | 242 | 1-242 (Landry) | 242 | - |
| SIRT I | **HST2** | - | - | *S. cerevisiae* | 357 | 8-298 (Wilson) | 291 | - |
| SIRT III | **SIRT5** | H4K8Ac | - | *H. sapiens* | 310 | 37-310 (Gertz) | 274 | 41-309: SIRT |
| | | H4K16Ac | | | | | | |
| SIRT III | **Sir2A** | - | - | *P. falciparum* | 273 | 1-273 (Zhu) | 273 | 19-273: SIRT |
| SIRT IV | **SIRT6** | H3K9Ac | - | *H. sapiens* | 355 | 1-289 (Tennen) | 289 | 35-274: SIRT |
| | | H3K56Ac | | | | | | |

| | | | | | | | | |
|---|---|---|---|---|---|---|---|---|
| Vannier, D., Balderes, D. & Shore, D. Evidence that the transcriptional regulators SIN3 and RPD3, and a novel gene (SDS3) with similar functions, are involved in transcriptional silencing in S. cerevisiae. Genetics 144, 1343-1353 (1996). Gregoretti, I., Lee, Y.-M. & Goodson, H. V. Molecular evolution of the histone deacetylase family: functional implications of phylogenetic analysis. Journal of molecular biology 338, 17-31 (2004). Gao, L., Cueto, M. A., Asselbergs, F. & Atadja, P. Cloning and functional characterization of HDAC11, a novel member of the human histone deacetylase family. Journal of Biological Chemistry 277, 25748-25755 (2002). Wu, K., Tian, L., Malik, K., Brown, D. & Miki, B. Functional analysis of HD2 histone deacetylase homologues in Arabidopsis thaliana. The Plant Journal 22, 19-27 (2000). Scher, M. B., Vaquero, A. & Reinberg, D. SirT3 is a nuclear NAD+-dependent histone deacetylase that translocates to the mitochondria upon cellular stress. Genes & development 21, 920-928 (2007). Hnisz, D., Schwarzm√°ller, T. & Kuchler, K. Transcriptional loops meet chromatin: a dual,Äêlayer network controls white,Äìopaque switching in Candida albicans. Molecular microbiology 74, 1-15 (2009). Landry, J. et al. The silencing protein SIR2 and its homologs are NAD-dependent protein deacetylases. Proceedings of the National Academy of Sciences 97, 5807-5811 (2000). Wilson, J. M., Le, V. Q., Zimmerman, C., Marmorstein, R. & Pillus, L. Nuclear export modulates the cytoplasmic Sir2 homologue Hst2. EMBO reports 7, 1247-1251 (2006). Gertz, M. & Steegborn, C. Function and regulation of the mitochondrial Sirtuin isoform Sirt5 in Mammalia. Biochimica et Biophysica Acta (BBA)-Proteins and Proteomics 1804, 1658-1665 (2010). Zhu, A. Y. et al. Plasmodium falciparum Sir2A preferentially hydrolyzes medium and long chain fatty acyl lysine. ACS chemical biology 7, 155-159 (2011). Tennen, R. I., Berber, E. & Chua, K. F. Functional dissection of SIRT6: identification of domains that regulate histone deacetylase activity and chromatin localization. Mechanisms of ageing and development 131, 185-192 (2010). | | | | | | | | |

**Table 10 Histone Methyltransferase (HMT) Effector Domains**

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| SET | **NUE** | H2B, H3, H4 | - | *C. trachomatis* | 219 | 1-219 (Pennini) | 219 | - |
| SET | **vSET** | - | H3K27me3 | *P. bursaria chlorella virus* | 119 | 1-119 (Mujtaba) | 119 | 4-112: SET2 |
| SUV39 family | **EHMT 2/G9A** | H1.4K2, H3K9, H3K27 | H3K9me1/ 2, H1K25me1 | *M. musculus* | 1263 | 969-1263 (Tachibana) | 295 | 1025-1233: preSET, SET, postSET |
| SUV39 | **SUV39 H1** | - | H3K9me2/ 3 | *H. sapiens* | 412 | 79-412 (Snowden) | 334 | 172-412: preSET, SET, postSET |
| Suvar3-9 | **dim-5** | - | H3K9me3 | *N. crassa* | 331 | 1-331 (Rathert) | 331 | 77-331 : preSET, SET, postSET |
| Suvar3-9 (SUVH subfamily) | **KYP** | - | H3K9me1/ 2 | *A. thaliana* | 624 | 335-601 | 267 (Jackso n) | - |
| Suvar3-9 (SUVR subfamily) | **SUVR4** | H3K9me 1 | H3K9me2/ 3 | *A. thaliana* | 492 | 180-492 | 313 (Thorst ensen) | 192-462: preSET, SET, postSET |
| Suvar4-20 | **SET4** | - | H4K20me3 | *C. elegans* | 288 | 1-288 (Vielle) | 288 | - |
| SET8 | **SET1** | - | H4K20me1 | *C. elegans* | 242 | 1-242 (Vielle) | 242 | - |
| SET8 | **SETD8** | - | H4K20mel | *H. sapiens* | 393 | 185-393 | 209 (Coutur e) | 256-382: SET |
| SET8 | **TgSET 8** | - | H4K20me1 /2/3 | *T. gondii* | 1893 | 1590-1893 (Sautel) | 304 | 1749-1884: SET |

| | | | | | | | | |
|---|---|---|---|---|---|---|---|---|
| Pennini, M. E., Perrinet, S. p., Dautry-Varsat, A. & Subtil, A. Histone methylation by NUE, a novel nuclear effector of the intracellular pathogen Chlamydia trachomatis. PLoS pathogens 6, e1000995 (2010). Mujtaba, S. et al. Epigenetic transcriptional repression of cellular genes by a viral SET protein. Nature cell biology 10, 1114-1122 (2008). Tachibana, M., Matsumura, Y., Fukuda, M., Kimura, H. & Shinkai, Y. G9a/GLP complexes independently mediate H3K9 and DNA methylation to silence transcription. The EMBO journal 27, 2681-2690 (2008). Snowden, A. W., Gregory, P. D., Case, C. C. & Pabo, C. O. Gene-specific targeting of H3K9 methylation is sufficient for initiating repression in vivo. Current biology 12, 2159-2166 (2002). Rathert, P., Zhang, X., Freund, C., Cheng, X. & Jeltsch, A. Analysis of the substrate specificity of the Dim-5 histone lysine methyltransferase using peptide arrays. Chemistry & biology 15, 5-11 (2008). Jackson, J. P. et al. Dimethylation of histone H3 lysine 9 is a critical mark for DNA methylation and gene silencing in Arabidopsis thaliana. Chromosoma 112, 308-315 (2004). Thorstensen, T. et al. The Arabidopsis SUVR4 protein is a nucleolar histone methyltransferase with preference for monomethylated H3K9. Nucleic acids research 34, 5461-5470 (2006). Vielle, A. et al. H4K20me1 Contributes to Downregulation of X-Linked Genes for C. elegans Dosage Compensation. PLoS Genetics 8, e1002933 (2012). Couture, J.-F., Collazo, E., Brunzelle, J. S. & Trievel, R. C. Structural and functional analysis of SET8, a histone H4 Lys-20 methyltransferase. Genes & development 19, 1455-1465 (2005). Sautel, C. 1. F. et al. SET8-mediated methylations of histone H4 lysine 20 mark silent heterochromatic domains in apicomplexan genomes. Molecular and cellular biology 27, 5711-5724 (2007). | | | | | | | | |

**Table 11 Histone Methyltransferase (HMT) Recruiter Effector Domains**

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| - | **Hp1a** | - | H3K9me3 | *M. musculus* | 191 | 73-191 | 119 (Hatha way) | 121-179: chromoshadow |
| - | **PHF19** | - | H3K27me3 | *H. sapiens* | 580 | (1-250) + GGSG linker + (500-580) | 335 (Ballaré ) | 163-250: PHD2 |
| - | **NIPP1** | - | H3K27me3 | *H. sapiens* | 351 | 1-329 (Jin) | 329 | 310-329: EED |

| | | | | | | | | |
|---|---|---|---|---|---|---|---|---|
| Hathaway, N. A. et al. Dynamics and memory of heterochromatin in living cells. Cell (2012). Ballaré, C. et al. Phf19 links methylated Lys36 of histone H3 to regulation of Polycomb activity. Nature structural & molecular biology 19, 1257-1265 (2012). Jin, Q. et al. The protein phosphatase-1 (PP1) regulator, nuclear inhibitor of PP1 (NIPP1), interacts with the polycomb group protein, embryonic ectoderm development (EED), and functions as a transcriptional repressor. Journal of Biological Chemistry 278, 30677-30685 (2003). | | | | | | | | |

**Table 12 Histone Acetyltransferase Inhibitor Effector Domains**

| Subtype/ Complex | Name | Substrate (if known) | Modification (if known) | Organism | Full size (aa) | Selected truncation (aa) | Final size (aa) | Catalytic domain |
|---|---|---|---|---|---|---|---|---|
| - | **SET/TA F-1β** | - | - | *M. musculus* | 289 | 1-289 (Cervoni) | 289 | - |

| | | | | | | | | |
|---|---|---|---|---|---|---|---|---|
| Cervoni, N., Detich, N., Seo, S.-B., Chakravarti, D. & Szyf, M. The oncoprotein Set/TAF-1Œ≤, an inhibitor of histone acetyltransferase, inhibits active demethylation of DNA, integrating DNA methylation and transcriptional silencing. Journal of Biological Chemistry 277, 25026-25031 (2002) | | | | | | | | |

### Supplementary Sequences

> TALE(*Ngn2*)-NLS-CRY2 
MSRTRLPSPPAPSPAFSADSFSDLLRQFDPSLFNTSLFDSLPPFGAHHTEAATG
EWDEVQSGLRAADAPPPTMRVAVTAARPPRAKPAPRRRAAQPSDASPAAQVDLRTLGY
SQQQQEKIKPKVRSTVAQHHEALVGHGFTHAHIVALSQHPAALGTVAVKYQDMIAALP
EATHEAIVGVGKQWSGARALEALLTVAGELRGPPLQLDTGQLLKIAKRGGVTAVEAVH
 
AWRNALTGAPLNLTPEQVVAIASNNGGKQALETVQRLLPVLCQAHGLTPEQVVAIASN
GGGKQALETVQRLLPVLCQAHGLTPEQVVAIASNNGGKQALETVQRLLPVLCQAHGLT
PEQVVAIASNIGGKQALETVQRLLPVLCQAHGLTPEQVVAIASNIGGKQALETVQRLLPV
LCQAHGLTPEQVVAIASNGGGKQALETVQRLLPVLCQAHGLTPEQVVAIASNNGGKQA
LETVQRLLPVLCQAHGLTPEQVVAIASNIGGKQALETVQRLLPVLCQAHGLTPEQVVAI
ASNGGGKQALETVQRLLPVLCQAHGLTPEQVVAIASNNGGKQALETVQRLLPVLCQAH
GLTPEQVVAIASNIGGKQALETVQRLLPVLCQAHGLTPEQVVAIASNGGGKQALETVQR
LLPVLCQAHGLTPEQVVAIASNIGGKQALETVQRLLPVLCQAHGLTPEQVVAIASNIGGK
QALETVQRLLPVLCQAHGLTPEQVVAIASNGGGKQALETVQRLLPVLCQAHGLTPEQV
VAIASNIGGKQALETVQRLLPVLCQAHGLTPEQVVAIASHDGGRPALESIVAQLSRPDPA
LAALTNDHLVALACLGGRPALDAVKKGLPHAPALIKRTNRRIPERTSHRVADHAQVVR
VLGFFQCHSHPAQAFDDAMTQFGMSRHGLLQLFRRVGVTELEARSGTLPPASQRWDRI
LQASGMKRAKPSPTSTQTPDQASLHAFADSLERDLDAPSPMHEGDQTRASASPKKKRK
VEASKMDKKTIVWFRRDLRIEDNPALAAAAHEGSVFPVFIWCPEEEGQFYPGRASRWW
MKQSLAHLSQSLKALGSDLTLIKTHNTISAILDCIRVTGATKVVFNHLYDPVSLVRDHTV
KEKLVERGISVQSYNGDLLYEPWEIYCEKGKPFTSFNSYWKKCLDMSIESVMLPPPWRL
MPITAAAEAIWACSIEELGLENEAEKPSNALLTRAWSPGWSNADKLLNEFIEKQLIDYAK
NSKKVVGNSTSLLSPYLHFGEISVRHVFQCARMKQIIWARDKNSEGEESADLFLRGIGLR
EYSRYICFNFPFTHEQSLLSHLRFFPWDADVDKFKAWRQGRTGYPLVDAGMRELWATG
WMHNRIRVIVSSFAVKFLLLPWKWGMKYFWDTLLDADLECDILGWQYISGSIPDGHEL
DRLDNPALQGAKYDPEGEYIRQWLPELARLPTEWIHHPWDAPLTVLKASGVELGTNYA
KPIVDIDTARELLAKAISRTREAQIMIGAAPDEIVADSFEALGANTIKEPGLCPSVSSNDQ
QVPSAVRYNGSKRVKPEEEEERDMKKSRGFDERELFSTAESSSSSSVFFVSQSCSLASEG
KNLEGIQDSSDQITTSLGKNG
> TALE(*Ngn2*)-NLS-CRY2PHR 
MSRTRLPSPPAPSPAFSADSFSDLLRQFDPSLFNTSLFDSLPPFGAHHTEAATG
EWDEVQSGLRAADAPPPTMRVAVTAARPPRAKPAPRRRAAQPSDASPAAQVDLRTLGY
SQQQQEKIKPKVRSTVAQHHEALVGHGFTHAHIVALSQHPAALGTVAVKYQDMIAALP
EATHEAIVGVGKQWSGARALEALLTVAGELRGPPLQLDTGQLLKIAKRGGVTAVEAVH
AWRNALTGAPLNLTPEQVVAIASNNGGKQALETVQRLLPVLCQAHGLTPEQVVAIASN
GGGKQALETVQRLLPVLCQAHGLTPEQVVAIASNNGGKQALETVQRLLPVLCQAHGLT
 
PEQVVAIASNIGGKQALETVQRLLPVLCQAHGLTPEQVVAIASNIGGKQALETVQRLLPV
LCQAHGLTPEQVVAIASNGGGKQALETVQRLLPVLCQAHGLTPEQVVAIASNNGGKQA
LETVQRLLPVLCQAHGLTPEQVVAIASNIGGKQALETVQRLLPVLCQAHGLTPEQVVAI
ASNGGGKQALETVQRLLPVLCQAHGLTPEQVVAIASNNGGKQALETVQRLLPVLCQAH
GLTPEQVVAIASNIGGKQALETVQRLLPVLCQAHGLTPEQVVAIASNGGGKQALETVQR
LLPVLCQAHGLTPEQVVAIASNIGGKQALETVQRLLPVLCQAHGLTPEQVVAIASNIGGK
QALETVQRLLPVLCQAHGLTPEQVVAIASNGGGKQALETVQRLLPVLCQAHGLTPEQV
VAIASNIGGKQALETVQRLLPVLCQAHGLTPEQVVAIASHDGGRPALESIVAQLSRPDPA
LAALTNDHLVALACLGGRPALDAVKKGLPHAPALIKRTNRRIPERTSHRVADHAQVVR
VLGFFQCHSHPAQAFDDAMTQFGMSRHGLLQLFRRVGVTELEARSGTLPPASQRWDRI
LQASGMKRAKPSPTSTQTPDQASLHAFADSLERDLDAPSPMHEGDQTRASASPKKKRK
VEASKMDKKTIVWFRRDLRIEDNPALAAAAHEGSVFPVFIWCPEEEGQFYPGRASRWW
MKQSLAHLSQSLKALGSDLTLIKTHNTISAILDCIRVTGATKVVFNHLYDPVSLVRDHTV
KEKLVERGISVQSYNGDLLYEPWEIYCEKGKPFTSFNSYWKKCLDMSIESVMLPPPWRL
MPITAAAEAIWACSIEELGLENEAEKPSNALLTRAWSPGWSNADKLLNEFIEKQLIDYAK
NSKKVVGNSTSLLSPYLHFGEISVRHVFQCARMKQIIWARDKNSEGEESADLFLRGIGLR
EYSRYICFNFPFTHEQSLLSHLRFFPWDADVDKFKAWRQGRTGYPLVDAGMRELWATG
WMHNRIRVIVSSFAVKFLLLPWKWGMKYFWDTLLDADLECDILGWQYISGSIPDGHEL
DRLDNPALQGAKYDPEGEYIRQWLPELARLPTEWIHHPWDAPLTVLKASGVELGTNYA
KPIVDIDTARELLAKAISRTREAQIMIGAAP
> CIB1 -NLS-VP64_2A_GFP 
MNGAIGGDLLLNFPDMSVLERQRAHLKYLNPTFDSPLAGFFADSSMITGGEM
DSYLSTAGLNLPMMYGETTVEGDSRLSISPETTLGTGNFKKRKFDTETKDCNEKKKKMT
MNRDDLVEEGEEEKSKITEQNNGSTKSIKKMKHKAKKEENNFSNDSSKVTKELEKTDYI
HVRARRGQATDSHSIAERVRREKISERMKFLQDLVPGCDKITGKAGMLDEIINYVQSLQ
RQIEFLSMKLAIVNPRPDFDMDDIFAKEVASTPMTVVPSPEMVLSGYSHEMVHSGYSSE
MVNSGYLHVNPMQQVNTSSDPLSCFNNGEAPSMWDSHVQNLYGNLGVASPKKKRKV
EASGSGRADALDDFDLDMLGSDALDDFDLDMLGSDALDDFDLDMLGSDALDDFDLDM
LINSRGSGEGRGSLLTCGDVEENPGPVSKGEELFTGVVPILVELDGDVNGHKFSVSGEGE
GDATYGKLTLKFICTTGKLPVPWPTLVTTLTYGVQCFSRYPDHMKQHDFFKSAMPEGY
VQERTIFFKDDGNYKTRAEVKFEGDTLVNRIELKGIDFKEDGNILGHKLEYNYNSHNVYI
 
MADKQKNGIKVNFKIRHNIEDGSVQLADHYQQNTPIGDGPVLLPDNHYLSTQSALSKDP
NEKRDHMVLLEFVTAAGITLGMDELYK
> CIBN-NLS-VP64_2A_GFP 
MNGAIGGDLLLNFPDMSVLERQRAHLKYLNPTFDSPLAGFFADSSMITGGEM
DSYLSTAGLNLPMMYGETTVEGDSRLSISPETTLGTGNFKKRKFDTETKDCNEKKKKMT
MNRDDLVEEGEEEKSKITEQNNGSTKSIKKMKHKAKKEENNFSNDSSKVTKELEKTDYI
ASPKKKRKVEASGSGRADALDDFDLDMLGSDALDDFDLDMLGSDALDDFDLDMLGSD
ALDDFDLDMLINSRGSGEGRGSLLTCGDVEENPGPVSKGEELFTGVVPILVELDGDVNG
HKFSVSGEGEGDATYGKLTLKFICTTGKLPVPWPTLVTTLTYGVQCFSRYPDHMKQHDF
FKSAMPEGYVQERTIFFKDDGNYKTRAEVKFEGDTLVNRIELKGIDFKEDGNILGHKLE
YNYNSHNVYIMADKQKNGIKVNFKIRHNIEDGSVQLADHYQQNTPIGDGPVLLPDNHY
LSTQSALSKDPNEKRDHMVLLEFVTAAGITLGMDELYK
> CIB1-NLS-VP16_2A_GFP 
MNGAIGGDLLLNFPDMSVLERQRAHLKYLNPTFDSPLAGFFADSSMITGGEM
DSYLSTAGLNLPMMYGETTVEGDSRLSISPETTLGTGNFKKRKFDTETKDCNEKKKKMT
MNRDDLVEEGEEEKSKITEQNNGSTKSIKKMKHKAKKEENNFSNDSSKVTKELEKTDYI
HVRARRGQATDSHSIAERVRREKISERMKFLQDLVPGCDKITGKAGMLDEIINYVQSLQ
RQIEFLSMKLAIVNPRPDFDMDDIFAKEVASTPMTVVPSPEMVLSGYSHEMVHSGYSSE
MVNSGYLHVNPMQQVNTSSDPLSCFNNGEAPSMWDSHVQNLYGNLGVASPKKKRKV
EASAPPTDVSLGDELHLDGEDVAMAHADALDDFDLDMLGDGDSPGPGFTPHDSAPYG
ALDMADFEFEQMFTDALGIDEYGGEFPGIRRSRGSGEGRGSLLTCGDVEENPGPVSKGE
ELFTGVVPILVELDGDVNGHKFSVSGEGEGDATYGKLTLKFICTTGKLPVPWPTLVTTLT
YGVQCFSRYPDHMKQHDFFKSAMPEGYVQERTIFFKDDGNYKTRAEVKFEGDTLVNRI
ELKGIDFKEDGNILGHKLEYNYNSHNVYIMADKQKNGIKVNFKIRHNIEDGSVQLADHY
QQNTPIGDGPVLLPDNHYLSTQSALSKDPNEKRDHMVLLEFVTAAGITLGMDELYK
> CIB1-NLS-p65_2A_GFP 
MNGAIGGDLLLNFPDMSVLERQRAHLKYLNPTFDSPLAGFFADSSMITGGEM
DSYLSTAGLNLPMMYGETTVEGDSRLSISPETTLGTGNFKKRKFDTETKDCNEKKKKMT
MNRDDLVEEGEEEKSKITEQNNGSTKSIKKMKHKAKKEENNFSNDSSKVTKELEKTDYI
HVRARRGQATDSHSIAERVRREKISERMKFLQDLVPGCDKITGKAGMLDEIINYVQSLQ
RQIEFLSMKLAIVNPRPDFDMDDIFAKEVASTPMTVVPSPEMVLSGYSHEMVHSGYSSE
 
MVNSGYLHVNPMQQVNTSSDPLSCFNNGEAPSMWDSHVQNLYGNLGVASPKKKRKV
EASPSGQISNQALALAPSSAPVLAQTMVPSSAMVPLAQPPAPAPVLTPGPPQSLSAPVPK
STQAGEGTLSEALLHLQFDADEDLGALLGNSTDPGVFTDLASVDNSEFQQLLNQGVSMS
HSTAEPMLMEYPEAITRLVTGSQRPPDPAPTPLGTSGLPNGLSGDEDFSSIADMDFSALLS
QISSSGQSRGSGEGRGSLLTCGDVEENPGPVSKGEELFTGVVPILVELDGDVNGHKFSVS
GEGEGDATYGKLTLKFICTTGKLPVPWPTLVTTLTYGVQCFSRYPDHMKQHDFFKSAM
PEGYVQERTIFFKDDGNYKTRAEVKFEGDTLVNRIELKGIDFKEDGNILGHKLEYNYNS
HNVYIMADKQKNGIKVNFKIRHNIEDGSVQLADHYQQNTPIGDGPVLLPDNHYLSTQSA
LSKDPNEKRDHMVLLEFVTAAGITLGMDELYK
> HA-TALE(12mer)-NLS-VP64_2A_GFP 
MYPYDVPDYAVDLRTLGYSQQQQEKIKPKVRSTVAQHHEALVGHGFTHAHI
VALSQHPAALGTVAVKYQDMIAALPEATHEAIVGVGKQWSGARALEALLTVAGELRGP
PLQLDTGQLLKIAKRGGVTAVEAVHAWRNALTGAPLNLTPEQVVAIASXXGGKQALET
VQRLLPVLCQAHGLTPEQVVAIASXXGGKQALETVQRLLPVLCQAHGLTPEQVVAIASX
XGGKQALETVQRLLPVLCQAHGLTPEQVVAIASXXGGKQALETVQRLLPVLCQAHGLT
PEQVVAIASXXGGKQALETVQRLLPVLCQAHGLTPEQVVAIASXXGGKQALETVQRLLP
VLCQAHGLTPEQVVAIASXXGGKQALETVQRLLPVLCQAHGLTPEQVVAIASXXGGKQ
ALETVQRLLPVLCQAHGLTPEQVVAIASXXGGKQALETVQRLLPVLCQAHGLTPEQVV
AIASXXGGKQALETVQRLLPVLCQAHGLTPEQVVAIASXXGGKQALETVQRLLPVLCQ
AHGLTPEQVVAIASXXGGKQALETVQRLLPVLCQAHGLTPEQVVAIASXXGGRPALESI
VAQLSRPDPALAALTNDHLVALACLGGRPALDAVKKGLPHAPALIKRTNRRIPERTSHR
VAASPKKKRKVEASGSGRADALDDFDLDMLGSDALDDFDLDMLGSDALDDFDLDMLG
SDALDDFDLDMLINSRGSGEGRGSLLTCGDVEENPGPVSKGEELFTGVVPILVELDGDV
NGHKFSVSGEGEGDATYGKLTLKFICTTGKLPVPWPTLVTTLTYGVQCFSRYPDHMKQ
HDFFKSAMPEGYVQERTIFFKDDGNYKTRAEVKFEGDTLVNRIELKGIDFKEDGNILGH
KLEYNYNSHNVYIMADKQKNGIKVNFKIRHNIEDGSVQLADHYQQNTPIGDGPVLLPDN
HYLSTQSALSKDPNEKRDHMVLLEFVTAAGITLGMDELYK
> HA-TALE(12mer)-NLS-SID4X_2A_phiLOV2.1 
MYPYDVPDYAVDLRTLGYSQQQQEKIKPKVRSTVAQHHEALVGHGFTHAHI
VALSQHPAALGTVAVKYQDMIAALPEATHEAIVGVGKQWSGARALEALLTVAGELRGP
PLQLDTGQLLKIAKRGGVTAVEAVHAWRNALTGAPLNLTPEQVVAIASXXGGKQALET
 
VQRLLPVLCQAHGLTPEQVVAIASXXGGKQALETVQRLLPVLCQAHGLTPEQVVAIASX
XGGKQALETVQRLLPVLCQAHGLTPEQVVAIASXXGGKQALETVQRLLPVLCQAHGLT
PEQVVAIASXXGGKQALETVQRLLPVLCQAHGLTPEQVVAIASXXGGKQALETVQRLLP
VLCQAHGLTPEQVVAIASXXGGKQALETVQRLLPVLCQAHGLTPEQVVAIASXXGGKQ
ALETVQRLLPVLCQAHGLTPEQVVAIASXXGGKQALETVQRLLPVLCQAHGLTPEQVV
AIASXXGGKQALETVQRLLPVLCQAHGLTPEQVVAIASXXGGKQALETVQRLLPVLCQ
AHGLTPEQVVAIASXXGGKQALETVQRLLPVLCQAHGLTPEQVVAIASXXGGRPALESI
VAQLSRPDPALAALTNDHLVALACLGGRPALDAVKKGLPHAPALIKRTNRRIPERTSHR
VAASPKKKRKVEASPKKKRKVEASGSGMNIQMLLEAADYLERREREAEHGYASMLPG
SGMNIQMLLEAADYLERREREAEHGYASMLPGSGMNIQMLLEAADYLERREREAEHG
YASMLPGSGMNIQMLLEAADYLERREREAEHGYASMLPSRSRGSGEGRGSLLTCGDVE
ENPGPIEKSFVITDPRLPDYPIIFASDGFLELTEYSREEIMGRNARFLQGPETDQATVQKIR
DAIRDQRETTVQLINYTKSGKKFWNLLHLQPVRDRKGGLQYFIGVQLVGSDHV
> HA-TALE(12mer)-NLS-CIB1 
MYPYDVPDYAVDLRTLGYSQQQQEKIKPKVRSTVAQHHEALVGHGFTHAHI
VALSQHPAALGTVAVKYQDMIAALPEATHEAIVGVGKQWSGARALEALLTVAGELRGP
PLQLDTGQLLKIAKRGGVTAVEAVHAWRNALTGAPLNLTPEQVVAIASXXGGKQALET
VQRLLPVLCQAHGLTPEQVVAIASXXGGKQALETVQRLLPVLCQAHGLTPEQVVAIASX
XGGKQALETVQRLLPVLCQAHGLTPEQVVAIASXXGGKQALETVQRLLPVLCQAHGLT
PEQVVAIASXXGGKQALETVQRLLPVLCQAHGLTPEQVVAIASXXGGKQALETVQRLLP
VLCQAHGLTPEQVVAIASXXGGKQALETVQRLLPVLCQAHGLTPEQVVAIASXXGGKQ
ALETVQRLLPVLCQAHGLTPEQVVAIASXXGGKQALETVQRLLPVLCQAHGLTPEQVV
AIASXXGGKQALETVQRLLPVLCQAHGLTPEQVVAIASXXGGKQALETVQRLLPVLCQ
AHGLTPEQVVAIASXXGGKQALETVQRLLPVLCQAHGLTPEQVVAIASXXGGRPALESI
VAQLSRPDPALAALTNDHLVALACLGGRPALDAVKKGLPHAPALIKRTNRRIPERTSHR
VAASPKKKRKVEASNGAIGGDLLLNFPDMSVLERQRAHLKYLNPTFDSPLAGFFADSSM
ITGGEMDSYLSTAGLNLPMMYGETTVEGDSRLSISPETTLGTGNFKKRKFDTETKDCNE
KKKKMTMNRDDLVEEGEEEKSKITEQNNGSTKSIKKMKHKAKKEENNFSNDSSKVTKE
LEKTDYIHVRARRGQATDSHSIAERVRREKISERMKFLQDLVPGCDKITGKAGMLDEIIN
YVQSLQRQIEFLSMKLAIVNPRPDFDMDDIFAKEVASTPMTVVPSPEMVLSGYSHEMVH
SGYSSEMVNSGYLHVNPMQQVNTSSDPLSCFNNGEAPSMWDSHVQNLYGNLGV
> CRY2PHR-NLS-VP64_2A_GFP 
MKMDKKTIVWFRRDLRIEDNPALAAAAHEGSVFPVFIWCPEEEGQFYPGRAS
RWWMKQSLAHLSQSLKALGSDLTLIKTHNTISAILDCIRVTGATKVVFNHLYDPVSLVR
DHTVKEKLVERGISVQSYNGDLLYEPWEIYCEKGKPFTSFNSYWKKCLDMSIESVMLPP
PWRLMPITAAAEAIWACSIEELGLENEAEKPSNALLTRAWSPGWSNADKLLNEFIEKQLI
DYAKNSKKVVGNSTSLLSPYLHFGEISVRHVFQCARMKQIIWARDKNSEGEESADLFLR
GIGLREYSRYICFNFPFTHEQSLLSHLRFFPWDADVDKFKAWRQGRTGYPLVDAGMREL
WATGWMHNRIRVIVSSFAVKFLLLPWKWGMKYFWDTLLDADLECDILGWQYISGSIPD
GHELDRLDNPALQGAKYDPEGEYIRQWLPELARLPTEWIHHPWDAPLTVLKASGVELG
TNYAKPIVDIDTARELLAKAISRTREAQIMIGAAPASPKKKRKVEASGSGRADALDDFDL
DMLGSDALDDFDLDMLGSDALDDFDLDMLGSDALDDFDLDMLINSRGSGEGRGSLLTC
GDVEENPGPVSKGEELFTGVVPILVELDGDVNGHKFSVSGEGEGDATYGKLTLKFICTT
GKLPVPWPTLVTTLTYGVQCFSRYPDHMKQHDFFKSAMPEGYVQERTIFFKDDGNYKT
RAEVKFEGDTLVNRIELKGIDFKEDGNILGHKLEYNYNSHNVYIMADKQKNGIKVNFKI
RHNIEDGSVQLADHYQQNTPIGDGPVLLPDNHYLSTQSALSKDPNEKRDHMVLLEFVTA
AGITLGMDELYKV
> CRY2PHR-NLS-SID4X_2A_phiLOV2.1 
MKMDKKTIVWFRRDLRIEDNPALAAAAHEGSVFPVFIWCPEEEGQFYPGRAS
RWWMKQSLAHLSQSLKALGSDLTLIKTHNTISAILDCIRVTGATKVVFNHLYDPVSLVR
DHTVKEKLVERGISVQSYNGDLLYEPWEIYCEKGKPFTSFNSYWKKCLDMSIESVMLPP
PWRLMPITAAAEAIWACSIEELGLENEAEKPSNALLTRAWSPGWSNADKLLNEFIEKQLI
DYAKNSKKVVGNSTSLLSPYLHFGEISVRHVFQCARMKQIIWARDKNSEGEESADLFLR
GIGLREYSRYICFNFPFTHEQSLLSHLRFFPWDADVDKFKAWRQGRTGYPLVDAGMREL
WATGWMHNRIRVIVSSFAVKFLLLPWKWGMKYFWDTLLDADLECDILGWQYISGSIPD
GHELDRLDNPALQGAKYDPEGEYIRQWLPELARLPTEWIHHPWDAPLTVLKASGVELG
TNYAKPIVDIDTARELLAKAISRTREAQIMIGAAPASPKKKRKVEASGSGMNIQMLLEAA
DYLERREREAEHGYASMLPGSGMNIQMLLEAADYLERREREAEHGYASMLPGSGMNIQ
MLLEAADYLERREREAEHGYASMLPGSGMNIQMLLEAADYLERREREAEHGYASMLP
SRSRGSGEGRGSLLTCGDVEENPGPIEKSFVITDPRLPDYPIIFASDGFLELTEYSREEIMG
RNARFLQGPETDQATVQKIRDAIRDQRETTVQLINYTKSGKKFWNLLHLQPVRDRKGGL
QYFIGVQLVGSDHV
> TALE(KLF4)-NLS-CRY2PHR 
MSRTRLPSPPAPSPAFSADSFSDLLRQFDPSLFNTSLFDSLPPFGAHHTEAATG
EWDEVQSGLRAADAPPPTMRVAVTAARPPRAKPAPRRRAAQPSDASPAAQVDLRTLGY
SQQQQEKIKPKVRSTVAQHHEALVGHGFTHAHIVALSQHPAALGTVAVKYQDMIAALP
EATHEAIVGVGKQWSGARALEALLTVAGELRGPPLQLDTGQLLKIAKRGGVTAVEAVH
AWRNALTGAPLNLTPEQVVAIASNGGGKQALETVQRLLPVLCQAHGLTPEQVVAIASH
DGGKQALETVQRLLPVLCQAHGLTPEQVVAIASNGGGKQALETVQRLLPVLCQAHGLT
PEQVVAIASNGGGKQALETVQRLLPVLCQAHGLTPEQVVAIASNIGGKQALETVQRLLP
VLCQAHGLTPEQVVAIASHDGGKQALETVQRLLPVLCQAHGLTPEQVVAIASNGGGKQ
ALETVQRLLPVLCQAHGLTPEQVVAIASNGGGKQALETVQRLLPVLCQAHGLTPEQVV
AIASNIGGKQALETVQRLLPVLCQAHGLTPEQVVAIASNGGGKQALETVQRLLPVLCQA
HGLTPEQVVAIASNIGGKQALETVQRLLPVLCQAHGLTPEQVVAIASNIGGKQALETVQ
RLLPVLCQAHGLTPEQVVAIASHDGGRPALESIVAQLSRPDPALAALTNDHLVALACLG
GRPALDAVKKGLPHAPALIKRTNRRIPERTSHRVADHAQVVRVLGFFQCHSHPAQAFDD
AMTQFGMSRHGLLQLFRRVGVTELEARSGTLPPASQRWDRILQASGMKRAKPSPTSTQ
TPDQASLHAFADSLERDLDAPSPMHEGDQTRASASPKKKRKVEASKMDKKTIVWFRRD
LRIEDNPALAAAAHEGSVFPVFIWCPEEEGQFYPGRASRWWMKQSLAHLSQSLKALGS
DLTLIKTHNTISAILDCIRVTGATKVVFNHLYDPVSLVRDHTVKEKLVERGISVQSYNGD
LLYEPWEIYCEKGKPFTSFNSYWKKCLDMSIESVMLPPPWRLMPITAAAEAIWACSIEEL
GLENEAEKPSNALLTRAWSPGWSNADKLLNEFIEKQLIDYAKNSKKVVGNSTSLLSPYL
HFGEISVRHVFQCARMKQIIWARDKNSEGEESADLFLRGIGLREYSRYICFNFPFTHEQSL
LSHLRFFPWDADVDKFKAWR[?]GRTGYPLVDAGMRELWATGWMHNRIRVIVSSFAVKF
LLLPWKWGMKYFWDTLLDADLECDILGWQYISGSIPDGHELDRLDNPALQGAKYDPEG
EYIRQWLPELARLPTEWIHHPWDAPLTVLKASGVELGTNYAKPIVDIDTARELLAKAISR
TREAQIMIGAAP
> HA-NLS-TALE(*p11*, N136)-SID 
MYPYDVPDYASPKKKRKVEASVDLRTLGYSQQQQEKIKPKVRSTVAQHHEA
LVGHGFTHAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPEQVVAIA
SNNGGKQALETVQRLLPVLCQAHGLTPEQVVAIASHDGGKQALETVQRLLPVLCQAHG
LTPEQVVAIASHDGGKQALETVQRLLPVLCQAHGLTPEQVVAIASNGGGKQALETVQR
 
LLPVLCQAHGLTPEQVVAIASNNGGKQALETVQRLLPVLCQAHGLTPEQVVAIASHDGG
KQALETVQRLLPVLCQAHGLTPEQVVAIASHDGGKQALETVQRLLPVLCQAHGLTPEQ
VVAIASHDGGKQALETVQRLLPVLCQAHGLTPEQVVAIASNGGGKQALETVQRLLPVL
CQAHGLTPEQVVAIASHDGGKQALETVQRLLPVLCQAHGLTPEQVVAIASHDGGKQAL
ETVQRLLPVLCQAHGLTPEQVVAIASNIGGKQALETVQRLLPVLCQAHGLTPEQVVAIA
SNNGGKQALETVQRLLPVLCQAHGLTPEQVVAIASNNGGKQALETVQRLLPVLCQAHG
LTPEQVVAIASHDGGKQALETVQRLLPVLCQAHGLTPEQVVAIASNGGGKQALETVQR
LLPVLCQAHGLTPEQVVAIASHDGGKQALETVQRLLPVLCQAHGLTPEQVVAIASHDGG
KQALETVQRLLPVLCQAHGLTPEQVVAIASHDGGRPALESIVAQLSRPDPALAALTNDH
LVALACLGGRPALDAVKKGLPHAPALIKRTNRRIPERTSHRVADHAQVVRVLGFFQCHS
HPAQAFDDAMTQFGMSRHGLLQLFRRVGVTELEARSGTLPPASQRWDRILQASGMKRA
KPSPTSTQTPDQASLHAFADSLERDLDAPSPMHEGDQTRASASGSGMNIQMLLEAADYL
ERREREAEHGYASMLP.
> HA-NLS-TALE(*p11*, N136)-SID4X 
MYPYDVPDYASPKKKRKVEASVDLRTLGYSQQQQEKIKPKVRSTVAQHHEA
LVGHGFTHAHIVALSQHPAALGTVAVKYQDMIAALPEATHEAIVGVGKQWSGARALEA
LLTVAGELRGPPLQLDTGQLLKIAKRGGVTAVEAVHAWRNALTGAPLNLTPEQVVAIA
SNNGGKQALETVQRLLPVLCQAHGLTPEQVVAIASHDGGKQALETVQRLLPVLCQAHG
LTPEQVVAIASHDGGKQALETVQRLLPVLCQAHGLTPEQVVAIASNGGGKQALETVQR
LLPVLCQAHGLTPEQVVAIASNNGGKQALETVQRLLPVLCQAHGLTPEQVVAIASHDGG
KQALETVQRLLPVLCQAHGLTPEQVVAIASHDGGKQALETVQRLLPVLCQAHGLTPEQ
VVAIASHDGGKQALETVQRLLPVLCQAHGLTPEQVVAIASNGGGKQALETVQRLLPVL
CQAHGLTPEQVVAIASHDGGKQALETVQRLLPVLCQAHGLTPEQVVAIASHDGGKQAL
ETVQRLLPVLCQAHGLTPEQVVAIASNIGGKQALETVQRLLPVLCQAHGLTPEQVVAIA
SNNGGKQALETVQRLLPVLCQAHGLTPEQVVAIASNNGGKQALETVQRLLPVLCQAHG
LTPEQVVAIASHDGGKQALETVQRLLPVLCQAHGLTPEQVVAIASNGGGKQALETVQR
LLPVLCQAHGLTPEQVVAIASHDGGKQALETVQRLLPVLCQAHGLTPEQVVAIASHDGG
KQALETVQRLLPVLCQAHGLTPEQVVAIASHDGGRPALESIVAQLSRPDPALAALTNDH
LVALACLGGRPALDAVKKGLPHAPALIKRTNRRIPERTSHRVADHAQVVRVLGFFQCHS
HPAQAFDDAMTQFGMSRHGLLQLFRRVGVTELEARSGTLPPASQRWDRILQASGMKRA
KPSPTSTQTPDQASLHAFADSLERDLDAPSPMHEGDQTRASASGSGMNIQMLLEAADYL
 
ERRERREAEHGYASMLPGSGMNIQMLLEAADYLERRERREAEHGYASMLPGSGMNIQML
LEAADYLERRERREAEHGYASMLPGSGMNIQMLLEAADYLERRERREAEHGYASMLPSR
>HA-TALE(*ng2*, C63)-GS-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRQIEFLSMKLAIVNPRPDFDMDDIFAKEVAS
TPMTVVPSPEMVLSGYSHEMVHSGYSSEMVNSGYLHVNPMQQVNTSSDPLSCFNNGEA
PSMWDSHVQNLYGNLGV
> HA-TALE(*ng2*, C63)-wNES-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
 
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLYPERLRRILTNGAIGGDLLLNFPDMSVLERQRA
HLKYLNPTFDSPLAGFFADSSMITGGEMDSYLSTAGLNLPMMYGETTVEGDSRLSISPET
TLGTGNFKAAKFDTETKDCNEAAKKMTMNRDDLVEEGEEEKSKITEQNNGSTKSIKKM
KHKAKKEENNFSNDSSKVTKELEKTDYIHVRARRGQATDSHSIAERVRREKISERMKFL
QDLVPGCDKITGKAGMLDEIINYVQSLQRQIEFLSMKLAIVNPRPDFDMDDIFAKEVAST
PMTVVPSPEMVLSGYSHEMVHSGYSSEMVNSGYLHVNPMQQVNTSSDPLSCFNNGEAP
SMWDSHVQNLYGNLGV
> HA-TALE(*ng2*, C63)-mNES-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLQLPPLERLTLNGAIGGDLLLNFPDMSVLERQR
 
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRQIEFLSMKLAIVNPRPDFDMDDIFAKEVAS
TPMTVVPSPEMVLSGYSHEMVHSGYSSEMVNSGYLHVNPMQQVNTSSDPLSCFNNGEA
PSMWDSHVQNLYGNLGV
> HA-TALE(*ng2*, C63)-ptk2NES-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLDLASLILNGAIGGDLLLNFPDMSVLERQRAHL
KYLNPTFDSPLAGFFADSSMITGGEMDSYLSTAGLNLPMMYGETTVEGDSRLSISPETTL
GTGNFKAAKFDTETKDCNEAAKKMTMNRDDLVEEGEEEKSKITEQNNGSTKSIKKMKH
KAKKEENNFSNDSSKVTKELEKTDYIHVRARRGQATDSHSIAERVRREKISERMKFLQD
LVPGCDKITGKAGMLDEIINYVQSLQRQIEFLSMKLAIVNPRPDFDMDDIFAKEVASTPM
TVVPSPEMVLSGYSHEMVHSGYSSEMVNSGYLHVNPMQQVNTSSDPLSCFNNGEAPSM
WDSHVQNLYGNLGV
> HA-TALE(*ng2*, C63)-mapkkNES-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
 
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLQKKLEELELNGAIGGDLLLNFPDMSVLERQRA
HLKYLNPTFDSPLAGFFADSSMITGGEMDSYLSTAGLNLPMMYGETTVEGDSRLSISPET
TLGTGNFKAAKFDTETKDCNEAAKKMTMNRDDLVEEGEEEKSKITEQNNGSTKSIKKM
KHKAKKEENNFSNDSSKVTKELEKTDYIHVRARRGQATDSHSIAERVRREKISERMKFL
QDLVPGCDKITGKAGMLDEIINYVQSLQRQIEFLSMKLAIVNPRPDFDMDDIFAKEVAST
PMTVVPSPEMVLSGYSHEMVHSGYSSEMVNSGYLHVNPMQQVNTSSDPLSCFNNGEAP
SMWDSHVQNLYGNLGV
> HA-TALE(*ng2*, C63)-GS-cib1Δ3-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
 
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRQIEFLSMKLAIVNPRPDFDMDDIFAKEVAS
TPMTVVPSPEMVLSGYSHEMVHSGYSSEMVNSGYLHVNPMQQVNTSS
> HA-TALE(*ng2*, C63)-wNLS-cib1Δ3-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLYPERLRRILTNGAIGGDLLLNFPDMSVLERQRA
HLKYLNPTFDSPLAGFFADSSMITGGEMDSYLSTAGLNLPMMYGETTVEGDSRLSISPET
TLGTGNFKAAKFDTETKDCNEAAKKMTMNRDDLVEEGEEEKSKITEQNNGSTKSIKKM
KHKAKKEENNFSNDSSKVTKELEKTDYIHVRARRGQATDSHSIAERVRREKISERMKFL
QDLVPGCDKITGKAGMLDEIINYVQSLQRQIEFLSMKLAIVNPRPDFDMDDIFAKEVAST
PMTVVPSPEMVLSGYSHEMVHSGYSSEMVNSGYLHVNPMQQVNTSS
> HA-TALE(*ng2*, C63)-mNLS-cib1Δ3-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASSPKKKRKVEASNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRQIEFLSMKLAIVNPRPDFDMDDIFAKEVAS
TPMTVVPSPEMVLSGYSHEMVHSGYSSEMVNSGYLHVNPMQQVNTSS
> HA-TALE(*ng2*, C63)-GS-cib1-mutNLS-mutbHLH 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
 
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARAAQATDSHSIAEAVAREKISERMK
FLQDLVPGCDKITGKAGMLDEIINYVQSLQRQIEFLSMKLAIVNPRPDFDMDDIFAKEVA
STPMTVVPSPEMVLSGYSHEMVHSGYSSEMVNSGYLHVNPMQQVNTSSDPLSCFNNGE
APSMWDSHVQNLYGNLGV
> HA-TALE(*ng2*, C63)-wNES-cibl-mutNLS-mutbHLH 
SRTRLPSPPAPSPAFSADSFSDLLRQFDPSLFNTSLFDSLPPFGAHHTEAATGE
WDEVQSGLRAADAPPPTMRVAVTAARPPRAKPAPRRRAAQPSDASPAAQVDLRTLGYS
QQQQEKIKPKVRSTVAQHHEALVGHGFTHAHIVALSQHPAALGTVAVKYQDMIAALPE
ATHEAIVGVGKQWSGARALEALLTVAGELRGPPLQLDTGQLLKIAKRGGVTAVEAVHA
WRNALTGAPLNLTPEQVVAIASNGGGKQALETVQRLLPVLCQAHGLTPEQVVAIASNN
GGKQALETVQRLLPVLCQAHGLTPEQVVAIASNIGGKQALETVQRLLPVLCQAHGLTPE
QVVAIASNIGGKQALETVQRLLPVLCQAHGLTPEQVVAIASNGGGKQALETVQRLLPVL
CQAHGLTPEQVVAIASNNGGKQALETVQRLLPVLCQAHGLTPEQVVAIASNIGGKQALE
TVQRLLPVLCQAHGLTPEQVVAIASNGGGKQALETVQRLLPVLCQAHGLTPEQVVAIAS
NNGGKQALETVQRLLPVLCQAHGLTPEQVVAIASNIGGKQALETVQRLLPVLCQAHGL
TPEQVVAIASNGGGKQALETVQRLLPVLCQAHGLTPEQVVAIASNIGGKQALETVQRLL
PVLCQAHGLTPEQVVAIASNIGGKQALETVQRLLPVLCQAHGLTPEQVVAIASNGGGKQ
ALETVQRLLPVLCQAHGLTPEQVVAIASNIGGKQALETVQRLLPVLCQAHGLTPEQVVA
IASHDGGRPALESIVAQLSRPDPALAALTNDHLVALACLGGRPALDAVKKGLPHAPALI
KRTNRRIPERTSHRVAASLYPERLRRILTNGAIGGDLLLNFPDMSVLERQRAHLKYLNPT
FDSPLAGFFADSSMITGGEMDSYLSTAGLNLPMMYGETTVEGDSRLSISPETTLGTGNFK
AAKFDTETKDCNEAAKKMTMNRDDLVEEGEEEKSKITEQNNGSTKSIKKMKHKAKKE
ENNFSNDSSKVTKELEKTDYIHVRARAAQATDSHSIAEAVAREKISERMKFLQDLVPGC
DKITGKAGMLDEIINYVQSLQRQIEFLSMKLAIVNPRPDFDMDDIFAKEVASTPMTVVPS
 
PEMVLSGYSHEMVHSGYSSEMVNSGYLHVNPMQQVNTSSDPLSCFNNGEAPSMWDSH
VQNLYGNLGV
> HA-TALE(*ng2*, C63)-GS-cib1Δ1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRGGSVASTPMTVVPSPEMVLSGYSHEMVHS
GYSSEMVNSGYLHVNPMQQVNTSSDPLSCFNNGEAPSMWDSHVQNLYGNLGV
> HA-TALE(*ng2*, C63)-wNLS-cib1Δ1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
 
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLYPERLRRILTNGAIGGDLLLNFPDMSVLERQRA
HLKYLNPTFDSPLAGFFADSSMITGGEMDSYLSTAGLNLPMMYGETTVEGDSRLSISPET
TLGTGNFKAAKFDTETKDCNEAAKKMTMNRDDLVEEGEEEKSKITEQNNGSTKSIKKM
KHKAKKEENNFSNDSSKVTKELEKTDYIHVRARRGQATDSHSIAERVRREKISERMKFL
QDLVPGCDKITGKAGMLDEIINYVQSLQRGGSGEEEKSKITEQNNGSTKSIKKMKHKAK
KEENNFSNDSSKVTKELEKTDYIHVRARRGQATDSHSIAERVRREKISERMKFLQDLVP
GCDKITGKAGMLDEIINYVQSLQRGGSVASTPMTVVPSPEMVLSGYSHEMVHSGYSSE
MVNSGYLHVNPMQQVNTSSDPLSCFNNGEAPSMWDSHVQNLYGNLGV
>HA-TALE(*ng2*, C63)-GS-cib1Δ2-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
 
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRQIEFLSMKLAIVNPRPDFDMDDIFAKEVAS
TPMTVVPSPEMVLSGYGGSPLSCFNNGEAPSMWDSHVQNLYGNLGV
> HA-TALE(*ng2*, C63)-wNES-cib1Δ2-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLYPERLRRILTNGAIGGDLLLNFPDMSVLERQRA
HLKYLNPTFDSPLAGFFADSSMITGGEMDSYLSTAGLNLPMMYGETTVEGDSRLSISPET
TLGTGNFKAAKFDTETKDCNEAAKKMTMNRDDLVEEGEEEKSKITEQNNGSTKSIKKM
KHKAKKEENNFSNDSSKVTKELEKTDYIHVRARRGQATDSHSIAERVRREKISERMKFL
QDLVPGCDKITGKAGMLDEIINYVQSLQRQIEFLSMKLAIVNPRPDFDMDDIFAKEVAST
PMTVVPSPEMVLSGYGGSPLSCFNNGEAPSMWDSHVQNLYGNLGV
> HA-TALE(*ng2*, C63)-NLS-cib1-mutNLS-mutbHLH 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
 
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASSPKKKRKVEASNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARAAQATDSHSIAEAVAREKISERMK
FLQDLVPGCDKITGKAGMLDEIINYVQSLQRQIEFLSMKLAIVNPRPDFDMDDIFAKEVA
STPMTVVPSPEMVLSGYSHEMVHSGYSSEMVNSGYLHVNPMQQVNTSSDPLSCFNNGE
APSMWDSHVQNLYGNLGV
> HA-TALE(*ng2*, C63)-NLS-cib1Δ1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
 
GLTPEQVVAIASHDGGRPALESIVAQLSRPDPALAALTNDHLVALACLGGRPALDAVKK
GLPHAPALIKRTNRRIPERTSHRVAASSPKKKRKVEASNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRGGSVASTPMTVVPSPEMVLSGYSHEMVHS
GYSSEMVNSGYLHVNPMQQVNTSSDPLSCFNNGEAPSMWDSHVQNLYGNLGV
> HA-TALE(*ng2*, C63)-NLS-cib1Δ2-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASLYPERLRRILTNGAIGGDLLLNFPDMSVLERQRA
HLKYLNPTFDSPLAGFFADSSMITGGEMDSYLSTAGLNLPMMYGETTVEGDSRLSISPET
TLGTGNFKAAKFDTETKDCNEAAKKMTMNRDDLVEEGEEEKSKITEQNNGSTKSIKKM
KHKAKKEENNFSNDSSKVTKELEKTDYIHVRARRGQATDSHSIAERVRREKISERMKFL
QDLVPGCDKITGKAGMLDEIINYVQSLQRQIEFLSMKLAIVNPRPDFDMDDIFAKEVAST
PMTVVPSPEMVLSGYGGSPLSCFNNGEAPSMWDSHVQNLYGNLGV
> HA-TALE(*ng2*, C63)-GS-iNES1-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
 
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLLYPERLRRILTNPTFDSPLAGFFADSSMITGGEMDSYLSTAGLNLPMMYGETT
VEGDSRLSISPETTLGTGNFKAAKFDTETKDCNEAAKKMTMNRDDLVEEGEEEKSKITE
QNNGSTKSIKKMKHKAKKEENNFSNDSSKVTKELEKTDYIHVRARRGQATDSHSIAERV
RREKISERMKFLQDLVPGCDKITGKAGMLDEIINYVQSLQRQIEFLSMKLAIVNPRPDFD
MDDIFAKEVASTPMTVVPSPEMVLSGYSHEMVHSGYSSEMVNSGYLHVNPMQQVNTS
SDPLSCFNNGEAPSMWDSHVQNLYGNLGV
> HA-TALE(*ng2*, C63)-GS-iNES2-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
 
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDLYPERLRRILTSYLSTAGLNLPMMYGETT
VEGDSRLSISPETTLGTGNFKAAKFDTETKDCNEAAKKMTMNRDDLVEEGEEEKSKITE
QNNGSTKSIKKMKHKAKKEENNFSNDSSKVTKELEKTDYIHVRARRGQATDSHSIAERV
RREKISERMKFLQDLVPGCDKITGKAGMLDEIINYVQSLQRQIEFLSMKLAIVNPRPDFD
MDDIFAKEVASTPMTVVPSPEMVLSGYSHEMVHSGYSSEMVNSGYLHVNPMQQVNTS
SDPLSCFNNGEAPSMWDSHVQNLYGNLGV
> HA-TALE(*ng2*, C63)-GS-iNES3-cibl-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDSYLSTAGLNLPMMYGETTVEGLYPERLRR
ILTDSRLSISPETTLGTGNFKAAKFDTETKDCNEAAKKMTMNRDDLVEEGEEEKSKITEQ
NNGSTKSIKKMKHKAKKEENNFSNDSSKVTKELEKTDYIHVRARRGQATDSHSIAERVR
REKISERMKFLQDLVPGCDKITGKAGMLDEIINYVQSLQRQIEFLSMKLAIVNPRPDFDM
 
DDIFAKEVASTPMTVVPSPEMVLSGYSHEMVHSGYSSEMVNSGYLHVNPMQQVNTSSD
PLSCFNNGEAPSMWDSHVQNLYGNLGV
> HA-TALE(*ng2*, C63)-GS-iNES4-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAAAKKMTMNRDDLVEEGLYPERLRRILTEEEKSKIT
EQNNGSTKSIKKMKHKAKKEENNFSNDSSKVTKELEKTDYIHVRARRGQATDSHSIAER
VRREKISERMKFLQDLVPGCDKITGKAGMLDEIINYVQSLQRQIEFLSMKLAIVNPRPDF
DMDDIFAKEVASTPMTVVPSPEMVLSGYSHEMVHSGYSSEMVNSGYLHVNPMQQVNT
SSDPLSCFNNGEAPSMWDSHVQNLYGNLGV
> HA-TALE(*ng2*, C63)-GS-iNES5-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
 
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
LYPERLRRILTMKHKAKKEENNFSNDSSKVTKELEKTDYIHVRARRGQATDSHSIAERV
RREKISERMKFLQDLVPGCDKITGKAGMLDEIINYVQSLQRQIEFLSMKLAIVNPRPDFD
MDDIFAKEVASTPMTVVPSPEMVLSGYSHEMVHSGYSSEMVNSGYLHVNPMQQVNTS
SDPLSCFNNGEAPSMWDSHVQNLYGNLGV
> HA-TALE(*ng2*, C63)-GS-iNES6-cib1-mutNLS 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASGGGGSGGGGSNGAIGGDLLLNFPDMSVLERQR
 
AHLKYLNPTFDSPLAGFFADSSMITGGEMDSYLSTAGLNLPMMYGETTVEGDSRLSISPE
TTLGTGNFKAAKFDTETKDCNEAAKKMTMNRDDLVEEGEEEKSKITEQNNGSTKSIKK
MKHKAKKEENNFSNDSSKVTLYPERLRRILTKELEKTDYIHVRARRGQATDSHSIAERV
RREKISERMKFLQDLVPGCDKITGKAGMLDEIINYVQSLQRQIEFLSMKLAIVNPRPDFD
MDDIFAKEVASTPMTVVPSPEMVLSGYSHEMVHSGYSSEMVNSGYLHVNPMQQVNTS
SDPLSCFNNGEAPSMWDSHVQNLYGNLGV
> HA-TALE(*ng2*, C63)-NLS-cib1Δ1 
MYPYDVPDYASRTRLPSPPAPSPAFSADSFSDLLRQFDPSLFNTSLFDSLPPFG
AHHTEAATGEWDEVQSGLRAADAPPPTMRVAVTAARPPRAKPAPRRRAAQPSDASPA
AQVDLRTLGYSQQQQEKIKPKVRSTVAQHHEALVGHGFTHAHIVALSQHPAALGTVAV
KYQDMIAALPEATHEAIVGVGKQWSGARALEALLTVAGELRGPPLQLDTGQLLKIAKR
GGVTAVEAVHAWRNALTGAPLNLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNIGGKQALETVQRLLPVLCQAHGLTPEQVVAIASNGGGKQ
ALETVQRLLPVLCQAHGLTPEQVVAIASNNGGKQALETVQRLLPVLCQAHGLTPEQVV
AIASNIGGKQALETVQRLLPVLCQAHGLTPEQVVAIASNGGGKQALETVQRLLPVLCQA
HGLTPEQVVAIASNNGGKQALETVQRLLPVLCQAHGLTPEQVVAIASNIGGKQALETVQ
RLLPVLCQAHGLTPEQVVAIASNGGGKQALETVQRLLPVLCQAHGLTPEQVVAIASNIG
GKQALETVQRLLPVLCQAHGLTPEQVVAIASNIGGKQALETVQRLLPVLCQAHGLTPEQ
VVAIASNGGGKQALETVQRLLPVLCQAHGLTPEQVVAIASNIGGKQALETVQRLLPVLC
QAHGLTPEQVVAIASHDGGRPALESIVAQLSRPDPALAALTNDHLVALACLGGRPALDA
VKKGLPHAPALIKRTNRRIPERTSHRVAASSPKKKRKVEASNGAIGGDLLLNFPDMSVLE
RQRAHLKYLNPTFDSPLAGFFADSSMITGGEMDSYLSTAGLNLPMMYGETTVEGDSRLS
ISPETTLGTGNFKKRKFDTETKDCNEKKKKMTMNRDDLVEEGEEEKSKITEQNNGSTKS
IKKMKHKAKKEENNFSNDSSKVTKELEKTDYIHVRARRGQATDSHSIAERVRREKISER
MKFLQDLVPGCDKITGKAGMLDEIINYVQSLQRGGSVASTPMTVVPSPEMVLSGYSHE
MVHSGYSSEMVNSGYLHVNPMQQVNTSSDPLSCFNNGEAPSMWDSHVQNLYGNLGV
> HA-TALE(*ng2*, C63)-NLS-cib1Δ2 
YPYDVPDYASRTRLPSPPAPSPAFSADSFSDLLRQFDPSLFNTSLFDSLPPFGA
HHTEAATGEWDEVQSGLRAADAPPPTMRVAVTAARPPRAKPAPRRRAAQPSDASPAA
QVDLRTLGYSQQQQEKIKPKVRSTVAQHHEALVGHGFTHAHIVALSQHPAALGTVAVK
 
YQDMIAALPEATHEAIVGVGKQWSGARALEALLTVAGELRGPPLQLDTGQLLKIAKRG
GVTAVEAVHAWRNALTGAPLNLTPEQVVAIASNGGGKQALETVQRLLPVLCQAHGLTP
EQVVAIASNNGGKQALETVQRLLPVLCQAHGLTPEQVVAIASNIGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASNNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNNGGKQALETVQRLLPVLCQAHGLTPEQVVAIASNIGGKQALETVQRLL
PVLCQAHGLTPEQVVAIASNGGGKQALETVQRLLPVLCQAHGLTPEQVVAIASNIGGKQ
ALETVQRLLPVLCQAHGLTPEQVVAIASNIGGKQALETVQRLLPVLCQAHGLTPEQVVA
IASNGGGKQALETVQRLLPVLCQAHGLTPEQVVAIASNIGGKQALETVQRLLPVLCQAH
GLTPEQVVAIASHDGGRPALESIVAQLSRPDPALAALTNDHLVALACLGGRPALDAVKK
GLPHAPALIKRTNRRIPERTSHRVAASSPKKKRKVEASNGAIGGDLLLNFPDMSVLERQR
AHLKYLNPTFDSPLAGFFADSSMITGGEMDSYLSTAGLNLPMMYGETTVEGDSRLSISPE
TTLGTGNFKKRKFDTETKDCNEKKKKMTMNRDDLVEEGEEEKSKITEQNNGSTKSIKK
MKHKAKKEENNFSNDSSKVTKELEKTDYIHVRARRGQATDSHSIAERVRREKISERMKF
LQDLVPGCDKITGKAGMLDEIINYVQSLQRQIEFLSMKLAIVNPRPDFDMDDIFAKEVAS
TPMTVVPSPEMVLSGYGGSPLSCFNNGEAPSMWDSHVQNLYGNLGV
> alpha-importin-NLS-CRY2PHR-NLS-VP64_2A_GFP 
MKRPAATKKAGQAKKKKKMDKKTIVWFRRDLRIEDNPALAAAAHEGSVFP
VFIWCPEEEGQFYPGRASRWWMKQSLAHLSQSLKALGSDLTLIKTHNTISAILDCIRVTG
ATKVVFNHLYDPVSLVRDHTVKEKLVERGISVQSYNGDLLYEPWEIYCEKGKPFTSFNS
YWKKCLDMSIESVMLPPPWRLMPITAAAEAIWACSIEELGLENEAEKPSNALLTRAWSP
GWSNADKLLNEFIEKQLIDYAKNSKKVVGNSTSLLSPYLHFGEISVRHVFQCARMKQII
WARDKNSEGEESADLFLRGIGLREYSRYICFNFPFTHEQSLLSHLRFFPWDADVDKFKA
WRQGRTGYPLVDAGMRELWATGWMHNRIRVIVSSFAVKFLLLPWKWGMKYFWDTLL
DADLECDILGWQYISGSIPDGHELDRLDNPALQGAKYDPEGEYIRQWLPELARLPTEWIH
HPWDAPLTVLKASGVELGTNYAKPIVDIDTARELLAKAISRTREAQIMIGAAPASPKKKR
KVEASGSGRADALDDFDLDMLGSDALDDFDLDMLGSDALDDFDLDMLGSDALDDFDL
DMLINSRGSGEGRGSLLTCGDVEENPGPVSKGEELFTGVVPILVELDGDVNGHKFSVSG
EGEGDATYGKLTLKFICTTGKLPVPWPTLVTTLTYGVQCFSRYPDHMKQHDFFKSAMP
EGYVQERTIFFKDDGNYKTRAEVKFEGDTLVNRIELKGIDFKEDGNILGHKLEYNYNSH
 
NVYIMADKQKNGIKVNFKIRHNIEDGSVQLADHYQQNTPIGDGPVLLPDNHYLSTQSAL
SKDPNEKRDHMVLLEFVTAAGITLGMDELYKV
> mutNES-CRY2PHR-NLS-VP64_2A_GFP 
MEQKLISEEDLKMDKKTIVWFRRDLRIEDNPALAAAAHEGSVFPVFIWCPEEE
GQFYPGRASRWWMKQSLAHLSQSLKAAGSDATLIKTHNTISAILDCIRVTGATKVVFNH
LYDPVSLVRDHTVKEKLVERGISVQSYNGDLLYEPWEIYCEKGKPFTSFNSYWKKCLD
MSIESVMLPPPWRLMPITAAAEAIWACSIEELGLENEAEKPSNALLTRAWSPGWSNADK
LLNEFIEKQLIDYAKNSKKVVGNSTSLLSPYLHFGEISVRHVFQCARMKQIIWARDKNSE
GEESADLFLRGIGLREYSRYICFNFPFTHEQSLLSHLRFFPWDADVDKFKAWRQGRTGYP
LVDAGMRELWATGWMHNRIRVIVSSFAVKFLLLPWKWGMKYFWDTLLDADLECDILG
WQYISGSIPDGHELDRLDNPALQGAKYDPEGEYIRQWLPELARLPTEWIHHPWDAPLTV
LKASGVELGTNYAKPIVDIDTARELLAKAISRTREAQIMIGAAPASPKKKRKVEASGSGR
ADALDDFDLDMLGSDALDDFDLDMLGSDALDDFDLDMLGSDALDDFDLDMLINSRGS
GEGRGSLLTCGDVEENPGPVSKGEELFTGVVPILVELDGDVNGHKFSVSGEGEGDATYG
KLTLKFICTTGKLPVPWPTLVTTLTYGVQCFSRYPDHMKQHDFFKSAMPEGYVQERTIF
FKDDGNYKTRAEVKFEGDTLVNRIELKGIDFKEDGNILGHKLEYNYNSHNVYIMADKQ
KNGIKVNFKIRHNIEDGSVQLADHYQQNTPIGDGPVLLPDNHYLSTQSALSKDPNEKRD
HMVLLEFVTAAGITLGMDELYKV
> CRY2PHR-NLS-VP64-NLS_2A_GFP 
MKMDKKTIVWFRRDLRIEDNPALAAAAHEGSVFPVFIWCPEEEGQFYPGRAS
RWWMKQSLAHLSQSLKALGSDLTLIKTHNTISAILDCIRVTGATKVVFNHLYDPVSLVR
DHTVKEKLVERGISVQSYNGDLLYEPWEIYCEKGKPFTSFNSYWKKCLDMSIESVMLPP
PWRLMPITAAAEAIWACSIEELGLENEAEKPSNALLTRAWSPGWSNADKLLNEFIEKQLI
DYAKNSKKVVGNSTSLLSPYLHFGEISVRHVFQCARMKQIIWARDKNSEGEESADLFLR
GIGLREYSRYICFNFPFTHEQSLLSHLRFFPWDADVDKFKAWRQGRTGYPLVDAGMREL
WATGWMHNRIRVIVSSFAVKFLLLPWKWGMKYFWDTLLDADLECDILGWQYISGSIPD
GHELDRLDNPALQGAKYDPEGEYIRQWLPELARLPTEWIHHPWDAPLTVLKASGVELG
TNYAKPIVDIDTARELLAKAISRTREAQIMIGAAPASPKKKRKVEASGSGRADALDDFDL
DMLGSDALDDFDLDMLGSDALDDFDLDMLGSDALDDFDLDMLINSPKKKRKVEASSR
GSGEGRGSLLTCGDVEENPGPVSKGEELFTGVVPILVELDGDVNGHKFSVSGEGEGDAT
YGKLTLKFICTTGKLPVPWPTLVTTLTYGVQCFSRYPDHMKQHDFFKSAMPEGYVQER
 
TIFFKDDGNYKTRAEVKFEGDTLVNRIELKGIDFKEDGNILGHKLEYNYNSHNVYIMAD
KQKNGIKVNFKIRHNIEDGSVQLADHYQQNTPIGDGPVLLPDNHYLSTQSALSKDPNEK
RDHMVLLEFVTAAGITLGMDELYKV
> Neurog2-TALE(N240,C63)-PYL 
MSRTRLPSPPAPSPAFSADSFSDLLRQFDPSLFNTSLFDSLPPFGAHHTEAATG
EWDEVQSGLRAADAPPPTMRVAVTAARPPRAKPAPRRRAAQPSDASPAAQVDLRTLGY
SQQQQEKIKPKVRSTVAQHHEALVGHGFTHAHIVALSQHPAALGTVAVKYQDMIAALP
EATHEAIVGVGKQWSGARALEALLTVAGELRGPPLQLDTGQLLKIAKRGGVTAVEAVH
AWRNALTGAPLNLTPEQVVAIASHNGGKQALETVQRLLPVLCQAHGLTPEQVVAIASNI
GGKQALETVQRLLPVLCQAHGLTPEQVVAIASNIGGKQALETVQRLLPVLCQAHGLTPE
QVVAIASNGGGKQALETVQRLLPVLCQAHGLTPEQVVAIASHNGGKQALETVQRLLPV
LCQAHGLTPEQVVAIASNIGGKQALETVQRLLPVLCQAHGLTPEQVVAIASNGGGKQAL
ETVQRLLPVLCQAHGLTPEQVVAIASHNGGKQALETVQRLLPVLCQAHGLTPEQVVAIA
SNIGGKQALETVQRLLPVLCQAHGLTPEQVVAIASNGGGKQALETVQRLLPVLCQAHGL
TPEQVVAIASNIGGKQALETVQRLLPVLCQAHGLTPEQVVAIASNIGGKQALETVQRLLP
VLCQAHGLTPEQVVAIASNGGGRPALESIVAQLSRPDPALAALTNDHLVALACLGGRPA
LDAVKKGLPHAPALIKRTNRRIPERTSHRVAASMANSESSSSPVNEEENSQRISTLHHQT
MPSDLTQDEFTQLSQSIAEFHTYQLGNGRCSSLLAQRIHAPPETVWSVVRRFDRPQIYKH
FIKSCNVSEDFEMRVGCTRDVNVISGLPANTSRERLDLLDDDRRVTGFSITGGEHRLRNY
KSVTTVHRFEKEEEEERIWTVVLESYVVDVPEGNSEEDTRLFADTVIRLNLQKLASITEA
MNRNNNNNNNSSQVR
>ABI-NLS-VP64 
MVPLYGFTSICGRRPEMEAAVSTIPRFLQSSSGSMLDGRFDPQSAAHFFGVYD
GHGGSQVANYCRERMHLALAEEIAKEKPMLCDGDTWLEKWKKALFNSFLRVDSEIESV
APETVGSTSVVAVVFPSHIFVANCGDSRAVLCRGKTALPLSVDHKPDREDEAARIEAAG
GKVIQWNGARVFGVLAMSRSIGDRYLKPSIIPDPEVTAVKRVKEDDCLILASDGVWDV
MTDEEACEMARKRILLWHKKNAVAGDASLLADERRKEGKDPAAMSAAEYLSKLAIQR
GSKDNISVVVVDLKPRRKLKSKPLNASPKKKRKVEASGSGRADALDDFDLDMLGSDAL
DDFDLDMLGSDALDDFDLDMLGSDALDDFDLDMLIN
>hSpCas9(D)0A,H840A)-Linker-NLS-VP64 
MDKKYSIGLAIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKK
HERHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFL
AAKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSEETI
TPWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNASL
GTYHDLLKIIKDKDFLDNEENEDILEDIVLTLTLFEDREMIEERLKTYAHLFDDKVMKQL
KRRRYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQEL
DINRLSDYDVDAIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKL
ESEFVYGDYKVYDVRKMIAKSEQEIGKATAKYFFYSNIMNFFKTEITLANGEIRKRPLIET
NGETGEIVWDKGRDFATVRKVLSMPQVNIVKKTEVQTGGFSKESILPKRNSDKLIARKK
DWDPKKYGGFDSPTVAYSVLVVAKVEKGKSKKLKSVKELLGITIMERSSFEKNPIDFLE
AKGYKEVKKDLIIKLPKYSLFELENGRKRMLASAGELQKGNELALPSKYVNFLYLASHY
EKLKGSPEDNEQKQLFVEQHKHYLDEIIEQISEFSKRVILADANLDKVLSAYNKHRDKPI
REQAENIIHLFTLTNLGAPAAFKYFDTTIDRKRYTSTKEVLDATLIHQSITGLYETRIDLSQ
LGGDSAGGGGSGGGGSGGGGSGPKKKRKVAAAGSGRADALDDFDLDMLGSDALDDF
DLDMLGSDALDDFDLDMLGSDALDDFDLDMLIN
>SID4X-NLS-FLAG-Linker-hSpCas9(D10A,H840A)-NLS

MGSGMNIQMLLEAADYLERREREAEHGYASMLPGSGMNIQMLLEAADYLE
RREREAEHGYASMLPGSGMNIQMLLEAADYLERREREAEHGYASMLPGSGMNIQMLL
EAADYLERREREAEHGYASMLPSRSAGPKKKRKVAAADYKDDDDKSRLEPGEKPYKC
PECGKSFSQSGALTRHQRTHTRDKKYSIGLAIGTNSVGWAVITDEYKVPSKKFKVLGNT
DRHSIKKNLIGALLFDSGETAEATRLKRTARRRYTRRKNRICYLQEIFSNEMAKVDDSFF

HRLEESFLVEEDKKHERHPIFGNIVDEVAYHEKYPTIYHLRKKLVDSTDKADLRLIYLAL
AHMIKFRGHFLIEGDLNPDNSDVDKLFIQLVQTYNQLFEENPINASGVDAKAILSARLSK
SRRLENLIAQLPGEKKNGLFGNLIALSLGLTPNFKSNFDLAEDAKLQLSKDTYDDDLDNL
LAQIGDQYADLFLAAKNLSDAILLSDILRVNTEITKAPLSASMIKRYDEHHQDLTLLKAL
VRQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGNS
RFAWMTRKSEETITPWNFEEVVDKGASAQSFIERMTNFDKNLPNEKVLPKHSLLYEYFT
VYNELTKVKYVTEGMRKPAFLSGEQKKAIVDLLFKTNRKVTVKQLKEDYFKKIECFDS
VEISGVEDRFNASLGTYHDLLKIIKDKDFLDNEENEDILEDIVLTLTLFEDREMIEERLKT
YAHLFDDKVMKQLKRRRYTGWGRLSRKLINGIRDKQSGKTILDFLKSDGFANRNFMQL
IHDDSLTFKEDIQKAQVSGQGDSLHEHIANLAGSPAIKKGILQTVKVVDELVKVMGRHK
PENIVIEMARENQTTQKGQKNSRERMKRIEEGIKELGSQILKEHPVENTQLQNEKLYLYY
LQNGRDMYVDQELDINRLSDYDVDAIVPQSFLKDDSIDNKVLTRSDKNRGKSDNVPSEE
VVKKMKNYWRQLLNAKLITQRKFDNLTKAERGGLSELDKAGFIKRQLVETRQITKHVA
QILDSRMNTKYDENDKLIREVKVITLKSKLVSDFRKDFQFYKVREINNYHHAHDAYLNA
VVGTALIKKYPKLESEFVYGDYKVYDVRKMIAKSEQEIGKATAKYFFYSNIMNFFKTEIT
LANGEIRKRPLIETNGETGEIVWDKGRDFATVRKVLSMPQVNIVKKTEVQTGGFSKESIL
PKRNSDKLIARKKDWDPKKYGGFDSPTVAYSVLVVAKVEKGKSKKLKSVKELLGITIM
ERSSFEKNPIDFLEAKGYKEVKKDLIIKLPKYSLFELENGRKRMLASAGELQKGNELALP
SKYVNFLYLASHYEKLKGSPEDNEQKQLFVEQHKHYLDEIIEQISEFSKRVILADANLDK
VLSAYNKHRDKPIREQAENIIHLFTLTNLGAPAAFKYFDTTIDRKRYTSTKEVLDATLIHQ
SITGLYETRIDLSQLGGDKRPAATKKAGQAKKKK

### Epigenetic effector domain sequences

>hs_NCoR 
ASSPKKKRKVEASMNGLMEDPMKVYKDRQFMNVWTDHEKEIFKDKFIQHP
KNFGLIASYLERKSVPDCVLYYYLTKKNENYKEF
>pf_Sir2A 
ASSPKKKRKVEASMGNLMISFLKKDTQSITLEELAKIIKKCKHVVALTGSGTS
AESNIPSFRGSSNSIWSKYDPRIYGTIWGFWKYPEKIWEVIRDISSDYEIEINNGHVALSTL
ESLGYLKSVVTQNVDGLHEASGNTKVISLHGNVFEAVCCTCNKIVKLNKIMLQKTSHFM
 
HQLPPECPCGGIFKPNIILFGEVVSSDLLKEAEEEIAKCDLLLVIGTSSTVSTATNLCHFAC
KKKKKIVEINISKTYITNKMSDYHVCAKFSELTKVANILKGSSEKNKKIMEF
>nc_DIM5 
ASSPKKKRKVEASMEKAFRPHFFNHGKPDANPKEKKNCHWCQIRSFATHAQ
LPISIVNREDDAFLNPNFRFIDHSIIGKNVPVADQSFRVGCSCASDEECMYSTCQCLDEMA
PDSDEEADPYTRKKRFAYYSQGAKKGLLRDRVLQSQEPIYECHQGCACSKDCPNRVVE
RGRTVPLQIFRTKDRGWGVKCPVNIKRGQFVDRYLGEIITSEEADRRRAESTIARRKDVY
LFALDKFSDPDSLDPLLAGQPLEVDGEYMSGPTRFINHSCDPNMAIFARVGDHADKHIH
DLALFAIKDIPKGTELTFDYVNGLTGLESDAHDPSKISEMTKCLCGTAKCRGYLWEF
>sc_HST2 
ASSPKKKRKVEASTEMSVRKIAAHMKSNPNAKVIFMVGAGISTSCGIPDFRSP
GTGLYHNLARLKLPYPEAVFDVDFFQSDPLPFYTLAKELYPGNFRPSKFHYLLKLFQDK
DVLKRVYTQNIDTLERQAGVKDDLIIEAHGSFAHCHCIGCGKVYPPQVFKSKLAEHPIKD
FVKCDVCGELVKPAIVFFGEDLPDSFSETWLNDSEWLREKITTSGKHPQQPLVIVVGTSL
AVYPFASLPEEIPRKVKRVLCNLETVGDFKANKRPTDLIVHQYSDEFAEQLVEELGWQE
DFEKILTAQGGMGEF
>hs_SIRT3 
ASSPKKKRKVEASMVGAGISTPSGIPDFRSPGSGLYSNLQQYDLPYPEAIFELP
FFFHNPKPFFTLAKELYPGNYKPNVTHYFLRLLHDKGLLLRLYTQNIDGLERVSGIPASK
LVEAHGTFASATCTVCQRPFPGEDIRADVMADRVPRCPVCTGVVKPDIVFFGEPLPQRFL
LHVVDFPMADLLLILGTSLEVEPFASLTEAVRSSVPRLLINRDLVGPLAWHPRSRDVAQL
GDVVHGVESLVELLGWTEEMRDLVQRETGKLDGPDKEF
>hs_NIPP1 
ASSPKKKRKVEASMAAAANSGSSLPLFDCPTWAGKPPPGLHLDVVKGDKLIE
KLIIDEKKYYLFGRNPDLCDFTIDHQSCSRVHAALVYHKHLKRVFLIDLNSTHGTFLGHI
RLEPHKPQQIPIDSTVSFGASTRAYTLREKPQTLPSAVKGDEKMGGEDDELKGLLGLPEE
ETELDNLTEFNTAHNKRISTLTIEEGNLDIQRPKRKRKNSRVTFSEDDEIINPEDVDPSVGR
FRNMVQTAVVPVKKKRVEGPGSLGLEESGSRRMQNFAFSGGLYGGLPPTHSEAGSQPH
GIHGTALIGGLPMPYPNLAPDVDLTPVVPSAVNMNPAPNPAVYNPEAVNEEF
>ct_NUE 
ASSPKKKRKVEASMTTNSTQDTLYLSLHGGIDSAIPYPVRRVEQLLQFSFLPE
LQFQNAAVKQRIQRLCYREEKRLAVSSLAKWLGQLHKQRLRAPKNPPVAICWINSYVG
YGVFARESIPAWSYIGEYTGILRRRQALWLDENDYCFRYPVPRYSFRYFTIDSGMQGNV
TRFINHSDNPNLEAIGAFENGIFHIIIRAIKDILPGEELCYHYGPLYWKHRKKREEFVPQEE
EF
>hs_MBD2b 
ASSPKKKRKVEASARYLGNTVDLSSFDFRTGKMMPSKLQKNKQRLRNDPLN
QNKGKPDLNTTLPIRQTASIFKQPVTKVTNHPSNKVKSDPQRMNEQPRQLFWEKRLQGL
SASDVTEQIIKTMELPKGLQGVGPGSNDETLLSAVASALHTSSAPITGQVSAAVEKNPAV
WLNTSQPLCKAFIVTDEDIRKQEERVQQVRKILEDALMADILSRAADTEEMDIEMDSGD
EAEF
>ca_HST2 
ASSPKKKRKVEASMPSLDDILKPVAEAVKNGKKVTFFNGAGISTGAGIPDFRS
PDTGLYANLAKLNLPFAEAVFDIDFFKEDPKPFYTLAEELYPGNFAPTKFHHFIKLLQDQ
GSLKRVYTQNIDTLERLAGVEDKYIVEAHGSFASNHCVDCHKEMTTETLKTYMKDKKI
PSCQHCEGYVKPDIVFFGEGLPVKFFDLWEDDCEDVEVAIVAGTSLTVFPFASLPGEVNK
KCLRVLVNKEKVGTFKHEPRKSDIIALHDCDIVAERLCTLLGLDDKLNEVYEKEKIKYSK
AETKEIKMHEIEDKLKEEAHLKEDKHTTKVDKKEKQNDANDKELEQLIDKAKAEF
>hs_PHF19 
ASSPKKKRKVEASMENRALDPGTRDSYGATSHLPNKGALAKVKNNFKDLMS
KLTEGQYVLCRWTDGLYYLGKIKRVSSSKQSCLVTFEDNSKYWVLWKDIQHAGVPGEE
PKCNICLGKTSGPLNEILICGKCGLGYHQQCHIPIAGSADQPLLTPWFCRRCIFALAVRKG
GALKKGAIARTLQAVKMVLSYQPEELEWDSPHRTNQQQCYCYCGGPGEWYLRMLQCY
RCRQWFHEACTQCLNEPMMFGDRFYLFFCSVCNQGPGGSGSDSSAEGASVPERPDEGID
SHTFESISEDDSSLSHLKSSITNYFGAAGRLACGEKYQVLARRVTPEGKVQYLVEWEGTT
PYEF
>hs_HDAC11 
ASSPKKKRKVEASMLHTTQLYQHVPETRWPIVYSPRYNITFMGLEKLHPFDA
GKWGKVINFLKEEKLLSDSMLVEAREASEEDLLVVHTRRYLNELKWSFAVATITEIPPVI
FLPNFLVQRKVLRPLRTQTGGTIMAGKLAVERGWAINVGGGFHHCSSDRGGGFCAYAD
ITLAIKFLFERVEGISRATIIDLDAHQGNGHERDFMDDKRVYIMDVYNRHIYPGDRFAKQ
 
AIRRKVELEWGTEDDEYLDKVERNIKKSLQEHLPDVVVYNAGTDILEGDRLGGLSISPA
GIVKRDELVFRMVRGRRVPILMVTSGGYQKRTARIIADSILNLFGLGLIGPESPSVSAQNS
DTPLLPPAVPEF
>mL_MesoLo4 
ASSPKKKRKVEASMPLQIVHHPDYDAGFATNHRFPMSKYPLLMEALRARGL
ASPDALNTTEPAPASWLKLAHAADYVDQVISCSVPEKIEREIGFPVGPRVSLRAQLATGG
TILAARLALRHGIACNTAGGSHHARRAQGAGFCTFNDVAVASLVLLDEGAAQNILVVD
LDVHQGDGTADILSDEPGVFTFSMHGERNYPVRKIASDLDIALPDGTGDAAYLRRLATIL
PELSARARWDIVFYNAGVDVHAEDRLGRLALSNGGLRARDEMVIGHFRALGIPVCGVI
GGGYSTDVPALASRHAILFEVASTYAEF
>pbcv1_vSET 
ASSPKKKRKVEASMFNDRVIVKKSPLGGYGVFARKSFEKGELVEECLCIVRH
NDDWGTALEDYLFSRKNMSAMALGFGAIFNHSKDPNARHELTAGLKRMRIFTIKPIAIG
EEITISYGDDYWLSRPRLTQNEF
>at_KYP 
ASSPKKKRKVEASDISGGLEFKGIPATNRVDDSPVSPTSGFTYIKSLIIEPNVIIP
KSSTGCNCRGSCTDSKKCACAKLNGGNFPYVDLNDGRLIESRDVVFECGPHCGCGPKC
VNRTSQKRLRFNLEVFRSAKKGWAVRSWEYIPAGSPVCEYIGVVRRTADVDTISDNEYI
FEIDCQQTMQGLGGRQRRLRDVAVPMNNGVSQSSEDENAPEFCIDAGSTGNFARFINHS
CEPNLFVQCVLSSHQDIRLARVVLFAADNISPMQELTYDYGYALDSVHEF
>tg_TgSET8 
ASSPKKKRKVEASASRRTGEFLRDAQAPSRWLKRSKTGQDDGAFCLETWLA
GAGDDAAGGERGRDREGAADKAKQREERRQKELEERFEEMKVEFEEKAQRMIARRAA
LTGEIYSDGKGSKKPRVPSLPENDDDALIEIIIDPEQGILKWPLSVMSIRQRTVIYQECLRR
DLTACIHLTKVPGKGRAVFAADTILKDDFVVEYKGELCSEREAREREQRYNRSKVPMGS
FMFYFKNGSRMMAIDATDEKQDFGPARLINHSRRNPNMTPRAITLGDFNSEPRLIFVARR
NIEKGEELLVDYGERDPDVIKEHPWLNSEF
>hs_SIRT6 
ASSPKKKRKVEASMSVNYAAGLSPYADKGKCGLPEIFDPPEELERKVWELAR
LVWQSSSVVFHTGAGISTASGIPDFRGPHGVWTMEERGLAPKFDTTFESARPTQTHMAL
VQLERVGLLRFLVSQNVDGLHVRSGFPRDKLAELHGNMFVEECAKCKTQYVRDTVVG
 
TMGLKATGRLCTVAKARGLRACRGELRDTILDWEDSLPDRDLALADEASRNADLSITL
GTSLQIRPSGNLPLATKRRGGRLVIVNLQPTKHDRHADLRIHGYVDEVMTRLMKHLGLE
IPAWDGPRVLERALPPLEF
>ce_Set1 
ASSPKKKRKVEASMKVAAKKLATSRMRKDRAAAASPSSDIENSENPSSLASH
SSSSGRMTPSKNTRSRKGVSVKDVSNHKITEFFQVRRSNRKTSKQISDEAKHALRDTVL
KGTNERLLEVYKDVVKGRGIRTKVNFEKGDFVVEYRGVMMEYSEAKVIEEQYSNDEEI
GSYMYFFEHNNKKWCIDATKESPWKGRLINHSVLRPNLKTKVVEIDGSHHLILVARRQI
AQGEELLYDYGDRSAETIAKNPWLVNTEF
>mm_G9a 
ASSPKKKRKVEASVRTEKIICRDVARGYENVPIPCVNGVDGEPCPEDYKYISE
NCETSTMNIDRNITHLQHCTCVDDCSSSNCLCGQLSIRCWYDKDGRLLQEFNKIEPPLIFE
CNQACSCWRSCKNRVVQSGIKVRLQLYRTAKMGWGVRALQTIPQGTFICEYVGELISD
AEADVREDDSYLFDLDNKDGEVYCIDARYYGNISRFINHLCDPNIIPVRVFMLHQDLRFP
RIAFFSSRDIRTGEELGFDYGDRFWDIKSKYFTCQCGSEKCKHSAEAIALEQSRLARLDP
HPELLPDLSSLPPINTEF
>hs_SIRT5 
ASSPKKKRKVEASSSSMADFRKFFAKAKHIVIISGAGVSAESGVPTFRGAGGY
WRKWQAQDLATPLAFAHNPSRVWEFYHYRREVMGSKEPNAGHRAIAECETRLGKQGR
RVVVITQNIDELHRKAGTKNLLEIHGSLFKTRCTSCGVVAENYKSPICPALSGKGAPEPG
TQDASIPVEKLPRCEEAGCGGLLRPHVVWFGENLDPAILEEVDRELAHCDLCLVVGTSS
VVYPAAMFAPQVAARGVPVAEFNTETTPATNRFRFHFQGPCGTTLPEALACHENETVSE
F
>xl_HDAC8 
ASSPKKKRKVEASMSRVVKPKVASMEEMAAFHTDAYLQHLHKVSEEGDND
DPETLEYGLGYDCPITEGIYDYAAAVGGATLTAAEQLIEGKTRIAVNWPGGWHHAKKD
EASGFCYLNDAVLGILKLREKFDRVLYVDMDLHHGDGVEDAFSFTSKVMTVSLHKFSP
GFFPGTGDVSDIGLGKGRYYSINVPLQDGIQDDKYYQICEGVLKEVFTTFNPEAVVLQLG
ADTIAGDPMCSFNMTPEGIGKCLKYVLQWQLPTLILGGGGYHLPNTARCWTYLTALIVG
RTLSSEIPDHEFFTEYGPDYVLEITPSCRPDRNDTQKVQEILQSIKGNLKRVVEF
>mm_HPla 
ASSPKKKRKVEASMKEGENNKPREKSEGNKRKSSFSNSADDIKSKKKREQSN
DIARGFERGLEPEKIIGATDSCGDLMFLMKWKDTDEADLVLAKEANVKCPQIVIAFYEE
RLTWHAYPEDAENKEKESAKSEF
>at_HDT1 
ASSPKKKRKVEASMEFWGIEVKSGKPVTVTPEEGILIHVSQASLGECKNKKG
EFVPLHVKVGNQNLVLGTLSTENIPQLFCDLVFDKEFELSHTWGKGSVYFVGYKTPNIEP
QGYSEEEEEEEEEVPAGNAAKAVAKPKAKPAEVKPAVDDEEDESDSDGMDEDDSDGE
DSEEEEPTPKKPASSKKRANETTPKAPVSAKKAKVAVTPQKTDEKKKGGKAANQSEF
>mm_SAll 
ASSPKKKRKVEASMSRRKQAKPQHFQSDPEVASLPRRDGDTEKGQPSRPTKS
KDAHVCGRCCAEFFELSDLLLHKKSCTKNQLVLIVNESPASPAKTFPPGPSLNDEF
>hs_SETD8 
ASSPKKKRKVEASSCDSTNAAIAKQALKKPIKGKQAPRKKAQGKTQQNRKL
TDFYPVRRSSRKSKAELQSEERKRIDELIESGKEEGMKIDLIDGKGRGVIATKQFSRGDFV
VEYHGDLIEITDAKKREALYAQDPSTGCYMYYFQYLSKTYCVDATRETNRLGRLINHSK
CGNCQTKLHDIDGVPHLILIASRDIAAGEELLYDYGDRSKASIEAFPWLKHEF
>sc_RPD3 
ASSPKKKRKVEASRRVAYFYDADVGNYAYGAGHPMKPHRIRMAHSLIMNY
GLYKKMEIYRAKPATKQEMCQFHTDEYIDFLSRVTPDNLEMFKRESVKFNVGDDCPVF
DGLYEYCSISGGGSMEGAARLNRGKCDVAVNYAGGLHHAKKSEASGFCYLNDIVLGIIE
LLRYHPRVLYIDIDVHHGDGVEEAFYTTDRVMTCSFHKYGEFFPGTGELRDIGVGAGKN
YAVNVPLRDGIDDATYRSVFEPVIKKIMEWYQPSAVVLQCGGDSLSGDRLGCFNLSME
GHANCVNYVKSFGIPMMVVGGGGYTMRNVARTWCFETGLLNNVVLDKDLPYEF
>ec_CobB 
ASSPKKKRKVEASMEKPRVLVLTGAGISAESGIRTFRAADGLWEEHRVEDVA
TPEGFDRDPELVQAFYNARRRQLQQPEIQPNAAHLALAKLQDALGDRFLLVTQNIDNLH
ERAGNTNVIHMHGELLKVRCSQSGQVLDWTGDVTPEDKCHCCQFPAPLRPHVVWFGE
MPLGMDEIYMALSMADIFIAIGTSGHVYPAAGFVHEAKLHGAHTVELNLEPSQVGNEFA
EKYYGPASQVVPEFVEKLLKGLKAGSIAEF
>hs_SUV39H1 
ASSPKKKRKVEASNLKCVRILKQFHKDLERELLRRHHRSKTPRHLDPSLANY
LVQKAKQRRALRRWEQELNAKRSHLGRITVENEVDLDGPPRAFVYINEYRVGEGITLNQ
VAVGCECQDCLWAPTGGCCPGASLHKFAYNDQGQVRLRAGLPIYECNSRCRCGYDCP
NRVVQKGIRYDLCIFRTDDGRGWGVRTLEKIRKNSFVMEYVGEIITSEEAERRGQIYDRQ
GATYLFDLDYVEDVYTVDAAYYGNISHFVNHSCDPNLQVYNVFIDNLDERLPRIAFFAT
RTIRAGEELTFDYNMQVDPVDMESTRMDSNFGLAGLPGSPKKRVRIECKCGTESCRKYL
FEF
>hs_RCOR1 
ASSPKKKRKVEASSNSWEEGSSGSSSDEEHGGGGMRVGPQYQAVVPDFDPA
KLARRSQERDNLGMLVWSPNQNLSEAKLDEYIAIAKEKHGYNMEQALGMLFWHKHNI
EKSLADLPNFTPFPDEWTVEDKVLFEQAFSFHGKTFHRIQQMLPDKSIASLVKFYYSWK
KTRTKTSVMDRHARKQKREREESEDELEEANGNNPIDIEVDQNKESKKEVPPTETVPQV
KKEKHSTEF
>hs_sin3a 
ASSPKKKRKVEASYKESVHLETYPKERATEGIAMEIDYASCKRLGSSYRALP
KSYQQPKCTGRTPLCKEVLNDTWVSFPSWSEDSTFVSSKKTQYEEHIYRCEDERFELDV
VLETNLATIRVLEAIQKKLSRLSAEEQAKFRLDNTLGGTSEVIHRKALQRIYADKAADIID
GLRKNPSIAVPIVLKRLKMKEEEWREAQRGFNKVWREQNEKYYLKSLDHQGINFKQND
TKVLRSKSLLNEIESIYDERQEQATEENAGVPVGPHLSLAYEDKQILEDAAALIIHHVKR
QTGIQKEDKYKIKQIMHHFIPDLLFAQRGDLSDVEEEEEEEMDVDEATGAVEF
>at_SUVR4 
ASSPKKKRKVEASQSAYLHVSLARISDEDCCANCKGNCLSADFPCTCARETS
GEYAYTKEGLLKEKFLDTCLKMKKEPDSFPKVYCKDCPLERDHDKGTYGKCDGHLIRK
FIKECWRKCGCDMQCGNRVVQRGIRCQLQVYFTQEGKGWGLRTLQDLPKGTFICEYIG
EILTNTELYDRNVRSSSERHTYPVTLDADWGSEKDLKDEEALCLDATICGNVARFINHR
CEDANMIDIPIEIETPDRHYYHIAFFTLRDVKAMDELTWDYMIDFNDKSHPVKAFRCCC
GSESCRDRKIKGSQGKSIERRKIVSAKKQQGSKEVSKKRKEF
>rn_MeCP2_NLS 
ASSPKKKRKVEASVQVKRVLEKSPGKLLVKMPFQASPGGKGEGGGATTSAQ
VMVIKRPGRKRKAEADPQAIPKKRGRKPGSVVAAAAAEAKKKAVKESSIRSVQETVLPI
KKRKTRETVSIEVKEVVKPLLVSTLGEKSGKGLKTCKSPGRKSKESSPKGRSSSASSPPK
 
KEHHHHHHHAESPKAPMPLLPPPPPPEPQSSEDPISPPEPQDLSSSICKEEKMPRAGSLESD
GCPKEPAKTQPMVAAAATTTTTTTTTVAEKYKHRGEGERKDIVSSSMPRPNREEPVDSR
TPVTERVSEF
>mm_SET-TAF1B 
ASSPKKKRKVEASMAPKRQSAILPQPKKPRPAAAPKLEDKSASPGLPKGEKE
QQEAIEHIDEVQNEIDRLNEQASEEILKVEQKYNKLRQPFFQKRSELIAKIPNFWVTTFVN
HPQVSALLGEEDEEALHYLTRVEVTEFEDIKSGYRIDFYFDENPYFENKVLSKEFHLNES
GDPSSKSTEIKWKSGKDLTKRSSQTQNKASRKRQHEEPESFFTWFTDHSDAGADELGEV
IKDDIWPNPLQYYLVPDMDDEEGEAEDDDDDDEEEEGLEDIDEEGDEDEGEEDDDEDE
GEEGEEDEGEDDEF
>ce_Set4

ASSPKKKRKVEASMQLHEQIANISVTFNDIPRSDHSMTPTELCYFDDFATTLV
VDSVLNFTTHKMSKKRRYLYQDEYRTARTVMKTFREQRDWTNAIYGLLTLRSVSHFLS
KLPPNKLFEFRDHIVRFLNMFILDSGYTIQECKRYSQEGHQGAKLVSTGVWSRGDKIERL
SGVVCLLSSEDEDSILAQEGSDFSVMYSTRKRCSTLWLGPGAYINHDCRPTCEFVSHGST
AHIRVLRDMVPGDEITCFYGSEFFGPNNIDCECCTCEKNMNGAFSYLRGNENAEPIISEK
KTKYELRSRSEF

### Photostimulation Hardware Control Scripts

The following Arduino script was used to enable the individual control of each 4-well column of a light-stimulated 24-well plate

```
 //Basic control code for LITE LED array using Arduino UNO
 //LED column address initialization to PWM-ready Arduino outputs
int led1_pin = 3;
 int led2_pin = 5;
 int led3_pin = 6;
 int led4_pin = 9:
 int led5_pin = 10;
 int led6_pin = 11;
 //Maximum setting for Arduino PWM
 int uniform_brightness = 255:
 //PWM settings for individual LED columns
 int Led1_brightness = uniform_brightness/2:
 int led2_brightness = uniform_brightness/2:
 int led3_brightness = uniform_brightness/2:
 int led4_brightness = uniform_brightness/2:
 int led5_brightness = uniform_brightness/2:
 int led6_brightness = uniform_brightness/2:
 //'on' time in msec
 unsigned long uniform_stim_time = 1000: /
 //individual 'on' time settings for LED columns
 unsigned long led1_stim_time = uniform_stim_time:
 unsigned long led2_stim_time = uniform_stim_time:
 unsigned long led3_stim_time = uniform_stim_time:
 unsigned long led4_stim_time = uniform_stim_time:
 unsigned long led5_stim_time = uniform_stim_time:
 unsigned long led6_stim_time = uniform_stim_time:
 //'off' time in msec
 unsigned long uniform_off_time = 14000:
 //individual 'off' time settings for LED columns
 unsigned long led1_off_time = uniform_off_time:
 unsigned long led2_off_time = uniform_off_time:
 unsigned long led3_off_time = uniform_off_time:
 unsigned long led4_off_time = uniform_off_time:
 unsigned long led5_off_time = uniform_off_time:
 unsigned long led6_off_time = uniform_off_time:
 unsigned long currentMillis = 0:
 //initialize timing and state variables
 unsigned long led1_last_change = 0:
 unsigned long led2_last_change = 0:
 unsigned long led3_last_change = 0;
 unsigned long led4_last_change = 0:
 unsigned long led5_last_change = 0;
 unsigned long led6_last_change = 0:
 int led1_state = HIGH:
 int led2_state = HIGH;
 int led3_state = HIGH:
 int led4_state = HIGH;
 int led5_state = HIGH:
 int led6_state = HIGH:
 unsigned long led1_timer = 0;
 unsigned long led2_timer = 0:
 unsigned long led3_timer = 0:
 unsigned long led4_timer = 0:
 unsigned long led5-timer = 0:
 unsigned long led6_timer = 0:
 void setup() [
 // setup PWM pins for output
 pinMode(led1_pin, OUTPUT):
 pinMode(led2_pin, OUTPUT):
 pinMode(led3_pin, OUTPUT):
 pinMode(led4_pin, OUTPUT):
 pinMode(led5_pin, OUTPUT):
 pinMode(led6_pin, OUTPUT):
 //LED starting state
 analogWrite(led1_pin, led1_brightness);
 analogWrite(led2_pin, ed2_brightness);
 analogWrite(led3_pin, led3_brightness);
 analogWrite(led4_pin, led4_brightness);
 analogWrite(led5_pin, led5_brightness);
 analogWrite(led6_pin, led6_brightness);
 }
   void loop () [
     currentMillis = millis() ;
    //identical timing loops for the 6 PWM output pins
    led1_timer = currentMillis - led1_last_change ;
    if (led1_state == HIGH) (//led state is on
      if (Ied1_timer >= led1_stim_time) ( //TRUE if stim time is complete
       analogWrite(led1_pin, 0) : //turn LED off
       Ied1_state = LOW:         //change LED state variable
       led1_last_change = currentmillis: //mark time of most recent change
      }
 }
   else[ //led1 state is off
     if (led1_timer >= led1_off_time){ //TRUE if off time is complete
        analogWrite(led1_pin, led1_brightness) : //turn LED on
        led1_state = HIGH;                      //change LED state variable
 led1_last_change = currentMillis;             //mark time of most recent change
    }
 }
   led2_timer = currentMillis - led2_last_change ;
   if (led2_state == HIGH) {
     if (led2_timer >= led2_stim_time) {
       analogWrite(led2_pin, 0) ;
       led2_state = LOW;
       led2_last_change = currentmillis:
    }
 }
   else[ //led2 state is off
    if (led2_timer >= led2_off_time) {
       anamogWrite(led2_pin, led2_brightness) ;
       led2_state = HIGH:
       led2_last_change = currentmillis:
    }
 }
   led3_timer = currentMillis - led3_last_change;
    if (led3_state == HIGH) {
      if (led3_timer >= led3_stim_time) {
        analogWrite(led3_pin, 0) ;
        led3_state = LOW:
        led3_last_change = currentmillis:
    }
 }
   else[ //led3 state is off
    if (led3_timer >= led3_off_time) {
       analogWrite(led3_pin, led3_brightness);
       led3_state = HIGH:
       led3_last_change = ourrentMillis;
    }
 }
   led4_timer = currentMillis - led4_last_change:
   if (led4_state = HIGH) {
      if (led4_timer >= led4_stim_time){
         analogWrite(led4_pin, 0) ;
         led4_state = LOW:
         led4_last_change = currentMillis:
    }
 }
   else[ //led4 state is off
    if (led4_timer >= led4_off_time) {
       anamogWrite(led4_pin, led4_brightness);
       led4_state = HIGH:
       led4_last_change = currentMillis;
    }
 }
   led5_timer = currentMillis - led5_last_change;
   if (led5_state == HIGH) {
     if (led5_timer >= led5_stim_titne) {
       analogWrite(led5_pin, 0) ;
       led5_state = LOW:
       led5_last_change = currentMillis:
     }
 }
   else{ //led5 state is off
    if (led5_timer >= led5_off_time) [
       analogWrite(led5_pin, led5_brightness);
       led5_state = HIGH:
       led5_last_change = currentMillis;
     }
 }
   led6_timer = currentMillis - led6_last_charge:
    if (led6_state == HIGH) {
       if (led6_timer >= led6_stim_time) {
       analogWrite(led6_pin, 0) ;
       led6_state = LOW:
       led6_last_change = currentMillis;
     }
 }
   else{ //led6 state is off
    if (led6_timer >= led6_off_time){
       analogWrite(led6_pin, led6_brightness);
       led6_state = HIGH;
       led6_last_change = currentMillis:
     }
   }
 }
```

### Example 12

### Optical Control of Endogenous Mammalian Transcription

To test the efficacy of AAV-mediated TALE delivery for modulating transcription in primary mouse cortical neurons, Applicants constructed six TALE-DNA binding domains targeting the genetic loci of three mouse neurotransmitter receptors: *Grm5, Grin2a,* and *Grm2,* which encode mGluR5, NMDA subunit 2A and mGluR2, respectively (**FIG. 58**). To increase the likelihood of a target site accessibility, Applicants used mouse cortex DNase I sensitivity data from the UCSC genome browser to identify putative open chromatin regions. DNase I sensitive regions in the promoter of each target gene provided a guide for the selection of TALE binding sequences (**FIG. 46**). For each TALE, Applicants employed VP64 as a transcriptional activator or a quadruple tandem repeat of the mSin3 interaction domain (SID) (Beerli, R.R., Segal, D.J., Dreier, B. & Barbas, C.F., 3rd Toward controlling gene expression at will: specific regulation of the erbB-2/HER-2 promoter by using polydactyl zinc finger proteins constructed from modular building blocks. Proc Natl Acad Sci U S A 95, 14628-14633 (1998) and Ayer, D.E., Laherty, C.D., Lawrence, Q.A., Armstrong, A.P. & Eisenman, R.N. Mad proteins contain a dominant transcription repression domain. Molecular and Cellular Biology 16, 5772-5781 (1996)) as a repressor. Applicants have previously shown that a single SID fused to TALE downregulated a target gene effectively in 293FT cells (Cong, L., Zhou, R., Kuo, Y.-c., Cunniff, M. & Zhang, F. Comprehensive interrogation of natural TALE DNA-binding modules and transcriptional repressor domains. Nat Commun 3, 968 (2012)). Hoping to further improve this TALE repressor, Applicants reasoned that four repeats of SID - analogous to the successful quadruple VP16 repeat architecture of VP64 (Beerli, R.R., Segal, D.J., Dreier, B. & Barbas, C.F., 3rd Toward controlling gene expression at will: specific regulation of the erbB-2/HER-2 promoter by using polydactyl zinc finger proteins constructed from modular building blocks. Proc Natl Acad Sci U S A 95, 14628-14633 (1998) - might augment its repressive activity. This was indeed the case, as TALE-SID4X constructs enhanced repression ∼2-fold over TALE-SID in 293FT cells (**FIG. 54**).

Applicants found that four out of six TALE-VP64 constructs (T1, T2, T5 and T6) efficiently activated their target genes *Grm5* and *Grm2* in AAV-transduced primary neurons by up to 3- and 8-fold, respectively (**FIG. 58**). Similarly, four out of six TALE-SID4X repressors (T9, T10, T11, T12) reduced the expression of their endogenous targets *Grin2a* and *Grm2* by up to 2- and 8-fold, respectively (**FIG. 58**). Together, these results indicate that constitutive TALEs can positively or negatively modulate endogenous target gene expression in neurons. Notably, efficient activation or repression by a given TALE did not predict its efficiency at transcriptional modulation in the opposite direction. Therefore, multiple TALEs may need to be screened to identify the most effective TALE for a particular locus.

For a neuronal application of LITEs, Applicants selected the *Grm2* TALE (T6), which exhibited the strongest level of target upregulation in primary neurons, based on Applicants' comparison of 6 constitutive TALE activators (**FIG. 58**). Applicants investigated its function using 2 light pulsing frequencies with the same duty cycle of 0.8%. Both stimulation conditions achieved a ∼7-fold light-dependent increase in *Grm2* mRNA levels (**FIG. 38C**). Further study confirmed that, significant target gene expression increases could be attained quickly (4-fold upregulation within 4 h; **FIG. 38D**). In addition, Applicants observed significant upregulation of mGluR2 protein after stimulation, demonstrating that changes effected by LITEs at the mRNA level are translated to the protein domain (**FIG. 38E**). Taken together, these results confirm that LITEs enable temporally precise optical control of endogenous gene expression in neurons.

As a compliment to Applicants' previously implemented LITE activators, Applicants next engineered a LITE repressor based on the TALE-SID4X constructs. Constitutive *Grm2* TALEs (T11 and T12, **FIG. 59A**) mediated the highest level of transcription repression, and were chosen as LITE repressors (**FIG. 59A****, B**). Both light-induced repressors mediated significant downregulation of *Grm2* expression, with 1.95-fold and 1.75-fold reductions for T11 and T12, respectively, demonstrating the feasibility of optically controlled repression in neurons (**FIG**. **38G**)**.**

In order to deliver LITEs into neurons using AAV, Applicants had to ensure that the total viral genome size, with the LITE transgenes included, did not exceed 4.8 kb (Wu, Z., Yang, H. & Colosi, P. Effect of Genome Size on AAV Vector Packaging. Mol Ther 18, 80-86 (2009) and Dong JY, F.P., Frizzell RA Quantitative analysis of the packaging capacity of recombinant adeno-associated virus. Human Gene Therapy 7, 2101-2112 (1996)). To that end, Applicants shortened the TALE N- and C-termini (keeping 136 aa in the N-terminus and 63 aa in the C-terminus) and exchanged the CRY2PHR and CIB1 domains (TALE-CIB1 and CRY2PHR-VP64; **FIG. 38A**). This switch allowed each component of LITE to fit into AAV vectors and did not reduce the efficacy of light-mediated transcription modulation (**FIG. 60**). These LITEs can be efficiently delivered into primary cortical neurons via co-transduction by a combination of two AAV vectors (**FIG. 38B**; delivery efficiencies of 83-92% for individual components with >80% co-transduction efficiency).

### Reference example 13 (disclosure)

### Inducible lentiviral Cas9

Lentivirus preparation. After cloning pCasES10 (which contains a lentiviral transfer plasmid backbone), HEK293FT. at low passage (p=5) were seeded in a T-75 flask to 50% confluence the day before transfection in DMEM with 10% fetal bovine serum and without antibiotics. After 20 hours, media was changed to OptiMEM (serum-free) media and transfection was done 4 hours later. Cells were transfected with 10ug of lentiviral transfer plasmid (pCasES10) and the following packaging plasmids: 5ug of pMD2.G (VSV-g pseudotype), and 7.5ug of psPAX2 (gag/pol/rev/tat). Transfection was done in 4mL OptiMEM with a cationic lipid delivery agent (50uL Lipofectamine 2000 and 100ul Plus reagent). After 6 hours, the media was changed to antibiotic-free DMEM with 10% fetal bovine serum.

Lentivirus purification. Viral supernatants were harvested after 48 hours. Supernatants were first cleared of debris and filtered through a 0.45um low protein binding (PVDF) filter. They were then spun in a ultracentrifuge for 2 hours at 24,000 rpm. Viral pellets were resuspended in 50ul of DMEM overnight at 4C. They were then aliquotted and immediately frozen at -80C.

Clonal isolation using FACS. For clonal isolation of HEK293FT and HUES64 human embryonic stem cells, cells were infected in suspension with either 1ul or 5ul of purified virus. Twenty-four hours post infection, 1uM doxycycline was added to the cell culture media. After 24 or 48 hours more, cells underwent fluorescence-assisted cell sorting (FACS) on a BD FACSAria IIu instrument to isolate single cells that robustly expressed EGFP (and hence Cas9) after doxycycline treatment. Cell were plated either in bulk or into individual wells to allow selection of clonal populations with an integrated inducible Cas9 for further use. Sort efficiency was always >95% and cells were visualized immediately after plating to verify EGFP fluorescence.

FIG. 61 depicts Tet Cas9 vector designs

FIG. 62 depicts a vector and EGFP expression in 293FT cells.

Sequence of pCasES020 inducible Cas9:

caactttgtatagaaaagttggctccgaattcgcccttcaggtccgaggttctagacgagtttactccctatcagtgatagaga
acgatgtcgagtttactccctatcagtgatagagaacgtatgtcgagtttactccctatcagtgatagagaacgtatgtcgagtttactccctatc
agtgatagagaacgtatgtcgagtttatccctatcagtgatagagaacgtatgtcgagtttactccctatcagtgatagagaacgtatgtcgag
gtaggcgtgtacggtgggaggcctatataagcagagctcgtttagtgaaccgtcagatcgcaaagggcgaattcgacccaagtttgtacag
ccaccATGGACTATAAGGACCACGACGGAGACTACAAGGATCATGATATTGATTACAA
AGACGATGACGATAAGATGGCCCCAAAGAAGAAGCGGAAGGTCGGTATCCACGGA
GTCCCAGCAGCCGACAAGAAGTACAGCATCGGCCTGGACATCGGCACCAACTCTGT
GGGCTGGGCCGTGATCACCGACGAGTACAAGGTGCCCAGCAAGAAATTCAAGGTGC
TGGGCAACACCGACCGGCACAGCATCAAGAAGAACCTGATCGGAGCCCTGCTGTTC
GACAGCGGCGAAACAGCCGAGGCCACCCGGCTGAAGAGAACCGCCAGAAGAAGAT
ACACCAGACGGAAGAACCGGATCTGCTATCTGCAAGAGATCTTCAGCAACGAGATG
GCCAAGGTGGACGACAGCTTCTTCCACAGACTGGAAGAGTCCTTCCTGGTGGAAGA

GGATAAGAAGCACGAGCGGCACCCCATCTTCGGCAACATCGTGGACGAGGTGGCCT
ACCACGAGAAGTACCCCACCATCTACCACCTGAGAAAGAAACTGGTGGACAGCACC
GACAAGGCCGACCTGCGGCTGATCTATCTGGCCCTGGCCCACATGATCAAGTTCCGG
GGCCACTTCCTGATCGAGGGCGACCTGAACCCCGACAACAGCGACGTGGACAAGCT
GTTCATCCAGCTGGTGCAGACCTACAACCAGCTGTTCGAGGAAAACCCCATCAACG
CCAGCGGCGTGGACGCCAAGGCCATCCTGTCTGCCAGACTGAGCAAGAGCAGACGG
CTGGAAAATCTGATCGCCCAGCTGCCCGGCGAGAAGAAGAATGGCCTGTTCGGCAA
CCTGATTGCCCTGAGCCTGGGCCTGACCCCCAACTTCAAGAGCAACTTCGACCTGGC
CGAGGATGCCAAACTGCAGCTGAGCAAGGACACCTACGACGACGACCTGGACAACC
TGCTGGCCCAGATCGGCGACCAGTACGCCGACCTGTTTCTGGCCGCCAAGAACCTGT
CCGACGCCATCCTGCTGAGCGACATCCTGAGAGTGAACACCGAGATCACCAAGGCC
CCCCTGAGCGCCTCTATGATCAAGAGATACGACGAGCACCACCAGGACCTGACCCT
GCTGAAAGCTCTCGTGCGGCAGCAGCTGCCTGAGAAGTACAAAGAGATTTTCTTCGA
CCAGAGCAAGAACGGCTACGCCGGCTACATTGACGGCGGAGCCAGCCAGGAAGAG
TTCTACAAGTTCATCAAGCCCATCCTGGAAAAGATGGACGGCACCGAGGAACTGCT
CGTGAAGCTGAACAGAGAGGACCTGCTGCGGAAGCAGCGGACCTTCGACAACGGCA
GCATCCCCCACCAGATCCACCTGGGAGAGCTGCACGCCATTCTGCGGCGGCAGGAA
GATTTTTACCCATTCCTGAAGGACAACCGGGAAAAGATCGAGAAGATCCTGACCTTC
CGCATCCCCTACTACGTGGGCCCTCTGGCCAGGGGAAACAGCAGATTCGCCTGGAT
GACCAGAAAGAGCGAGGAAACCATCACCCCCTGGAACTTCGAGGAAGTGGTGGAC
AAGGGCGCTTCCGCCCAGAGCTTCATCGAGCGGATGACCAACTTCGATAAGAACCT
GCCCAACGAGAAGGTGCTGCCCAAGCACAGCCTGCTGTACGAGTACTTCACCGTGT
ATAACGAGCTGACCAAAGTGAAATACGTGACCGAGGGAATGAGAAAGCCCGCCTTC
CTGAGCGGCGAGCAGAAAAAGGCCATCGTGGACCTGCTGTTCAAGACCAACCGGAA
AGTGACCGTGAAGCAGCTGAAAGAGGACTACTTCAAGAAAATCGAGTGCTTCGACT
CCGTGGAAATCTCCGGCGTGGAAGATCGGTTCAACGCCTCCCTGGGCACATACCAC
GATCTGCTGAAAATTATCAAGGACAAGGACTTCCTGGACAATGAGGAAAACGAGGA
CATTCTGGAAGATATCGTGCTGACCCTGACACTGTTTGAGGACAGAGAGATGATCGA
GGAACGGCTGAAAACCTATGCCCACCTGTTCGACGACAAAGTGATGAAGCAGCTGA
AGCGGCGGAGATACACCGGCTGGGGCAGGCTGAGCCGGAAGCTGATCAACGGCATC
CGGGACAAGCAGTCCGGCAAGACAATCCTGGATTTCCTGAAGTCCGACGGCTTCGC

CAACAGAAACTTCATGCAGCTGATCCACGACGACAGCCTGACCTTTAAAGAGGACA
TCCAGAAAGCCCAGGTGTCCGGCCAGGGCGATAGCCTGCACGAGCACATTGCCAAT
CTGGCCGGCAGCCCCGCCATTAAGAAGGGCATCCTGCAGACAGTGAAGGTGGTGGA
CGAGCTCGTGAAAGTGATGGGCCGGCACAAGCCCGAGAACATCGTGATCGAAATGG
CCAGAGAGAACCAGACCACCCAGAAGGGACAGAAGAACAGCCGCGAGAGAATGAA
GCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATG
TGGACCATATCGTGCCTCAGAGCTTTCTGAAGGACGACTCCATCGACAACAAGGTGC
TGACCAGAAGCGACAAGAACCGGGGCAAGAGCGACAACGTGCCCTCCGAAGAGGT
CGTGAAGAAGATGAAGAACTACTGGCGGCAGCTGCTGAACGCCAAGCTGATTACCC
AGAGAAAGTTCGACAATCTGACCAAGGCCGAGAGAGGCGGCCTGAGCGAACTGGAT
AAGGCCGGCTTCATCAAGAGACAGCTGGTGGAAACCCGGCAGATCACAAAGCACGT
GGCACAGATCCTGGACTCCCGGATGAACACTAAGTACGACGAGAATGACAAGCTGA
TCCGGGAAGTGAAAGTGATCACCCTGAAGTCCAAGCTGGTGTCCGATTTCCGGAAG
GATTTCCAGTTTTACAAAGTGCGCGAGATCAACAACTACCACCACGCCCACGACGCC
TACCTGAACGCCGTCGTGGGAACCGCCCTGATCAAAAAGTACCCTAAGCTGGAAAG
CGAGTTCGTGTACGGCGACTACAAGGTGTACGACGTGCGGAAGATGATCGCCAAGA
GCGAGCAGGAAATCGGCAAGGCTACCGCCAAGTACTTCTTCTACAGCAACATCATG
AACTTTTTCAAGACCGAGATTACCCTGGCCAACGGCGAGATCCGGAAGCGGCCTCT
GATCGAGACAAACGGCGAAACCGGGGAGATCGTGTGGGATAAGGGCCGGGATTTTG
CCACCGTGCGGAAAGTGCTGAGCATGCCCCAAGTGAATATCGTGAAAAAGACCGAG
GTGCAGACAGGCGGCTTCAGCAAAGAGTCTATCCTGCCCAAGAGGAACAGCGATAA
GCTGATCGCCAGAAAGAAGGACTGGGACCCTAAGAAGTACGGCGGCTTCGACAGCC
CCACCGTGGCCTATTCTGTGCTGGTGGTGGCCAAAGTGGAAAAGGGCAAGTCCAAG
AAACTGAAGAGTGTGAAAGAGCTGCTGGGGATCACCATCATGGAAAGAAGCAGCTT
CGAGAAGAATCCCATCGACTTTCTGGAAGCCAAGGGCTACAAAGAAGTGAAAAAGG
ACCTGATCATCAAGCTGCCTAAGTACTCCCTGTTCGAGCTGGAAAACGGCCGGAAG
AGAATGCTGGCCTCTGCCGGCGAACTGCAGAAGGGAAACGAACTGGCCCTGCCCTC
CAAATATGTGAACTTCCTGTACCTGGCCAGCCACTATGAGAAGCTGAAGGGCTCCCC
CGAGGATAATGAGCAGAAACAGCTGTTTGTGGAACAGCACAAGCACTACCTGGACG

AGATCATCGAGCAGATCAGCGAGTTCTCCAAGAGAGTGATCCTGGCCGACGCTAAT
CTGGACAAAGTGCTGTCCGCCTACAACAAGCACCGGGATAAGCCCATCAGAGAGCA
GGCCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGTGC
TGGACGCCACCCTGATCCACCAGAGCATCACCGGCCTGTACGAGACACGGATCGAC
CTGTCTCAGCTGGGAGGCGACAAAAGGCCGGCGGCCACGAAAAAGGCCGGCCAGG
CAAAAAAGAAAAAGgaattctctagaGGCAGTGGAGAGGGCAGAGGAAGTCTGCTAACAT
GCGGTGACGTCGAGGAGAATCCTGGCCCAGTGAGCAAGGGCGAGGAGCTGTTCACC
GGGGTGGTGCCCATCCTGGTCGAGCTGGACGGCGACGTAAACGGCCACAAGTTCAG
CGTGTCCGGCGAGGGCGAGGGCGATGCCACCTACGGCAAGCTGACCCTGAAGTTCA
TCTGCACCACCGGCAAGCTGCCCGTGCCCTGGCCCACCCTCGTGACCACCCTGACCT
ACGGCGTGCAGTGCTTCAGCCGCTACCCCGACCACATGAAGCAGCACGACTTCTTCA
AGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGC
TGGAGTACAACTACAACAGCCACAACGTCTATATCATGGCCGACAAGCAGAAGAAC
GGCATCAAGGTGAACTTCAAGATCCGCCACAACATCGAGGACGGCAGCGTGCAGCT
CGCCGACCACTACCAGCAGAACACCCCCATCGGCGACGGCCCCGTGCTGCTGCCCG
ACAACCACTACCTGAGCACCCAGTCCGCCCTGAGCAAAGACCCCAACGAGAAGCGC
GATCACATGGTCCTGCTGGAGTTCGTGACCGCCGCCGGGATCACTCTCGGCATGGAC
GAGCTGTACAAGCTCGAGGGAAGCGGAGCTACTAACTTCAGCCTGCTGAAGCAGGC
TGGCGACGTGGAGGAGAACCCTGGACCTatgtctaggctggacaagagcaaagtcataaacggagctctggaa
ttactcaatggtgtcggtatcgaaggcctgacgacaaggaaactcgctcaaaagctgggagttgagcagcctaccctgtactggcacgtga
agaacaagcgggccctgctcgatgccctgccaatcgagatgctggacaggcatcatacccacttctgcccccctggaaggcgagtcatggc
aagactttctgcggaacaacgccaagtcataccgctgtgctctcctctcacatcgcgacggggctaaagtgcatctcggcacccgcccaac
agagaaacagtacgaaaccctggaaaatcagctcgcgttcctgtgtcagcaaggcttctccctggagaacgcactgtacgctctgtccgcc
gtgggccactttacactgggctgcgtattggaggaacaggagcatcaagtagcaaaagaggaaagagagacacctaccaccgattctatg
cccccacttctgagacaagcaattgagctgttcgaccggcagggagccgaacctgccttccttttcggcctggaactaatcatatgtggcctg
gagaaacagctaaagtgcgaaagcggcgggccgaccgacgcccttgacgattttgacttagacatgctcccagccgatgcccttgacgatt
ttgaccttgacatgctccccgggtaatgtacaaagtggtgaattccggcaattcgatatcaagcttatcgataatcaacctctggattacaaaatt
tgtgaaagattgactggtattcttaactatgttgctccttttacgctatgtggatacgctgctttaatgcctttgtatcatgctattgcttcccgtatgg

ctttcattttctcctccttgtataaatcctggttgctgtctctttatgaggagttgtggcccgttgtcaggcaacgtggcgtggtgtgcactgtgtttg
ctgacgcaacccccactggttggggcattgccaccacctgtcagctcctttccgggactttcgctttccccctccctattgccacggcggaac
tcatcgccgcctgccttgcccgctgctggacaggggctcggctgttgggcactgacaattccgtggtgttgtcggggaaatcatcgtcctttc
cttggctgctcgcctgtgttgccacctggattctgcgcgggacgtccttctgctacgtcccttcggccctcaatccagcggaccttccttcccg
cggcctgctgccggctctgcggcctcttccgcgtcttcgccttcgccctcagacgagtcggatctccctttgggccgcctccccgcatcgata
ccgtcgacctcgagacctagaaaaacatggagcaatcacaagtagcaatacagcagctaccaatgctgattgtgcctggctagaagcaca
agaggaggaggaggtgggttttccagtcacacctcaggtacctttaagaccaatgacttacaaggcagctgtagatcttagccactttttaaa
agaaaaggggggactggaagggctaattcactcccaacgaagacaagatatccttgatctgtggatctaccacacacaaggctacttccct
gattggcagaactacacaccagggccagggatcagatatccactgacctttggatggtgctacaagctagtaccagttgagcaagagaag
gtagaagaagccaatgaaggagagaacacccgcttgttacaccctgtgagcctgcatgggatggatgacccggagagagaagtattaga
gtggaggtttgacagccgcctagcatttcatcacatggcccgagagctgcatccggactgtactgggtctctctggttagaccagatctgagc
ctgggagctctctggctaactagggaacccactgcttaagcctcaataaagcttgccttgagtgcttcaagtagtgtgtgcccgtctgttgtgtg
actctggtaactagagatccctcagacccttttagtcagtgtggaaaatctctagcagggcccgtttaaacccgctgatcagcctcgactgtgc
cttctagttgccagccatctgttgtttgcccctcccccgtgccttccttgaccctggaaggtgccactcccactgtcctttcctaataaaatgagg
aaattgcatcgcattgtctgagtaggtgtcattctattctggggggtggggtggggcaggacagcaagggggaggattgggaagacaatag
caggcatgctggggatgcggtgggctctatggcttctgaggcggaaagaaccagctggggctctagggggtatccccacgcgccctgta
gcggcgcattaagcgcggcgggtgtggtggttacgcgcagcgtgaccgctacacttgccagcgccctagcgcccgctcctttcgctttcttc
ccttcctttctcgccacgttcgccggctttccccgtcaagctctaaatcgggggctccctttagggttccgatttagtgctttacggcacctcgac
cccaaaaaacttgattagggtgatggttcacgtagtgggccatcgccctgatagacggtttttcgccctttgacgttggagtccacgttctttaat
agtggactcttgttccaaactggaacaacactcaaccctatctcggtctattcttttgatttataagggattttgccgatttcggcctattggttaaa
aaatgagctgatttaacaaaaatttaacgcgaattaattctgtggaatgtgtgtcagttagggtgtggaaagtccccaggctccccagcaggc
agaagtatgcaaagcatgcatctcaattagtcagcaaccaggtgtggaaagtccccaggctccccagcaggcagaagtatgcaaagcatg
catctcaattagtcagcaaccatagtcccgcccctaactccgcccatcccgcccctaactccgcccagttccgcccattctccgccccatggc
tgactaattttttttatttatgcagaggccgaggccgcctctgcctctgagctattccagaagtagtgaggaggcttttttggaggcctaggctttt
gcaaaaagctcccgggagcttgtatatccattttcggatctgatcagcacgtgttgacaattaatcatcggcatagtatatcggcatagtataat
acgacaaggtgaggaactaaaccatggccaagttgaccagtgccgttccggtgctcaccgcgcgcgacgtcgccggagcggtcgagttc
tggaccgaccggctcgggttctcccgggacttcgtggaggacgacttcgccggtgtggtccgggacgacgtgaccctgttcatcagcgcg
gtccaggaccaggtggtgccggacaacaccctggcctgggtgtgggtgcgcggcctggacgagctgtacgccgagtggtcggaggtcg
tgtccacgaacttccgggacgcctccgggccggccatgaccgagatcggcgagcagccgtgggggcgggagttcgccctgcgcgacc
cggccggcaactgcgtgcacttcgtggccgaggagcaggactgacacgtgctacgagatttcgattccaccgccgccttctatgaaaggtt
gggcttcggaatcgttttccgggacgccggctggatgatcctccagcgcggggatctcatgctggagttcttcgcccaccccaacttgtttatt

gcagcttataatggttacaaataaagcaatagcatcacaaatttcacaaataaagcatttttttcactgcattctagttgtggtttgtccaaactcat
caatgtatcttatcatgtctgtataccgtcgacctctagctagagcttggcgtaatcatggtcatagctgtttcctgtgtgaaattgttatccgctca
caattccacacaacatacgagccggaagcataaagtgtaaagcctggggtgcctaatgagtgagctaactcacattaattgcgttgcgctca
ctgcccgctttccagtcgggaaacctgtcgtgccagctgcattaatgaatcggccaacgcgcggggagaggcggtttgcgtattgggcgct
cttccgcttcctcgctcactgactcgctgcgctcggtcgttcggctgcggcgagcggtatcagctcactcaaaggcggtaatacggttatcca
cagaatcaggggataacgcaggaaagaacatgtgagcaaaaggccagcaaaaggccaggaaccgtaaaaaggccgcgttgctggcgtt
tttccataggctccgcccccctgacgagcatcacaaaaatcgacgctcaagtcagaggtggcgaaacccgacaggactataaagatacca
ggcgtttccccctggaagctccctcgtgcgctctcctgttccgaccctgccgcttaccggatacctgtccgcctttctcccttcgggaagcgtg
gcgctttctcatagctcacgctgtaggtatctcagttcggtgtaggtcgttcgctccaagctgggctgtgtgcacgaaccccccgttcagccc
gaccgctgcgccttatccggtaactatcgtcttgagtccaacccggtaagacacgacttatcgccactggcagcagccactggtaacaggat
tagcagagcgaggtatgtaggcggtgctacagagttcttgaagtggtggcctaactacggctacactagaagaacagtatttggtatctgcg
ctctgctgaagccagttaccttcggaaaaagagttggtagctcttgatccggcaaacaaaccaccgctggtagcggtggtttttttgtttgcaa
gcagcagattacgcgcagaaaaaaaggatctcaagaagatcctttgatcttttctacggggtctgacgctcagtggaacgaaaactcacgtt
aagggattttggtcatgagattatcaaaaaggatcttcacctagatccttttaaattaaaaatgaagttttaaatcaatctaaagtatatatgagtaa
acttggtctgacagttaccaatgcttaatcagtgaggcacctatctcagcgatctgtctatttcgttcatccatagttgcctgactccccgtcgtgt
agataactacgatacgggagggcttaccatctggccccagtgctgcaatgataccgcgagacccacgctcaccggctccagatttatcagc
aataaaccagccagccggaagggccgagcgcagaagtggtcctgcaactttatccgcctccatccagtctattaattgttgccgggaagcta
gagtaagtagttcgccagttaatagtttgcgcaacgttgttgccattgctacaggcatcgtggtgtcacgctcgtcgtttggtatggcttcattca
gctccggttcccaacgatcaaggcgagttacatgatcccccatgttgtgcaaaaaagcggttagctccttcggtcctccgatcgttgtcagaa
gtaagttggccgcagtgttatcactcatggttatggcagcactgcataattctcttactgtcatgccatccgtaagatgcttttctgtgactggtga
gtactcaaccaagtcattctgagaatagtgtatgcggcgaccgagttgctcttgcccggcgtcaatacgggataataccgcgccacatagca
gaactttaaaagtgctcatcattggaaaacgttcttcggggcgaaaactctcaaggatcttaccgctgttgagatccagttcgatgtaacccact
cgtgcacccaactgatcttcagcatcttttactttcaccagcgtttctgggtgagcaaaaacaggaaggcaaaatgccgcaaaaaagggaat
aagggcgacacggaaatgttgaatactcatactcttcctttttcaatattattgaagcatttatcagggttattgtctcatgagcggatacatatttg
aatgtatttagaaaaataaacaaataggggttccgcgcacatttccccgaaaagtgccacctgacgtcgacggatcgggagatctcccgatc
ccctatggtgcactctcagtacaatctgctctgatgccgcatagttaagccagtatctgctccctgcttgtgtgttggaggtcgctgagtagtgc
gcgagcaaaatttaagctacaacaaggcaaggcttgaccgacaattgcatgaagaatctgcttagggttaggcgttttgcgctgcttcgcgat
gtacgggccagatatacgcgttgacattgattattgactagttattaatagtaatcaattacggggtcattagttcatagcccatatatggagttcc
gcgttacataacttacggtaaatggcccgcctggctgaccgcccaacgacccccgcccattgacgtcaataatgacgtatgttcccatagta
acgccaatagggactttccattgacgtcaatgggtggagtatttacggtaaactgcccacttggcagtacatcaagtgtatcatatgccaagta
cgccccctattgacgtcaatgacggtaaatggcccgcctggcattatgcccagtacatgaccttatgggactttcctacttggcagtacatcta

cgtattagtcatcgctattaccatggtgatgcggttttggcagtacatcaatgggcgtggatagcggtttgactcacggggatttccaagtctcc
accccattgacgtcaatgggagtttgttttggcaccaaaatcaacgggactttccaaaatgtcgtaacaactccgccccattgacgcaaatgg
gcggtaggcgtgtacggtgggaggtctatataagcagcgcgttttgcctgtactgggtctctctggttagaccagatctgagcctgggagctc
tctggctaactagggaacccactgcttaagcctcaataaagcttgccttgagtgcttcaagtagtgtgtgcccgtctgttgtgtgactctggtaa
ctagagatccctcagacccttttagtcagtgtggaaaatctctagcagtggcgcccgaacagggacttgaaagcgaaagggaaaccagag
gagctctctcgacgcaggactcggcttgctgaagcgcgcacggcaagaggcgaggggcggcgactggtgagtacgccaaaaattttgac
tagcggaggctagaaggagagagatgggtgcgagagcgtcagtattaagcgggggagaattagatcgcgatgggaaaaaattcggttaa
ggccagggggaaagaaaaaatataaattaaaacatatagtatgggcaagcagggagctagaacgattcgcagttaatcctggcctgttaga
aacatcagaaggctgtagacaaatactgggacagctacaaccatcccttcagacaggatcagaagaacttagatcattatataatacagtag
caaccctctattgtgtgcatcaaaggatagagataaaagacaccaaggaagctttagacaagatagaggaagagcaaaacaaaagtaaga
ccaccgcacagcaagcggccgctgatcttcagacctggaggaggagatatgagggacaattggagaagtgaattatataaatataaagta
gtaaaaattgaaccattaggagtagcacccaccaaggcaaagagaagagtggtgcagagagaaaaaagagcagtgggaataggagcttt
gttccttgggttcttgggagcagcaggaagcactatgggcgcagcgtcaatgacgctgacggtacaggccagacaattattgtctggtatag
tgcagcagcagaacaatttgctgagggctattgaggcgcaacagcatctgttgcaactcacagtctggggcatcaagcagctccaggcaa
gaatcctggctgtggaaagatacctaaaggatcaacagctcctggggatttggggttgctctggaaaactcatttgcaccactgctgtgcctt
ggaatgctagttggagtaataaatctctggaacagatttggaatcacacgacctggatggagtgggacagagaaattaacaattacacaagc
ttaatacactccttaattgaagaatcgcaaaaccagcaagaaaagaatgaacaagaattattggaattagataaatgggcaagtttgtggaatt
ggtttaacataacaaattggctgtggtatataaaattattcataatgatagtaggaggcttggtaggtttaagaatagtttttgctgtactttctatag
tgaatagagttaggcagggatattcaccattatcgtttcagacccacctcccaaccccgaggggacccgacaggcccgaaggaatagaag
aagaaggtggagagagagacagagacagatccattcgattagtgaacggatcggcactgcgtgcgccaattctgcagacaaatggcagt
attcatccacaattttaaaagaaaaggggggattggggggtacagtgcaggggaaagaatagtagacataatagcaacagacatacaaact
aaagaattacaaaaacaaattacaaaaattcaaaattttcgggtttattacagggacagcagagatccagtttggttaattaa

## Claims

1. A non-naturally occurring or engineered Transcription activator-like effector (TALE) system comprising at least one switch wherein the activity of said TALE system is controlled by contact with at least one inducer energy source as to the switch, wherein the inducer energy source is electromagnetic energy,
wherein the TALE system comprises:
[A]
(i) a DNA binding domain comprising at least ten or more Transcription activator-like effector (TALE) monomers and at least one or more half-monomers specifically ordered to target a locus of interest
linked to an energy sensitive protein or fragment thereof, wherein the energy sensitive protein or fragment thereof undergoes a conformational change upon induction by an inducer energy source allowing it to bind an interacting partner, and
(ii) at least one or more effector domains
linked to the interacting partner, wherein the energy sensitive protein or fragment thereof binds to the interacting partner upon induction by the inducer energy source;
or,
[B]
(i) at least one or more effector domains
linked to an energy sensitive protein or fragment thereof, wherein the energy sensitive protein or fragment thereof undergoes a conformational change upon induction by an inducer energy source allowing it to bind an interacting partner, and
(ii) a DNA binding domain comprising at least ten or more TALE monomers or half-monomers specifically ordered to target the locus of interest
linked to the interacting partner, wherein the energy sensitive protein or fragment thereof binds to the interacting partner upon induction by the inducer energy source.

2. The system according to claim 1, wherein the DNA binding polypeptide comprises
(a) a N-terminal capping region
(b) a DNA binding domain comprising at least 10 to 40 Transcription activator-like effector (TALE) monomers and at least one or more half-monomers specifically ordered to target the locus of interest, and
(c) a C-terminal capping region
wherein (a), (b) and (c) are arranged in a predetermined N-terminus to C-terminus orientation,
wherein the genomic locus comprises a target DNA sequence 5'-T₀N₁N₂....N_{z} N_{z+1} -3', where T0 and N = A, G, T or C,
wherein the DNA binding domain comprises (X₁₋₁₁-X₁₂X₁₃-X_{14-33 or 34 or 35})_{z},
wherein X₁₋₁₁ is a chain of 11 contiguous amino acids,
wherein X₁₂X₁₃ is a repeat variable diresidue (RVD),
wherein X_{14-33 or 34 or 35} is a chain of 21, 22 or 23 contiguous amino acids,
wherein z is at least 10 to 40.

3. A method of controlling a non-naturally occurring or engineered Transcription activator-like effector (TALE) system comprising at least one switch wherein the activity of said TALE system is controlled by contact with at least one inducer energy source as to the switch,
wherein the TALE system comprises:
[A]
(i) a DNA binding domain comprising at least ten or more Transcription activator-like effector (TALE) monomers and at least one or more half-monomers specifically ordered to target a locus of interest
linked to an energy sensitive protein or fragment thereof, wherein the energy sensitive protein or fragment thereof undergoes a conformational change upon induction by an inducer energy source allowing it to bind an interacting partner, and
(ii) at least one or more effector domains
linked to the interacting partner, wherein the energy sensitive protein or fragment thereof binds to the interacting partner upon induction by the inducer energy source;
or,
[B]
(i) at least one or more effector domains
linked to an energy sensitive protein or fragment thereof, wherein the energy sensitive protein or fragment thereof undergoes a conformational change upon induction by an inducer energy source allowing it to bind an interacting partner, and
(ii) a DNA binding domain comprising at least ten or more TALE monomers or half-monomers specifically ordered to target the locus of interest
linked to the interacting partner, wherein the energy sensitive protein or fragment thereof binds to the interacting partner upon induction by the inducer energy source,
wherein the inducer energy source is electromagnetic energy,
wherein the method is not a method for treatment of the human or animal body by therapy, and wherein the method is not a process for modifying the germ line genetic identity of human beings.

4. The method according to claim 3, wherein the DNA binding polypeptide comprises
(a) a N-terminal capping region
(b) a DNA binding domain comprising at least 10 to 40 Transcription activator-like effector (TALE) monomers and at least one or more half-monomers specifically ordered to target the locus of interest, and
(c) a C-terminal capping region
wherein (a), (b) and (c) are arranged in a predetermined N-terminus to C-terminus orientation,
wherein the genomic locus comprises a target DNA sequence 5'-T₀N₁N₂....N_{z} N_{z+1} -3', where T₀ and N = A, G, T or C,
wherein the DNA binding domain comprises (X₁₋₁₁-X₁₂X₁₃-X_{14-33 or 34 or 35})_{z},
wherein X₁₋₁₁ is a chain of 11 contiguous amino acids,
wherein X₁₂X₁₃ is a repeat variable diresidue (RVD),
wherein X_{14-33 or 34 or 35} is a chain of 21, 22 or 23 contiguous amino acids,
wherein z is at least 10 to 40.

5. The system or method according to any one of claims 1-4,
wherein the control as to the at least one switch or the activity of said system is activated, enhanced, terminated or repressed.

6. The system or method according to any one of claims 1-5,
wherein the effector domain is selected from the group consisting of: transposase domain, integrase domain, recombinase domain, resolvase domain, invertase domain, protease domain, DNA methyltransferase domain, DNA demethylase domain, histone acetylase domain, histone deacetylases domain, nuclease domain, repressor domain, activator domain, nuclear-localization signal domains, cellular uptake activity associated domain.

7. The system or method according to any one of claims 1-6,
wherein the electromagnetic energy is a component of visible light.

8. The system or method according to any one of claims 1-7,
wherein the TALE DNA binding domain is fused to one half of the cryptochrome heterodimer (cryptochrome-2 or CIB1), while the remaining cryptochrome partner is fused to a transcriptional effector domain.

9. The system or method according to any one of claims 7-8,
wherein the component of visible light has a wavelength in the range of 450nm-700nm or wherein the component of visible light has a wavelength in the range of 450nm-500nm.

10. The system or method according to any one of claims 7-9,
wherein the component of visible light has a wavelength in the range of 450nm-500nm, optionally wherein the component of visible light is blue light,
further optionally wherein the blue light has an intensity of at least 0.2mW/cm² or of at least 4mW/cm².

11. The system or method according to any one of claims 1-10,
wherein said system further comprises at least one nuclear localization signal (NLS), nuclear export signal (NES), functional domain, flexible linker, mutation, deletion, alteration or truncation,
wherein the at least one functional domain may optionally be selected from the group consisting of: transposase domain, integrase domain, recombinase domain, resolvase domain, invertase domain, protease domain, DNA methyltransferase domain, DNA demethylase domain, histone acetylase domain, histone deacetylases domain, nuclease domain, repressor domain, activator domain, nuclear-localization signal domains, cellular uptake activity associated domain.

12. The system or method according to any one of claims 1-11,
wherein the TALE system can be or is delivered into an organism or a cell via a delivery system.

13. The system or method according to any one of claims 1-12, wherein the system is a TALE system that can be or is delivered via a AAV or a lentivirus vector.

14. The system according to any one of claims 1-2 or 5-13 for use in therapy.

15. The system for use according to claim 14, for perturbing a genomic or epigenomic locus of interest.

16. Use of a system according to any one of claims 1-2 or 5-13, for perturbing a genomic or epigenomic locus,
wherein the use is not a method for treatment of the human or animal body by therapy, and wherein the use is not a process for modifying the germ line genetic identity of human beings.

17. A cell, a cell line or a non-human transgenic animal containing a system according to any one of claims 1-2 or 5-13.

## Patentansprüche

1. Nicht natürlich vorkommendes oder konstruiertes Transkriptionsaktivator-ähnlicher-Effektor (TALE)-System, das wenigstens einen Schalter umfasst, wobei die Aktivität des TALE-Systems durch Kontakt mit wenigstens einer Induktor-Energiequelle den Schalter betreffend gesteuert wird, wobei die Induktor-Energiequelle elektromagnetische Energie ist,
wobei das TALE-System Folgendes umfasst:
[A]
(i) eine DNA-Bindedomäne umfassend wenigstens zehn oder mehr Transkriptionsaktivator-ähnlicher-Effektor (TALE)-Monomere und wenigstens ein oder mehrere Halbmonomere, die spezifisch angeordnet sind, um an einen Locus von Interesse zu binden,
verbunden mit einem energieempfindlichen Protein oder einem Fragment davon, wobei das energieempfindliche Protein oder das Fragment davon bei Induktion durch eine Induktor-Energiequelle eine Konformationsänderung unterläuft, die es ihm erlaubt, einen Interaktionspartner zu binden, und
(ii) wenigstens eine oder mehrere Effektordomänen verbunden mit dem Interaktionspartner, wobei das energieempfindliche Protein oder das Fragment davon bei Induktion durch die Induktor-Energiequelle den Interaktionspartner bindet;
oder,
[B]
(i) wenigstens eine oder mehrere Effektordomänen verbunden mit einem energieempfindlichen Protein oder einem Fragment davon, wobei das energieempfindliche Protein oder das Fragment davon bei Induktion durch eine Induktor-Energiequelle eine Konformationsänderung unterläuft, die es ihm erlaubt, einen Interaktionspartner zu binden, und
(ii) eine DNA-Bindedomäne umfassend wenigstens zehn oder mehr TALE-Monomere oder Halbmonomere, die spezifisch angeordnet sind, um den Locus von Interesse zu binden, verbunden mit dem Interaktionspartner, wobei das energieempfindliche Protein oder das Fragment davon bei Induktion durch die Induktor-Energiequelle den Interaktionspartner bindet.

2. System nach Anspruch 1, wobei das DNA-bindende Polypeptid Folgendes umfasst:
(a) eine N-terminale Capping-Region,
(b) eine DNA-Bindedomäne umfassend wenigstens 10 bis 40 Transkriptionsaktivator-ähnlicher-Effektor (TALE)-Monomere und wenigstens ein oder mehrere Halbmonomere, die spezifisch angeordnet sind, um den Locus von Interesse zu binden, und
(c) eine C-terminale Capping-Region,
wobei (a), (b) und (c) in einer vorher festgelegten Orientierung vom N-Terminus zum C-Terminus angeordnet sind,
wobei der genomische Locus eine Ziel-DNA-Sequenz 5'-T₀N₁N₂...N_{z}N_{z+1} -3' umfasst,
worin T₀ und N = A, G, T oder C sind,
wobei die DNA-Bindedomäne (X₁₋₁₁-X₁₂X₁₃-X_{14-33 oder 34 oder 35})_{z} umfasst,
wobei X₁₋₁₁ eine Kette von 11 zusammenhängenden Aminosäuren ist,
wobei X₁₂X₁₃ ein Repeat Variable Diresidue (RVD) ist, wobei X_{14-33 oder 34 oder 35} eine Kette von 21, 22 oder 23 zusammenhängenden Aminosäuren ist,
wobei z wenigstens 10 bis 40 ist.

3. Verfahren zur Steuerung eines nicht natürlich vorkommenden oder konstruierten Transkriptionsaktivator-ähnlicher-Effektor (TALE)-Systems, das wenigstens einen Schalter umfasst, wobei die Aktivität des TALE-Systems durch Kontakt mit wenigstens einer Induktor-Energiequelle den Schalter betreffend gesteuert wird, wobei das TALE-System Folgendes umfasst:
[A]
(i) eine DNA-Bindedomäne umfassend wenigstens zehn oder mehr Transkriptionsaktivator-ähnliche-Effektor (TALE)-Monomere und wenigstens ein oder mehrere Halbmonomere, die spezifisch angeordnet sind, um einen Locus von Interesse zu binden,
verbunden mit einem energieempfindlichen Protein oder einem Fragment davon, wobei das energieempfindliche Protein oder das Fragment davon bei Induktion durch eine Induktor-Energiequelle eine Konformationsänderung unterläuft, die es ihm erlaubt, einen Interaktionspartner zu binden, und
(ii) wenigstens eine oder mehrere Effektordomänen
verbunden mit dem Interaktionspartner, wobei das energieempfindliche Protein oder das Fragment davon bei Induktion durch die Induktor-Energiequelle den Interaktionspartner bindet;
oder,
[B]
(i) wenigstens eine oder mehrere Effektordomänen
verbunden mit einem energieempfindlichen Protein oder einem Fragment davon, wobei das energieempfindliche Protein oder das Fragment davon bei Induktion durch eine Induktor-Energiequelle eine Konformationsänderung unterläuft, die es ihm erlaubt, einen Interaktionspartner zu binden, und
(ii) eine DNA-Bindedomäne umfassend wenigstens zehn oder mehr TALE-Monomere oder Halbmonomere, die spezifisch angeordnet sind, um den Locus von Interesse zu binden,
verbunden mit dem Interaktionspartner, wobei das energieempfindliche Protein oder das Fragment davon bei Induktion durch die Induktor-Energiequelle den Interaktionspartner bindet,
wobei die Induktor-Energiequelle elektromagnetische Energie ist,
wobei das Verfahren kein Verfahren zur therapeutischen Behandlung des menschlichen oder tierischen Körpers ist, und wobei das Verfahren kein Verfahren zur Veränderung der genetischen Keimbahnidentität von Menschen ist.

4. Verfahren nach Anspruch 3, wobei das DNA-bindende Polypeptid Folgendes umfasst:
(a)eine N-terminale Capping-Region,
(b) eine DNA-Bindedomäne umfassend wenigstens 10 bis 40 Transkriptionsaktivator-ähnliche-Effektor (TALE)-Monomere und wenigstens ein oder mehrere Halbmonomere, die spezifisch angeordnet sind, um den Locus von Interesse anzusteuern, und
(c) eine C-terminale Capping-Region,
wobei (a), (b) und (c) in einer vorher festgelegten Orientierung vom N-Terminus zum C-Terminus angeordnet sind,
wobei der genomische Locus eine Ziel-DNA-Sequenz 5'-T₀N₁N₂...N_{z}N_{z+1} -3' umfasst,
worin T₀ und N = A, G, T oder C sind,
wobei die DNA-Bindedomäne (X₁₋₁₁-X₁₂X₁₃-X_{14-33 oder 34 oder 35})_{z} umfasst,
wobei X₁₋₁₁ eine Kette von 11 zusammenhängenden Aminosäuren ist,
wobei X₁₂X₁₃ ein Repeat Variable Diresidue (RVD) ist, wobei X_{14-33 oder 34 oder 35} eine Kette von 21, 22 oder 23 zusammenhängenden Aminosäuren ist,
wobei z wenigstens 10 bis 40 ist.

5. System oder Verfahren nach einem der Ansprüche 1-4, wobei die Steuerung den wenigstens einen Schalter betreffend oder die Aktivität des Systems aktiviert, verstärkt, beendet oder unterdrückt wird.

6. System oder Verfahren nach einem der Ansprüche 1-5, wobei die Effektordomäne ausgewählt ist aus der Gruppe bestehend aus: Transposasedomäne, Integrasedomäne, Rekombinasedomäne, Resolvasedomäne, Invertasedomäne, Proteasedomäne, DNA-Methyltransferasedomäne, DNA-Demethylasedomäne, Histonacetylasedomäne, Histondeacetylasedomäne, Nukleasedomäne, Repressordomäne, Aktivatordomäne, Kernlokalisationssignaldomänen, mit der zellulären Aufnahmeaktivität assoziierte Domäne.

7. System oder Verfahren nach einem der Ansprüche 1-6, wobei die elektromagnetische Energie ein Bestandteil des sichtbaren Lichts ist.

8. System oder Verfahren nach einem der Ansprüche 1-7, wobei die TALE-DNA-Bindedomäne mit einer Hälfte des Cryptochrom-Heterodimers (Cryptochrom-2 oder CIB1) fusioniert ist, während der verbleibende Cryptochrompartner mit einer Transkriptionseffektordomäne fusioniert ist.

9. System oder Verfahren nach einem der Ansprüche 7-8, wobei der Bestandteil des sichtbaren Lichts eine Wellenlänge im Bereich von 450 - 700 nm aufweist oder wobei der Bestandteil des sichtbaren Lichts eine Wellenlänge im Bereich von 450 - 500 nm aufweist.

10. System oder Verfahren nach einem der Ansprüche 7-9,
wobei der Bestandteil des sichtbaren Lichts eine Wellenlänge im Bereich von 450 - 500 nm aufweist,
wobei wahlweise der Bestandteil des sichtbaren Lichts blaues Licht ist,
wobei ferner wahlweise das blaue Licht eine Intensität von wenigstens 0,2 mW/cm² oder von wenigstens 4 mW/cm² aufweist.

11. System oder Verfahren nach einem der Ansprüche 1-10, wobei das System ferner wenigstens ein Kernlokalisationssignal (NLS), Kernexportsignal (NES), eine funktionelle Domäne, einen flexiblen Linker, eine Mutation, Deletion, Veränderung oder Trunkierung umfasst, wobei die wenigstens eine funktionelle Domäne wahlweise ausgewählt ist aus der Gruppe bestehend aus: Transposasedomäne, Integrasedomäne, Rekombinasedomäne, Resolvasedomäne, Invertasedomäne, Proteasedomäne, DNA-Methyltransferasedomäne, DNA-Demethylasedomäne, Histonacetylasedomäne, Histondeacetylasedomäne, Nukleasedomäne, Repressordomäne, Aktivatordomäne, Kernlokalisationssignaldomänen, mit der zellulären Aufnahmeaktivität assoziierte Domäne.

12. System oder Verfahren nach einem der Ansprüche 1-11, wobei das TALE-System über ein Verabreichungssystem in einen Organismus oder eine Zelle verabreicht wird oder werden kann.

13. System oder Verfahren nach einem der Ansprüche 1-12, wobei das System ein TALE-System ist, das über einen AAV oder einen Lentivirus-Vektor verabreicht wird oder werden kann.

14. System nach einem der Ansprüche 1-2 oder 5-13 zur therapeutischen Verwendung.

15. System zur Verwendung nach Anspruch 14, zur Unterbrechung eines genomischen oder epigenomischen Locus von Interesse.

16. Verwendung eines Systems gemäß eines der Ansprüche 1-2 oder 5-13, zur Störung eines genomischen oder epigenomischen Locuses,
wobei die Verwendung kein Verfahren zur therapeutischen Behandlung des menschlichen oder tierischen Körpers ist, und
wobei die Verwendung kein Verfahren zur Veränderung der genetischen Identität der Keimbahn von Menschen ist.

17. Zelle, Zelllinie oder nichtmenschliches transgenes Tier, das ein System nach einem der Ansprüche 1-2 oder 5-13 umfasst.

## Revendications

1. Système d'effecteur du type activateur de la transcription (TALE) n'existant pas naturellement ou étant manipulé par génie génétique, comprenant au moins un interrupteur, dans lequel l'activité dudit système de TALE est contrôlée par un contact avec au moins une source d'énergie inductrice pour ce qui est de l'interrupteur, dans lequel la source d'énergie inductrice est l'énergie électromagnétique,
le système de TALE comprenant :
[A]
(i) un domaine de liaison à l'ADN comprenant au moins dix monomères ou plus d'effecteur du type activateur de la transcription (TALE) et au moins un ou plusieurs demi-monomère(s) ayant pour ordre spécifique de cibler un locus d'intérêt
lié à une protéine sensible à une énergie ou un fragment de celle-ci, dans lequel la protéine sensible à une énergie ou le fragment de celle-ci subit un changement de conformation lors d'une induction par une source d'énergie inductrice, ce qui lui permet de se lier à un partenaire d'interaction, et
(ii) au moins un ou plusieurs domaine(s) effecteur(s) lié(s) au partenaire d'interaction, dans lequel la protéine sensible à une énergie ou le fragment de celle-ci se lie au partenaire d'interaction lors d'une induction par la source d'énergie inductrice ;
ou
[B]
(i) au moins un ou plusieurs domaine(s) effecteur(s) lié(s) à une protéine sensible à une énergie ou un fragment de celle-ci, dans lequel la protéine sensible à une énergie ou le fragment de celle-ci subit un changement de conformation lors d'une induction par une source d'énergie inductrice, ce qui lui permet de se lier à un partenaire d'interaction, et
(ii) un domaine de liaison à l'ADN comprenant au moins dix ou plus monomères ou demi-monomères de TALE ayant pour ordre spécifique de cibler le locus d'intérêt
lié au partenaire d'interaction, dans lequel la protéine sensible à une énergie ou le fragment de celle-ci se lie au partenaire d'interaction lors d'une induction par la source d'énergie inductrice.

2. Système selon la revendication 1, dans lequel le polypeptide de liaison à l'ADN comprend
(a) une région de coiffage N-terminale
(b) un domaine de liaison à l'ADN comprenant au moins 10 à 40 monomères d'effecteur du type activateur de la transcription (TALE) et au moins un ou plusieurs demi-monomère(s) ayant pour ordre spécifique de cibler le locus d'intérêt et
(c) une région de coiffage C-terminale
dans lequel les (a), (b) et (c) sont rangés dans une orientation prédéterminée de l'extrémité N-terminale vers l'extrémité C-terminale,
dans lequel le locus génomique comprend une séquence d'ADN cible 5' -T₀N₁N₂....N_{z} N_{z+1}-3', où T₀ et N = A, G, T ou C,
dans lequel le domaine de liaison à l'ADN comprend (X₁₋₁₁-X₁₂X₁₃-X_{14-33 ou 34 ou 35})_{z},
dans lequel X₁₋₁₁ est une chaîne de 11 acides aminés contigus,
dans lequel X₁₂X₁₃ est un di-résidu variable de répétition (DVR),
dans lequel X_{14-33 ou 34 ou 35} est une chaîne de 21, 22 ou 23 acides aminés contigus,
dans lequel z est au moins 10 à 40.

3. Procédé de contrôle d'un système d'effecteur du type activateur de la transcription (TALE) n'existant pas naturellement ou étant manipulé par génie génétique, comprenant au moins un interrupteur, dans lequel l'activité dudit système de TALE est contrôlée par un contact avec au moins une source d'énergie inductrice pour ce qui est de l'interrupteur,
le système de TALE comprenant :
[A]
(i) un domaine de liaison à l'ADN comprenant au moins dix monomères ou plus d'effecteur du type activateur de la transcription (TALE) et au moins un ou plusieurs demi-monomère(s) ayant pour ordre spécifique de cibler un locus d'intérêt
lié à une protéine sensible à une énergie ou un fragment de celle-ci, dans lequel la protéine sensible à une énergie ou le fragment de celle-ci subit un changement de conformation lors d'une induction par une source d'énergie inductrice, ce qui lui permet de se lier à un partenaire d'interaction, et
(ii) au moins un ou plusieurs domaine(s) effecteur(s)
lié(s) au partenaire d'interaction, dans lequel la protéine sensible à une énergie ou le fragment de celle-ci se lie au partenaire d'interaction lors d'une induction par la source d'énergie inductrice ;
ou
[B]
(i) au moins un ou plusieurs domaine(s) effecteur(s)
lié(s) à une protéine sensible à une énergie ou un fragment de celle-ci, dans lequel la protéine sensible à une énergie ou le fragment de celle-ci subit un changement de conformation lors d'une induction par une source d'énergie inductrice, ce qui lui permet de se lier à un partenaire d'interaction, et
(ii) un domaine de liaison à l'ADN comprenant au moins dix ou plus monomères ou demi-monomères de TALE ayant pour ordre spécifique de cibler le locus d'intérêt
lié au partenaire d'interaction, dans lequel la protéine sensible à une énergie ou le fragment de celle-ci se lie au partenaire d'interaction lors d'une induction par la source d'énergie inductrice,
dans lequel la source d'énergie inductrice est l'énergie électromagnétique,
le procédé n'étant pas un procédé destiné à un traitement du corps humain ou animal par une thérapie, et le procédé n'étant pas un processus destiné à modifier l'identité génétique de la lignée germinale d'êtres humains.

4. Procédé selon la revendication 3, dans lequel le polypeptide de liaison à l'ADN comprend
(a) une région de coiffage N-terminale
(b) un domaine de liaison à l'ADN comprenant au moins 10 à 40 monomères d'effecteur du type activateur de la transcription (TALE) et au moins un ou plusieurs demi-monomère(s) ayant pour ordre spécifique de cibler le locus d'intérêt, et
(c) une région de coiffage C-terminale
dans lequel les (a), (b) et (c) sont rangés dans une orientation prédéterminée de l'extrémité N-terminale vers l'extrémité C-terminale,
dans lequel le locus génomique comprend une séquence d'ADN cible 5'-T₀N₁N₂....N_{z} N_{z+1} -3', où T₀ et N = A, G, T ou C,
dans lequel le domaine de liaison à l'ADN comprend (X₁-₁₁-X₁₂X₁₃-X_{14-33 ou 34 ou 35})_{z},
dans lequel X₁₋₁₁ est une chaîne de 11 acides aminés contigus,
dans lequel X₁₂X₁₃ est un di-résidu variable de répétition (DVR),
dans lequel X_{14-33 ou 34 ou 35} est une chaîne de 21, 22 ou 23 acides aminés contigus,
dans lequel z est au moins 10 à 40.

5. Système ou procédé selon l'une quelconque des revendications 1 à 4,
dans lequel le contrôle, pour ce qui est de l'au moins un interrupteur, ou l'activité dudit système est activé(e), amplifié(e), terminé(e) ou réprimé(e).

6. Système ou procédé selon l'une quelconque des revendications 1 à 5,
dans lequel le domaine effecteur est choisi dans le groupe constitué par : un domaine de transposase, un domaine d'intégrase, un domaine de recombinase, un domaine de résolvase, un domaine d'invertase, un domaine de protéase, un domaine d'ADN méthyl transférase, un domaine d'ADN déméthylase, un domaine d'histone acétylase, un domaine d'histone désacétylase, un domaine de nucléase, un domaine répresseur, un domaine activateur, des domaines de signaux de localisation nucléaire, un domaine associé à une activité d'absorption cellulaire.

7. Système ou procédé selon l'une quelconque des revendications 1 à 6,
dans lequel l'énergie électromagnétique est un composant de la lumière visible.

8. Système ou procédé selon l'une quelconque des revendications 1 à 7,
dans lequel le domaine de liaison à l'ADN de TALE est fusionné avec une moitié de l'hétérodimère de cryptochrome (cryptochrome-2 ou CIB1), alors que le partenaire du cryptochrome restant est fusionné avec un domaine effecteur de la transcription.

9. Système ou procédé selon l'une quelconque des revendications 7 à 8,
dans lequel le composant de la lumière visible a une longueur d'onde comprise dans la plage des 450 nm - 700 nm ou
dans lequel le composant de la lumière visible a une longueur d'onde comprise dans la plage des 450 nm - 500 nm.

10. Système ou procédé selon l'une quelconque des revendications 7 à 9,
dans lequel le composant de la lumière visible a une longueur d'onde comprise dans la plage des 450 nm - 500 nm, en option dans lequel le composant de la lumière visible est la lumière bleue,
en option en outre dans lequel la lumière bleue a une intensité d'au moins 0,2 mW/cm² ou d'au moins 4 mW/cm².

11. Système ou procédé selon l'une quelconque des revendications 1 à 10,
ledit système comprenant en outre au moins un signal de localisation nucléaire (SLN), un signal d'exportation nucléaire (SEN), un domaine fonctionnel, un lieur flexible, une mutation, une délétion, une altération ou une troncature,
dans lequel l'au moins un domaine fonctionnel peut être choisi, en option, dans le groupe constitué par : un domaine de transposase, un domaine d'intégrase, un domaine de recombinase, un domaine de résolvase, un domaine d'invertase, un domaine de protéase, un domaine d'ADN méthyl transférase, un domaine d'ADN déméthylase, un domaine d'histone acétylase, un domaine d'histone désacétylase, un domaine de nucléase, un domaine répresseur, un domaine activateur, des domaines de signaux de localisation nucléaire, un domaine associé à une activité d'absorption cellulaire.

12. Système ou procédé selon l'une quelconque des revendications 1 à 11,
le système de TALE pouvant être ou étant délivré dans un organisme ou une cellule via un système de délivrance.

13. Système ou procédé selon l'une quelconque des revendications 1 à 12, le système étant un système de TALE qui peut être ou qui est délivré via un VAA ou un vecteur à lentivirus.

14. Système selon l'une quelconque des revendications 1 à 2 ou 5 à 13 destiné à être utilisé dans une thérapie.

15. Système destiné à être utilisé selon la revendication 14, destiné à perturber un locus génomique ou épigénomique d'intérêt.

16. Utilisation d'un système, selon l'une quelconque des revendications 1 à 2 ou 5 à 13, destiné à perturber un locus génomique ou épigénomique,
l'utilisation n'étant pas un procédé destiné à un traitement du corps humain ou animal par une thérapie, et
l'utilisation n'étant pas un processus destiné à modifier l'identité génétique de la lignée germinale d'êtres humains.

17. Cellule, lignée de cellules ou animal transgénique non humain contenant un système selon l'une quelconque des revendications 1 à 2 ou 5 à 13.
